(19) Europäisches Patentamt
European Patent Office
Office européen des brevets

(11) EP 1 219 294 A1

(12) # EUROPEAN PATENT APPLICATION
published in accordance with Art. 158(3) EPC

(43) Date of publication:
**03.07.2002 Bulletin 2002/27**

(21) Application number: **00961076.7**

(22) Date of filing: **19.09.2000**

(51) Int Cl.7: **A61K 31/137**, A61K 31/27, A61K 31/4035, A61K 31/44, A61K 31/445, A61K 31/4453, A61K 31/472, A61P 43/00, A61P 31/04, C07D 211/14, C07D 211/18, C07D 211/46, C07D 211/58, C07D 211/70, C07D 401/12, C07D 405/12, C07D 409/12, C07D 417/12

(86) International application number:
**PCT/JP00/06376**

(87) International publication number:
**WO 01/21169 (29.03.2001 Gazette 2001/13)**

(84) Designated Contracting States:
**AT BE CH CY DE DK ES FI FR GB GR IE IT LI LU MC NL PT SE**
Designated Extension States:
**AL LT LV MK RO SI**

(30) Priority: **20.09.1999 JP 26627899**
**17.07.2000 JP 2000221055**

(71) Applicant: **Takeda Chemical Industries, Ltd.**
**Osaka-shi, Osaka 541-0045 (JP)**

(72) Inventors:
- **KATO, Kaneyoshi**
**Kawanishi-shi, Hyogo 666-0152 (JP)**
- **MORI, Masaaki**
**Tsukuba-shi, Ibaraki 305-0821 (JP)**
- **SUZUKI, Nobuhiro**
**Nishinomiya-shi, Hyogo 663-8031 (JP)**
- **SHIMOMURA, Yukio**
**Takeda Yakuhin Matsushiro Res.410**
**Tsukuba-shi Ibaraki 305-0035 (JP)**
- **TAKEKAWA, Shiro**
**Nishinomiya-shi, Hyogo 662-0976 (JP)**
- **CHOH, Nobuo**
**Tsukuba-shi, Ibaraki 305-0035 (JP)**

(74) Representative: **Lewin, John Harvey**
**Takeda Patent Office,**
**11-12 Charles II Street**
**London SW1Y 4QU (GB)**

(54) **MELANIN CONCENTRATING HORMONE ANTAGONISTS**

(57) A compound represented by the formula

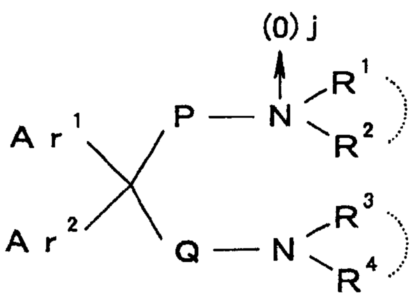

wherein $Ar^1$ and $Ar^2$ are each an aromatic group optionally having substituents, P and Q are each a divalent aliphatic hydrocarbon group which optionally contains ether oxygen or ether sulfur in a carbon chain and which optionally has substituents, $R^1$ and $R^3$ are each (i) a hydrogen atom, (ii) an acyl group or (iii) an hydrocarbon group optionally having substituents, $R^2$ and $R^4$ are each (i) a hydrogen atom, (ii) an alkyl group optionally having substituents or (iii) an alkylcarbonyl optionally having substituents, $R^1$ and $R^2$ or $R^3$ and $R^4$ each optionally forms, together with the adjacent nitrogen atom, a monocyclic or fused nitrogen-containing heterocyclic group optionally having substituents, j is 0 or 1, a salt thereof or a prodrug thereof are useful as melanin-concentrating hormone antagonists.

EP 1 219 294 A1

SEQUENCE LISTING

<110> Takeda Chemical Industries, Ltd.

<120> MCH Receptor Antagonist

<130> Case2651

<150> JP 11-266278

<151> 1999-09-20

<150> JP 2000-221055

<151> 2000-07-17

<160> 16

<210> 1

<211> 32

<212> DNA

<213> Artificial Sequence

<220>

<223>

<400> 1

GTCGACATGG ATCTGCAAAC CTCGTTGCTG TG 32

<210> 2

<211> 32

<212> DNA

<213> Artificial Sequence

<220>

<223>

<400> 2

ACTAGTTCAG GTGCCTTTGC TTTCTGTCCT CT 32

<210> 3

<211> 353

<212> PRT

<213> Rat

<400> 3

Met Asp Leu Gln Thr Ser Leu Leu Ser Thr Gly Pro Asn Ala Ser Asn
1 5 10 15

Ile Ser Asp Gly Gln Asp Asn Leu Thr Leu Pro Gly Ser Pro Pro Arg
20 25 30

Thr Gly Ser Val Ser Tyr Ile Asn Ile Ile Met Pro Ser Val Phe Gly
35 40 45

Thr Ile Cys Leu Leu Gly Ile Val Gly Asn Ser Thr Val Ile Phe Ala
50 55 60

Val Val Lys Lys Ser Lys Leu His Trp Cys Ser Asn Val Pro Asp Ile
65 70 75 80

Phe Ile Ile Asn Leu Ser Val Val Asp Leu Leu Phe Leu Leu Gly Met
85 90 95

Pro Phe Met Ile His Gln Leu Met Gly Asn Gly Val Trp His Phe Gly
100 105 110

Glu Thr Met Cys Thr Leu Ile Thr Ala Met Asp Ala Asn Ser Gln Phe
115 120 125

Thr Ser Thr Tyr Ile Leu Thr Ala Met Thr Ile Asp Arg Tyr Leu Ala
130 135 140

Thr Val His Pro Ile Ser Ser Thr Lys Phe Arg Lys Pro Ser Met Ala
145 150 155 160

Thr Leu Val Ile Cys Leu Leu Trp Ala Leu Ser Phe Ile Ser Ile Thr
165 170 175

Pro Val Trp Leu Tyr Ala Arg Leu Ile Pro Phe Pro Gly Gly Ala Val
180 185 190

Gly Cys Gly Ile Arg Leu Pro Asn Pro Asp Thr Asp Leu Tyr Trp Phe
195 200 205

Thr Leu Tyr Gln Phe Phe Leu Ala Phe Ala Leu Pro Phe Val Val Ile
210 215 220

Thr Ala Ala Tyr Val Lys Ile Leu Gln Arg Met Thr Ser Ser Val Ala
225 230 235 240
Pro Ala Ser Gln Arg Ser Ile Arg Leu Arg Thr Lys Arg Val Thr Arg
245 250 255
Thr Ala Ile Ala Ile Cys Leu Val Phe Phe Val Cys Trp Ala Pro Tyr
260 265 270
Tyr Val Leu Gln Leu Thr Gln Leu Ser Ile Ser Arg Pro Thr Leu Thr
275 280 285
Phe Val Tyr Leu Tyr Asn Ala Ala Ile Ser Leu Gly Tyr Ala Asn Ser
290 295 300
Cys Leu Asn Pro Phe Val Tyr Ile Val Leu Cys Glu Thr Phe Arg Lys
305 310 315 320
Arg Leu Val Leu Ser Val Lys Pro Ala Ala Gln Gly Gln Leu Arg Thr
325 330 335
Val Ser Asn Ala Gln Thr Ala Asp Glu Glu Arg Thr Glu Ser Lys Gly
340 345 350
Thr
<210> 4
<211> 1074
<212> DNA
<213> Rat
<400> 4
GTCGACATGG ATCTGCAAAC CTCGTTGCTG TCCACTGGCC CCAATGCCAG CAACATCTCC 60
GATGGCCAGG ATAATCTCAC ATTGCCGGGG TCACCTCCTC GCACAGGGAG TGTCTCCTAC 120
ATCAACATCA TTATGCCTTC CGTGTTTGGT ACCATCTGTC TCCTGGGCAT CGTGGGAAAC 180
TCCACGGTCA TCTTTGCTGT GGTGAAGAAG TCCAAGCTAC ACTGGTGCAG CAACGTCCCC 240
GACATCTTCA TCATCAACCT CTCTGTGGTG GATCTGCTCT TCCTGCTGGG CATGCCTTTC 300
ATGATCCACC AGCTCATGGG GAACGGCGTC TGGCACTTTG GGGAAACCAT GTGCACCCTC 360
ATCACAGCCA TGGACGCCAA CAGTCAGTTC ACTAGCACCT ACATCCTGAC TGCCATGACC 420

ATTGACCGCT ACTTGGCCAC CGTCCACCCC ATCTCCTCCA CCAAGTTCCG GAAGCCCTCC 480
ATGGCCACCC TGGTGATCTG CCTCCTGTGG GCGCTCTCCT TCATCAGTAT CACCCCTGTG 540
TGGCTCTACG CCAGGCTCAT TCCCTTCCCA GGGGGTGCTG TGGGCTGTGG CATCCGCCTG 600
CCAAACCCGG ACACTGACCT CTACTGGTTC ACTCTGTACC AGTTTTTCCT GGCCTTTGCC 660
CTTCCGTTTG TGGTCATTAC CGCCGCATAC GTGAAAATAC TACAGCGCAT GACGTCTTCG 720
GTGGCCCCAG CCTCCCAACG CAGCATCCGG CTTCGGACAA AGAGGGTGAC CCGCACGGCC 780
ATTGCCATCT GTCTGGTCTT CTTTGTGTGC TGGGCACCCT ACTATGTGCT GCAGCTGACC 840
CAGCTGTCCA TCAGCCGCCC GACCCTCACG TTTGTCTACT TGTACAACGC GGCCATCAGC 900
TTGGGCTATG CTAACAGCTG CCTGAACCCC TTTGTGTACA TAGTGCTCTG TGAGACCTTT 960
CGAAAACGCT TGGTGTTGTC AGTGAAGCCT GCAGCCCAGG GGCAGCTCCG CACGGTCAGC 1020
AACGCTCAGA CAGCTGATGA GGAGAGGACA GAAAGCAAAG GCACCTGAAC TAGT 1074

<210> 5

<211> 262

<212> RNA

<213> Rat

<400> 5

GCGAAUUGGG UACCGGGCCC CCCCUCGAGG UCGACGGUAU CGAUAAGCUU GAUAUCGAAU 60
UCCUGCAGCC CGGGGGAUCC GCCCACUAGU UCAGGUGCCU UUGCUUUCUG UCCUCUCCUC 120
AUCAGCUGUC UGAGCGUUGC UGACCGUGCG GAGCUGCCCC UGGGCUGCAG GCUUCACUGA 180
CAACACCAAG CGUUUUCGAA AGGUCUCACA GAGCACUAUG UACACAAAGG GGUUCAGGCA 240
GCUGUUAGCA UAGCCCAAGC UG 262

<210> 6

<211> 18

<212> DNA

<213> Artificial Sequence

<220>

<223>

<400> 6

CAACAGCTGC CTCAACCC 18

<210> 7

<211> 18

<212> DNA

<213> Artificial Sequence

<220>

<223>

<400> 7

CCTGGTGATC TGCCTCCT 18

<210> 8

<211> 1275

<212> DNA

<213> Human

<400> 8

TAGGTGATGT CAGTGGGAGC CATGAAGAAG GGAGTGGGGA GGGCAGTTGG GCTTGGAGGC 60

GGCAGCGGCT GCCAGGCTAC GGAGGAAGAC CCCCTTCCCA ACTGCGGGGC TTGCGCTCCG 120

GGACAAGGTG GCAGGCGCTG GAGGCTGCCG CAGCCTGCGT GGGTGGAGGG GAGCTCAGCT 180

CGGTTGTGGG AGCAGGCGAC CGGCACTGGC TGGATGGACC TGGAAGCCTC GCTGCTGCCC 240

ACTGGTCCCA ACGCCAGCAA CACCTCTGAT GGCCCCGATA ACCTCACTTC GGCAGGATCA 300

CCTCCTCGCA CGGGGAGCAT CTCCTACATC AACATCATCA TGCCTTCGGT GTTCGGCACC 360

ATCTGCCTCC TGGGCATCAT CGGGAACTCC ACGGTCATCT TCGCGGTCGT GAAGAAGTCC 420

AAGCTGCACT GGTGCAACAA CGTCCCCGAC ATCTTCATCA TCAACCTCTC GGTAGTAGAT 480

CTCCTCTTTC TCCTGGGCAT GCCCTTCATG ATCCACCAGC TCATGGGCAA TGGGGTGTGG 540

CACTTTGGGG AGACCATGTG CACCCTCATC ACGGCCATGG ATGCCAATAG TCAGTTCACC 600

AGCACCTACA TCCTGACCGC CATGGCCATT GACCGCTACC TGGCCACTGT CCACCCCATC 660

TCTTCCACGA AGTTCCGGAA GCCCTCTGTG GCCACCCTGG TGATCTGCCT CCTGTGGGCC 720

CTCTCCTTCA TCAGCATCAC CCCTGTGTGG CTGTATGCCA GACTCATCCC CTTCCCAGGA 780

GGTGCAGTGG GCTGCGGCAT ACGCCTGCCC AACCCAGACA CTGACCTCTA CTGGTTCACC 840

CTGTACCAGT TTTTCCTGGC CTTTGCCCTG CCTTTTGTGG TCATCACAGC CGCATACGTG 900

AGGATCCTGC AGCGCATGAC GTCCTCAGTG GCCCCCGCCT CCCAGCGCAG CATCCGGCTG 960

CGGACAAAGA GGGTGACCCG CACAGCCATC GCCATCTGTC TGGTCTTCTT TGTGTGCTGG 1020
GCACCCTACT ATGTGCTACA GCTGACCCAG TTGTCCATCA GCCGCCCGAC CCTCACCTTT 1080
GTCTACTTAT ACAATGCGGC CATCAGCTTG GGCTATGCCA ACAGCTGCCT CAACCCCTTT 1140
GTGTACATCG TGCTCTGTGA GACGTTCCGC AAACGCTTGG TCCTGTCGGT GAAGCCTGCA 1200
GCCCAGGGGC AGCTTCGCGC TGTCAGCAAC GCTCAGACGG CTGACGAGGA GAGGACAGAA 1260
AGCAAAGGCA CCTGA 1275

<210> 9
<211> 422
<212> PRT
<213> Human
<400> 9

MeT Ser Val Gly Ala MeT Lys Lys Gly Val Gly Arg Ala Val Gly Leu
1 5 10 15
Gly Gly Gly Ser Gly Cys Gln Ala Thr Glu Glu Asp Pro Leu Pro Asn
20 25 30
Cys Gly Ala Cys Ala Pro Gly Gln Gly Gly Arg Arg Trp Arg Leu Pro
35 40 45
Gln Pro Ala Trp Val Glu Gly Ser Ser Ala Arg Leu Trp Glu Gln Ala
50 55 60
Thr Gly Thr Gly Trp MeT Asp Leu Glu Ala Ser Leu Leu Pro Thr Gly
65 70 75 80
Pro Asn Ala Ser Asn Thr Ser Asp Gly Pro Asp Asn Leu Thr Ser Ala
85 90 95
Gly Ser Pro Pro Arg Thr Gly Ser Ile Ser Tyr Ile Asn Ile Ile MeT
100 105 110
Pro Ser Val Phe Gly Thr Ile Cys Leu Leu Gly Ile Ile Gly Asn Ser
115 120 125
Thr Val Ile Phe Ala Val Val Lys Lys Ser Lys Leu His Trp Cys Asn
130 135 140

Asn Val Pro Asp Ile Phe Ile Ile Asn Leu Ser Val Val Asp Leu Leu
145 150 155 160
Phe Leu Leu Gly MeT Pro Phe MeT Ile His Gln Leu MeT Gly Asn Gly
165 170 175
Val Trp His Phe Gly Glu Thr MeT Cys Thr Leu Ile Thr Ala MeT Asp
180 185 190
Ala Asn Ser Gln Phe Thr Ser Thr Tyr Ile Leu Thr Ala MeT Ala Ile
195 200 205
Asp Arg Tyr Leu Ala Thr Val His Pro Ile Ser Ser Thr Lys Phe Arg
210 215 220
Lys Pro Ser Val Ala Thr Leu Val Ile Cys Leu Leu Trp Ala Leu Ser
225 230 235 240
Phe Ile Ser Ile Thr Pro Val Trp Leu Tyr Ala Arg Leu Ile Pro Phe
245 250 255
Pro Gly Gly Ala Val Gly Cys Gly Ile Arg Leu Pro Asn Pro Asp Thr
260 265 270
Asp Leu Tyr Trp Phe Thr Leu Tyr Gln Phe Phe Leu Ala Phe Ala Leu
275 280 285
Pro Phe Val Val Ile Thr Ala Ala Tyr Val Arg Ile Leu Gln Arg MeT
290 295 300
Thr Ser Ser Val Ala Pro Ala Ser Gln Arg Ser Ile Arg Leu Arg Thr
305 310 315 320
Lys Arg Val Thr Arg Thr Ala Ile Ala Ile Cys Leu Val Phe Phe Val
325 330 335
Cys Trp Ala Pro Tyr Tyr Val Leu Gln Leu Thr Gln Leu Ser Ile Ser
340 345 350
Arg Pro Thr Leu Thr Phe Val Tyr Leu Tyr Asn Ala Ala Ile Ser Leu
355 360 365
Gly Tyr Ala Asn Ser Cys Leu Asn Pro Phe Val Tyr Ile Val Leu Cys

370 375 380

Glu Thr Phe Arg Lys Arg Leu Val Leu Ser Val Lys Pro Ala Ala Gln

385 390 395 400

Gly Gln Leu Arg Ala Val Ser Asn Ala Gln Thr Ala Asp Glu Glu Arg

405 410 415

Thr Glu Ser Lys Gly Thr

420

<210> 10

<211> 31

<212> DNA

<213> Artificial Sequence

<220>

<223>

<400> 10

GTCGACATGG ACCTGGAAGC CTCGCTGCTG C 31

<210> 11

<211> 31

<212> DNA

<213> Artificial Sequence

<220>

<223>

<400> 11

ACTAGTTCAG GTGCCTTTGC TTTCTGTCCT C 31

<210> 12

<211> 33

<212> DNA

<213> Artificial Sequence

<220>

<223>

<400> 12

AGTCGACATG TCAGTGGGAG CCATGAAGAA GGG 33

<210> 13

<211> 33

<212> DNA

<213> Artificial Sequence

<220>

<223>

<400> 13

AACTAGTTCA GGTGCCTTTG CTTTCTGTCC TCT 33

<210> 14

<211> 1074

<212> DNA

<213> Human

<400> 14

GTCGACATGG ACCTGGAAGC CTCGCTGCTG CCCACTGGTC CCAACGCCAG CAACACCTCT 60
GATGGCCCCG ATAACCTCAC TTCGGCAGGA TCACCTCCTC GCACGGGGAG CATCTCCTAC 120
ATCAACATCA TCATGCCTTC GGTGTTCGGC ACCATCTGCC TCCTGGGCAT CATCGGGAAC 180
TCCACGGTCA TCTTCGCGGT CGTGAAGAAG TCCAAGCTGC ACTGGTGCAA CAACGTCCCC 240
GACATCTTCA TCATCAACCT CTCGGTAGTA GATCTCCTCT TTCTCCTGGG CATGCCCTTC 300
ATGATCCACC AGCTCATGGG CAATGGGGTG TGGCACTTTG GGGAGACCAT GTGCACCCTC 360
ATCACGGCCA TGGATGCCAA TAGTCAGTTC ACCAGCACCT ACATCCTGAC CGCCATGGCC 420
ATTGACCGCT ACCTGGCCAC TGTCCACCCC ATCTCTTCCA CGAAGTTCCG GAAGCCCTCT 480
GTGGCCACCC TGGTGATCTG CCTCCTGTGG GCCCTCTCCT TCATCAGCAT CACCCCTGTG 540
TGGCTGTATG CCAGACTCAT CCCCTTCCCA GGAGGTGCAG TGGGCTGCGG CATACGCCTG 600
CCCAACCCAG ACACTGACCT CTACTGGTTC ACCCTGTACC AGTTTTTCCT GGCCTTTGCC 660
CTGCCTTTTG TGGTCATCAC AGCCGCATAC GTGAGGATCC TGCAGCGCAT GACGTCCTCA 720
GTGGCCCCCG CCTCCCAGCG CAGCATCCGG CTGCGGACAA AGAGGGTGAC CCGCACAGCC 780
ATCGCCATCT GTCTGGTCTT CTTTGTGTGC TGGGCACCCT ACTATGTGCT ACAGCTGACC 840

CAGTTGTCCA TCAGCCGCCC GACCCTCACC TTTGTCTACT TATACAATGC GGCCATCAGC 900
TTGGGCTATG CCAACAGCTG CCTCAACCCC TTTGTGTACA TCGTGCTCTG TGAGACGTTC 960
CGCAAACGCT TGGTCCTGTC GGTGAAGCCT GCAGCCCAGG GGCAGCTTCG CGCTGTCAGC 1020
AACGCTCAGA CGGCTGACGA GGAGAGGACA GAAAGCAAAG GCACCTGAAC TAGT 1074

<210> 15
<211> 1283
<212> DNA
<213> Human
<400> 15

AGTCGACATG TCAGTGGGAG CCATGAAGAA GGGAGTGGGG AGGGCAGTTG GGCTTGGAGG 60
CGGCAGCGGC TGCCAGGCTA CGGAGGAAGA CCCCCTTCCC AACTGCGGGG CTTGCGCTCC 120
GGGACAAGGT GGCAGGCGCT GGAGGCTGCC GCAGCCTGCG TGGGTGGAGG GGAGCTCAGC 180
TCGGTTGTGG GAGCAGGCGA CCGGCACTGG CTGGATGGAC CTGGAAGCCT CGCTGCTGCC 240
CACTGGTCCC AACGCCAGCA ACACCTCTGA TGGCCCCGAT AACCTCACTT CGGCAGGATC 300
ACCTCCTCGC ACGGGGAGCA TCTCCTACAT CAACATCATC ATGCCTTCGG TGTTCGGCAC 360
CATCTGCCTC CTGGGCATCA TCGGGAACTC CACGGTCATC TTCGCGGTCG TGAAGAAGTC 420
CAAGCTGCAC TGGTGCAACA ACGTCCCCGA CATCTTCATC ATCAACCTCT CGGTAGTAGA 480
TCTCCTCTTT CTCCTGGGCA TGCCCTTCAT GATCCACCAG CTCATGGGCA ATGGGGTGTG 540
GCACTTTGGG GAGACCATGT GCACCCTCAT CACGGCCATG GATGCCAATA GTCAGTTCAC 600
CAGCACCTAC ATCCTGACCG CCATGGCCAT TGACCGCTAC CTGGCCACTG TCCACCCCAT 660
CTCTTCCACG AAGTTCCGGA AGCCCTCTGT GGCCACCCTG GTGATCTGCC TCCTGTGGGC 720
CCTCTCCTTC ATCAGCATCA CCCCTGTGTG GCTGTATGCC AGACTCATCC CCTTCCCAGG 780
AGGTGCAGTG GGCTGCGGCA TACGCCTGCC CAACCCAGAC ACTGACCTCT ACTGGTTCAC 840
CCTGTACCAG TTTTTCCTGG CCTTTGCCCT GCCTTTTGTG GTCATCACAG CCGCATACGT 900
GAGGATCCTG CAGCGCATGA CGTCCTCAGT GGCCCCCGCC TCCCAGCGCA GCATCCGGCT 960
GCGGACAAAG AGGGTGACCC GCACAGCCAT CGCCATCTGT CTGGTCTTCT TTGTGTGCTG 1020
GGCACCCTAC TATGTGCTAC AGCTGACCCA GTTGTCCATC AGCCGCCCGA CCCTCACCTT 1080
TGTCTACTTA TACAATGCGG CCATCAGCTT GGGCTATGCC AACAGCTGCC TCAACCCCTT 1140
TGTGTACATC GTGCTCTGTG AGACGTTCCG CAAACGCTTG GTCCTGTCGG TGAAGCCTGC 1200

AGCCCAGGGG CAGCTTCGCG CTGTCAGCAA CGCTCAGACG GCTGACGAGG AGAGGACAGA 1260

AAGCAAAGGC ACCTGAACTA GTT 1283

<210> 16

<211> 420

<212> RNA

<213> Human

<400> 16

CAAAAGCUGG AGCUCCACCG CGGUGGCGGC CGCUCUAGCC CACUAGUUCA GGUGCCUUUG 60

CUUUCUGUCC UCUCCUCGUC AGCCGUCUGA GCGUUGCUGA CAGCGCGAAG CUGCCCCUGG 120

GCUGCAGGCU UCACCGACAG GACCAAGCGU UUGCGGAACG UCUCACAGAG CACGAUGUAC 180

ACAAAGGGGU UGAGGCAGCU GUUGGCAUAG CCCAAGCUGA UGGCCGCAUU GUAUAAGUAG 240

ACAAAGGUGA GGGUCGGGCG GCUGAUGGAC AACUGGGUCA GCUGUAGCAC AUAGUAGGGU 300

GCCCAGCACA CAAAGAAGAC CAGACAGAUG GCGAUGGCUG UGCGGGUCAC CCUCUUUGUC 360

CGCAGCCGGA UGCUGCGCUG GGAGGCGGGG GCCACUGAGG ACGUCAUGCG CUGCAGGAUC 420

## Description

### Technical Field

The present invention relates to a melanin-concentrating hormone antagonist containing an aromatic amine derivative, an agent for the prophylaxis or therapy of obesity or hyperphagia and an agent for improving emotional disorder or sexual dysfunction.

### Background Art

Feeding behavior is an indispensable action for many organisms including human. An abnormality in feeding behavior causes deviation from normal life support activities, which in most cases results in diseases. Along with the recent changes in feeding environment, obesity is becoming a social problem. It is widely known that obesity is not only a serious risk factor of life-style related diseases, such as diabetes, hypertension, arteriosclerosis and the like, but also causes arthritis and pain due to excessive burden on joints of knee etc. created by increased body weight. In addition, the dieting boom and the like have increased the potential population that desires weight loss. There are many reports on eating disorders, such as hyperorexia and the like, due to neuropathy and the like, which are genetic or caused by stress.

Consequently, the development and investigation of an agent for the prophylaxis or therapy of obesity or feeding deterrents started some time ago and mazindol has been on the market as a centrally acting anorexiant.

Along therewith, a number of appetite-regulating factors represented by leptin have been found in recent years, and new anti-obesity agents and anorexiants that suppress the activity of such appetite-regulating factors have been developed. Among others, a melanin-concentrating hormone (MCH) is known to be a hormone derived from hypothalamus and promote appetite. Furthermore, MCH knockout mouse has been reported to show significantly decreased food intake and be lean, as compared to normal mouse, though normal in daily behavior [Nature, vol. 396, p. 670, 1998]. From the foregoing, an MCH antagonist, once completed, is expected to be a superior anorexiant or anti-obesity agent. However, a compound having an MCH antagonistic action, particularly a non-peptide compound, has not been known as yet.

JP-A-8-253447 discloses a compound having a gonadotropin releasing hormone antagonistic action, which is represented by the formula

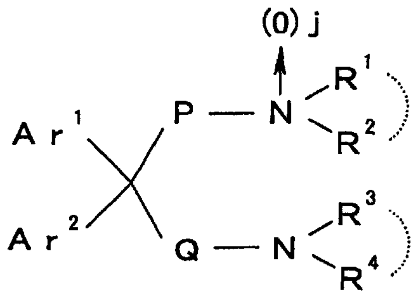

wherein

Ar$^1$ and Ar$^2$ are each an aromatic group optionally having substituents,
P and Q are each a divalent aliphatic hydrocarbon group having 2 or more carbon atoms, which optionally contains ether oxygen or ether sulfur in a carbon chain,
R$^1$ and R$^3$ are each i) an acyl group represented by -CO-R or -CONH-R (R is hydrocarbon group optionally having substituents or heterocyclic group optionally having substituents) or ii) a hydrocarbon group optionally having substituents,
R$^2$ and R$^4$ are each a hydrogen atom or an alkyl group optionally having substituents,
R$^1$ and R$^2$ or R$^3$ and R$^4$ may form, together with the adjacent nitrogen atom, a nitrogen-containing heterocyclic group optionally having substituents, and
j is 0 or 1, or a salt thereof.

JP-A-10-81665 discloses a compound having an MIP-1α/RANTES antagonistic action, which is represented by the formula

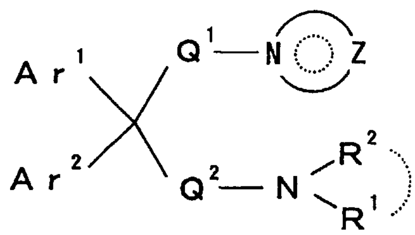

wherein $Ar^1$ and $Ar^2$ are each an aromatic group optionally having substituents, $Q^1$ and $Q^2$ are each a divalent $C_{1-6}$ aliphatic hydrocarbon group which optionally contains ether oxygen or ether sulfur in a carbon chain and which optionally has substituents, $R^1$ is a hydrogen atom, a lower alkyl group optionally having substituents or a lower alkyl-carbonyl group optionally having substituents, $R^2$ is a hydrocarbon group optionally having substituents or an acyl group, or $R^1$ and $R^2$ optionally form, together with the adjacent nitrogen atom, a nitrogen-containing heterocycle optionally having substituents, and a group of the formula

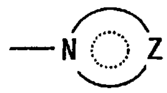

is a monocyclic or fused nitrogen-containing heterocycle optionally having substituents, or a salt thereof.

However, a compound having a sufficiently superior MCH antagonistic action as a pharmaceutical product has not been found. Thus, the development of a clinically useful and safe compound having a superior MCH antagonistic action has been demanded.

**Disclosure of the Invention**

The present inventors have intensively studied from various aspects, seeking a compound having an MCH antagonistic action, and found that a compound of the formula

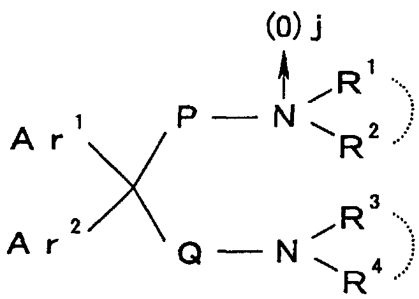

(I)

wherein $Ar^1$ and $Ar^2$ are each an aromatic group optionally having substituents, P and Q are each a divalent aliphatic hydrocarbon group which optionally contains ether oxygen or ether sulfur in a carbon chain and which optionally has substituents, $R^1$ and $R^3$ are each (i) a hydrogen atom, (ii) an acyl group or (iii) a hydrocarbon group optionally having substituents, $R^2$ and $R^4$ are each (i) a hydrogen atom, (ii) an alkyl group optionally having substituents or (iii) an alkyl-carbonyl group optionally having substituents, $R^1$ and $R^2$ or $R^3$ and $R^4$ optionally form, together with the adjacent nitrogen atom, a monocyclic or fused nitrogen-containing heterocyclic group optionally having substituents, and j is 0 or 1, or a salt thereof, is not influenced by the presence or the kind of cyclic substituent, but unexpectedly has a superior MCH antagonistic action and low toxicity, and is clinically useful as an agent for the prophylaxis or therapy of obesity or hyperphagia, an agent for improving emotional disorder or sexual dysfunction and the like. Based on the finding, the present inventors have further studied and completed the present invention.

Accordingly, the present invention provides

[1] a melanin-concentrating hormone antagonist containing a compound of the formula

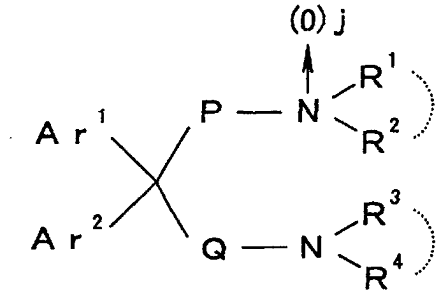

wherein

| | |
|---|---|
| $Ar^1$ and $Ar^2$ | are each an aromatic group optionally having substituents, |
| P and Q | are each a divalent aliphatic hydrocarbon group which optionally contains ether oxygen or ether sulfur in a carbon chain and which optionally has substituents, |
| $R^1$ and $R^3$ | are each (i) a hydrogen atom, (ii) an acyl group or (iii) a hydrocarbon group optionally having substituents, |
| $R^2$ and $R^4$ | are each (i) a hydrogen atom, (ii) an alkyl group optionally having substituents or (iii) an alkylcarbonyl group optionally having substituents, |
| $R^1$ and $R^2$ or $R^3$ and $R^4$ | optionally form, together with the adjacent nitrogen atom, a monocyclic or fused nitrogen-containing heterocyclic group optionally having substituents, and |
| j | is 0 or 1, |

or a salt thereof or a prodrug thereof;

[2] The antagonist of [1] , wherein $Ar^1$ and $Ar^2$ are each (i) a $C_{6-14}$ aryl group or (ii) a 5 to 14-membered monocyclic or fused aromatic heterocyclic group containing, besides carbon atom, 1 to 4 heteroatom(s) selected from the group consisting of nitrogen atom, sulfur atom and oxygen atom, which optionally has 1 to 5 substituent(s) selected from the group (group Aa) consisting of

(a) a halogen atom,
(b) a $C_{1-3}$ alkylenedioxy group,
(c) a nitro group,
(d) a cyano group,
(e) an optionally halogenated $C_{1-6}$ alkyl group,
(f) an optionally halogenated $C_{3-6}$ cycloalkyl group,
(g) an optionally halogenated $C_{1-6}$ alkoxy group,
(h) an optionally halogenated $C_{1-6}$ alkylthio group,
(i) a hydroxy group,
(j) an amino group,
(k) a mono-$C_{1-6}$ alkylamino group,
(l) a di-$C_{1-6}$ alkylamino group,
(m) an optionally halogenated $C_{1-6}$ alkyl-carbonylamino group,
(n) a formyl group,
(o) a $C_{1-6}$ alkyl-carbonyl group optionally substituted by halogen atom or $C_{1-6}$ alkoxy-carbonyl group,
(p) a $C_{1-6}$ alkyl-carbonyloxy group,
(q) a carboxyl group,
(r) a $C_{1-6}$ alkoxy-carbonyl group,
(s) a carbamoyl group,
(t) a mono-$C_{1-6}$ alkyl-carbamoyl group optionally substituted by $C_{1-6}$ alkoxy-carbonyl group,
(u) a di-$C_{1-6}$ alkyl-carbamoyl group optionally substituted by $C_{1-6}$ alkoxy-carbonyl group,
(v) a sulfo group,
(w) a $C_{1-6}$ alkylsulfonyl group,
(x) a $C_{1-6}$ alkylsulfinyl group,
(y) a $C_{6-10}$ aryl group optionally having 1 to 4 substituent(s) selected from the above-mentioned (a) to (x),
(z) a $C_{6-10}$ aryloxy group optionally having 1 to 4 substituent(s) selected from the above-mentioned (a) to (x),

(aa) an optionally halogenated $C_{6-10}$ aryl-carbonyl group,
(ab) an optionally halogenated 5 or 6-membered heterocyclic ring-carbonyl group,
(ac) a $C_{1-6}$ alkoxy-carbonylamino group,
(ad) a $C_{6-10}$ aryl-carbonylamino group and
(ae) a $C_{7-16}$ aralkyloxy-carbonyl group,

P and Q are each a divalent $C_{1-6}$ aliphatic hydrocarbon group which optionally contains ether oxygen or ether sulfur in a carbon chain and which is optionally substituted by oxo group or thioxo group;
$R^1$ and $R^3$ are each (i) hydrogen atom, (ii) acyl group represented by $-CO-R^a$, $-CONR^aR^b$, $-SO-R^a$, $-SO_2-R^a$, $-CONR^aR^b$, $-COO-R^a$, $-(C=S)O-R^a$, $-(C=S)NR^aR^b$, $-SONR^aR^b$, $-SO_2NR^aR^b$, $-SO-O-R^a$ or $-SO_2-O-R^a$, wherein $R^a$ is (A) hydrogen atom; (B) carboxyl group; (C) (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group, which optionally has 1 to 5 substituent(s) selected from the group (group Ba) consisting of

(a) a halogen atom,
(b) a $C_{1-3}$ alkylenedioxy group,
(c) a nitro group,
(d) a cyano group,
(e) an optionally halogenated $C_{1-6}$ alkyl group,
(f) an optionally halogenated $C_{3-6}$ cycloalkyl group,
(g) an optionally halogenated $C_{1-6}$ alkoxy group,
(h) an optionally halogenated $C_{1-6}$ alkylthio group,
(i) a hydroxy group,
(j) an amino group,
(k) a mono-$C_{1-6}$ alkylamino group,
(l) a di-$C_{1-6}$ alkylamino group,
(m) a $C_{1-6}$ alkyl-carbonylamino group,
(n) a formyl group,
(o) a $C_{1-6}$ alkyl-carbonyl group,
(p) a $C_{1-6}$ alkyl-carbonyloxy group,
(q) a carboxyl group,
(r) a $C_{1-6}$ alkoxy-carbonyl group,
(s) a carbamoyl group,
(t) a mono-$C_{1-6}$ alkyl-carbamoyl group,
(u) a di-$C_{1-6}$ alkyl-carbamoyl group,
(v) a sulfo group,
(w) a $C_{1-6}$ alkylsulfonyl group,
(x) a $C_{1-6}$ alkylsulfinyl group,
(y) a $C_{6-10}$ aryl group optionally having 1 to 4 substituent(s) selected from the aforementioned (a) to (x),
(z) a $C_{6-10}$ aryloxy group optionally having 1 to 4 substituent(s) selected from the aforementioned (a) to (x),
(zz) a 5 to 7-membered heterocyclic group optionally having 1 to 4 substituent(s) selected from the aforementioned (a) to (x),

(aa) a di-$C_{1-6}$ alkyl-carbonylamino group,
(ab) a sulfamoyl group,
(ac) a $C_{1-6}$ alkoxy-carbonylamino group,
(ad) a $C_{7-16}$ aralkyloxy-carbonylamino group,
(ae) a $C_{7-16}$ aralkyloxy group,
(af) a $C_{6-10}$ aryl-carbonyl group,
(ag) a $C_{1-6}$ alkyl-carbonyloxy group,
(ah) a $C_{6-10}$ aryl-carbonylamino group,
(ai) a $C_{6-10}$ aryl-carbamoyl group,
(aj) a $C_{7-16}$ aralkylaminocarbonyl group,
(ak) a $C_{7-16}$ aralkylcarbonylamino group and
(al) a $C_{7-16}$ aralkyloxy-carbonyloxy group;

(D) a 5 to 10-membered heterocyclic group containing, besides carbon atom, 1 to 4 heteroatom(s) selected from the group consisting of nitrogen atom, oxygen atom and sulfur atom, which optionally has 1 to 5 substituent(s)

selected from the group consisting of (a) substituent selected from group Aa,

(b) (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group, which optionally has 1 to 5 substituent(s) selected from group Ba,
(c) oxo group and
(d) thioxo group; or

(E) a $C_{1-6}$ alkoxy-carbonyl group;
$R^b$ is a hydrogen atom or a $C_{1-6}$ alkyl group, or (iii) (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group optionally having 1 to 5 substituent (s) selected from group Ba;
$R^2$ and $R^4$ are each (i) a hydrogen atom, (ii) $C_{1-6}$ alkyl group optionally having substituents selected from group Ba or (iii) $C_{1-6}$ alkyl-carbonyl group optionally having substituents selected from group Ba;
$R^1$ and $R^2$ or $R^3$ and $R^4$ may form, together with the adjacent nitrogen atom, a group of

(i) the formula

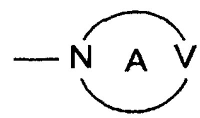

wherein ring A is a 4 to 8-membered ring optionally substituted by hydroxy or oxo, V is a group represented by the formula >O, >C=O, >C(W)-$W^a$ or >N-W (W is (a) hydrogen atom, (b) (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group, which optionally has 1 to 5 substituent(s) selected from group Ba, or (c) 5 to 10-membered heterocyclic group containing, besides carbon atom, 1 to 4 heteroatom(s) selected from nitrogen, oxygen and sulfur, which optionally has 1 to 5 substituent(s) selected from group Aa, $W^a$ is hydrogen atom, hydroxy group or $C_{1-6}$ alkyl group),
(ii) the formula

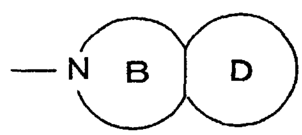

wherein ring B is monocyclic or bicyclic 4 to 12-membered ring optionally substituted by 1 or 2 oxo group(s) or 1 to 5 $C_{1-6}$ alkyl group(s), ring D is a 4 to 12-membered aromatic ring optionally having 1 to 5 substituent (s) selected from group Aa or
(iii) the formula

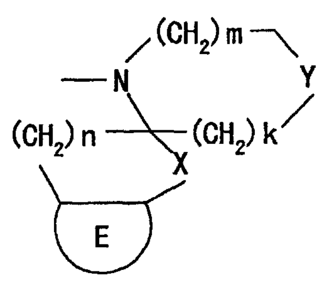

wherein ring E is a 4 to 12-membered aromatic ring optionally having 1 to 5 substituent(s) selected from group Aa;

X is $-CH_2-$, -CO- or -CH(OH)-;
Y is $-CH_2-$, -O- or $-NW^b-$ ($W^b$ is (a) hydrogen atom or (b) $C_{1-6}$ alkyl group optionally having substituents selected from group Ba);

k and m are each an integer of 0 to 4, and k+m is an integer of 1 to 4;
n is an integer of 1 to 3,

[3] the antagonist of [1] wherein $Ar^1$ and $Ar^2$ are each (i) a $C_{6-14}$ aryl group or (ii) a 5 to 14-membered monocyclic or fused aromatic heterocyclic group containing, besides carbon atom, 1 to 4 heteroatom(s) selected from the group consisting of nitrogen atom, sulfur atom and oxygen atom, which optionally has 1 to 5 substituent(s) selected from the group (group A) consisting of

(a) a halogen atom,
(b) a $C_{1-3}$ alkylenedioxy group,
(c) a nitro group,
(d) a cyano group,
(e) an optionally halogenated $C_{1-6}$ alkyl group,
(f) an optionally halogenated $C_{3-6}$ cycloalkyl group,
(g) an optionally halogenated $C_{1-6}$ alkoxy group,
(h) an optionally halogenated $C_{1-6}$ alkylthio group,
(i) a hydroxy group,
(j) an amino group,
(k) a mono-$C_{1-6}$ alkylamino group,
(l) a di-$C_{1-6}$ alkylamino group,
(m) a $C_{1-6}$ alkyl-carbonylamino group,
(n) a formyl group,
(o) a $C_{1-6}$ alkyl-carbonyl group,
(p) a $C_{1-6}$ alkyl-carbonyloxy group,
(q) a carboxyl group,
(r) a $C_{1-6}$ alkoxy-carbonyl group,
(s) a carbamoyl group,
(t) a mono-$C_{1-6}$ alkylcarbamoyl group,
(u) a di-$C_{1-6}$ alkylcarbamoyl group,
(v) a sulfo group,
(w) a $C_{1-6}$ alkylsulfonyl group,
(x) a $C_{1-6}$ alkylsulfinyl group,
(y) a $C_{6-10}$ aryl group optionally having 1 to 4 substituent(s) selected from the above-mentioned (a) to (x) and
(z) a $C_{6-10}$ aryloxy group optionally having 1 to 4 substituent(s) selected from the above-mentioned (a) to (x),

P and Q are each a $C_{1-6}$ aliphatic hydrocarbon group which optionally contains ether oxygen or ether sulfur in a carbon chain and which is optionally substituted by oxo group or thioxo group,
$R^1$ and $R^3$ are each (i) hydrogen atom, (ii) an acyl group represented by $-CO-R^a$, $-CONR^aR^b$, $-SO-R^a$, $-SO_2-R^a$, $-CONR^aR^b$, $-COO-R^a$, $-(C=S)O-R^a$ or $-(C=S)NR^aR^b$ wherein $R^a$ is

(a) hydrogen atom,
(b) carboxyl group,
(c) a (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group, which optionally has 1 to 5 substituent(s) selected from the group (group B) consisting of

(a) a halogen atom,
(b) a $C_{1-3}$ alkylenedioxy group,
(c) a nitro group,
(d) a cyano group,
(e) an optionally halogenated $C_{1-6}$ alkyl group,
(f) an optionally halogenated $C_{3-6}$ cycloalkyl group,
(g) an optionally halogenated $C_{1-6}$ alkoxy group,
(h) an optionally halogenated $C_{1-6}$ alkylthio group,
(i) a hydroxy group,
(j) an amino group,
(k) a mono-$C_{1-6}$ alkylamino group,
(l) a di-$C_{1-6}$ alkylamino group,

(m) a $C_{1-6}$ alkyl-carbonylamino group,
(n) a formyl group,
(o) a $C_{1-6}$ alkyl-carbonyl group,
(p) a $C_{1-6}$ alkyl-carbonyloxy group,
(q) a carboxyl group,
(r) a $C_{1-6}$ alkoxy-carbonyl group,
(s) a carbamoyl group,
(t) a mono-$C_{1-6}$ alkylcarbamoyl group,
(u) a di-$C_{1-6}$ alkylcarbamoyl group,
(v) a sulfo group,
(w) a $C_{1-6}$ alkylsulfonyl group,
(x) a $C_{1-6}$ alkylsulfinyl group,
(y) a $C_{6-10}$ aryl group optionally having 1 to 4 substituent(s) selected from the aforementioned (a) to (x),
(z) a $C_{6-10}$ aryloxy group optionally having 1 to 4 substituent(s) selected from the aforementioned (a) to (x) and
(zz) a 5 to 7-membered heterocyclic group optionally having 1 to 4 substituent(s) selected from the aforementioned (a) to (x), or
(d) a 5 to 10-membered heterocyclic group containing, besides carbon atom, 1 to 4 heteroatom(s) selected from the group consisting of nitrogen atom, oxygen atom and sulfur atom, which optionally has 1 to 5 substituent(s) selected from the group (group C) consisting of

(a) a halogen atom,
(b) a $C_{1-3}$ alkylenedioxy group,
(c) a nitro group,
(d) a cyano group,
(e) a $C_{1-6}$ alkyl group optionally having substituents selected from the group consisting of (aa) a halogen atom, (bb) $C_{1-3}$ alkylenedioxy group, (cc) nitro group, (dd) cyano group, (ee) an optionally halogenated $C_{1-6}$ alkyl group, (ff) an optionally halogenated $C_{3-6}$ cycloalkyl group, (gg) an optionally halogenated $C_{1-6}$ alkoxy group, (hh) an optionally halogenated $C_{1-6}$ alkylthio group, (ii) a hydroxy group, (jj) amino group, (kk) a mono-$C_{1-6}$ alkylamino group, (ll) a di-$C_{1-6}$ alkylamino group, (mm) $C_{1-6}$ alkyl-carbonylamino group, (nn) a formyl group, (oo) $C_{1-6}$ alkyl-carbonyl group, (pp) $C_{1-6}$ alkyl-carbonyloxy group, (qq) carboxyl group, (rr) $C_{1-6}$ alkoxy-carbonyl group, (ss) carbamoyl group, (tt) a mono-$C_{1-6}$ alkylcarbamoyl group, (uu) a di-$C_{1-6}$ alkylcarbamoyl group, (vv) a sulfo group, (ww) $C_{1-6}$ alkylsulfonyl group, (xx) $C_{1-6}$ alkylsulfinyl group, (yy) $C_{6-10}$ aryl group optionally having 1 to 4 substituent(s) selected from the aforementioned (aa) to (xx), (zz) $C_{6-10}$ aryloxy group optionally having 1 to 4 substituent(s) selected from the aforementioned (aa) to (xx) and (zzz) 5 to 7-membered heterocyclic group optionally having 1 to 4 substituent(s) selected from the aforementioned (aa) to (xx),
(f) an optionally halogenated $C_{3-6}$ cycloalkyl group,
(g) an optionally halogenated $C_{1-6}$ alkoxy group,
(h) an optionally halogenated $C_{1-6}$ alkylthio group,
(i) a hydroxy group,
(j) an amino group,
(k) a mono-$C_{1-6}$ alkylamino group,
(l) a di-$C_{1-6}$ alkylamino group,
(m) an optionally halogenated $C_{1-6}$ alkyl-carbonylamino group,
(n) a formyl group,
(o) a $C_{1-6}$ alkyl-carbonyl group,
(p) a $C_{1-6}$ alkyl-carbonyloxy group,
(q) a carboxyl group,
(r) a $C_{1-6}$ alkoxy-carbonyl group,
(s) a carbamoyl group,
(t) a mono-$C_{1-6}$ alkylcarbamoyl group,
(u) a di-$C_{1-6}$ alkylcarbamoyl group,
(v) a sulfo group,
(w) a $C_{1-6}$ alkylsulfonyl group,
(x) a $C_{1-6}$ alkylsulfinyl group,
(y) a $C_{6-10}$ aryl group optionally having 1 to 4 substituent(s) selected from the aforementioned (a) to (x) and
(z) a $C_{6-10}$ aryloxy group optionally having 1 to 4 substituent(s) selected from the aforementioned (a) to (x), and $R^b$ is a hydrogen atom or a $C_{1-6}$ alkyl group) or (iii) (1) $C_{1-6}$ alkyl group, (2) $C_{2-6}$ alkenyl group, (3) $C_{2-6}$

alkynyl group, (4) $C_{3-6}$ cycloalkyl group, (5) $C_{6-14}$ aryl group or (6) $C_{7-16}$ aralkyl group, which optionally has 1 to 5 substituent(s) selected from group B,

$R^2$ and $R^4$ are each (i) hydrogen atom, (ii) $C_{1-6}$ alkyl optionally having substituents selected from group B or (iii) $C_{1-6}$ alkyl-carbonyl group optionally having substituents selected from group B,
$R^1$ and $R^2$ or $R^3$ and $R^4$ form, together with the adjacent nitrogen atom, a nitrogen-containing heterocyclic group represented by

(i) the formula

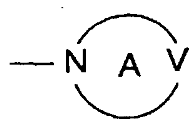

wherein ring A is a 4 to 8-membered ring optionally substituted by hydroxy or oxo, V is a group represented by the formula >O, >C=O, >C-(W)$W^a$ or >N-W (W is (a) hydrogen atom, (b) (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group, which optionally has 1 to 5 substituent(s) selected from group B, or (c) 5 to 10-membered heterocyclic group containing, besides carbon atom, 1 to 4 heteroatom(s) selected from nitrogen, oxygen and sulfur, which optionally has 1 to 5 substituent(s) selected from group A, $W^a$ is hydrogen atom or hydroxy group),
(ii) the formula

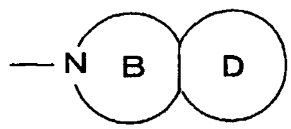

wherein ring B is monocyclic or bicyclic 4 to 12-membered ring optionally substituted by oxo group or 1 to 5 $C_{1-6}$ alkyl group(s), ring D is a 4 to 12-membered aromatic ring optionally having 1 to 5 substituent(s) selected from group A or
(iii) the formula

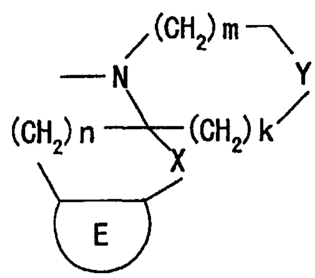

wherein ring E is a 5 to 10-membered aromatic ring optionally having 1 to 5 substituent(s) selected from group A

X is $-CH_2-$, -CO- or -CH(OH)-,
Y is $-CH_2-$, -O- or $-NW^b-$ ($W^b$ is (a) hydrogen atom or (b) $C_{1-6}$ alkyl group optionally having substituents selected from group B);
k+m is an integer of 1 to 4; and
n is an integer of 1 to 3,

[4] the antagonist of [1], wherein $Ar^1$ and $Ar^2$ are each (i) a phenyl group optionally substituted by halogen atom or $C_{1-6}$ alkoxy group or (ii) a 5 or 6-membered heterocyclic group containing, besides carbon atom, 1 to 3 heteroatom(s) selected from nitrogen atom, oxygen atom and sulfur atom,
[5] the antagonist of [1], wherein P and Q are each a $C_{1-6}$ alkylene group,
[6] the antagonist of [1], wherein j is 0,
[7] the antagonist of [1], wherein

$R^1$ is (i) $C_{1-6}$ alkyl group optionally having a 5 or 6-membered nitrogen-containing heterocyclic group, (ii) $C_{7-16}$ aralkyl group optionally having nitro, amino or $C_{1-6}$ alkoxy-carbonyl or (iii) cyclohexyl group fused with benzene ring optionally having $C_{1-6}$ alkoxy;
$R^2$ is (i) hydrogen atom, (ii) $C_{1-6}$ alkyl group or (iii) $C_{7-16}$ aralkyl group; or $R^1$ and $R^2$ form, together with the adjacent nitrogen atom, a nitrogen-containing heterocyclic group represented by

(i) the formula

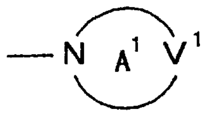

wherein ring $A^1$ is a 4 to 8-membered ring optionally substituted by hydroxy or oxo, $V^1$ is a group represented by the formula >O, >C($W^1$)-$W^{a1}$ or >N-$W^1$ ($W^1$ is (a) hydrogen atom, (b) $C_{6-14}$ aryl group optionally having 1 or 2 substituent(s) selected from the group consisting of a halogen atom, optionally halogenated $C_{1-6}$ alkyl group and optionally halogenated $C_{1-6}$ alkoxy group, (c) $C_{1-6}$ alkyl group optionally having 1 or 2 $C_{6-10}$ aryl group(s) or (d) pyridyl group, $W^{a1}$ is hydrogen atom, hydroxy group or $C_{1-6}$ alkyl group),
(ii) the formula

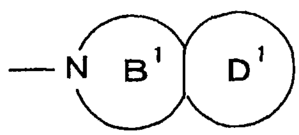

wherein ring $B^1$ is a monocyclic or bicyclic 5 to 10-membered ring optionally substituted by oxo group or 1 or 2 $C_{1-6}$ alkyl group(s), ring $D^1$ is a benzene ring optionally having 1 or 2 substituent(s) selected from the group consisting of $C_{1-6}$ alkyl group, $C_{1-6}$ alkoxy group and $C_{1-6}$ alkyl-carbonyl group or
(iii) the formula

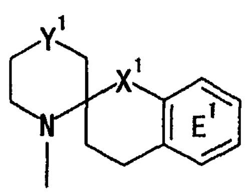

wherein ring $E^1$ is a benzene ring optionally having 1 to 3 substituent(s) selected from the group consisting of $C_{1-3}$ alkylenedioxy group, nitro group, $C_{1-6}$ alkoxy group, amino group, $C_{1-6}$ alkyl-carbonylamino group and $C_{1-6}$ alkoxy-carbonyl group,

$X^1$ is $-CH_2-$ or -CO-, and
$Y^1$ is $-CH_2-$ or -O-,
$R^3$ is

(i) hydrogen atom,
(ii) a group represented by the formula -CO-$R^5$ ($R^5$ is (a) hydrogen atom, (b) carboxyl group, (c) $C_{1-6}$ alkyl group, (d) $C_{5-6}$ cycloalkyl group optionally having $C_{1-6}$ alkoxy, and which is fused with benzene ring or (e) 5 or 6-membered aromatic heterocyclic group containing, besides carbon

atom, 1 to 3 heteroatom(s) selected from the group consisting of nitrogen atom, oxygen atom and sulfur atom, which optionally has 1 or 2 substituent(s) selected from the group consisting of a halogen atom, $C_{6-10}$ aryl group, $C_{6-10}$ aryl-carbonylamino group),

(iii) a group represented by the formula -CO-$Alk_0$-$R^6$ [$Alk_0$ is $C_{1-6}$ alkylene group optionally having hydroxy group, $R^6$ is (a) $C_{6-14}$ aryl group optionally having 1 or 2 substituent (s) selected from the group consisting of a halogen atom, optionally halogenated $C_{1-6}$ alkyl, nitro, $C_{1-6}$ alkoxy, $C_{1-3}$ alkylenedioxy and $C_{6-10}$ aryl group, (b) $C_{6-10}$ aryloxy group, (c) 5 or 6-membered aromatic heterocyclic group containing, besides carbon atom, 1 or 2 heteroatom(s) selected from nitrogen atom, oxygen atom and sulfur atom (d) $C_{1-6}$ alkyl-carbonyl group, (e) carboxyl group, (f) $C_{1-6}$ alkoxy-carbonyl group, (g) amino group optionally having 1 or 2 substituent(s) selected from the group consisting of $C_{1-6}$ alkyl and $C_{1-6}$ alkyl-carbonyl, (h) 5 to 7-membered heterocyclic group optionally having hydroxy, (i) $C_{7-16}$ aralkyloxy group, (j) $C_{6-10}$ aryl-carbonyl group or (k) $C_{1-6}$ alkyl-carbonyloxy group],

(iv) a group represented by the formula

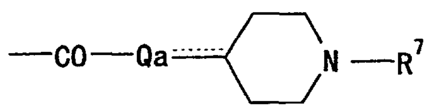

wherein Qa is a group represented by the formula -$(CH_2)$s- (s is an integer of 1 to 3) or -$(CH_2)$t-CH= (t is an integer of 0 to 2) and $R^7$ is hydrogen atom or $C_{1-6}$ alkoxy-carbonyl group,

(v) a group represented by the formula

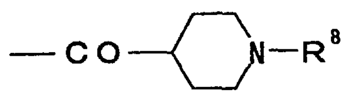

wherein $R^8$ is (a) hydrogen atom, (b) $C_{1-6}$ alkyl group optionally having substituents selected from the group consisting of $C_{1-6}$ alkoxy-carbonyl, morpholino and mono- or di-$C_{1-6}$ alkylamino, (c) $C_{1-6}$ alkoxy-carbonyl group, (d) a group represented by the formula -CO-$R^d$ ($R^d$ is $C_{6-10}$ aryl group optionally having halogen atom or 5 or 6-membered heterocyclic group containing, besides carbon atom, 1 or 2 heteroatom(s) selected from nitrogen atom, oxygen atom and sulfur atom), (e) a group represented by the formula -CO-$(CH_2)r^1$-$R^e$ ($r^1$ is an integer of 1 to 3, $R^e$ is $C_{1-6}$ alkoxy-carbonyl group or 5 or 6-membered heterocyclic group containing, besides carbon atom, 1 or 2 heteroatom(s) selected from nitrogen atom, oxygen atom and sulfur atom) or (f) a group represented by -CONH-$R^f$ ($R^f$ is $C_{1-6}$ alkyl group or $C_{6-14}$ aryl group),

(vi) a group represented by the formula -$COOR^9$ ($R^9$ is optionally halogenated $C_{1-6}$ alkyl group),

(vii) a group represented by the formula

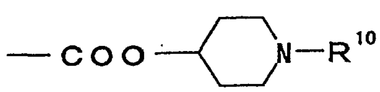

wherein $R^{10}$ is hydrogen atom, $C_{1-6}$ alkoxy-carbonyl group, mono-or di-$C_{1-6}$ alkyl-carbamoyl group, optionally halogenated nicotinoyl group or optionally halogenated isonicotinoyl group,

(viii) a group represented by the formula -$CONR^{11}$-$R^{12}$ ($R^{11}$ is hydrogen atom or $C_{1-6}$ alkyl group, $R^{12}$ is $C_{1-6}$ alkyl group optionally having substituents selected from the group consisting of (a) hydroxy, (b) amino, (c) a mono- or di-$C_{1-6}$ alkyl-amino, (d) $C_{1-6}$ alkyl-carbonyl, (e) $C_{1-6}$ alkoxy-carbonyl, (f) $C_{1-6}$ alkyl-carbonyloxy, (g) sulfamoyl and (h) 5 to 7-membered heterocyclic group optionally substituted by oxo, and (i) $C_{6-14}$ aryl group) ,

(ix) a group represented by the formula

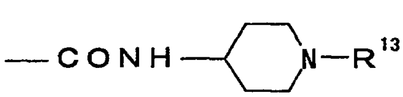

wherein $R^{13}$ is (a) hydrogen atom, (b) $C_{1-6}$ alkyl group optionally having substituents selected from the group consisting of a hydroxy and $C_{1-6}$ alkoxy-carbonyl, (c) $C_{7-16}$ aralkyl group, (d) $C_{1-6}$ alkyl-carbonyl group optionally having substituents selected from the group consisting of a halogen atom and $C_{1-6}$ alkoxy-carbonyl or (e) $C_{1-6}$ alkyl-carbamoyl group optionally having $C_{1-6}$ alkoxy-carbonyl,
(x) a group represented by the formula

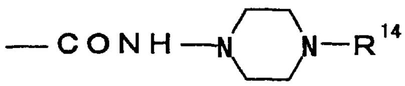

wherein $R^{14}$ is $C_{1-6}$ alkyl group or $C_{7-16}$ aralkyl group,
(xi) a group represented by the formula

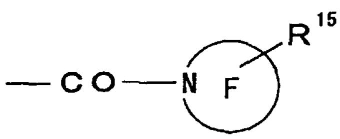

wherein ring F is 5 to 7-membered non-aromatic heterocyclic group optionally fused with benzene ring and $R^{15}$ is hydrogen atom, $C_{1-6}$ alkoxy-carbonylamino group or optionally halogenated $C_{1-6}$ alkyl-carbonylamino group,
(xii) a group represented by the formula

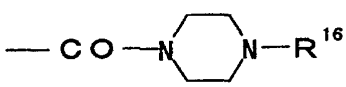

wherein $R^{16}$ is (a) $C_{1-6}$ alkyl group optionally having substituents selected from the group consisting of a hydroxy and $C_{1-6}$ alkoxy-carbonyl, (b) a formyl group, (c) $C_{1-6}$ alkoxy-carbonyl group or (d) 5 or 6-membered heterocyclic ring-carbonyl group containing, besides carbon atom, 1 to 3 heteroatom(s) selected from the group consisting of nitrogen atom, oxygen atom and sulfur atom,
(xiii) a group represented by the formula $-SO_2-R^{17}$ ($R^{17}$ is (i) $C_{1-6}$ alkyl group optionally having 5 or 6-membered heterocyclic group, (ii) $C_{2-6}$ alkenyl group or (iii) $C_{6-14}$ aryl group optionally having $C_{1-6}$ alkyl),
(xiv) $C_{7-16}$ aralkyl group optionally having 1 to 3 halogen atom(s) or $C_{1-6}$ alkoxy group,
(xv) $C_{1-6}$ alkyl group substituted by 5 or 6-membered heterocyclic group containing, besides carbon atom, 1 to 3 heteroatom(s) selected from the group consisting of nitrogen atom, oxygen atom and sulfur atom,
(xvi) a group represented by the formula

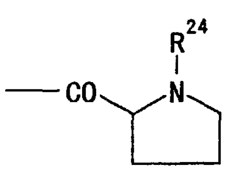

wherein $R^{24}$ is hydrogen atom or $C_{7-16}$ aralkyloxy-carbonyl group;
(xvii) a group represented by the formula

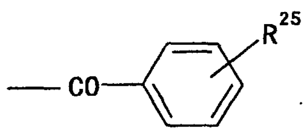

wherein $R^{25}$ is hydrogen atom, $C_{6-10}$ aryl group, $C_{7-16}$ aralkyloxy group, $C_{6-10}$ aryloxy group, halogen atom, $C_{6-10}$ aryl-carbonylamino group or $C_{6-10}$ aryl-carbamoyl group;
(xviii) a group represented by the formula $-CO-Alk-NR^{27}-CO-Alk_2-O-Alk_3-R^{28}$
[Alk is $C_{1-6}$ alkylene group optionally having substituents; $R^{27}$ is hydrogen atom or $C_{1-6}$ alkyl group; $Alk_2$ and $Alk_3$ are the same or different and each is a bond or $C_{1-6}$ alkylene group optionally having substituents; $R^{28}$ is $C_{6-10}$ aryl group optionally having substituents or hydrogen atom];
(xix) a group represented by the formula $-CO-Alk_2-NR^{27}-CO-Alk_3-R^{29}$
[$Alk_2$, $Alk_3$ and $R^{27}$ are as defined above; $R^{29}$ is (1) $C_{6-10}$ aryl group optionally having substituent or (2) 5 to 10-membered aromatic heterocyclic group optionally having substituent, which contains, besides carbon atom, 1 to 3 heteroatom(s) selected from the group consisting of nitrogen atom, oxygen atom and sulfur atom] ;
(xx) a group represented by the formula $-CO-Alk-NR^{27}-CO-Alk_2-NR^{30}-Alk_3-R^{31}$
[Alk, $R^{27}$, $Alk_2$, $Alk_3$ are as defined above; $R^{30}$ is hydrogen atom, $C_{1-6}$ alkyl group or optionally halogenated $C_{1-6}$ alkyl-carbonyl group; and $R^{31}$ is $C_{6-10}$ aryl group optionally having substituents] ;
(xxi) a group represented by the formula $-CO-Alk-NR^{27}-CO-Alk_2-NR^{32}-CO-O-Alk_3-R^{31}$
[Alk, $R^{27}$, $Alk_2$, $Alk_3$ and $R^{31}$ are as defined above; and $R^{32}$ is the same as the aforementioned $R^{27}$];
(xxii) a group represented by the formula $-CO-Alk-CO-NR^{27}-Alk_2-R^{31}$
[Alk, $R^{27}$, $Alk_2$ and $R^{31}$ are as defined above]; or
(xxiii) a group represented by the formula $-CO-Alk-O-CO-O-Alk_2-R^{31}$
[Alk, $Alk_2$ and $R^{31}$ are as defined above];

$R^4$ is hydrogen atom or $C_{1-6}$ alkyl group;
or $R^3$ and $R^4$ may form, together with the adjacent nitrogen atom, a group represented by the formula

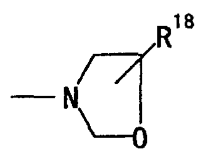

wherein $R^{18}$ is halogen atom, oxo group, optionally halogenated $C_{1-6}$ alkyl group or optionally halogenated $C_{1-6}$ alkoxy group,

[8] the antagonist of [1] wherein $R^1$ is (i) $C_{1-6}$ alkyl group optionally having 5 or 6-membered nitrogen-containing heterocyclic group, (ii) $C_{7-16}$ aralkyl group optionally having nitro, amino or $C_{1-6}$ alkoxy-carbonyl or (iii) cyclohexyl group fused with benzene ring optionally having $C_{1-6}$ alkoxy,
$R^2$ is (i) hydrogen atom, (ii) $C_{1-6}$ alkyl group or (iii) $C_{7-16}$ aralkyl group, or $R^1$ and $R^2$ may form, together with the adjacent nitrogen atom, a nitrogen-containing heterocyclic group represented by

(i) the formula

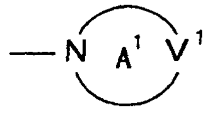

wherein ring $A^1$ is a 4 to 8-membered ring optionally substituted by hydroxy or oxo, $V^1$ is a group represented by the formula >O, $>C-(W^1)W^{a1}$ or $>N-W^1$ ($W^1$ is (a) hydrogen atom, (b) $C_{6-14}$ aryl group optionally having 1 or 2 substituent(s) selected from the group consisting of halogen atom, optionally halogenated $C_{1-6}$ alkyl group

and $C_{1-6}$ alkoxy group or (c) $C_{1-6}$ alkyl group optionally having 1 or 2 $C_{6-10}$ aryl group(s), $W^{a1}$ is hydrogen atom or hydroxy group),
(ii) the formula

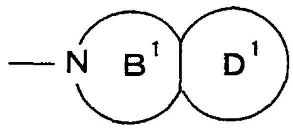

wherein ring $B^1$ is a monocyclic or bicyclic 5 to 10-membered ring optionally substituted by oxo group or 1 or 2 $C_{1-6}$ alkyl group(s), ring $D^1$ is a benzene ring optionally having 1 or 2 substituent(s) selected from the group consisting of $C_{1-6}$ alkyl group, $C_{1-6}$ alkoxy group and $C_{1-6}$ alkyl-carbonyl group or
(iii) the formula

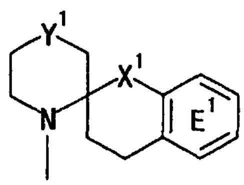

wherein ring $E^1$ is a benzene ring optionally having 1 or 2 substituent(s) selected from the group consisting of $C_{1-3}$ alkylenedioxy group, nitro group, $C_{1-6}$ alkoxy group, amino group, $C_{1-6}$ alkyl-carbonylamino group and $C_{1-6}$ alkoxy-carbonyl group

$X^1$ is $-CH_2-$ or -CO-,
$Y^1$ is $-CH_2-$ or -O-,

$R^3$ is (i) hydrogen atom, (ii) a group represented by the formula $-CO-R^5$ ($R^5$ is (a) hydrogen atom, (b) carboxyl group, (c) $C_{1-6}$ alkyl group, (d) $C_{5-7}$ cycloalkyl group optionally having alkoxy, and which is fused with benzene ring or (e) 5 or 6-membered aromatic heterocyclic group containing, besides carbon atom, 1 to 3 heteroatom(s) selected from the group consisting of nitrogen atom, oxygen atom and sulfur atom),

(iii) a group represented by the formula $-CO-(CH_2)r^1-R^6$ ($r^1$ is an integer of 1 to 3, $R^6$ is (a) $C_{6-14}$ aryl group optionally having 1 or 2 substituent(s) selected from the group consisting of halogen atom, optionally halogenated $C_{1-6}$ alkyl, nitro, $C_{1-6}$ alkoxy and $C_{1-3}$ alkylenedioxy, (b) $C_{6-14}$ aryloxy group, (c) 5 or 6-membered aromatic heterocyclic group containing, besides carbon atom, 1 or 2 heteroatom(s) selected from nitrogen atom, oxygen atom and sulfur atom, (d) $C_{1-6}$ alkyl-carbonyl group, (e) carboxyl group, (f) $C_{1-6}$ alkoxy-carbonyl group, (g) amino group optionally having 1 or 2 substituent(s) selected from the group consisting of $C_{1-6}$ alkyl and $C_{1-6}$ alkyl-carbonyl or (h) 5 or 6-membered cyclic amino group optionally having hydroxy),
(iv) a group represented by the formula

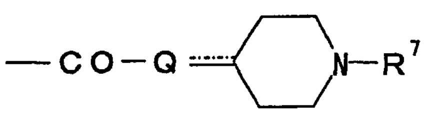

(Q is a group represented by the formula $-(CH_2)s-$ (s is an integer of 1 to 3) or $-(CH_2)t-CH=$ (t is an integer of 0 to 2),
$R^7$ is hydrogen atom or $C_{1-6}$ alkoxy-carbonyl group),
(v) a group represented by the formula

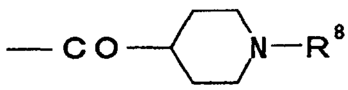

($R^8$ is (a) hydrogen atom, (b) $C_{1-6}$ alkyl group optionally having substituents selected from the group consisting of $C_{1-6}$ alkoxy-carbonyl, morpholino and mono- or di-$C_{1-6}$ alkylamino, (c) $C_{1-6}$ alkoxy-carbonyl group, (d) a group represented by the formula -CO-$R^d$ ($R^d$ is $C_{6-14}$ aryl group optionally having halogen atom or 5 or 6-membered heterocyclic group containing, besides carbon atom, 1 or 2 heteroatom(s) selected from nitrogen atom, oxygen atom and sulfur atom),
(e) a group represented by the formula -CO-$(CH_2)r^1$-$R^e$ ($r^1$ is an integer of 1 to 3, $R^e$ is $C_{1-6}$ alkoxy-carbonyl group or 5 or 6-membered heterocyclic group containing, besides carbon atom, 1 or 2 heteroatom(s) selected from nitrogen atom, oxygen atom and sulfur atom) or (f) a group represented by -CONH-$R^f$ ($R^f$ is $C_{1-6}$ alkyl group or $C_{6-14}$ aryl group)),
(vi) a group represented by the formula -$COOR^9$ ($R^9$ is optionally halogenated $C_{1-6}$ alkyl group),
(vii) a group represented by the formula

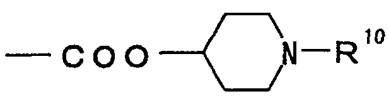

wherein $R^{10}$ is hydrogen atom, $C_{1-6}$ alkoxy-carbonyl group, mono or di-$C_{1-6}$ alkyl-carbamoyl group, optionally halogenated nicotinoyl group or optionally halogenated isonicotinoyl group,
(viii) a group represented by the formula -$CONR^{11}$-$R^{12}$ ($R^{11}$ is hydrogen atom or $C_{1-6}$ alkyl group, $R^{12}$ is $C_{1-6}$ alkyl optionally having substituents selected from the group consisting of (a) hydroxy, (b) amino, (c) a mono- or di-$C_{1-6}$ alkyl-amino, (d) $C_{1-6}$ alkyl-carbonyl, (e) $C_{1-6}$ alkoxy-carbonyl, (f) $C_{1-6}$ alkyl-carbonyloxy, (g) sulfamoyl and (f) 5 or 6-membered cyclic amine optionally substituted by oxo),
(ix) a group represented by the formula

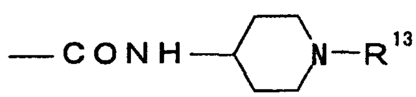

wherein $R^{13}$ is (a) a hydrogen atom, (b) $C_{1-6}$ alkyl group optionally having substituents selected from the group consisting of a hydroxy and $C_{1-6}$ alkoxy-carbonyl, (c) $C_{7-16}$ aralkyl group, (d) $C_{1-6}$ alkyl-carbonyl group optionally having substituents selected from the group consisting of a halogen and $C_{1-6}$ alkoxy-carbonyl or (e) $C_{1-6}$ alkyl-carbamoyl group optionally having $C_{1-6}$ alkoxy-carbonyl,
(x) a group represented by the formula

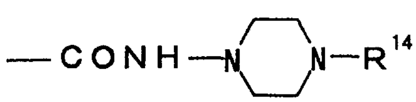

wherein $R^{14}$ is $C_{1-6}$ alkyl group or $C_{7-16}$ aralkyl group,
(xi) a group represented by the formula

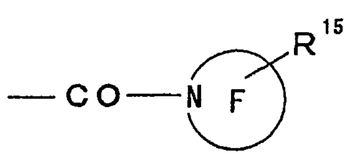

wherein ring F is 5 to 7-membered cyclic amino group optionally fused with benzene ring, $R^{15}$ is hydrogen

atom, $C_{1-6}$ alkoxy-carbonylamino group or optionally halogenated $C_{1-6}$ alkyl-carbonylamino group,
(xii) a group represented by the formula

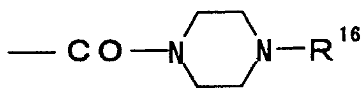

wherein $R^{16}$ is (a) $C_{1-6}$ alkyl group optionally having substituents selected from the group consisting of a hydroxy and $C_{1-6}$ alkoxy-carbonyl, (b) a formyl group, (c) $C_{1-6}$ alkoxy-carbonyl group or (d) a 5 or 6-membered heterocyclic ring-carbonyl group containing, besides carbon atom, 1 to 3 heteroatom(s) selected from the group consisting of nitrogen atom, oxygen atom and sulfur atom,
(xiii) a group represented by the formula $-SO_2-R^{17}$ ($R^{17}$ is (i) $C_{1-6}$ alkyl group optionally having 5 or 6-membered nitrogen-containing ring group, (ii) $C_{2-6}$ alkenyl group or (iii) $C_{6-14}$ aryl group optionally having $C_{1-6}$ alkyl),
(xiv) $C_{7-16}$ aralkyl group optionally having 1 to 3 halogen atom(s), or
(xv) $C_{1-6}$ alkyl group substituted by 5 or 6-membered heterocyclic group containing, besides carbon atom, 1 to 3 heteroatom(s) selected from the group consisting of nitrogen atom, oxygen atom and sulfur atom,

$R^4$ is hydrogen atom or $C_{1-6}$ alkyl group,
or $R^3$ and $R^4$ may form, together with the adjacent nitrogen atom, a group of the formula

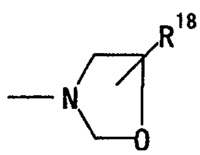

wherein $R^{18}$ is halogen atom, oxo group, optionally halogenated $C_{1-6}$ alkyl group or optionally halogenated $C_{1-6}$ alkoxy group,
[9] the antagonist of [1] wherein $R^1$ and $R^2$ form, together with the adjacent nitrogen atom, a nitrogen-containing heterocyclic group represented by

(i) the formula

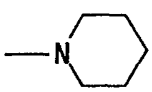 , 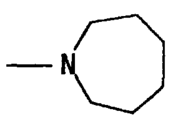 , 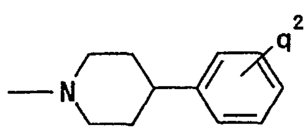 , 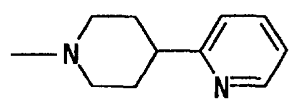 ,

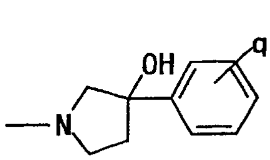 , 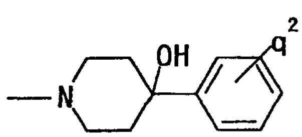 , 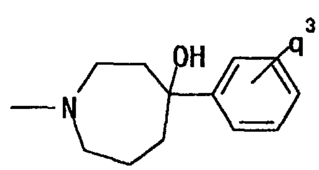 ,

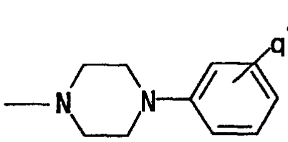 , 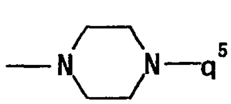 or 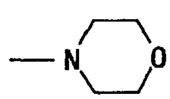

wherein $q^1$ is hydrogen atom or halogen atom, $q^2$ is hydrogen atom, halogen atom, optionally halogenated $C_{1-6}$ alkyl group or $C_{1-6}$ alkoxy group, $q^3$ is hydrogen atom or halogen atom, $q^4$ is hydrogen atom, halogen atom or $C_{1-6}$ alkoxy group, $q^5$ is hydrogen atom or $C_{1-6}$ alkyl group optionally having 1 or 2 $C_{6-10}$ aryl group(s), (ii) the formula

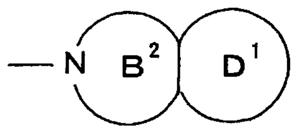

wherein ring $B^2$ is represented by the formula

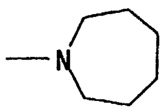
,
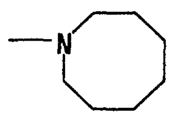
,
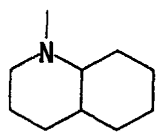
or
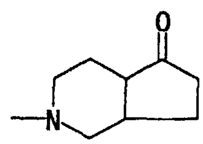

wherein ring $D^1$ is benzene ring optionally having 1 or 2 substituent(s) selected from the group consisting of $C_{1-6}$ alkyl group, $C_{1-6}$ alkoxy group and $C_{1-6}$ alkyl-carbonyl group or
(iii) the formula

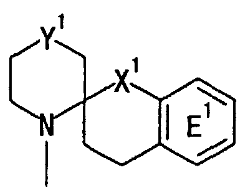

wherein ring $E^1$ is benzene ring optionally having 1 or 2 substituent(s) selected from the group consisting of $C_{1-3}$ alkylenedioxy group, nitro group, $C_{1-6}$ alkoxy group, amino group, $C_{1-6}$ alkyl-carbonylamino group and $C_{1-6}$ alkoxy-carbonyl group, $X^1$ is $-CH_2-$ or -CO-, and $Y^1$ is $-CH_2-$ or -O-),

[10] the antagonist of [1] wherein the compound is represented by the formula

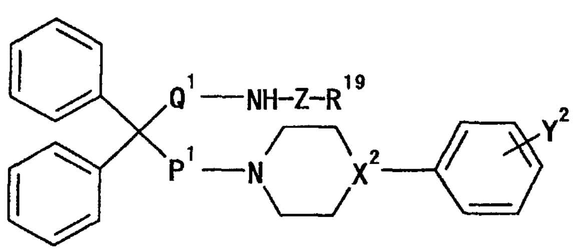

wherein $R^{19}$ is (i) hydrogen atom, (ii) carboxyl, (iii) $C_{1-6}$ alkoxy-carbonyl group, (iv) $C_{1-6}$ alkyl group optionally having substituents selected from the group consisting of carboxyl, $C_{1-6}$ alkyl-carbonyl, $C_{1-6}$ alkoxy-carbonyl, $C_{1-6}$ alkoxy-carbonylamino and $C_{7-16}$ aralkyloxy-carbonylamino, (v) a mono- or di-$C_{1-6}$ alkylamino group or (iv) $C_{6-14}$ aryloxy group; $P^1$ is $C_{1-3}$ alkylene group; $Q^1$ is $C_{1-3}$ alkylene group; $X^2$ is CH, C-OH or N; $Y^2$ is hydrogen atom, halogen atom, optionally halogenated $C_{1-6}$ alkyl group or $C_{1-6}$ alkoxy group; and Z is CO, SO or $SO_2$,
[11] the antagonist of [1] wherein the compound is represented by the formula

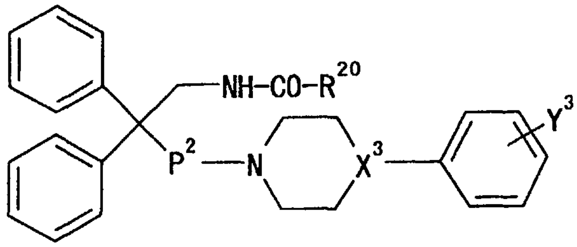

wherein $R^{20}$ is (i) hydrogen atom or (ii) $C_{1-6}$ alkyl group optionally having substituents selected from the group consisting of $C_{1-6}$ alkoxy-carbonylamino and $C_{7-16}$ aralkyloxy-carbonylamino; $P^2$ is $C_{1-3}$ alkylene group; $X^3$ is CH, C-OH or N; $Y^3$ is hydrogen atom, halogen atom or $C_{1-6}$ alkoxy group,
[12] the antagonist of [1] wherein the compound is represented by the formula

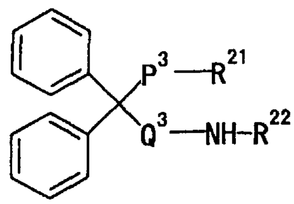

wherein $R^{21}$ is a nitrogen-containing heterocyclic group represented by (i) the formula

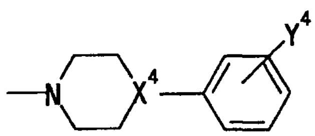

wherein $X^4$ is CH or N, $Y^4$ is hydrogen atom, halogen atom or $C_{1-6}$ alkoxy group or (ii) the formula

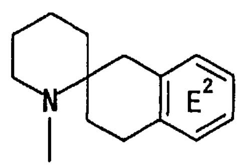

wherein ring $E^2$ is benzene ring optionally having 1 to 3 $C_{1-6}$ alkoxy,
$R^{22}$ is (i) hydrogen atom, (ii) $C_{7-16}$ aralkyl group, (iii) formyl group, (iv) $C_{1-6}$ alkyl-carbonyl group, (v) $C_{6-14}$ aryl-carbonyl group optionally having $C_{1-6}$ alkyl or (vi) $C_{6-14}$ aryl-sulfonyl group optionally having 1 to 4 $C_{1-6}$ alkyl; $P^3$ is $C_{1-3}$ alkylene group; and $Q^3$ is $C_{1-3}$ alkylene group,
[13] the antagonist of [1] wherein the compound is

1- (5-amino-4,4-diphenylpentyl)-4-phenylpiperidine, 3,4-dihydro-6-methoxy-1'-(5-amino-4,4-diphenylpentyl) spiro[naphthalene-2(1H),2'-piperidine] or a salt thereof,
1-[5-amino-4-(4-methoxyphenyl)-4-phenylpentyl]-4-phenylpiperidine or a salt thereof,
1-[5-amino-4,4-bis(4-chlorophenyl)pentyl]-4-(4-fluorophenyl)piperazine or a salt thereof,
3,4-dihydro-6-methoxy-1'-(6-amino-4,4-diphenylhexyl)spiro[naphthalene-2(1H),2'-piperidine] or a salt thereof,
3,4-dihydro-6,7-dimethoxy-1'-(7-amino-4,4-diphenylheptyl)spiro[naphthalene-2(1H),2'-piperidine] or a salt thereof,

4,4-diphenyl-5-formylamino-1-(4-phenylpiperidino)pentane or a salt thereof (e.g., hydrochloride),
1-[4-(4-fluorophenyl)piperazin-l-yl]-5-formylamino-4,4-diphenylpentane or a salt thereof (e.g., dihydrochloride),
4,4-diphenyl-1-(4-phenylpiperazin-1-yl)-5-(tosylamino)pentane or a salt thereof,
4,4-diphenyl-1-[4-(2-methoxyphenyl)piperazin-1-yl]-5-(tosylamino)pentane or a salt thereof (e.g., hydrochloride),
4-(4-chlorophenyl)-5-formylamino-4-phenyl-1-(4-phenylpiperidino)pentane or a salt thereof (e.g., hydrochloride),
4-(4-chlorophenyl)-5-formylamino-4-phenyl-1-(4-phenylpiperazin-1-yl)pentane or a salt thereof (e.g., dihydrochloride),
4-(4-chlorophenyl)-1-[4-(4-fluorophenyl)piperazin-1-yl]-5-formylamino-4-phenylpentane or a salt thereof (e.g., dihydrochloride),
4-(4-chlorophenyl)-1-[4-(diphenylmethyl)piperazin-1-yl]-5-formylamino-4-phenylpentane or a salt thereof,
5-formylamino-4-(4-methoxyphenyl)-4-phenyl-1- (4-phenylpiperidino)pentane or a salt thereof (e.g., hydrochloride),
4,4-bis(4-chlorophenyl)-1- [4-(4-fluorophenyl)piperazin-1-yl]-5-(formylamino)pentane or a salt thereof (e.g., dihydrochloride),
1-[4-(4-fluorophenyl)piperazin-1-yl]-6-formylamino-5,5-diphenylhexane or a salt thereof (e.g., dihydrochloride),
1-[4-(4-fluorophenyl)piperazin-1-yl]-6-formylamino-4,4-diphenylhexane or a salt thereof (e.g., dihydrochloride),
4,4-diphenyl-1-(4-phenylpiperidino]-6-(tosylamino)hexane or a salt thereof (e.g., hydrochloride),
5-[4-(4-chlorophenyl)-4-hydroxypiperidino]-1-formylamino-2,2-diphenylpentane or a salt thereof (e.g., hydrochloride),
5-[4-(4-fluorophenyl)piperazin-1-yl]-1-formylamino-2,2-diphenylpentane or a salt thereof (e.g., dihydrochloride),
1-formylamino-5-(4-hydroxy-4-phenylpiperidino)-2,2-diphenylpentane or a salt thereof (e.g., hydrochloride),
5-[4-(4-trifluoromethylphenyl) -4-hydroxypiperidinol -1-formylamino-2,2-diphenylpentane or a salt thereof (e.g., hydrochloride),
5-[4-[3,5-bis (trifluoromethyl) phenyl]-4-hydroxypiperidino]-1-formylamino-2,2-diphenylpentane or a salt thereof (e.g., hydrochloride),
5-[4-(3,5-dichlorophenyl)-4-hydroxypiperidino]-1-formylamino-2,2-diphenylpentane or a salt thereof (e.g., hydrochloride),
5-[4-(4-chlorophenyl)-1,2,3,6-tetrahydropyridin-1-yl]-1-formylamino-2,2-diphenylpentane or a salt thereof (e.g., hydrochloride),
l-formylamino-2,2-diphenyl-5-(4-phenylpiperidino)pentane or a salt thereof,
5-[4-(4-chlorophenyl)piperidino]-1-formylamino-2,2-diphenylpentane or a salt thereof (e.g., hydrochloride),
7-[4-(4-chlorophenyl)-4-hydroxypiperidino]-1-formylamino-4,4-diphenylheptane or a salt thereof (e.g., hydrochloride),
5-[4-(4-fluorophenyl)-4-hydroxypiperidino]-1-formylamino-2,2-diphenylpentane or a salt thereof (e.g., hydrochloride),
1-formylamino-5-[4-hydroxy-4-(4-methoxyphenyl)piperidino]-2,2-diphenylpentane or a salt thereof (e.g., hydrochloride),
1-formylamino-5-[4-hydroxy-4-(2-pyridyl)piperidino]-2,2-diphenylpentane or a salt thereof (e.g., dihydrochloride),
1-acetylamino-5-[4-(4-chlorophenyl)-4-hydroxypiperidino]-2,2-diphenylpentane or a salt thereof (e.g., hydrochloride),
1-acetoacetylamino-5-[4-(4-chlorophenyl)-4-hydroxypiperidino]-2,2-diphenylpentane or a salt thereof (e.g., hydrochloride),
ethyl N-[5-[4-(4-chlorophenyl)-4-hydroxypiperidino]-2,2-diphenylpentyl]succinamate or a salt thereof (e.g., hydrochloride),
N-[5-[4-(4-chlorophenyl)-4-hydroxypiperidino]-2,2-diphenylpentyl]succinamic acid or a salt thereof,
1-[5-[4-(4-chlorophenyl)-4-hydroxypiperidino]-2,2-diphenylpentyl]-3-ethylurea or a salt thereof,
N-[5-[4-(4-chlorophenyl)-4-hydroxypiperidino]-2,2-diphenylpentyl]methanesulfonamide or a salt thereof (e.g., hydrochloride),
phenyl N- [5- [4-(4-chlorophenyl)-4-hydroxypiperidino]-2,2-diphenylpentyl]carbamate or a salt thereof,
1-acetylamino-5- [4-(4-chlorophenyl)-4-hydroxypiperidino]-2-phenyl-2-(2-pyridyl)pentane or a salt thereof (e.g., dihydrochloride),

ethyl N-[5-[4-(4-chlorophenyl)-4-hydroxypiperidino]-2,2-diphenylpentyl]oxamate or a salt thereof (e.g., hydrochloride),
ethyl N-[5-[4-(4-chlorophenyl)-4-hydroxypiperidino]-2,2-diphenylpentyl]malonamate or a salt thereof (e.g., hydrochloride),
ethyl N-[5-[4-(4-chlorophenyl)-4-hydroxypiperidino]-2,2-diphenylpentyl]glutaramate or a salt thereof,
benzyl 2-((2,2-diphenyl-5-(4-phenylpiperidino)pentyl)amino)-2-oxoethylcarbamate or a salt thereof (e.g., hydrochloride),
tert-butyl 2-((2,2-diphenyl-5-(4-phenylpiperidino)pentyl)amino)-2-oxoethylcarbamate or a salt thereof,
4,4-diphenyl-7-(4-phenylpiperidino)heptylamine or a salt thereof (e.g., dihydrochloride),
N-(4,4-diphenyl-7-(4-phenylpiperidino)heptyl)-4-methylbenzenesulfonamide or a salt thereof (e.g., hydrochloride),
N-(4,4-diphenyl-7-(4-phenylpiperidino)heptyl)acetamide or a salt thereof (e.g., hydrochloride),
N-benzyl-N-(4,4-diphenyl-7-(4-phenylpiperidino)heptyl)amine or a salt thereof (e.g., dihydrochloride),
N- (4,4-diphenyl-7-(4-phenylpiperidino)heptyl) -N- (3-methoxybenzyl) amine or a salt thereof (e.g., dihydrochloride),
N-(4,4-diphenyl-7-(4-phenylpiperidino)heptyl)-N-(2-methoxybenzyl)amine or a salt thereof (e.g., dihydrochloride),
N-(4,4-diphenyl-7-(4-phenylpiperidino)heptyl)-N-(2-fluorobenzyl)amine or a salt thereof (e.g., dihydrochloride),
N-(4,4-diphenyl-7-(4-phenylpiperidino)heptyl)-2-thiophenecarboxamide or a salt thereof (e.g., hydrochloride),
N-(4,4-diphenyl-7-(4-phenylpiperidino)heptyl)-2-phenylacetamide or a salt thereof (e.g., hydrochloride),
N-(4,4-diphenyl-7-(4-phenylpiperidino)heptyl)-N-(2-thienylmethyl)amine or a salt thereof (e.g., dihydrochloride), or
N-benzyl-N-(4,4-diphenyl-7-(4-phenylpiperidino)heptyl)-N-methylamine or a salt thereof (e.g., dihydrochloride),

[14] the antagonist of [1] which is an agent for the prophylaxis or therapy of a disease caused by melanin-concentrating hormone,
[15] the antagonist of [1] which is an agent for the prophylaxis or therapy of obesity,
[16] the antagonist of [1] which is an agent for suppressing food intake,
[17] a compound represented by the formula

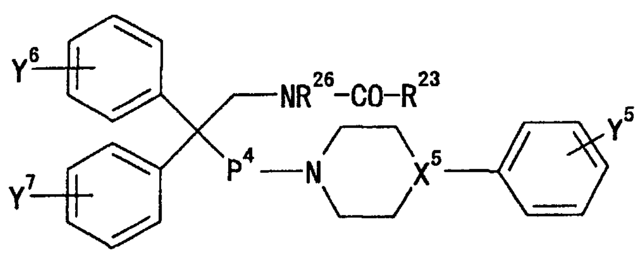

(Ia)

wherein $R^{23}$ is $C_{1-6}$ alkyl group having $C_{7-16}$ aralkyloxy-carbonylamino optionally having substituents selected from the group consisting of halogen atom, $C_{1-6}$ alkoxy and $C_{1-6}$ alkyl; $P^4$ is $C_{1-3}$ alkylene group; $X^5$ is CH, C-OH or N; $Y^5$ is hydrogen atom, halogen atom or $C_{1-6}$ alkoxy group; $R^{26}$ is hydrogen atom or $C_{1-6}$ alkyl group; $Y^6$ and $Y^7$ are the same or different and each is hydrogen atom, halogen atom, optionally halogenated $C_{1-6}$ alkyl group or optionally halogenated $C_{1-6}$ alkoxy group, or a salt thereof or a prodrug thereof,
[18] the compound of [17] wherein $R^{26}$ is hydrogen atom,
[19] benzyl 2-((2,2-diphenyl-5-(4-phenylpiperidino)pentyl)-amino)-2-oxoethylcarbamate (Example 1) or a salt thereof,

4-chlorobenzyl 2-((2,2-diphenyl-5-(4-phenylpiperidino)pentyl)-amino)-2-oxoethylcarbamate (Example 57) or a salt thereof,
3-chlorobenzyl 2-((2,2-diphenyl-5-(4-phenylpiperidino)pentyl)-amino)-2-oxoethylcarbamate (Example 58) or a salt thereof,
benzyl 2-(N-(2,2-diphenyl-5-(4-phenylpiperidino)pentyl)-N-methylamino)-2-oxoethylcarbamate (Example 75) or a salt thereof,
benzyl 2-((5- (4-(3-fluorophenyl)piperidino)-2,2-diphenylpentyl)amino)-2-oxoethylcarbamate (Example 76) or

a salt thereof,
benzyl 2-((5-(4-(2-fluorophenyl)piperidino)-2,2-diphenylpentyl)amino)-2-oxoethylcarbamate (Example 77) or a salt thereof,
benzyl 2-((5-(4-(2-methoxyphenyl)piperidino)-2,2-diphenylpentyl) amino)-2-oxoethylcarbamate(Example 80) or a salt thereof, or
3-chlorobenzyl 2-((2,2-bis(4-chlorophenyl)-5-(4-phenylpiperidino)pentyl)amino)-2-oxoethylcarbamate (Example 103) or a salt thereof,

[20] a production method of a compound of [17], which comprises reacting a compound represented by the formula

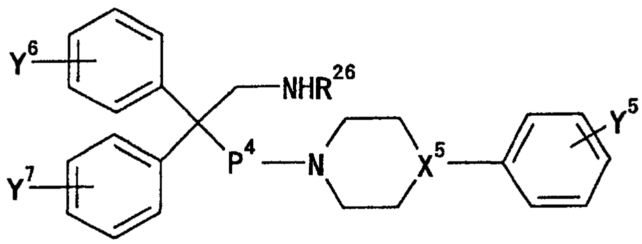

wherein each symbol is as defined in [17] or a salt thereof with a reactive derivative of an organic acid of the formula

$$R^{23}\text{-COOH}$$

wherein $R^{23}$ is as defined in [17],
[21] a production method of a compound of [17], which comprises reacting a compound represented by the formula

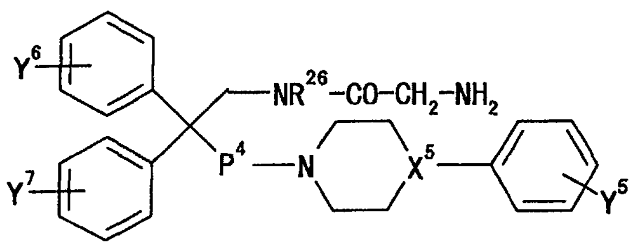

wherein each symbol is as defined in [17] or a salt thereof with a reactive derivative of the formula

$$R^{32}\text{-X}$$

wherein $R^{32}$ is $C_{7-16}$ aralkyloxy-carbonyl group, and X is a leaving group,
[22] a pharmaceutical composition containing a compound of [17],
[23] a compound represented by the formula

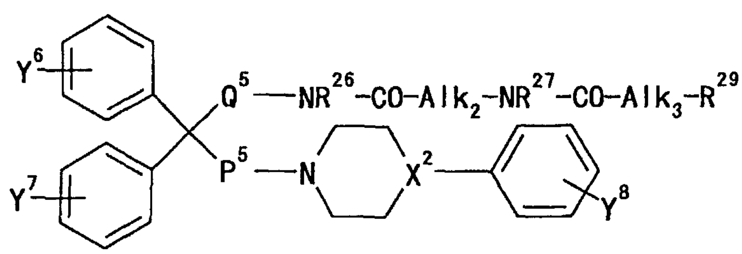

(Ib)

wherein $R^{26}$ and $R^{27}$ are the same or different and each is hydrogen atom or $C_{1-6}$ alkyl group; $Alk_2$ and $Alk_3$ are

the same or different and each is a bond or $C_{1-6}$ alkylene group optionally having substituents; $R^{29}$ is (1) $C_{6-10}$ aryl group optionally having substituents or (2) 5 to 10-membered aromatic heterocyclic group optionally having substituents, which contains, besides carbon atom, 1 to 3 heteroatom(s) selected from the group consisting of nitrogen atom, oxygen atom and sulfur atom; $X^2$ is CH, C-OH or N; $P^5$ and $Q^5$ are the same or different and each is $C_{1-6}$ alkylene group; $Y^6$, $Y^7$ and $Y^8$ are the same or different and each is hydrogen atom, halogen atom, optionally halogenated $C_{1-6}$ alkyl group or optionally halogenated $C_{1-6}$ alkoxy group, or a salt thereof or a prodrug thereof,
[24] the compound of [23], wherein $Alk_2$ and $Alk_3$ are the same or different and each is a bond, or $C_{1-6}$ alkylene group optionally having substituents selected from the group consisting of halogen atom, hydroxy, amino and $C_{6-10}$ aryl; $R^{29}$ is (1) $C_{6-10}$ aryl group or (2) 5 to 10-membered aromatic heterocyclic group containing, besides carbon atom, 1 to 3 heteroatom(s) selected from the group consisting of nitrogen atom, oxygen atom and sulfur atom, which optionally has substituents selected from the group consisting of nitro, halogen atom, $C_{1-6}$ alkyl, hydroxy, $C_{1-6}$ alkoxy and $C_{6-10}$ aryl,
[25] the compound of [23] or [24] , wherein $R^{29}$ is indol-2-yl optionally having substituents,
[26] the compound of [23] or [24], wherein $R^{29}$ is indol-2-yl optionally having substituents selected from halogen atom, $C_{1-6}$ alkyl, $C_{1-6}$ alkoxy and hydroxy,
[27] N-(2-((2,2-diphenyl-5-(4-phenylpiperidino)pentyl)amino)-2-oxoethyl)indole-2-carboxamide (Example 44) or a salt thereof,

N-(2-((2,2-diphenyl-5-(4-phenylpiperidino)pentyl)amino)-2-oxoethyl)-1-methylindole-2-carboxamide (Example 45) or a salt thereof,
5-chloro-N-(2-((2,2-diphenyl-5-(4-phenylpiperidino)pentyl)-amino)-2-oxoethyl)indole-2-carboxamide (Example 47) or a salt thereof,
N-(2-((2,2-bis(4-chlorophenyl)-5-(4-phenylpiperidino)pentyl)-amino)-2-oxoethyl)indole-2-carboxamide (Example 104) or a salt thereof,
N-(2-((2,2-bis(4-chlorophenyl)-5-(4-phenylpiperidino)pentyl)-amino)-2-oxoethyl)-5-chloroindole-2-carboxamide (Example 105) or a salt thereof,
N-(2-((2,2-bis(4-chlorophenyl)-5-(4-phenylpiperidino)pentyl)-amino)-2-oxoethyl)-1-methylindole-2-carboxamide (Example 106) or a salt thereof,
N-(2-((2,2-bis(4-chlorophenyl)-5-(4-phenylpiperidino)pentyl)-amino)-2-oxoethyl)-5-fluoroindole-2-carboxamide (Example 107) or a salt thereof,
N-(2-((2,2-bis(4-chlorophenyl)-5-(4-phenylpiperidino)pentyl)-amino)-2-oxoethyl)-5-methoxyindole-2-carboxamide (Example 108) or a salt thereof,
N-(2-((2,2-bis(4-chlorophenyl)-5-(4-phenylpiperidino)pentyl)-amino)-2-oxoethyl)-5-hydroxyindole-2-carboxamide (Example 109) or a salt thereof,
N-(2-((5-(4-(2-fluorophenyl)piperidino)-2,2-diphenylpentyl)-amino)-2-oxoethyl)indole-2-carboxamide (Example 115) or a salt thereof,
N-(2-((5-(4-(2-fluorophenyl)piperidino)-2,2-diphenylpentyl)-amino)-2-oxoethyl)-1-methylindole-2-carboxamide (Example 116) or a salt thereof,
5-chloro-N-(2-((5-(4-(2-fluorophenyl)piperidino)-2,2-diphenylpentyl)amino)-2-oxoethyl)-1-methylindole-2-carboxamide (Example 117) or a salt thereof,
5-chloro-N-(2-((5-(4-(2-fluorophenyl)piperidino)-2,2-diphenylpentyl)amino)-2-oxoethyl)indole-2-carboxamide (Example 118) or a salt thereof,
N-(2-((2,2-bis(4-chlorophenyl)-5(4-(2-fluorophenyl)-piperidino)pentyl)amino)-2-oxoethyl)-5-fluoroindole-2-carboxamide (Example 120) or a salt thereof,
N-(2-((2,2-bis(4-chlorophenyl)-5-(4-(2-fluorophenyl)-piperidino)pentyl)amino)-2-oxoethyl)-5-methoxyindole-2-carboxamide (Example 121) or a salt thereof,
N-(2-((2,2-bis(4-chlorophenyl)-5-(4-(2-fluorophenyl)-piperidino)pentyl)amino)-2-oxoethyl)indole-2-carboxamide (Example 122) or a salt thereof,
N-(2-((2,2-bis(4-fluorophenyl)-5-(4-phenylpiperidino)pentyl)-amino)-2-oxoethyl)indole-2-carboxamide (Example 124) or a salt thereof,
N-(2-((2,2-bis(4-fluorophenyl)-5-(4-phenylpiperidino)pentyl)-amino)-2-oxoethyl)-5-chloroindole-2-carboxamide (Example 125) or a salt thereof,
N-(2-((2,2-bis(4-fluorophenyl)-5-(4-(2-methoxyphenyl)-piperidino)pentyl)amino)-2-oxoethyl)indole-2-carboxamide (Example 127) or a salt thereof,
N-(2-((2,2-bis(4-fluorophenyl)-5-(4-(2-methoxyphenyl)-piperidino)pentyl)amino)-2-oxoethyl)-5-chloroindole-2-carboxamide (Example 128) or a salt thereof,
N-(2-((2,2-bis (4-fluorophenyl)-5-(4-(2-fluorophenyl)-piperidino)pentyl)amino)-2-oxoethyl)indole-2-carboxamide (Example 130) or a salt thereof, or

N-(2-((2,2-bis(4-fluorophenyl)-5-(4-(2-fluorophenyl)-piperidino)pentyl)amino)-2-oxoethyl)-5-chloroindole-2-carboxamide (Example 131) or a salt thereof,

[28] a production method of a compound of [23], which comprises reacting a compound represented by the formula

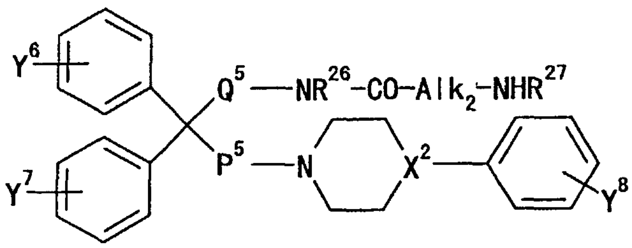

wherein each symbol is as defined in [23] or a salt thereof with a reactive derivative of an organic acid of the formula

$$R^{29}\text{-Alk}_3\text{-COOH}$$

wherein each symbol is as defined in [23],
[29] a pharmaceutical composition containing the compound of [23],
[30] a compound represented by the formula

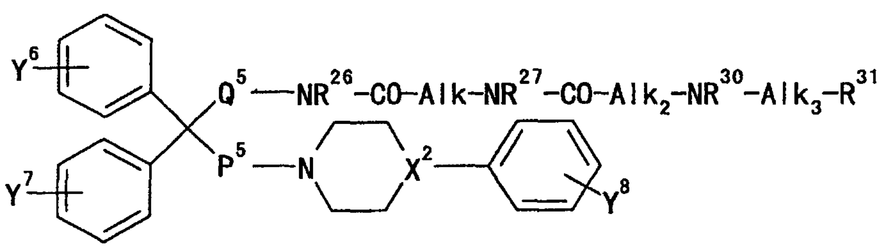

(Ic)

wherein $R^{26}$ and $R^{27}$ are the same or different and each is hydrogen atom or $C_{1-6}$ alkyl group; $R^{30}$ is hydrogen atom, $C_{1-6}$ alkyl group or optionally halogenated $C_{1-6}$ alkyl-carbonyl group; Alk is $C_{1-6}$ alkylene group optionally having substituents; $Alk_2$ and $Alk_3$ are the same or different and each is a bond or $C_{1-6}$ alkylene group optionally having substituents; $R^{31}$ is $C_{6-10}$ aryl group optionally having substituents; $X^2$ is CH, C-OH or N; $P^5$ and $Q^5$ are the same or different and each is $C_{1-6}$ alkylene group; $Y^6$, $Y^7$ and $Y^8$ are the same or different and each is hydrogen atom, halogen atom, optionally halogenated $C_{1-6}$ alkyl group or optionally halogenated $C_{1-6}$ alkoxy group, or a salt thereof or a prodrug thereof,
[31] the compound of [30] wherein Alk is $C_{1-6}$ alkylene group optionally having substituents selected from the group consisting of halogen atom, hydroxy, amino and $C_{6-10}$ aryl; $Alk_2$ and $Alk_3$ are the same or different and each is a bond or $C_{1-6}$ alkylene group optionally having substituents selected from the group consisting of halogen atom, hydroxy, amino and $C_{6-10}$ aryl; $R^{31}$ is $C_{6-10}$ aryl group optionally having substituents selected from the group consisting of halogen atom, $C_{1-6}$ alkyl, hydroxy, $C_{1-6}$ alkoxy and $C_{6-10}$ aryl,
[32] N-(2-((2-((2,2-diphenyl-5-(4-phenylpiperidino)pentyl)-amino)-2-oxoethyl)amino)-2-oxoethyl)-2,2,2-trifluoro-N-phenylacetamide (Example 51) or a salt thereof,
2-anilino-N-(2-((2,2-diphenyl-5-(4-phenylpiperidino)pentyl)-amino)-2-oxoethyl)acetamide (Example 59) or a salt thereof, or 2-(((benzylamino)carbonyl) amino)-N-(2,2-diphenyl-5-(4-phenylpiperidino)pentyl)acetamide (Example 65) or a salt thereof,
[33] a production method of a compound of [30], which comprises reacting a compound represented by the formula

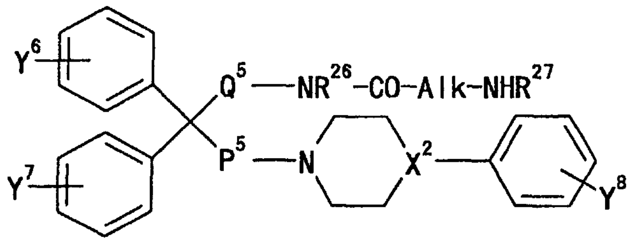

wherein each symbol is as defined in [30], or a salt thereof, with,

(1) when $Alk_2$ is $C_{1-6}$ alkylene group optionally having substituents, a reactive derivative of an organic acid compound of the formula

$$R^{31}\text{-}Alk_3\text{-}NR^{30}\text{-}Alk_2\text{-}COOH$$

wherein each symbol is as defined in [30],
(2) when $Alk_2$ is a bond, a reactive derivative of the formula

$$R^{31}\text{-}Alk_3\text{-}NR^{30}\text{-}CO\text{-}X$$

or

$$R^{31}\text{-}Alk_3\text{-}NCO$$

wherein X is leaving group, and other symbols are as defined in [30],

[34] a pharmaceutical composition containing a compound of [30],
[35] a method for antagonizing melanin-concentrating hormone, comprising administering, to a mammal, an effective amount of a compound of the formula

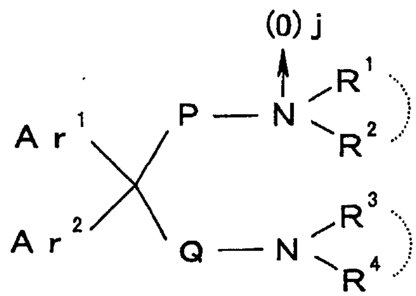

wherein

$Ar^1$ and $Ar^2$ are each an aromatic group optionally having substituents,
P and Q are each a divalent aliphatic hydrocarbon group which optionally contains ether oxygen or ether sulfur in a carbon chain and which optionally has substituents, ,
$R^1$ and $R^3$ are each (i) hydrogen atom, (ii) acyl group or (iii) a hydrocarbon group optionally having substituents,
$R^2$ and $R^4$ are each (i) hydrogen atom, (ii) an alkyl group optionally having substituents or (iii) an alkylcarbonyl group optionally having substituents, $R^1$ and $R^2$ or $R^3$ and $R^4$ optionally form, together with the adjacent nitrogen atom, a monocyclic or fused nitrogen-containing heterocyclic group optionally having substituents, and
j is 0 or 1, or a salt thereof or a prodrug thereof,

[36] use of a compound of the formula

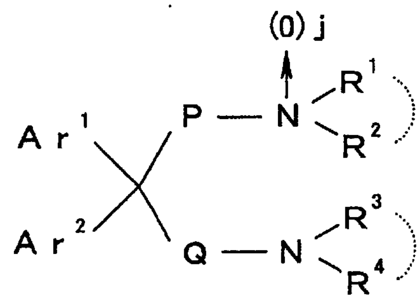

wherein

$Ar^1$ and $Ar^2$ are each an aromatic group optionally having substituents,
P and Q are each a divalent aliphatic hydrocarbon group which optionally contains ether oxygen or ether sulfur in a carbon chain and which optionally has substituents, ,
$R^1$ and $R^3$ are each (i) hydrogen atom, (ii) acyl group or (iii) hydrocarbon group optionally having substituents,
$R^2$ and $R^4$ are each (i) hydrogen atom, (ii) an alkyl group optionally having substituents or (iii) an alkylcarbonyl group optionally having substituents, $R^1$ and $R^2$ or $R^3$ and $R^4$ optionally form, together with the adjacent nitrogen atom, a monocyclic or fused nitrogen-containing heterocyclic group optionally having substituents, and
j is 0 or 1, or a salt thereof or a prodrug thereof, for production of a melanin-concentrating hormone antagonist and

[37] a compound represented by the formula

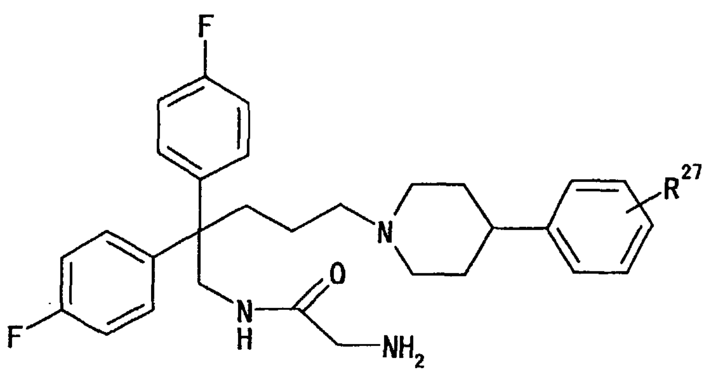

wherein $R^{27}$ is hydrogen atom, halogen atom, optionally halogenated $C_{1-6}$ alkyl group or optionally halogenated $C_{1-6}$ alkoxy group, or a salt thereof.

**Best Mode for Embodying the Invention**

As the "aromatic group" represented by $Ar^1$ and $Ar^2$, for example, aromatic hydrocarbon group, aromatic heterocyclic group and the like are used, and aromatic hydrocarbon group is particularly preferable.
As the "aromatic hydrocarbon group", for example, monocyclic or fused polycyclic aromatic hydrocarbon group having 6 to 14 carbon atoms and the like are used, which are specifically exemplified by $C_{6-14}$ aryl group such as phenyl, 1-naphthyl, 2-naphthyl, indenyl, anthryl and the like, and the like, wherein particularly phenyl is generally used.
As the "aromatic heterocyclic group", for example, 5 to 14-membered monocyclic or fused (e.g., bicylic, tricyclic) aromatic heterocyclic group containing, besides carbon atom, at least one (e.g., 1 to 4, preferably 1 to 3, more preferably 1 or 2) preferably 1 or 2 kinds of heteroatoms selected from nitrogen atom, sulfur atom and oxygen atom, and the like are used. Specific examples thereof include monovalent groups obtained by removing an optional hydrogen atom from aromatic heterocyclic rings, such as thiophene, benzo[b]thiophene, benzo[b]furan, benzimidazole, benzoxazole, benzothiazole, benzisothiazole, naphth[2,3-b]thiophene, thianthrene, furan, isoindolizidine, xanthrene, phenoxathiin, pyrrole, imidazole, pyrazole, pyridine, pyrazine, pyrimidine, pyridazine, indole, isoindole, 1H-indazole, purine, 4H-quinolizidine, isoquinoline, quinoline, phthalazine, naphthyridine, quinoxaline, quinazolin, cinnoline, carbazole, β-carboline, phenanthridine, acridine, phenazine, isothiazole, phenothiazine, isooxazole, furazan, phenoxazine, isochroman and the like, or a fused ring formed by fusing these rings (preferably the aforementioned monocyclic heterocyclic ring) with one or more (preferably 1 or 2, more preferably 1) aromatic ring (e.g., the above-mentioned aromatic hydrocarbon group and the like, preferably benzene ring and the like) and the like. Of these, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo [b] thienyl, benzo [b] furanyl, 2-thienyl, 3-thienyl and the like are mentioned. More preferably, 5 to 10-membered (monocyclic or bicyclic) aromatic heterocyclic group containing, besides carbon atom, 1 to 3 heteroatoms selected from nitrogen atom, oxygen atom and sulfur atom, such as 2-thienyl, 3-thienyl, 2-pyridyl, 3-pyridyl, 4-pyridyl, 2-furyl, 3-furyl, 2-quinolyl, pyrazinyl, 2-pyrimidinyl, 3-pyrrolyl, 2-imidazolyl, 3-pyridazinyl, 3-isothiazolyl, 3-isooxazolyl, 1-isoquinolyl, 1-indolyl, 2-indolyl, 2-benzothiazolyl and the like, and the like are used. Of these, 5 or 6-membered aromatic heterocyclic group containing, besides carbon atom, 1 to 3 (preferably 1) heteroatoms selected from nitrogen atom, oxygen atom and sulfur atom, such as 2-thienyl, 3-thienyl, 2-pyridyl, 4-pyridyl and the like, and the like are generally used.

As the substituent that the "aromatic group" represented by $Ar^1$ and $Ar^2$ optionally has, for example, halogen atom (e.g., fluorine, chlorine, bromine, iodine and the like); $C_{1-3}$ alkylenedioxy group (e.g., methylenedioxy, ethylenedioxy and the like); nitro group; cyano group; optionally halogenated $C_{1-6}$ alkyl group; optionally halogenated $C_{3-6}$ cycloalkyl group; optionally halogenated $C_{1-6}$ alkoxy group; optionally halogenated $C_{1-6}$ alkylthio group; hydroxy group; amino group; mono-$C_{1-6}$ alkylamino group (e.g., methylamino, ethylamino and the like); di-$C_{1-6}$ alkylamino group (e.g., dimethylamino, diethylamino and the like); optionally halogenated $C_{1-6}$ alkyl-carbonylamino group (e.g., acetylamino, propionylamino, butyrylamino and the like); formyl group; $C_{1-6}$ alkyl-carbonyl group (e.g., acetyl, propionyl, butyryl and the like) optionally substituted by halogen atom or $C_{1-6}$ alkoxy-carbonyl group (e.g., methoxycarbonyl, ethoxycarbonyl and the like); $C_{1-6}$ alkyl-carbonyloxy group (e.g., acetyloxy, propionyloxy, butyryloxy and the like); carboxyl group; $C_{1-6}$ alkoxy-carbonyl group (e.g., methoxycarbonyl, ethoxycarbonyl, propoxycarbonyl, butoxycarbonyl and the like); carbamoyl group; mono-$C_{1-6}$ alkyl-carbamoyl group (e.g., methylcarbamoyl, ethylcarbamoyl and the like) optionally substituted by $C_{1-6}$ alkoxy-carbonyl group; di-$C_{1-6}$ alkyl-carbamoyl group (e.g., dimethylcarbamoyl, diethylcarbamoyl and the like) optionally substituted by $C_{1-6}$ alkoxy-carbonyl group; sulfo group; $C_{1-6}$ alkylsulfonyl group (e.g., methylsulfonyl, ethylsulfonyl and the like); $C_{1-6}$ alkylsulfinyl group (e.g., methylsulfinyl, ethylsulfinyl and the like) ; $C_{6-10}$ aryl group (e.g., phenyl, naphthalene and the like); $C_{6-10}$ aryloxy group (e.g., phenyloxy, naphthyloxy and the like); optionally halogenated $C_{6-10}$ aryl-carbonyl group (e.g., benzoyl, naphthoyl and the like); optionally halogenated 5 or 6-membered heterocyclic ring-carbonyl group [preferably 5 or 6-membered heterocyclic ring containing, besides carbon atom, 1 or 2 heteroatom(s) selected from nitrogen atom, oxygen atom and sulfur atom-carbonyl group (e.g., nicotinoyl, isonicotinoyl, morpholinocarbonyl and the like)]; $C_{1-6}$ alkoxy-carbonylamino group (e.g., methoxycarbonylamino, ethoxycarbonylamino and the like); $C_{6-10}$ aryl-carbonylamino group (e.g., benzoylamino and the like); $C_{7-16}$ aralkyloxy-carbonyl group (e.g., benzyloxycarbonyl and the like) and the like are used.

The aforementioned $C_{6-10}$ aryl group and $C_{6-10}$ aryloxy group optionally have 1 to 4 substituents selected from halogen atom, $C_{1-3}$ alkylenedioxy, nitro, cyano, optionally halogenated $C_{1-6}$ alkyl, optionally halogenated $C_{3-6}$ cycloalkyl, optionally halogenated $C_{1-6}$ alkoxy, optionally halogenated $C_{1-6}$ alkylthio, hydroxy, amino, mono-$C_{1-6}$ alkylamino, di-$C_{1-6}$ alkylamino, $C_{1-6}$ alkyl-carbonylamino, formyl, $C_{1-6}$ alkyl-carbonyl, $C_{1-6}$ alkyl-carbonyloxy, carboxyl, $C_{1-6}$ alkoxy-carbonyl, carbamoyl, mono-$C_{1-6}$ alkyl-carbamoyl, di-$C_{1-6}$ alkyl-carbamoyl, sulfo, $C_{1-6}$ alkylsulfonyl, $C_{1-6}$ alkylsulfinyl and the like.

The "aromatic group" represented by $Ar^1$ and $Ar^2$ may have, for example, 1 to 5, preferably 1 to 3, suitable substituent(s) selected from the above-mentioned substituents at substitutable position(s) on the ring. When the number of the substituent is two or more, these substituents may be the same or different.

As the "optionally halogenated $C_{1-6}$ alkyl group" used in the present specification, for example, $C_{1-6}$ alkyl group optionally having 1 to 5 halogen atom(s) (e.g., fluorine, chlorine, bromine, iodine and the like), such as methyl, chloromethyl, difluoromethyl, trichloromethyl, trifluoromethyl, ethyl, 2-bromomethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like, and the like are used.

As the "optionally halogenated $C_{3-6}$ cycloalkyl group" used in the present specification, for example, $C_{3-6}$ cycloalkyl group optionally having 1 to 4 halogen atom(s) (e.g., fluorine, chlorine, bromine, iodine and the like), such as cyclopropyl, cyclobutyl, cyclopentyl, cyclohexyl, 2,2,3,3-tetrafluorocyclopentyl, 4-chlorocyclohexyl and the like, and the like are used.

As the "optionally halogenated $C_{1-6}$ alkoxy group" used in the present specification, for example, $C_{1-6}$ alkoxy group optionally having 1 to 3 halogen atom(s) (e.g., fluorine, chlorine, bromine, iodine and the like), such as methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy and the like, and the like are used.

As the "optionally halogenated $C_{1-6}$ alkylthio group" used in the present specification, for example, $C_{1-6}$ alkylthio group optionally having 1 to 3 halogen atom(s) (e.g., fluorine, chlorine, bromine, iodine and the like), such as methylthio, difluoromethylthio, trifluoromethylthio, ethylthio, propylthio, isopropylthio, butylthio, 4,4,4-trifluorobutylthio, pentylthio, hexylthio and the like, and the like are used.

As the "hydrocarbon group" represented by $R^1$ and $R^3$, for example, alkyl group, alkenyl group, alkynyl group, cycloalkyl group, aryl group, aralkyl group and the like are used. Specifically, for example, the following chain, branched or cyclic hydrocarbon group having 1 to 16 carbon atoms, and the like are preferable.

a) $C_{1-6}$ alkyl group (e.g., methyl, ethyl, propyl, isopropyl, butyl, isobutyl, sec-butyl, tert-butyl, pentyl, hexyl and the like),
b) $C_{2-6}$ alkenyl group (e.g., vinyl, allyl, isopropenyl, butenyl, isobutenyl, sec-butenyl and the like),
c) $C_{2-6}$ alkynyl group (e.g., propargyl, ethynyl, butynyl, 1-hexyl and the like),
d) $C_{3-6}$ cycloalkyl group (e.g., cyclopropyl, cyclobutyl, cyclopentyl, cyclohexyl and the like), this $C_{3-6}$ cycloalkyl group may be fused with benzene ring optionally having 1 to 3 $C_{1-6}$ alkoxy group(s) (e.g., methoxy and the like),
e) $C_{6-14}$ aryl group (e.g., phenyl, tolyl, xylyl, 1-naphthyl, 2-naphthyl, biphenylyl, 2-indenyl, 2-anthryl and the like), particularly phenyl group,
f) $C_{7-16}$ aralkyl group (e.g., benzyl, phenethyl, diphenylmethyl, triphenylmethyl, 1-naphthylmethyl, 2-naphthylmethyl, 2-diphenylethyl, 3-phenylpropyl, 4-phenylbutyl, 5-phenylpentyl and the like), particularly benzyl group.

As the substituent that the "hydrocarbon group" represented by $R^1$ and $R^3$ optionally has, for example, halogen atom (e.g., fluorine, chlorine, bromine, iodine and the like), $C_{1-3}$ alkylenedioxy group (e.g., methylenedioxy, ethylenedioxy and the like), nitro group, cyano group, optionally halogenated $C_{1-6}$ alkyl group, optionally halogenated $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group (e.g., methylamino, ethylamino and the like), di-$C_{1-6}$ alkylamino group (e.g., dimethylamino, diethylamino and the like), $C_{1-6}$ alkyl-carbonylamino group (e.g., acetylamino, propionylamino, butyrylamino and the like), formyl group, $C_{1-6}$ alkyl-carbonyl group (e.g., acetyl, propionyl, butyryl and the like), $C_{1-6}$ alkyl-carbonyloxy group (e.g., acetyloxy, propionyloxy, butyryloxy and the like), carboxyl group, $C_{1-6}$ alkoxy-carbonyl group (e.g., methoxycarbonyl, ethoxycarbonyl, propoxycarbonyl, butoxycarbonyl and the like), carbamoyl group, mono-$C_{1-6}$ alkyl-carbamoyl group (e.g., methylcarbamoyl, ethylcarbamoyl and the like), di-$C_{1-6}$ alkyl-carbamoyl group (e.g., dimethylcarbamoyl, diethylcarbamoyl and the like), sulfo group, $C_{1-6}$ alkylsulfonyl group (e.g., methylsulfonyl, ethylsulfonyl and the like), $C_{1-6}$ alkylsulfinyl group (e.g., methylsulfinyl, ethylsulfinyl and the like), $C_{6-10}$ aryl group (e.g., phenyl, naphthyl and the like), $C_{6-10}$ aryloxy group (e.g., phenyloxy, naphthyloxy and the like), 5 to 7-membered heterocyclic ring [e.g., 5 to 7-membered heterocyclic ring containing, besides carbon atom, 1 to 3 heteroatom(s) selected from the group consisting of nitrogen atom, oxygen atom and sulfur atom (e.g., 1-, 2- or 3-pyrrolidinyl, 2- or 4-imidazolidinyl, 2-, 3- or 4-pyrazolidinyl, 1-,2-,3- or 4-piperidyl, 1- or 2-piperazinyl, morpholinyl, 2-thienyl, 3-thienyl, 2-pyridyl, 4-pyridyl, 2-furyl, 3-furyl, pyrazinyl, 2-pyrimidinyl, 3-pyrrolyl, 2-imidazolyl, 3-pyridazinyl, 3-isothiazolyl, 3-isooxazolyl and the like) or fused ring group thereof (e.g., fused ring group with benzene ring and the like)], di-$C_{1-6}$ alkyl-carbonylamino group, sulfamoyl group, $C_{1-6}$ alkoxy-carbonylamino group (e.g., methoxycarbonylamino, ethoxycarbonylamino, propoxycarbonylamino, butoxycarbonylamino, tert-butoxycarbonylamino and the like), $C_{7-16}$ aralkyloxy-carbonylamino group (e.g., benzyloxycarbonylamino and the like), $C_{7-16}$ aralkyloxy group (e.g., benzyloxy and the like), $C_{6-10}$ aryl-carbonyl group (e.g., benzoyl and the like), $C_{1-6}$ alkyl-carbonyloxy group (e.g., acetoxy and the like), $C_{6-10}$ aryl-carbonylamino group (e.g., benzoylamino and the like), $C_{6-10}$ aryl-carbamoyl group (e.g., phenylcarbamoyl and the like) and the like are used.

The above-mentioned $C_{6-10}$ aryl group, $C_{6-10}$ aryloxy group and 5 to 7-membered heterocyclic ring may have 1 to 4 substituents selected from halogen atom, $C_{1-3}$ alkylenedioxy, nitro, cyano, optionally halogenated $C_{1-6}$ alkyl, optionally halogenated $C_{3-6}$ cycloalkyl, optionally halogenated $C_{1-6}$ alkoxy, optionally halogenated $C_{1-6}$ alkylthio, hydroxy, amino, mono-$C_{1-6}$ alkylamino, di-$C_{1-6}$ alkylamino, $C_{1-6}$ alkyl-carbonylamino, formyl, $C_{1-6}$ alkyl-carbonyl, $C_{1-6}$ alkyl-carbonyloxy, carboxyl, $C_{1-6}$ alkoxy-carbonyl, carbamoyl, mono-$C_{1-6}$ alkyl-carbamoyl, di-$C_{1-6}$ alkyl-carbamoyl, sulfo, $C_{1-6}$ alkylsulfonyl, $C_{1-6}$ alkylsulfinyl and the like.

The "hydrocarbon group" represented by $R^1$ and $R^3$ may have, for example, 1 to 5, preferably 1 to 3, suitable substituent(s) selected from the above-mentioned substituents at substitutable position(s) of the hydrocarbon group. When the number of the substituent is two or more, these substituents may be the same or different.

As the "acyl group" represented by $R^1$ and $R^3$, for example, a group represented by $-CO-R^a$, $-CONR^aR^b$, $-SO-R^a$, $-SO_2-R^a$, $-CONR^aR^b$, $-COO-R^a$, $-(C=S)O-R^a$, $-(C=S)NR^aR^b$, $-SONR^aR^b$, $-SO_2NR^aR^b$, $-SO-O-R^a$, $-SO_2-O-R^a$ ($R^a$ is hydrogen atom, carboxyl group, hydrocarbon group optionally having substituents, heterocyclic group optionally having substituents or $C_{1-6}$ alkoxy-carbonyl group, and $R^b$ is hydrogen atom or $C_{1-6}$ alkyl group) and the like is used. Particularly, $-CO-R^a$, $-CONH-R^a$ and the like are preferable.

As the "acyl group" represented by $R^3$,

(xvi) a group represented by the formula

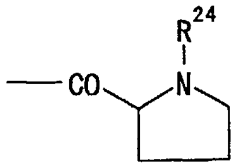

[$R^{24}$ is hydrogen atom or $C_{7-16}$ aralkyloxy-carbonyl group (e.g., benzyloxycarbonyl)];
(xvii) a group represented by the formula

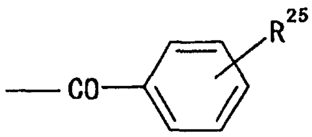

[$R^{25}$ is hydrogen atom, $C_{6-10}$ aryl group (e.g., phenyl), $C_{7-16}$ aralkyloxy group (e.g., benzyloxy), $C_{6-10}$ aryloxy group (e.g., phenoxy), halogen atom (e.g., bromine), $C_{6-10}$ aryl-carbonylamino group (e.g., benzoylamino) or $C_{6-10}$ aryl-carbamoyl group (e.g., phenylcarbamoyl)];
(xviii) a group represented by the formula

$$-CO-Alk-NR^{27}-CO-Alk_2-O-Alk_3-R^{28}$$

[Alk is a $C_{1-6}$ alkylene group optionally having a substituent (e.g., halogen atom, hydroxy group, amino group, $C_{6-10}$ aryl group (e.g., phenyl) and the like) ; $R^{27}$ is hydrogen atom or $C_{1-6}$ alkyl group; $Alk_2$ and $Alk_3$ are the same or different and each is a bond or $C_{1-6}$ alkylene group optionally having substituents (e.g., halogen atom, hydroxy group, amino group, $C_{6-10}$ aryl group (e.g., phenyl) and the like); $R^{28}$ is $C_{6-10}$ aryl group (e.g., phenyl) optionally having substituents (e.g., nitro group, halogen atom (e.g., fluorine, chlorine), $C_{1-6}$ alkyl group (e.g., methyl), hydroxy group, $C_{1-6}$ alkoxy group (e.g., methoxy), $C_{6-10}$ aryl group (e.g., phenyl) and the like) or hydrogen atom]; (xix) a group represented by the formula

$$-CO-Alk_2-NR^{27}-CO-Alk_3-R^{29}$$

[$Alk_2$, $Alk_3$ and $R^{27}$ are as defined above; and $R^{29}$ is (1) $C_{6-10}$ aryl group (e.g., phenyl, naphthyl) or (2) 5 to 10-membered aromatic heterocyclic group containing, besides carbon atom, 1 to 3 heteroatom(s) selected from the group consisting of nitrogen atom, oxygen atom and sulfur atom (e.g., indolyl, benzofuranyl, benzothienyl, pyrrolyl), which optionally has substituents (e.g., nitro group, halogen atom(e.g., fluorine, chlorine), $C_{1-6}$ alkyl group (e.g., methyl), hydroxy group, $C_{1-6}$ alkoxy group (e.g., methoxy), $C_{6-10}$ aryl group (e.g., phenyl) and the like)];
(xx) a group represented by the formula

$$-CO-Alk-NR^{27}-CO-Alk_2-NR^{30}-Alk_3-R^{31}$$

[Alk, $R^{27}$, $Alk_2$, $Alk_3$ are as defined above; $R^{30}$ is hydrogen atom, $C_{1-6}$ alkyl group (e.g., methyl) or optionally halogenated $C_{1-6}$ alkyl-carbonyl group (e.g., trifluoromethylcarbonyl); $R^{31}$ is $C_{6-10}$ aryl group (e.g., phenyl) optionally having substituents (e.g., halogen atom (e.g., fluorine, chlorine), $C_{1-6}$ alkyl group (e.g., methyl), hydroxy group, $C_{1-6}$ alkoxy group, $C_{6-10}$ aryl group (e.g., phenyl) and the like)];
(xxi) a group represented by the formula

$$-CO-Alk-NR^{27}-CO-Alk_2-NR^{32}-CO-O-Alk_3-R^{31}$$

[Alk, $R^{27}$, $Alk_2$, $Alk_3$ and $R^{31}$ are as defined above; and $R^{32}$ is the same as the aforementioned $R^{27}$];
(xxii) a group represented by the formula

$$-CO-Alk-CO-NR^{27}-Alk_2-R^{31}$$

[Alk, $R^{27}$, $Alk_2$ and $R^{31}$ are as defined above];
(xxiii) group represented by the formula

$$-CO-Alk-O-CO-O-Alk_2-R^{31}$$

[Alk, $Alk_2$ and $R^{31}$ are as defined above] and the like are mentioned.

As the "hydrocarbon group optionally having substituents "represented by the aforementioned $R^a$, those similar to the aforementioned "hydrocarbon group optionally having substituents "represented by $R^1$ and $R^3$ are used.

As the "heterocyclic group" represented by $R^a$, for example, 5 to 10-membered (monocyclic or bicyclic) heterocyclic group containing, besides carbon atom, 1 or 2 kinds of, preferably 1 to 4 heteroatom(s) selected from the group consisting of nitrogen atom, oxygen atom and sulfur atom, such as non-aromatic heterocyclic group (e.g., 1-, 2- or 3-pyrrolidinyl, 2- or 4-imidazolidinyl, 2-, 3- or 4-pyrazolidinyl, 1-, 2-, 3- or 4-piperidyl, 1- or 2-piperazinyl, morpholinyl and the like), aromatic heterocyclic group (e.g., 2-thienyl, 3-thienyl, 2-pyridyl, 4-pyridyl, 2-furyl, 3-furyl, 4-quinolyl, 8-quinolyl, 4-isoquinolyl, pyrazinyl, 2-pyrimidinyl, 3-pyrrolyl, 2-imidazolyl, 3-pyridazinyl, 3-isothiazolyl, 3-isooxazolyl, 1-indolyl, 2-isoindolyl and the like) and the like are used. Of these, non-aromatic heterocyclic group such as 1-, 2-, 3- or 4-piperidyl, 1- or 2-piperazinyl and the like are preferable, particularly, 1- or 4-piperidyl, 1-piperazinyl and the like are preferable.

As the substituent that the "heterocyclic group" optionally has, for example, (i) the substituent that the aforementioned "aromatic group" represented by $Ar^1$ and $Ar^2$ optionally have, (ii) the aforementioned "hydrocarbon group optionally having substituents" represented by $R^1$ and $R^3$, (iii) oxo group, (iv) thioxo group and the like are used.

As the "$C_{1-6}$ alkyl group" represented by $R^b$, for example, linear or branched $C_{1-6}$ alkyl group such as methyl, ethyl, propyl, isopropyl, butyl, isobutyl, sec-butyl, tert-butyl, pentyl, hexyl and the like are used.

As the "alkyl group" represented by $R^2$ and $R^4$, for example, linear or branched alkyl group having 1 to 6 carbon atoms (e.g., methyl, ethyl, propyl, isopropyl, butyl, isobutyl, sec-butyl, tert-butyl, pentyl, hexyl and the like) and the like are used.

As the substituent that the "alkyl group" optionally has, for example, those similar to the substituents that the aforementioned "hydrocarbon group" represented by $R^1$ and $R^3$ optionally have and the like are used.

As the "alkylcarbonyl group" represented by $R^2$ and $R^4$, for example, lower alkylcarbonyl group and the like are used, which is concretely exemplified by $C_{1-6}$ alkylcarbonyl group such as formyl, methylcarbonyl, ethylcarbonyl, propylcarbonyl, butylcarbonyl and the like, and the like.

As the substituent of the "alkylcarbonyl group", for example, those similar to the substituent that the aforementioned "hydrocarbon group" represented by $R^1$ and $R^3$ optionally has and the like are used.

As the $C_{1-6}$ alkylene group represented by Alk, $Alk_2$ and $Alk_3$, for example those exemplified for P or Q to be mentioned later are mentioned.

As the "divalent aliphatic hydrocarbon group" of the "divalent aliphatic hydrocarbon group which optionally contains ether oxygen or ether sulfur in a carbon chain" represented by P and Q, for example, a divalent group obtained by respectively removing one hydrogen atom bonded to the same or different carbon atom of saturated or unsaturated aliphatic hydrocarbon and the like are exemplified, with preference given to those having not more than 6 carbon atoms. Examples thereof include

(i) alkylene group (e,g., $-(CH_2)_2-$, $-(CH_2)_3-$, $-(CH_2)_4-$, $-CH_2-CH(CH_3)-CH_2-$, $-(CH_2)_5-$, $-CH_2-CH(CH_3)-CH_2-CH_2-$, $-(CH_2)_6-$ and the like),
(ii) alkenylene group (e.g., $-CH=CH-$, $-CH=C(CH_3)-$, $-CH_2-CH=CH-$, $-CH_2-CH=CH-CH_2-$ and the like),
(iii) alkynylene group (e.g., $-C=C-$, $-CH_2-C=C-$, $-CH_2-C=C-CH_2-$ and the like) and the like. Preferably, $C_{1-6}$ alkylene group (e.g., methylene, ethylene, propylene, trimethylene, tetramethylene, pentamethylene and the like), $C_{2-6}$ alkenylene group (e.g., vinylene, propenylene and the like), $C_{2-6}$ alkynylene group (e.g., ethynylene, propynylene and the like) and the like are generally used. More preferably, $C_{2-6}$ alkylene group is used.

The "divalent aliphatic hydrocarbon group" may contain ether oxygen or ether sulfur in a carbon chain, which is optionally substituted by oxo group or thioxo group.

To be specific, for example, $-CH_2-O-CH_2-$, $-CH_2-CH_2-O-CH_2-$, $-CH_2-O-CH_2-O-CH_2-$, $-CH_2-S-CH_2-$, $-CH_2-CH_2-$

S-$CH_2$-, -$CH_2$-S-$CH_2$-S-$CH_2$- and the like are used.

As the "monocyclic or fused nitrogen-containing heterocyclic group" of the "monocyclic or fused nitrogen-containing heterocyclic group optionally having substituents" formed by $R^1$ and $R^2$, and $R^3$ and $R^4$, together with the adjacent nitrogen atom, for example, monocyclic or fused 3 to 9-membered, preferably 5 to 7-membered, nitrogen-containing heterocyclic group optionally containing, besides the nitrogen atom of the bond moiety, 1 or 2 kinds of preferably 1 to 3 heteroatom(s) selected from nitrogen atom, oxygen atom and sulfur atom (e.g., pyrrolidyl, piperidyl, piperazyl and the like) and the like are used.

As the substituent that the "monocyclic or fused nitrogen-containing heterocyclic group" optionally has, for example, those similar to the substituent that the aforementioned $Ar^1$ and $Ar^2$ optionally have and the like are used.

As the "monocyclic or fused nitrogen-containing heterocyclic group optionally having substituents", for example

(i) a group represented by the formula

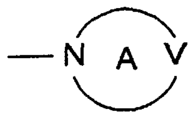

wherein ring A is a 4 to 8-membered ring optionally substituted by 1 or 2 hydroxy group(s) or oxo group(s); V is a group represented by the formula >O, >C=O, >C(W)-$W^a$ or >N-W (W is hydrogen atom, hydrocarbon group optionally having substituents or heterocyclic group optionally having substituents, $W^a$ is hydrogen atom, hydroxy group or $C_{1-6}$ alkyl group),

(ii) a group represented by the formula

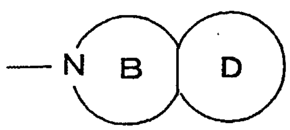

wherein ring B is a monocyclic or bicyclic 4 to 12-membered ring optionally substituted by 1 or 2 oxo group(s) or 1 to 5 $C_{1-6}$ alkyl group(s), ring D is a 4 to 12-membered aromatic ring optionally having substituents, preferably a group represented by the formula

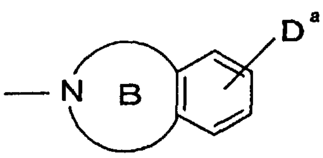

wherein ring B is monocyclic or bicyclic 4 to 12-membered ring optionally substituted by 1 or 2 oxo group(s) or 1 to 5 $C_{1-6}$ alkyl group(s), $D^a$ is a halogen atom (e.g., fluorine, chlorine, bromine, iodine and the like), a $C_{1-6}$ alkyl group (e.g., methyl, ethyl, propyl, isopropyl and the like), a $C_{1-6}$ alkoxy group (e.g., methoxy, ethoxy, propoxy, isopropoxy and the like), a $C_{1-3}$ alkylenedioxy group (e.g., methylenedioxy, ethylenedioxy and the like), a nitro group, an amino group or a $C_{1-6}$ alkyl-carbonyl group (e.g., acetyl, propionyl and the like),

(iii) a group represented by the formula

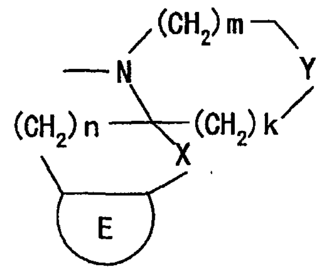

wherein ring E is a 4 to 12-membered aromatic ring optionally having substituents, X is $-CH_2-$, -CO- or -CH(OH)-, Y is $-CH_2-$, -O- or $-NW^b-$ ($W^b$ is hydrogen atom or $C_{1-6}$ alkyl group optionally having substituents), k and m are each an integer of 0 to 4, k+m is an integer of 1 to 4, and n is an integer of 1 to 3, or

(iv) a nitrogen-containing aromatic heterocyclic group optionally having substituents and the like are used. Of the above-mentioned, for example, (i), (ii), (iii) and the like are preferable, (i), (iii) and the like are more preferable, and (iii) is particularly preferable.

As the "4 to 8-membered ring optionally substituted by 1 or 2 hydroxy group(s) or oxo group(s)" represented by A, for example, a group represented by the formula

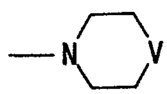 , 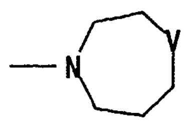

(V is as defined above), preferably the formula

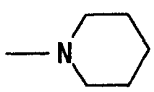 , 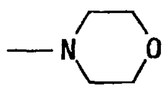 , 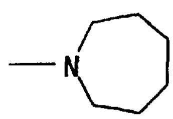 , 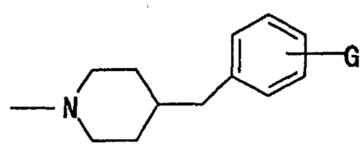 ,

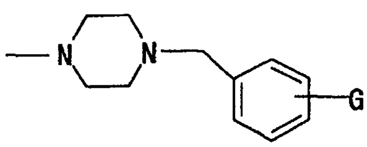 , 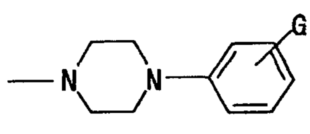 ,

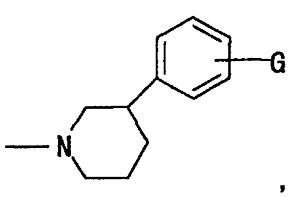 , 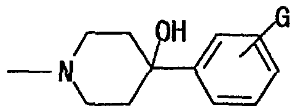 , 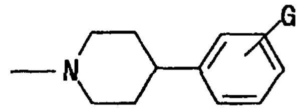

(V is as defined above, G is halogen atom (e.g., fluorine, chlorine and the like), $C_{1-6}$ alkyl group (e.g., methyl, ethyl, propyl, isopropyl and the like), optionally halogenated $C_{1-6}$ alkoxy group (e.g., methoxy, ethoxy, propoxy, isopropoxy, trifluoromethyl and the like), hydrogen atom, cyano group and the like) and the like are generally used.

G is preferably halogen atom such as fluorine, chlorine and the like; $C_{1-6}$ alkyl group such as methyl, ethyl, propyl, isopropyl and the like; $C_{1-6}$ alkoxy group such as methoxy, ethoxy, propoxy, isopropoxy and the like, and the like.

As the "hydrocarbon group optionally having substituents" represented by W, for example, those similar to the "hydrocarbon group optionally having substituents" represented by the aforementioned $R^1$ and $R^3$, and the like are used, and $C_{6-14}$ aryl group (e.g., phenyl and the like), $C_{7-16}$ aralkyl group (e.g., benzyl and the like) and the like are particularly preferable.

As the substituent that this hydrocarbon group optionally has, for example, those similar to the substituents that the aforementioned "hydrocarbon group" represented by $R^1$ and $R^3$ optionally have and the like are used.

As the "heterocyclic group" represented by W, for example, a 5 to 10-membered (monocyclic or bicyclic) heterocyclic group containing, besides carbon atom, 1 or 2 kinds of preferably 1 to 4 heteroatom(s) selected from nitrogen atom, oxygen atom and sulfur atom and the like are used. Specific examples include 1-, 2- or 3-pyrrolidinyl, 2- or 4-imidazolidinyl, 2-, 3- or 4-pyrazolidinyl, 1-, 2-, 3- or 4-piperidyl, 1- or 2-piperazinyl, morpholinyl, 2-thienyl, 3-thienyl, 2-pyridyl, 4-pyridyl, 2-furyl, 3-furyl, 4-quinolyl, 8-quinolyl, 4-isoquinolyl, pyrazinyl, 2-pyrimidinyl, 3-pyrrolyl, 2-imidazolyl, 3-pyridazinyl, 3-isothiazolyl, 3-isooxazolyl, 1-indolyl, 2-isoindolyl and the like, with preference given to aromatic ones. Particularly, for example, a 5 or 6-membered aromatic heterocyclic group containing, besides carbon atom, 1 to 3 heteroatom(s) selected from nitrogen atom, oxygen atom and sulfur atom (e.g., 2-thienyl, 3-thienyl, 2-pyridyl, 4-pyridyl and the like) and the like are preferable.

As the substituent that the "heterocyclic group" optionally has, for example, those similar to the substituents that the aforementioned "an aromatic group optionally having substituents" represented by $Ar^1$ and $Ar^2$ optionally have and the like are used.

As the $C_{1-6}$ alkyl group represented by $W^a$, for example, methyl, ethyl, propyl, isopropyl, butyl, isobutyl, sec-butyl, tert-butyl, pentyl, hexyl and the like are mentioned. $W^a$ is preferably a hydrogen atom or hydroxy group.

As the "monocyclic or bicyclic 4 to 12-membered ring optionally substituted by 1 or 2 oxo group(s) or 1 to 5 $C_{1-6}$ alkyl group(s)" represented by B, for example

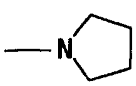 , 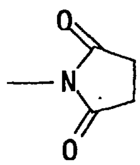 , 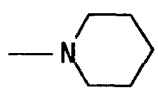 , 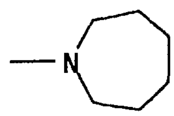 ,

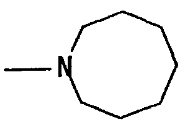 , 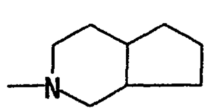 , 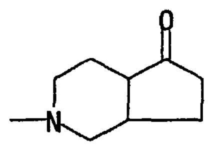 , 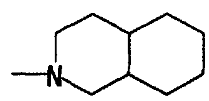

and the like are used.

As the "4 to 12-membered aromatic ring" represented by D and E, for example, benzene ring, naphthalene ring, 4 to 12-membered (preferably 5 to 10-membered) aromatic heterocyclic ring (e.g., a ring containing, besides carbon atom, 1 to 3 heteroatom(s) selected from nitrogen atom, oxygen atom and sulfur atom, which is specifically pyridine, pyrazine, pyrimidine, pyridazine, pyrrole, imidazole, pyrazole, thiophene, furan, thiazole, isothiazole, oxazole, isooxazole, quinoline, isoquinoline, indole, isoindole ring and the like) and the like are used. Particularly,

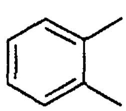 , 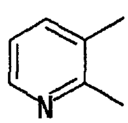 , 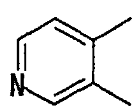 , 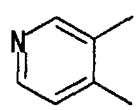 , 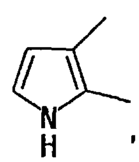 ,

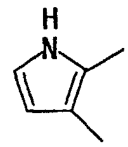 , 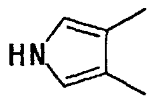 , 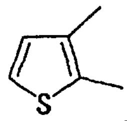 , 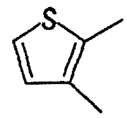 , 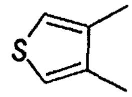 ,

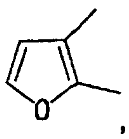 , 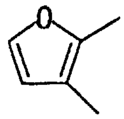 , 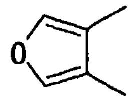

and the like are preferable. As the ring D, for example, benzene ring and pyridine ring are preferable, and particularly, benzene ring is generally used. As the ring E, for example, benzene ring is preferable.

As the substituent that the "4 to 12-membered aromatic ring" optionally has, for example, those similar to the substituents that the aforementioned "aromatic group" represented by $Ar^1$ and $Ar^2$ optionally has and the like are used in similar number.

As the "$C_{1-6}$ alkyl group" represented by $W^b$, for example, methyl, ethyl, propyl, isopropyl, butyl, isobutyl, sec-butyl, tert-butyl, pentyl, hexyl and the like are used.

As the substituent that the "$C_{1-6}$ alkyl group" optionally has, for example, 1 to 3 halogen atom(s), nitro group, cyano group, optionally halogenated $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group, di-$C_{1-6}$ alkylamino group, $C_{1-6}$ alkyl-carbonylamino group (e.g., acetylamino, propionylamino, butyrylamino and the like), carboxyl group, $C_{1-6}$ alkoxy-carbonyl group, carbamoyl group, mono-$C_{1-6}$ alkyl-carbamoyl group, di-$C_{1-6}$ alkyl-carbamoyl group, sulfo group, $C_{1-6}$ alkylsulfonyl group, $C_{1-6}$ alkylsulfinyl group, $C_{6-10}$ aryl group, $C_{6-10}$ aryloxy group and 5 or 6-membered heterocyclic group (e.g., thienyl, furyl, pyridyl and the like) and the like are used.

As the "nitrogen-containing aromatic heterocyclic group", for example, 5 to 10-membered (monocyclic or bicyclic) aromatic heterocyclic group containing one nitrogen atom besides carbon atom, and further, optionally containing 1 or 2 kinds of preferably 1 to 3 heteroatom(s) selected from nitrogen atom, oxygen atom and sulfur atom and the like are used. Specific examples include

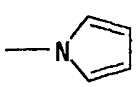 , 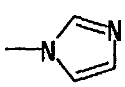 , 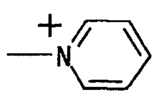 , 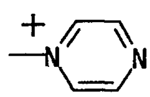

and the like. When a counter ion is necessary, for example, halogen ion (e.g., chlorine ion, bromine ion, iodine ion and the like) and the like are used.

As the substituent that the "nitrogen-containing aromatic heterocyclic group" optionally has, for example, those similar to the substituents that the aforementioned "aromatic group" represented by $Ar^1$ and $Ar^2$ optionally has and the like are used.

Of the aforementioned examples, a monocyclic or fused nitrogen-containing heterocyclic group optionally having substituents formed by $R^1$ and $R^2$ or $R^3$ and $R^4$, together with the adjacent nitrogen atom, for example, (i) a group represented by the formula

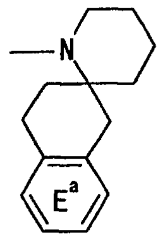

wherein ring $E^a$ is a benzene ring optionally having substituents, which is preferably, for example, benzene ring optionally having 1 to 4 substituents selected from the group consisting of halogen atom, $C_{1-3}$ alkylenedioxy group, nitro group, cyano group, optionally halogenated $C_{1-6}$ alkyl group, optionally halogenated $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group, di-$C_{1-6}$ alkylamino group, $C_{1-6}$ alkyl-carbonylamino group, formyl group, $C_{1-6}$ alkyl-carbonyl group, $C_{1-6}$ alkyl-carbonyloxy group, carboxyl group, $C_{1-6}$ alkoxy-carbonyl group, carbamoyl group, mono-$C_{1-6}$ alkyl-carbamoyl group, di-$C_{1-6}$ alkyl-carbamoyl group, sulfo group, $C_{1-6}$ alkylsulfonyl group, $C_{1-6}$ alkylsulfinyl group, $C_{6-10}$ aryl group and $C_{6-10}$ aryloxy group, and the like),
(ii) a group represented by the formula

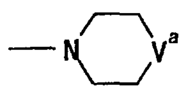

wherein $V^a$ is a group represented by the formula >C(W)-$W^a$ or >N-W (W is (a) hydrogen atom; (b) (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group, each of which optionally has 1 to 5 substituent(s) selected from a halogen atom, $C_{1-3}$ alkylenedioxy group, nitro group, cyano group, optionally halogenated $C_{1-6}$ alkyl group, optionally halogenated $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group, di-$C_{1-6}$ alkylamino group, $C_{1-6}$ alkyl-carbonylamino group, formyl group, $C_{1-6}$ alkyl-carbonyl group, $C_{1-6}$ alkyl-carbonyloxy group, carboxyl group, $C_{1-6}$ alkoxy-carbonyl group, carbamoyl group, mono-$C_{1-6}$ alkyl-carbamoyl group, di-$C_{1-6}$ alkyl-carbamoyl group, sulfo group, $C_{1-6}$ alkylsulfonyl group, $C_{1-6}$ alkylsulfinyl group and 5 to 7-membered heterocyclic ring (e.g., thienyl, furyl, pyridyl and the like); or (c) a 5 to 10-membered heterocyclic group containing, besides carbon atom, 1 to 4 heteroatom(s) selected from the group consisting of nitrogen, oxygen and sulfur, which optionally has 1 to 5 substituent(s) selected from the group consisting of halogen atom, $C_{1-3}$ alkylenedioxy group, nitro group, cyano group, optionally halogenated $C_{1-6}$ alkyl group, optionally halogenated $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group, di-$C_{1-6}$ alkylamino group, $C_{1-6}$ alkyl-carbonylamino group, formyl group, $C_{1-6}$ alkyl-carbonyl group, $C_{1-6}$ alkyl-carbonyloxy group, carboxyl group, $C_{1-6}$ alkoxy-carbonyl group, carbamoyl group, mono-$C_{1-6}$ alkyl-carbamoyl group, di-$C_{1-6}$ alkyl-carbamoyl group, sulfo group, $C_{1-6}$ alkylsulfonyl group, $C_{1-6}$ alkylsulfinyl group, $C_{6-10}$ aryl group and $C_{6-10}$ aryloxy group; $W^a$ is hydrogen atom, hydroxy group or $C_{1-6}$ alkyl group) and the like are preferable.

As W, for example, $C_{6-14}$ aryl group and $C_{7-16}$ aralkyl group, each of which optionally has 1 or 2 substituent(s) selected from the group consisting of halogen atom, cyano group, optionally halogenated $C_{1-6}$ alkyl group, optionally halogenated $C_{1-6}$ alkoxy group and the like, are preferable. Particularly, phenyl group optionally substituted by halogen atom or optionally halogenated $C_{1-6}$ alkoxy group, and the like are preferable.

Of the aforementioned examples, as the aromatic group represented by $Ar^1$ and $Ar^2$, $C_{6-10}$ aryl group (e.g., phenyl group and the like), 5 to 10-membered (monocyclic or bicyclic) aromatic heterocyclic group containing, besides carbon atom, 1 to 3 heteroatom(s) selected from nitrogen atom, oxygen atom and sulfur atom (particularly, thienyl group) and the like are preferable, with particular preference given to $C_{6-10}$ aryl group (e.g., phenyl group and the like).

As $Ar^1$ and $Ar^2$, for example, (i) $C_{6-14}$ aryl group (particularly, phenyl group) or (ii) 5 to 10-membered (monocyclic or bicyclic) aromatic heterocyclic group (particularly thienyl group) containing, besides carbon atom, 1 to 3 heteroatom(s) selected from nitrogen atom, oxygen atom and sulfur atom, which optionally has 1 to 3 substituent(s) selected from the group consisting of halogen atom, methylenedioxy group, nitro group, cyano group, optionally halogenated $C_{1-6}$ alkyl group, $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group, di-$C_{1-6}$ alkylamino group, formyl group, $C_{1-6}$ alkyl-carbonyl group, $C_{1-6}$ alkyl-carbonyloxy group, carboxyl group, $C_{1-6}$ alkoxy-carbonyl group, carbamoyl group, mo-

no-$C_{1-6}$ alkyl-carbamoyl group, di-$C_{1-6}$ alkyl-carbamoyl group, sulfo group, $C_{1-6}$ alkylsulfonyl group, phenyl group and phenyloxy group and the like are preferable. Of these, (i) phenyl group optionally having substituents selected from halogen atom, $C_{1-6}$ alkoxy group and optionally halogenated $C_{1-6}$ alkyl group or (ii) 5 or 6-membered aromatic heterocyclic group (particularly, thienyl group) containing, besides carbon atom, 1 to 3 heteroatom(s) selected from nitrogen atom, oxygen atom and sulfur atom are preferable.

$Ar^1$ and $Ar^2$ are more preferably phenyl group optionally having substituents selected from halogen atom, $C_{1-6}$ alkoxy group and optionally halogenated $C_{1-6}$ alkyl group, particularly preferably phenyl group optionally substituted by halogen atom or $C_{1-6}$ alkoxy group.

As P and Q, $C_{1-6}$ alkylene group or $C_{2-6}$ alkenylene group, which optionally contain ether oxygen or ether sulfur in a carbon chain, and the like are preferable. Of these, $C_{1-6}$ alkylene group or $C_{2-6}$ alkenylene group is preferable and particularly, $C_{1-6}$ alkylene group (e.g., methylene, ethylene, trimethylene, tetramethylene and the like) is generally used.

As P, $C_{3-5}$ alkylene group (e.g., trimethylene, tetramethylene and the like) and the like are preferable, and particularly, trimethylene and tetramethylene are preferable.

As Q, $C_{1-3}$ alkylene group (e.g., methylene, ethylene, trimethylene) and the like are preferable, and particularly, methylene is preferable.

As the acyl group represented by $R^1$, a group represented by -CO-$R^a$ or -CONH-$R^a$ ($R^a$ is as defined above), and the like are preferable.

As the "hydrocarbon group optionally having substituents" represented by $R^1$ and $R^a$, for example, (i) $C_{1-6}$ alkyl group, (ii) $C_{2-6}$ alkenyl group, (iii) $C_{2-6}$ alkynyl group, (iv) $C_{3-6}$ cycloalkyl group optionally fused with benzene ring, (v) $C_{6-14}$ aryl group or (vi) $C_{7-16}$ aralkyl group and the like, each of which optionally has 1 to 3 substituent(s) selected from halogen atom, $C_{1-3}$ alkylenedioxy group, nitro group, cyano group, optionally halogenated $C_{1-6}$ alkyl group, optionally halogenated $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group, di-$C_{1-6}$ alkylamino group, $C_{1-6}$ alkyl-carbonylamino group (e.g., acetylamino, propionylamino, butyrylamino and the like), formyl group, $C_{1-6}$ alkyl-carbonyl group, $C_{1-6}$ alkyl-carbonyloxy group, carboxyl group, $C_{1-6}$ alkoxy-carbonyl group, carbamoyl group, mono-$C_{1-6}$ alkyl-carbamoyl group, di-$C_{1-6}$ alkyl-carbamoyl group, sulfo group, $C_{1-6}$ alkylsulfonyl group, $C_{1-6}$ alkylsulfinyl group, $C_{6-10}$ aryl group, $C_{6-10}$ aryloxy group and 5 to 7-membered heterocyclic ring (e.g., thienyl, furyl, pyridyl and the like) is preferable.

Of the above-mentioned examples, as $R^1$, (i) $C_{1-6}$ alkyl group optionally having 5 or 6-membered nitrogen-containing heterocyclic group (e.g., pyridyl group), (ii) $C_{7-16}$ aralkyl group optionally having nitro, amino or $C_{1-6}$ alkoxy-carbonyl (particularly benzyl group), (iii) cyclohexyl group fused with benzene ring optionally having $C_{1-6}$ alkoxy and the like are preferable.

As the "alkyl group optionally having substituents" represented by $R^2$, for example, $C_{1-6}$ alkyl group (particularly $C_{1-3}$ alkyl group such as methyl and the like) optionally having 1 to 3 substituent(s) selected from the group consisting of halogen atom, nitro group, cyano group, optionally halogenated $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group, di-$C_{1-6}$ alkylamino group, $C_{1-6}$ alkyl-carbonylamino group, formyl group, $C_{1-6}$ alkyl-carbonyl group, $C_{1-6}$ alkyl-carbonyloxy group, carboxyl group, $C_{1-6}$ alkoxy-carbonyl group, carbamoyl group, mono-$C_{1-6}$ alkyl-carbamoyl group, di-$C_{1-6}$ alkyl-carbamoyl group, sulfo group, $C_{1-6}$ alkylsulfonyl group, $C_{1-6}$ alkylsulfinyl group and $C_{6-10}$ aryl group, and the like are preferable.

As the "alkylcarbonyl group optionally having substituents" represented by $R^2$, for example, $C_{1-6}$ alkyl-carbonyl group (e.g., formyl, acetyl and the like) optionally having 1 to 3 substituent(s) selected from the group consisting of halogen atom, nitro group, cyano group, optionally halogenated $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group, di-$C_{1-6}$ alkylamino group, $C_{1-6}$ alkyl-carbonylamino group, formyl group, $C_{1-6}$ alkyl-carbonyl group, $C_{1-6}$ alkyl-carbonyloxy group, carboxyl group, $C_{1-6}$ alkoxy-carbonyl group, carbamoyl group, mono-$C_{1-6}$ alkyl-carbamoyl group, di-$C_{1-6}$ alkyl-carbamoyl group, sulfo group, $C_{1-6}$ alkylsulfonyl group, $C_{1-6}$ alkylsulfinyl group and $C_{6-10}$ aryl group, and the like are preferable.

As $R^2$, (i) hydrogen atom, (ii) $C_{1-6}$ alkyl group (e.g., methyl), (iii) $C_{7-16}$ aralkyl group (e.g., benzyl) and the like are preferable.

As the "monocyclic or fused nitrogen-containing heterocyclic group optionally having substituents" formed by $R^1$ and $R^2$ together with the adjacent nitrogen, for example,

(i) a group represented by the formula

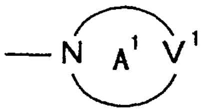

wherein ring $A^1$ is 4 to 8-membered ring optionally substituted by hydroxy or oxo, $V^1$ is a group represented by the formula >O, >C($W^1$)-$W^{a1}$ or >N-$W^1$ ($W^1$ is (a) hydrogen atom, (b) $C_{6-14}$ aryl group optionally having 1 or 2 substituent(s) selected from the group consisting of halogen atom, optionally halogenated $C_{1-6}$ alkyl group and optionally halogenated $C_{1-6}$ alkoxy group, (c) $C_{1-6}$ alkyl group optionally having 1 or 2 $C_{6-10}$ aryl group(s) or (d) pyridyl group, $W^{a1}$ is hydrogen atom, hydroxy group or $C_{1-6}$ alkyl group),
(ii) a group represented by the formula

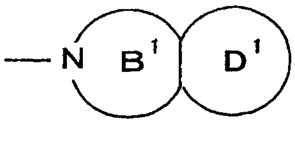

wherein ring $B^1$ is monocyclic or bicyclic 5 to 10-membered ring optionally substituted by oxo group or 1 or 2 $C_{1-6}$ alkyl group(s), ring $D^1$ is benzene ring optionally having 1 or 2 substituent(s) selected from the group consisting of $C_{1-6}$ alkyl group, $C_{1-6}$ alkoxy group and $C_{1-6}$ alkyl-carbonyl group, and
(iii) a group represented by the formula

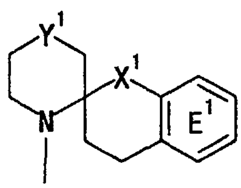

wherein ring $E^1$ is benzene ring optionally having 1 to 3 substituent(s) selected from $C_{1-3}$ alkylenedioxy group, nitro group, $C_{1-6}$ alkoxy group, amino group, $C_{1-6}$ alkyl-carbonylamino group and $C_{1-6}$ alkoxy-carbonyl group, $X^1$ is $-CH_2-$ or -CO-, $Y^1$ is $-CH_2-$ or -O-) and the like are preferable.

As the acyl group represented by $R^3$, a group represented by -CO-$R^a$ or -CONH-$R^a$ ($R^a$ is as defined above) and the like are preferable.

As the "hydrocarbon group optionally having substituents" represented by $R^a$ or $R^3$, for example, (i) $C_{1-6}$ alkyl group, (ii) $C_{2-6}$ alkenyl group, (iii) $C_{2-6}$ alkynyl group, (iv) $C_{3-6}$ cycloalkyl group optionally fused with benzene ring, (v) $C_{6-14}$ aryl group or (vi) $C_{7-16}$ aralkyl group, each optionally having 1 to 3 substituent(s) selected from, for example, halogen atom, $C_{1-3}$ alkylenedioxy group, nitro group, cyano group, optionally halogenated $C_{1-6}$ alkyl group, optionally halogenated $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group, di-$C_{1-6}$ alkylamino group, $C_{1-6}$ alkyl-carbonylamino group (e.g., acetylamino, propionylamino, butyrylamino and the like), formyl group, $C_{1-6}$ alkyl-carbonyl group, $C_{1-6}$ alkyl-carbonyloxy group, carboxyl group, $C_{1-6}$ alkoxy-carbonyl group, carbamoyl group, mono-$C_{1-6}$ alkyl-carbamoyl group, di-$C_{1-6}$ alkyl-carbamoyl group, sulfo group, $C_{1-6}$ alkylsulfonyl group, $C_{1-6}$ alkylsulfinyl group, $C_{6-10}$ aryl group, $C_{6-10}$ aryloxy group and 5 to 7-membered heterocyclic ring (e.g., thienyl, furyl, pyridyl and the like) and the like are preferable.

As the acyl group represented by $R^3$, for example, -CO-$R^a$ ($R^a$ is as defined above) and the like are preferable, and particularly, -CO-$R^c$ ($R^c$ is (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group, each optionally having 1 to 5 substituent(s) selected from the group consisting of halogen atom, $C_{1-3}$ alkylenedioxy group, nitro group, cyano group, optionally halogenated $C_{1-6}$ alkyl group, optionally halogenated $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group, di-$C_{1-6}$ alkylamino group, $C_{1-6}$ alkyl-carbonylamino group, formyl group, $C_{1-6}$ alkyl-carbonyl group, $C_{1-6}$ alkyl-carbonyloxy group, carboxyl group, $C_{1-6}$ alkoxy-carbonyl group, carbamoyl group, mono-$C_{1-6}$ alkyl-carbamoyl group, di-$C_{1-6}$ alkyl-carbamoyl group, sulfo group, $C_{1-6}$ alkylsulfonyl group, $C_{1-6}$ alkylsulfinyl group, $C_{6-10}$ aryl group, $C_{6-10}$ aryloxy group and 5 to 7-membered heterocyclic

ring (e.g., thienyl, furyl, pyridyl and the like) is preferable.

As $R^c$, for example, $C_{7-16}$ aralkyl group optionally having 1 to 3 substituent(s) selected from halogen atom, $C_{1-3}$ alkylenedioxy group, nitro group, cyano group, optionally halogenated $C_{1-6}$ alkyl group, optionally halogenated $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group, di-$C_{1-6}$ alkylamino group, $C_{1-6}$ alkyl-carbonylamino group (e.g., acetylamino, propionylamino, butyrylamino and the like), formyl group, $C_{1-6}$ alkyl-carbonyl group, $C_{1-6}$ alkyl-carbonyloxy group, carboxyl group, $C_{1-6}$ alkoxy-carbonyl group, carbamoyl group, mono-$C_{1-6}$ alkyl-carbamoyl group, di-$C_{1-6}$ alkyl-carbamoyl group, sulfo group, $C_{1-6}$ alkylsulfonyl group, $C_{1-6}$ alkylsulfinyl group, $C_{6-10}$ aryl group and $C_{6-10}$ aryloxy group, and the like are preferable. Particularly, $C_{7-16}$ aralkyl group optionally substituted by 1 to 3 halogen atom(s) or $C_{1-6}$ alkoxy group is preferable.

As $R^3$,

(i) hydrogen atom;

(ii) a group represented by the formula -CO-$R^5$ ($R^5$ is (a) hydrogen atom, (b) carboxyl group, (c) $C_{1-6}$ alkyl group (particularly, $C_{1-3}$ alkyl group such as methyl, ethyl and the like), (d) $C_{5-6}$ cycloalkyl group (e.g., cyclopentyl and cyclohexyl) optionally having $C_{1-6}$ alkoxy (e.g., methoxy) and fused with benzene ring, (e) 5 or 6-membered aromatic heterocyclic group containing, besides carbon atom, 1 to 3 heteroatom(s) selected from the group consisting of nitrogen atom, oxygen atom and sulfur atom (e.g., pyridyl, thienyl, furyl, pyrrolyl, thiazolyl and the like), which optionally has 1 or 2 substituent(s) selected from the group consisting of halogen atom (e.g., bromine and the like), $C_{6-10}$ aryl group (e.g., phenyl and the like), $C_{6-10}$ aryl-carbonylamino group (e.g., benzoylamino and the like));

(iii) a group represented by the formula -CO-$Alk_0$-$R^6$ [$Alk_0$ is $C_{1-6}$ alkylene group optionally having hydroxy group, preferably a group represented by the formula $(CH_2)r^1$ ($r^1$ is an integer of 1 to 3) ; $R^6$ is (a) $C_{6-10}$ aryl group (e.g., phenyl) optionally having 1 or 2 substituent(s) selected from the group consisting of halogen atom, optionally halogenated $C_{1-6}$ alkyl (e.g., trifluoromethyl), nitro, $C_{1-6}$ alkoxy (e.g., methoxy, ethoxy), $C_{1-3}$ alkylenedioxy (e.g., methylenedioxy) and $C_{6-10}$ aryl group (e.g., phenyl), (b) $C_{6-10}$ aryloxy group (e.g., phenyloxy) , (c) 5 or 6-membered aromatic heterocyclic group (e.g., pyridyl) containing, besides carbon atom, 1 or 2 heteroatom(s) selected from nitrogen atom, oxygen atom and sulfur atom, (d) $C_{1-6}$ alkyl-carbonyl group (e.g., acetyl), (e) carboxyl group, (f) $C_{1-6}$ alkoxy-carbonyl group (e.g., $C_{1-3}$ alkoxy-carbonyl such as methoxycarbonyl, ethoxycarbonyl and the like), (g) amino optionally having 1 or 2 substituent(s) selected from the group consisting of $C_{1-6}$ alkyl (e.g., $C_{1-3}$ alkyl such as methyl, ethyl and the like) and $C_{1-6}$ alkyl-carbonyl (e.g., acetyl), (h) 5 to 7-membered heterocyclic ring optionally having hydroxy, (i) $C_{7-16}$ aralkyloxy group (e.g., benzyloxy), (j) $C_{6-10}$ aryl-carbonyl group (e.g., benzoyl), (k) $C_{1-6}$ alkyl-carbonyloxy group (e.g., acetoxy)];

(iv) a group represented by the formula

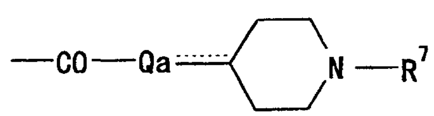

wherein Qa is a group represented by the formula $-(CH_2)s-$ (s is an integer of 1 to 3) or $-(CH_2)t-CH=$ (t is an integer of 0 to 2), $R^7$ is hydrogen atom or $C_{1-6}$ alkoxy-carbonyl group (e.g., $C_{1-3}$ alkoxy-carbonyl group such as methoxycarbonyl, ethoxycarbonyl and the like) ;

(v) a group represented the formula

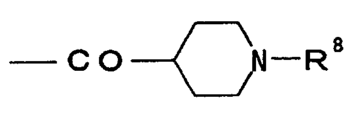

wherein $R^8$ is (a) hydrogen atom, (b) $C_{1-6}$ alkyl group (e.g., $C_{1-3}$ alkyl group such as methyl, ethyl, propyl and the like) optionally having substituents selected from the group consisting of $C_{1-6}$ alkoxy-carbonyl (e.g., $C_{1-3}$ alkoxy-carbonyl such as methoxycarbonyl, ethoxycarbonyl and the like), morpholino and mono- or di-$C_{1-6}$ alkylamino (e.g., methylamino, ethylamino, dimethylamino, diethylamino), (c) $C_{1-6}$ alkoxy-carbonyl group (e.g., $C_{1-3}$ alkoxy-carbonyl group such as methoxycarbonyl, ethoxycarbonyl and the like), (d) a group represented by the formula -CO-$R^d$ ($R^d$ is $C_{6-10}$ aryl group(e.g., phenyl, naphthyl) optionally having halogen atom (e.g., chlorine) or 5 or 6-membered heterocyclic group (e.g., pyridyl) containing, besides carbon atom, 1 or 2 heteroatom(s) selected from the group consisting of nitrogen atom, oxygen atom and sulfur atom), (e) a group represented by the formula -CO-$(CH_2)r^1$-

$R^e$ ($r^1$ is an integer of 1 to 3, $R^e$ is $C_{1-6}$ alkoxy-carbonyl group (e.g., $C_{1-3}$ alkoxy-carbonyl group such as methoxycarbonyl, ethoxycarbonyl and the like) or 5 or 6-membered heterocyclic group (e.g., pyridyl and the like) containing, besides carbon atom, 1 or 2 heteroatom(s) selected from nitrogen atom, oxygen atom and sulfur atom) or (f) a group represented by -CONH-$R^f$ ($R^f$ is $C_{1-6}$ alkyl group (e.g., $C_{1-3}$ alkyl group such as methyl, ethyl and the like) or $C_{6-14}$ aryl group (e.g., phenyl, naphthyl and the like);

(vi) a group represented by the formula -COOR$^9$ ($R^9$ is optionally halogenated $C_{1-6}$ alkyl group (e.g., methyl, ethyl, trifluoromethyl));

(vii) a group represented by the formula

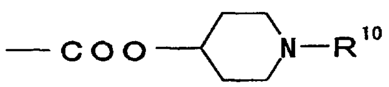

wherein $R^{10}$ is hydrogen atom, $C_{1-6}$ alkoxy-carbonyl group (e.g., $C_{1-3}$ alkoxy-carbonyl group such as methoxycarbonyl, ethoxycarbonyl and the like), mono- or di-$C_{1-6}$ alkyl-carbamoyl group (e.g., methylcarbamoyl, ethylcarbamoyl, dimethylcarbamoyl, diethylcarbamoyl), optionally halogenated nicotinoyl group or optionally halogenated isonicotinoyl group;

(viii) a group represented by the formula -CONR$^{11}$-R$^{12}$ ($R^{11}$ is hydrogen atom or $C_{1-6}$ alkyl group (e.g., $C_{1-3}$ alkyl group such as methyl, ethyl and the like), $R^{12}$ is $C_{1-6}$ alkyl group (e.g., $C_{1-3}$ alkyl group such as methyl, ethyl, propyl and the like) optionally having substituents selected from the group consisting of (a) hydroxy, (b) amino, (c) mono- or di-$C_{1-6}$ alkyl-amino (e.g., methylamino, ethylamino, dimethylamino, diethylamino), (d) $C_{1-6}$ alkyl-carbonyl (e.g., $C_{1-3}$ alkyl-carbonyl such as acetyl, ethylcarbonyl and the like), (e) $C_{1-6}$ alkoxy-carbonyl (e.g., $C_{1-3}$ alkoxy-carbonyl such as methoxycarbonyl, ethoxycarbonyl and the like), (f) $C_{1-6}$ alkyl-carbonyloxy (e.g., $C_{1-3}$ alkyl-carbonyloxy such as acetyloxy, ethylcarbonyloxy and the like), (g) sulfamoyl, (h) 5 to 7-membered heterocyclic group optionally substituted by oxo and (i) $C_{6-14}$ aryl (e.g., phenyl));

(ix) a group represented by the formula

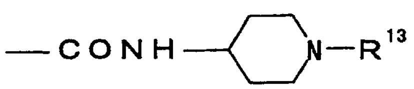

wherein $R^{13}$ is (a) hydrogen atom, (b) $C_{1-6}$ alkyl group (e.g., $C_{1-3}$ alkyl group such as methyl, ethyl, propyl and the like) optionally having substituents selected from the group consisting of a hydroxy and $C_{1-6}$ alkoxy-carbonyl (e. g., $C_{1-3}$ alkoxy-carbonyl such as methoxycarbonyl, ethoxycarbonyl and the like) , (c) $C_{7-16}$ aralkyl group (e.g., benzyl), (d) $C_{1-6}$ alkyl-carbonyl group (e.g., $C_{1-3}$ alkyl-carbonyl group such as acetyl, ethylcarbonyl and the like) optionally having substituents selected from the group consisting of a halogen atom (e.g., fluorine, chlorine) and $C_{1-6}$ alkoxy-carbonyl (e.g., $C_{1-3}$ alkoxy-carbonyl such as methoxycarbonyl, ethoxycarbonyl and the like) or (e) $C_{1-6}$ alkyl-carbamoyl group(e.g., $C_{1-3}$ alkyl-carbamoyl group such as methylcarbamoyl, ethylcarbamoyl and the like) optionally having $C_{1-6}$ alkoxy-carbonyl (e.g., $C_{1-3}$ alkoxy-carbonyl such as methoxycarbonyl, ethoxycarbonyl and the like);

(x) a group represented by the formula

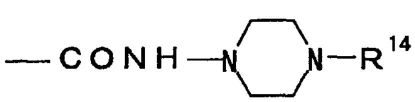

wherein $R^{14}$ is $C_{1-6}$ alkyl group (e.g., $C_{1-3}$ alkyl group such as methyl, ethyl, propyl and the like) or $C_{7-16}$ aralkyl group (e.g., benzyl) ;

(xi) a group represented by the formula

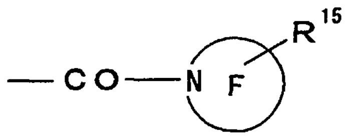

wherein ring F is 5 to 7-membered non-aromatic heterocyclic group (particularly piperidyl) optionally fused with benzene ring, $R^{15}$ is hydrogen atom, $C_{1-6}$ alkoxy-carbonylamino group (e.g., $C_{1-3}$ alkoxy-carbonylamino group such as methoxycarbonylamino, ethoxycarbonylamino and the like) or optionally halogenated $C_{1-6}$ alkyl-carbonylamino group (e.g., optionally halogenated $C_{1-3}$ alkoxy-carbonylamino group such as methylcarbonylamino, ethylcarbonylamino, trifluoromethylcarbonylamino and the like);
(xii) a group represented by the formula

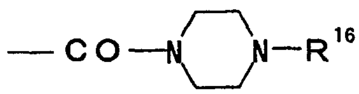

wherein $R^{16}$ is (a) $C_{1-6}$ alkyl group (e.g., $C_{1-3}$ alkyl group such as methyl, ethyl, propyl and the like) optionally having substituents selected from the group consisting of hydroxy and $C_{1-6}$ alkoxy-carbonyl (e.g., $C_{1-3}$ alkoxy-carbonyl such as methoxycarbonyl, ethoxycarbonyl and the like) (b) formyl group, (c) $C_{1-6}$ alkoxy-carbonyl group (e.g., $C_{1-3}$ alkoxy-carbonyl group such as methoxycarbonyl, ethoxycarbonyl and the like) or (d) 5 or 6-membered heterocyclic ring-carbonyl group containing, besides carbon atom, 1 to 3 heteroatom(s) selected from the group consisting of nitrogen atom, oxygen atom and sulfur atom (e.g., morpholinocarbonyl group);
(xiii) a group represented by the formula $-SO_2-R^{17}$ ($R^{17}$ is (i) $C_{1-6}$ alkyl group(e.g., $C_{1-3}$ alkyl group such as methyl, ethyl and the like) optionally having 5 or 6-membered heterocyclic group (e.g., 5 or 6-membered cyclic amino group), (ii) $C_{2-6}$ alkenyl group (e.g., ethenyl group) or (iii) $C_{6-14}$ aryl group (e.g., phenyl, naphthyl) optionally having $C_{1-6}$ alkyl (e.g., $C_{1-3}$ alkyl such as methyl, ethyl and the like));
(xiv) $C_{7-16}$ aralkyl group (preferably benzyl group) optionally having 1 to 3 halogen atom(s) (e.g., fluorine, chlorine and the like, preferably fluorine) or $C_{1-6}$ alkoxy group (e.g., methoxy and the like); and
(xv) $C_{1-6}$ alkyl group (preferably $C_{1-3}$ alkyl group such as methyl and the like) substituted by 5 or 6-membered heterocyclic group (e.g., thienyl) containing, besides carbon atom, 1 to 3 heteroatom(s) selected from the group consisting of nitrogen atom, oxygen atom and sulfur atom, and the like are preferable.

As preferable examples of $R^3$, moreover, the following group and the like are mentioned.
(xvi) a group represented by the formula

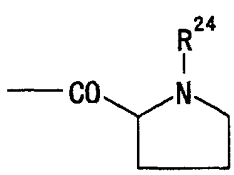

wherein the symbols are as defined above;
(xvii) a group represented by the formula

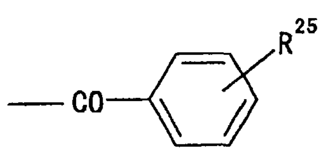

wherein the symbols are as defined above;
(xviii) a group represented by the formula

$$-CO\text{-}Alk\text{-}NR^{27}\text{-}CO\text{-}Alk_2\text{-}O\text{-}Alk_3\text{-}R^{28}$$

wherein the symbols are as defined above;
(xix) a group represented by the formula

$$-CO\text{-}Alk_2\text{-}NR^{27}\text{-}CO\text{-}Alk_3\text{-}R^{29}$$

wherein the symbols are as defined above;
(xx) a group represented by the formula

$$-CO\text{-}Alk\text{-}NR^{27}\text{-}CO\text{-}Alk_2\text{-}NR^{30}\text{-}Alk_3\text{-}R^{31}$$

wherein the symbols are as defined above;
(xxi) a group represented by the formula

$$-CO\text{-}Alk\text{-}NR^{27}\text{-}CO\text{-}Alk_2\text{-}NR^{32}\text{-}CO\text{-}O\text{-}Alk_3\text{-}R^{31}$$

wherein the symbols are as defined above;
(xxii) a group represented by the formula

$$-CO\text{-}Alk\text{-}CO\text{-}NR^{27}\text{-}Alk_2\text{-}R^{31}$$

wherein the symbols are as defined above; and
(xxiii) a group represented by the formula

$$-CO\text{-}Alk\text{-}O\text{-}CO\text{-}O\text{-}Alk_2\text{-}R^{31}$$

wherein the symbols are as defined above.

As the "alkyl group optionally having substituents" represented by $R^4$, for example, $C_{1-6}$ alkyl group optionally having 1 to 3 substituent(s) selected from halogen atom, nitro group, cyano group, optionally halogenated $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group, di-$C_{1-6}$ alkylamino group, $C_{1-6}$ alkyl-carbonylamino group (e.g., acetylamino, propionylamino, butyrylamino and the like), carboxyl group, $C_{1-6}$ alkoxy-carbonyl group, carbamoyl group, mono-$C_{1-6}$ alkyl-carbamoyl group, di-$C_{1-6}$ alkyl-carbamoyl group, sulfo group, $C_{1-6}$ alkylsulfonyl group, $C_{1-6}$ alkylsulfinyl group and $C_{6-10}$ aryl group, and the like are preferable.

As the "alkylcarbonyl group optionally having substituents" represented by $R^4$, for example, $C_{1-6}$ alkyl-carbonyl group (e.g., acetyl, ethylcarbonyl, propylcarbonyl and the like) optionally having 1 to 3 substituent(s) selected from halogen atom, nitro group, cyano group, optionally halogenated $C_{3-6}$ cycloalkyl group, optionally halogenated $C_{1-6}$ alkoxy group, optionally halogenated $C_{1-6}$ alkylthio group, hydroxy group, amino group, mono-$C_{1-6}$ alkylamino group, di-$C_{1-6}$ alkylamino group, $C_{1-6}$ alkyl-carbonylamino group (e.g., acetylamino, propionylamino, butyrylamino and the like), carboxyl group, $C_{1-6}$ alkoxy-carbonyl group, carbamoyl group, mono-$C_{1-6}$ alkyl-carbamoyl group, di-$C_{1-6}$ alkyl-carbamoyl group, sulfo group, $C_{1-6}$ alkylsulfonyl group, $C_{1-6}$ alkylsulfinyl group and $C_{6-10}$ aryl group, and the like are preferable.

As $R^4$, hydrogen atom, $C_{1-6}$ alkyl group (e.g., $C_{1-3}$ alkyl group such as methyl, ethyl and the like) and the like are preferable. Of these, hydrogen atom and methyl group are preferable, and hydrogen atom is particularly preferable.

The monocyclic or fused nitrogen-containing heterocyclic group optionally having substituents, which is formed by $R^3$ and $R^4$ together with the adjacent nitrogen atom is, for example, a group represented by the formula

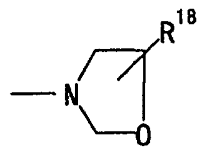

wherein $R^{18}$ is halogen atom, oxo group, optionally halogenated $C_{1-6}$ alkyl group or optionally halogenated $C_{1-6}$ alkoxy group, and the like are preferable.

As j, 0 is preferable.

Furthermore, a compound used for the preparation of the present invention, a compound wherein preferable groups represented by the aforementioned symbols are optionally combined and the like are preferably used. Specific examples include the following compounds and the like.

(1) A compound represented by the formula

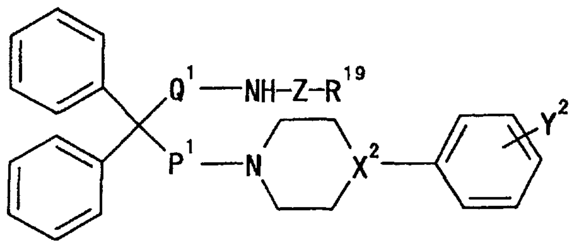

wherein $R^{19}$ is (i) hydrogen atom, (ii) carboxyl, (iii) $C_{1-6}$ alkoxy-carbonyl group (e.g., $C_{1-3}$ alkoxy-carbonyl such as methoxycarbonyl, ethoxycarbonyl and the like), (iv) $C_{1-6}$ alkyl group (e.g., $C_{1-3}$ alkyl group such as methyl, ethyl, propyl and the like) optionally having substituents selected from the group consisting of carboxyl, $C_{1-6}$ alkyl-carbonyl (e.g., $C_{1-3}$ alkyl-carbonyl such as acetyl, ethylcarbonyl and the like), $C_{1-6}$ alkoxy-carbonyl (e.g., $C_{1-3}$ alkoxy-carbonyl such as methoxycarbonyl, ethoxycarbonyl and the like), $C_{1-6}$ alkoxy-carbonylamino (e.g., methoxycarbonylamino, ethoxycarbonylamino, propoxycarbonylamino, butoxycarbonylamino, t-butoxycarbonylamino) and $C_{7-16}$ aralkyloxy-carbonylamino (e.g., benzyloxycarbonylamino), (v) a mono- or di-$C_{1-6}$ alkylamino group (e.g., mono- or di-$C_{1-3}$ alkylamino such as methylamino, ethylamino, dimethylamino, diethylamino and the like) or (iv) $C_{6-14}$ aryloxy group (e.g., phenyloxy), $P^1$ is $C_{1-3}$ alkylene group, $Q^1$ is $C_{1-3}$ alkylene group, $X^2$ is CH, C-OH or N, $Y^2$ is hydrogen atom, halogen atom (e.g., fluorine, chlorine), optionally halogenated $C_{1-6}$ alkyl group (e.g., optionally halogenated $C_{1-3}$ alkyl group such as methyl, ethyl, propyl, trifluoromethyl and the like) or $C_{1-6}$ alkoxy group (e.g., $C_{1-3}$ alkoxy group such as methoxy, ethoxy and the like), and Z is CO, SO or $SO_2$ (preferably CO)], or a salt thereof.

(2) A compound represented by the formula

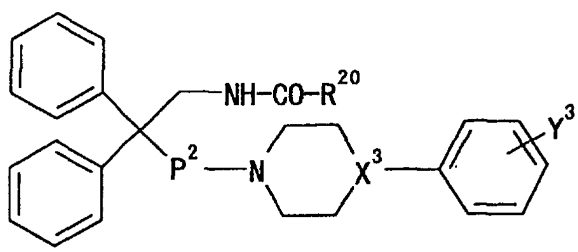

wherein $R^{20}$ is (i) hydrogen atom or (ii) $C_{1-6}$ alkyl group (e.g., $C_{1-3}$ alkyl group such as methyl, ethyl, propyl and the like) optionally having substituents selected from the group consisting of $C_{1-6}$ alkoxy-carbonylamino (e.g., methoxycarbonylamino, ethoxycarbonylamino, propoxycarbonylamino, butoxycarbonylamino, t-butoxycarbonylamino) and $C_{7-16}$ aralkyloxy-carbonylamino (e.g., benzyloxycarbonylamino), $P^2$ is $C_{1-3}$ alkylene group (e.g., methylene, ethylene and trimethylene, preferably trimethylene), $X^3$ is CH, C-OH or N (preferably CH), $Y^3$ is hydrogen atom, halogen atom (e.g., fluorine, chlorine) or $C_{1-6}$ alkoxy group (e.g., methoxy, ethoxy, propoxy), or a salt thereof.

(3) A compound represented by the formula

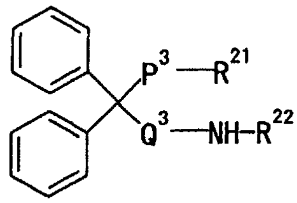

wherein $R^{21}$ is nitrogen-containing heterocyclic group represented by (i) the formula

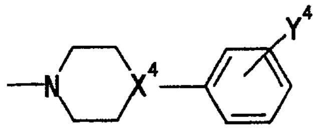

wherein $X^4$ is CH or N, and $Y^4$ is hydrogen atom, halogen atom or $C_{1-6}$ alkoxy group, or (ii) the formula

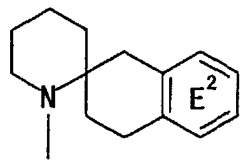

wherein ring $E^2$ is benzene ring optionally having 1 to 3 $C_{1-6}$ alkoxy (e.g., $C_{1-3}$ alkoxy such as methoxy and the like), $R^{22}$ is (i) hydrogen atom, (ii) $C_{7-16}$ aralkyl group (e.g., benzyl), (iii) a formyl group, (iv) $C_{1-6}$ alkyl-carbonyl group (e.g., $C_{1-3}$ alkyl-carbonyl such as acetyl, ethylcarbonyl and the like), (v) $C_{6-14}$ aryl-carbonyl group (e.g., phenylcarbonyl) optionally having $C_{1-6}$ alkyl (e.g., $C_{1-3}$ alkyl such as methyl and the like) or (vi) $C_{6-14}$ aryl-sulfonyl group (e.g., phenylsulfonyl, naphthylsulfonyl) optionally having 1 to 4 $C_{1-6}$ alkyl (e.g., $C_{1-3}$ alkyl such as methyl and the like), $P^3$ is $C_{1-3}$ alkylene group (e.g., methylene, ethylene, trimethylene, preferably trimethylene), $Q^3$ is $C_{1-3}$ alkylene group (e.g., methylene, ethylene, trimethylene) or a salt thereof.

(4) A compound represented by the formula

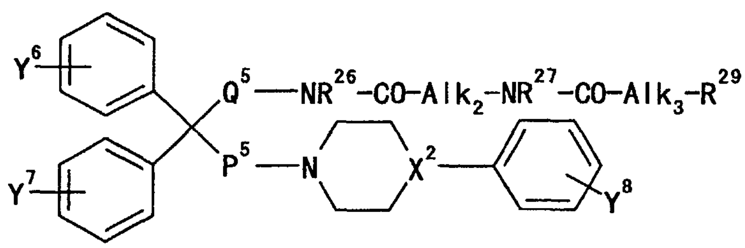

(Ib)

wherein $P^5$ and $Q^5$ are the same or different and each is $C_{1-6}$ alkylene group (e.g., methylene, ethylene, trimethylene); $Y^6$, $Y^7$ and $Y^8$ are the same or different and each is hydrogen atom, halogen atom (e.g., fluorine, chlorine), optionally halogenated $C_{1-6}$ alkyl group (e.g., methyl, ethyl, trifluoromethyl) or optionally halogenated $C_{1-6}$ alkoxy group (methoxy, ethoxy, trifluoromethoxy); other symbols are as defined above, or a salt thereof.

(5) A compound represented by the formula

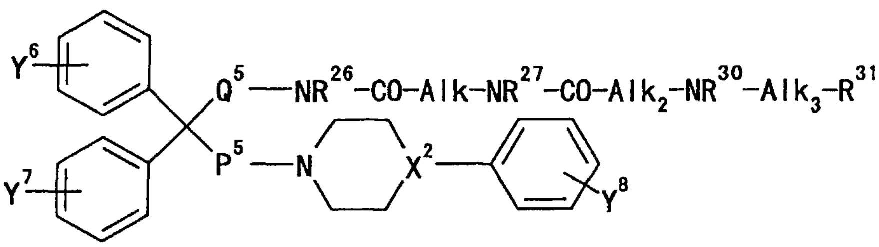

(Ic)

wherein the symbols in the formula are as defined above, or a salt thereof.

More preferable compounds are exemplified by, but not limited to, the following and the like.

Reference Example IA-1: 1-(5-amino-4,4-diphenylpentyl)-4-phenylpiperidine
Reference Example IA-2: 3,4-dihydro-6-methoxy-1'-(5-amino-4,4-diphenylpentyl)spiro[naphthalene-2(1H),2'-piperidine]
Reference Example IA-3: 1- [5-amino-4-(4-methoxyphenyl)-4-phenylpentyl]-4-phenylpiperidine
Reference Example IA-4: 1- [5-amino-4,4-bis(4-chlorophenyl)-pentyl]-4-(4-fluorophenyl)piperazine
Reference Example IA-5: 3,4-dihydro-6-methoxy-1'- (6-amino-4,4-diphenylhexyl)spiro[naphthalene-2(1H),2'-piperidine]
Reference Example IA-6: 3,4-dihydro-6,7-dimethoxy-1'-(7-amino-4,4-diphenylheptyl)spiro [naphthalene-2(1H),2'-piperidine]
Reference Example IIA-1:1-(N,N-dimethylamino)-4,4-diphenyl-5-(formylamino)pentane
Reference Example IIA-2: 1-(N-benzyl-N-methylamino)-4,4-diphenyl-5-(formylamino)pentane hydrochloride
Reference Example IIA-3: 4,4-diphenyl-5-formylamino-1-(morpholino)pentane hydrochloride
Reference Example IIA-4: 4,4-diphenyl-5-formylamino-1-(2,3,4,5-tetrahydro-IH-3-benzazepin-3-yl)pentane hydrochloride
Reference Example IIA-5: 4,4-diphenyl-5-formylamino-1-(4-phenylpiperidino)pentane hydrochloride
Reference Example IIA-6: 1-[4-(4-fluorophenyl)piperazin-1-yl]-5-formylamino-4,4-diphenylpentane dihydrochloride
Reference Example IIA-7: 3,4-dihydro-6-methoxy-1'-(5-formylamino-4,4-diphenylpentyl)spiro(naphthalene-2(1H), 2'-piperidine] dihydrochloride
Reference Example IIA-8: 1-benzylamino-4,4-diphenyl-5-(tosylamino)pentane hydrochloride
Reference Example IIA-9: 1-(N-benzyl-N-methylamino)-4,4-diphenyl-5-(tosylamino)pentane hydrochloride
Reference Example IIA-10: 4,4-diphenyl-1-(3-nitrobenzylamino)-5-(tosylamino)pentane hydrochloride
Reference Example IIA-11: 1-(3-aminobenzylamino)-4,4-diphenyl-5-(tosylamino)pentane
Reference Example IIA-12: 4,4-diphenyl-1-[3-(methoxycarbonyl)-benzylamino]-5-(tosylamino)pentane hydrochloride
Reference Example IIA-13: 4,4-diphenyl-1-(2-picolylamino)-5-(tosylamino)pentane dihydrochloride
Reference Example IIA-14: 4,4-diphenyl-1-(1-hexamethyleneimino)-5-(tosylamino)pentane hydrochloride
Reference Example IIA-15: 4,4-diphenyl-1-(4-phenylpiperazin-1-yl)-5-(tosylamino)pentane
Reference Example IIA-16: 4,4-diphenyl-1-[4-(2-methoxyphenyl)-piperazin-1-yl]-5-(tosylamino)pentane hydrochloride
Reference Example IIA-17: 4,4-diphenyl-5-mesylamino-1-(4-phenylpiperidino)pentane hydrochloride
Reference Example IIA-18: 5-benzenesulfonylamino-4,4-diphenyl-1-(4-phenylpiperidino)pentane
Reference Example IIA-19: 4,4-diphenyl-1-(4-phenylpiperidino)-5-(2,4,6-trimethylbenzenesulfonylamino)pentane
Reference Example IIA-20: 4,4-diphenyl-1-(4-phenylpiperidino)-5-(2,4,6-triisopropylbenzenesulfonylamino)pentane
Reference Example IIA-21: 4,4-diphenyl-5-(1-naphthylsulfonylamino)-1-(4-phenylpiperidino)pentane
Reference Example IIA-22: 4,4-diphenyl-5-(2-naphthylsulfonylamino)-1-(4-phenylpiperidino)pentane
Reference Example IIA-23: 3,4-dihydro-6-methoxy-1'-(5-acetylamino-4,4-diphenylpentyl)spiro[naphthalene-2(1H),2'-piperidine] dihydrochloride
Reference Example IIA-24: 3,4-dihydro-6-methoxy-1'-(5-tosylamino-4,4-diphenylpentyl)spiro [naphthalene-2(1H), 2'-piperidine] dihydrochloride
Reference Example IIA-25: 4-(4-chlorophenyl)-5-formylamino-4-phenyl-1-(4-phenylpiperidino)pentane hydrochloride
Reference Example IIA-26: 4- (4-chlorophenyl)-5-formylamino-4-phenyl-1-(4-phenylpiperazin-1-yl)pentane dihydrochloride

Reference Example IIA-27: 4-(4-chlorophenyl)-1-[4-(4-fluorophenyl)piperazin-1-yl]-5-formylamino-4-phenylpentane dihydrochloride
Reference Example IIA-28: 4-(4-chlorophenyl)-1- [4-(diphenylmethyl)piperazin-1-yl]-5-formylamino-4-phenylpentane
Reference Example IIA-29: 5-formylamino-4-(4-methoxyphenyl)-4-phenyl-1-(4-phenylpiperidino)pentane hydrochloride
Reference Example IIA-30: 4-(4-methoxyphenyl)-5-(1-naphthylsulfonylamino)-4-phenyl-1- (4-phenylpiperidino) pentane hydrochloride
Reference Example IIA-31: 4,4-bis(4-chlorophenyl)-1-[4-(4-fluorophenyl)piperazin-1-yl]-5-(formylamino)pentane dihydrochloride
Reference Example IIA-32: 4,4-bis(4-chlorophenyl)-1-[4- (4-fluorophenyl)piperazin-1-yl]-5-(mesylamino)pentane dihydrochloride
Reference Example IIA-33: 4,4-bis(4-chlorophenyl)-1-[4-(4-fluorophenyl)piperazin-1-yl]-5-(tosylamino)pentane dihydrochloride
Reference Example IIA-34: 1-[4-(4-fluorophenyl)piperazin-1-yl]-6-formylamino-5,5-diphenylhexane dihydrochloride
Reference Example IIA-35: 1-[4-(4-fluorophenyl)piperazin-1-yl]-6-formylamino-4,4-diphenylhexane dihydrochloride
Reference Example IIA-36: 4,4-diphenyl-1-(4-phenylpiperidino]-6-(tosylamino)hexane hydrochloride
Reference Example IIA-37: 3,4-dihydro-6-methoxy-1'-(6-acetylamino-4,4-diphenylhexyl) spiro[naphthalene-2(1H),2'-piperidine] dihydrochloride
Reference Example IIA-38: 3,4-dihydro-6-methoxy-1'-(6-tosylamino-4,4-diphenylhexyl)spiro [naphthalene-2(1H), 2'-piperidine] hydrochloride
Reference Example IIA-39: 3,4-dihydro-6-methoxy-1'-(6-benzylamino-4,4-diphenylhexyl)spiro[naphthalene-2(1H),2'-piperidine] dihydrochloride
Reference Example 1B-1: 5-[4-(4-chlorophenyl)-4-hydroxypiperidino]-1-formylamino-2,2-diphenylpentane hydrochloride
Reference Example 1B-2: 5-[4-(4-fluorophenyl)piperazin-1-yl]-1-formylamino-2,2-diphenylpentane dihydrochloride
Reference Example 1B-3: 1-formylamino-5-(4-hydroxy-4-phenylpiperidino)-2,2-diphenylpentane hydrochloride
Reference Example 1B-4: 5-[4-(4-trifluoromethylphenyl)-4-hydroxypiperidino]-1-formylamino-2,2-diphenylpentane hydrochloride
Reference Example 1B-5: 5-[4-[3,5-bis(trifluoromethyl)phenyl]-4-hydroxypiperidino]-1-formylamino-2,2-diphenylpentane hydrochloride
Reference Example 1B-6: 5-[4-(3,5-dichlorophenyl)-4-hydroxypiperidino]-1-formylamino-2,2-diphenylpentane hydrochloride
Reference Example 1B-7: 5-[4-(4-chlorophenyl)-1,2,3,6-tetrahydropyridin-1-yl]-1-formylamino-2,2-diphenylpentane hydrochloride
Reference Example 1B-8: 1-formylamino-2,2-diphenyl-5-(4-phenylpiperidino)pentane
Reference Example 1B-9: 5-[4-(4-chlorophenyl)piperidino]-1-formylamino-2,2-diphenylpentane hydrochloride
Reference Example 1B-10: 7-[4-(4-chlorophenyl)-4-hydroxypiperidino]-1-formylamino-4,4-diphenylheptane hydrochloride
Reference Example 2B-1: 5-[4-(4-fluorophenyl)-4-hydroxypiperidino]-1-formylamino-2,2-diphenylpentane hydrochloride
Reference Example 2B-2: 1-formylamino-5-[4-hydroxy-4-(4-methoxyphenyl)piperidino]-2,2-diphenylpentane hydrochloride
Reference Example 2B-3: 1-formylamino-5-[4-hydroxy-4-(2-pyridyl)piperidino]-2,2-diphenylpentane dihydrochloride
Reference Example 3B-1: 1-acetylamino-5-[4-(4-chlorophenyl)-4-hydroxypiperidino]-2,2-diphenylpentane hydrochloride
Reference Example 3B-2: 1-acetoacetylamino-5-[4-(4-chlorophenyl)-4-hydroxypiperidino]-2,2-diphenylpentane hydrochloride
Reference Example 3B-3: ethyl N-[5-[4-(4-chlorophenyl)-4-hydroxypiperidino]-2,2-diphenylpentyl]succinamate hydrochloride
Reference Example 3B-4: N-[5-[4-(4-chlorophenyl)-4-hydroxypiperidino]-2,2-diphenylpentyl]succinamic acid
Reference Example 3B-5: 1-[5-[4-(4-chlorophenyl)-4-hydroxypiperidino]-2,2-diphenylpentyl]-3-ethylurea
Reference Example 3B-6: N-[5-[4-(4-chlorophenyl)-4-hydroxypiperidino]-2,2-diphenylpentyl]methanesulfonamide hydrochloride

Reference Example 3B-7: phenyl N-[5-[4-(4-chlorophenyl)-4-hydroxypiperidino]-2,2-diphenylpentyl]carbamate
Reference Example 3B-8: 1-acetylamino-5-[4-(4-chlorophenyl)-4-hydroxypiperidino]-2-phenyl-2-(2-pyridyl)pentane dihydrochloride
Reference Example 3B-9: ethyl N-[5-[4-(4-chlorophenyl)-4-hydroxypiperidino]-2,2-diphenylpentyl]oxamate hydrochloride
Reference Example 3B-10: ethyl N-[5-[4-(4-chlorophenyl)-4-hydroxypiperidino]-2,2-diphenylpentyl]malonamate hydrochloride
Reference Example 3B-11: ethyl N-[5-[4-(4-chlorophenyl)-4-hydroxypiperidino]-2,2-diphenylpentyl]glutarmate
Example 1: benzyl 2-((2,2-diphenyl-5-(4-phenylpiperidino)-pentyl)amino)-2-oxoethylcarbamate hydrochloride
Example 2: tert-butyl 2-((2,2-diphenyl-5-(4-phenylpiperidino)-pentyl)amino)-2-oxoethylcarbamate
Example 3: 4,4-diphenyl-7-(4-phenylpiperidino)heptylamine dihydrochloride
Example 4: N-(4,4-diphenyl-7-(4-phenylpiperidino)heptyl)-4-methylbenzenesulfonamide hydrochloride
Example 5: N-(4,4-diphenyl-7-(4-phenylpiperidino)heptyl)-acetamide hydrochloride
Example 6: N-benzyl-N-(4,4-diphenyl-7-(4-phenylpiperidino)-heptyl)amine dihydrochloride
Example 7: N-(4,4-diphenyl-7- (4-phenylpiperidino)heptyl)-N-(3-methoxybenzyl)amine dihydrochloride
Example 8: N-(4,4-diphenyl-7-(4-phenylpiperidino)heptyl)-N-(2-methoxybenzyl)amine dihydrochloride
Example 9: N-(4,4-diphenyl-7-(4-phenylpiperidino)heptyl)-N-(2-fluorobenzyl)amine dihydrochloride
Example 10: N-(4,4-diphenyl-7-(4-phenylpiperidino)heptyl)-2-thiophenecarboxamide hydrochloride
Example 11: N-(4,4-diphenyl-7-(4-phenylpiperidino)heptyl)-2-phenylacetamide hydrochloride
Example 12: N-(4,4-diphenyl-7-(4-phenylpiperidino)heptyl)-N-(2-thienylmethyl)amine dihydrochloride
Example 13: N-benzyl-N-(4,4-diphenyl-7-(4-phenylpiperidino)-heptyl)-N-methylamine dihydrochloride
compounds of Example 14 - Example 131.

Of the above-mentioned compounds (I), a compound represented by the formula

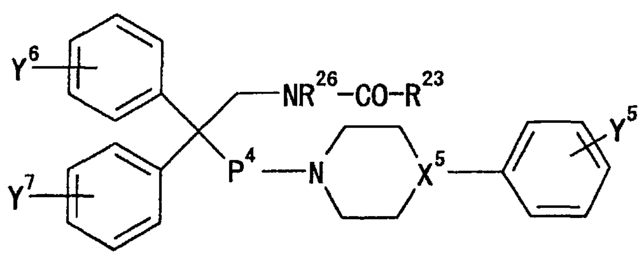

(Ia)

wherein $R^{23}$ is $C_{1-6}$ alkyl group (e.g., methyl, ethyl, propyl and the like) having $C_{7-16}$ aralkyloxy-carbonylamino (e.g., benzyloxycarbonylamino) optionally having substituents selected from the group consisting of halogen atom (e.g., fluorine atom, chlorine atom, iodine atom), $C_{1-6}$ alkoxy (e.g., methoxy, ethoxy) and $C_{1-6}$ alkyl (e.g., methyl, ethyl), $P^4$ is $C_{1-3}$ alkylene group (e.g., methylene, ethylene, trimethylene), $X^5$ is CH, C-OH or N, $Y^5$ is hydrogen atom, halogen atom (e.g., fluorine atom, chlorine atom, iodine atom) or $C_{1-6}$ alkoxy (e.g., methoxy, ethoxy, propoxy); $R^{26}$ is hydrogen atom or $C_{1-6}$ alkyl group; $Y^6$ and $Y^7$ are the same or different and each is hydrogen atom, halogen atom (e.g., fluorine atom, chlorine atom, iodine atom), optionally halogenated $C_{1-6}$ alkyl group (e.g., methyl, ethyl, trifluoromethyl) or optionally halogenated $C_{1-6}$ alkoxy group (methoxy, ethoxy, trifluoromethoxy) and a salt thereof are novel compounds.

As $R^{23}$, $C_{1-3}$ alkyl group (e.g., methyl, ethyl, propyl) having benzyloxycarbonylamino and the like, and the like are preferable.

As $P^4$, trimethylene is preferable.

As $Y^5$, hydrogen atom, fluorine atom and methoxy are preferable.

As $Y^6$ and $Y^7$, hydrogen atom is preferable.

As $R^{26}$, hydrogen atom is preferable.

As specific examples of compound (Ia), the compounds of Examples 1, 57, 58, 75, 76, 77, 80 and 103 and the like are preferable.

A compound represented by the aforementioned formula (Ib) or (Ic) and a salt thereof are also novel compounds.

As specific examples of compound (Ib), the compounds of Examples 44, 45, 47, 104, 105, 106, 107, 108, 109, 115, 116, 117, 118, 120, 121, 122, 124, 125, 127, 128, 130 and 131 and the like are preferable.

As specific examples of compound (Ic), the compounds of Examples 51, 59 and 65 and the like are preferable.

The prodrug of the compound (Ia) of the present invention may be a compound that is converted to compound

(Ia) by a reaction with an enzyme, gastric acid and the like in the body under physiological conditions. In other words, it may be a compound that undergoes enzymatic oxidation, reduction, hydrolysis and the like into compound (Ia), or a compound that undergoes hydrolysis and the like due to gastric acid and the like into compound (Ia).

Examples of the prodrug of- compound (Ia) includes compound (Ia) wherein amino group is acylated, alkylated or phosphorylated (e.g., compound (Ia) wherein amino group is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, tert-butylated and the like); compound (Ia) wherein hydroxyl group is acylated, alkylated, phosphorylated or borated (e.g., a compound (Ia) wherein hydroxyl group is acetylated, palmitoylated, propanoylated, pivaloylated, succinylated, fumarylated, alanylated, dimethylaminomethylcarbonylated and the like); compound (Ia) wherein carboxyl group is esterified or amidated (e.g., compound (Ia) wherein carboxyl group is ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified, methyl amidated and the like); and the like. These compounds can be produced from compound (Ia) by a method known *per se.*

The prodrug of compound (Ia) may be a compound that is converted to compound (Ia) under the physiological conditions described in Development of Pharmaceutical Products, vol. 7, Molecule Design, pp. 163-198, Hirokawa Shoten (1990).

The compounds (Ib) and (Ic) may be used as a prodrug, and as the prodrug, those similar to the prodrugs of the aforementioned compound (Ia) are mentioned.

When compound (I) forms a salt and the salt is used as a pharmaceutical product, it is preferably a pharmaceutically acceptable salt.

Examples of the pharmaceutically acceptable salt are, but not limited to, salts with inorganic acid salts such as hydrochloride, sulfate, phosphate, diphosphate, hydrobromate and nitrate; salts with organic acid such as acetate, malate, maleate, fumarate, tartrate, succinate, citrate, lactate, methanesulfonate, p-toluenesulfonate, palmitin acid, salicylate and stearate.

As the pharmaceutically acceptable salt, salts with inorganic base, salts with organic base and the like are also mentioned.

Preferable examples of salts with inorganic base include alkali metal salts such as sodium salt, potassium salt and the like; alkaline earth metal salts such as calcium salt, magnesium salt and the like; aluminum salt, ammonium salt and the like.

Preferable examples of salts with organic base include salts with trimethylamine, triethylamine, pyridine, picoline, ethanolamine, diethanolamine, triethanolamine, dicyclohexylamine, N,N-dibenzylethylenediamine and the like.

The compound (I) or a salt thereof may be labeled with an isotope (e.g., $^{3}H$, $^{14}C$, $^{35}S$, $^{125}I$ and the like).

The compound (I) or a salt thereof may be anhydrate or hydrate.

The compound (I) or a salt thereof to be used for the agent of the present invention can be produced according to a method known *per se,* such as a method described in, for example, JP-A-8-253447, JP-A-10-81665, JP-A-11-71350 and the like or a similar method.

For example, compound (Ia) or a salt thereof can be produced by reacting a compound represented by the formula

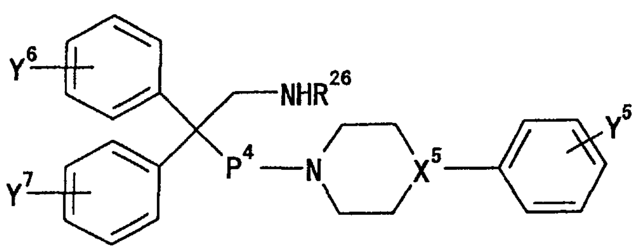 (Ia-1)

wherein each symbol is as defined above, or a salt thereof, with a reactive derivative of an organic acid represented by the formula

$$R^{23}\text{-COOH} \qquad \text{(Ia-2)}$$

wherein $R^{23}$ is as defined above, for acylation, or reacting a compound represented by the formula

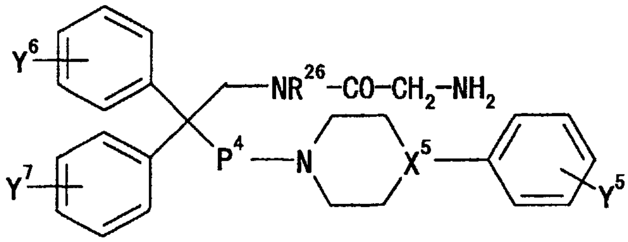

(Ia-3)

wherein each symbol is as defined above, or a salt thereof, with a reactive derivative represented by the formula

$$R^{32}\text{-X} \qquad \text{(Ia-4)}$$

wherein $R^{32}$ is $C_{7-16}$ aralkyloxy-carbonyl group and X is a leaving group;
the compound (Ib) or a salt thereof can be produced by reacting a compound represented by the formula

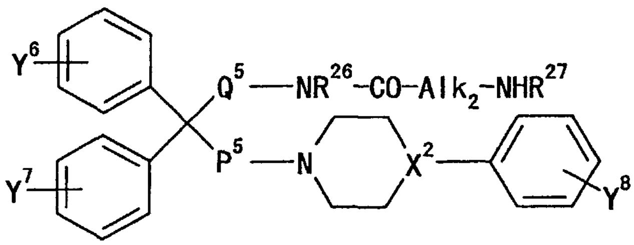

(Ib-1)

wherein each symbol is as defined above, or a salt thereof, with a reactive derivative of an organic acid represented by the formula

$$R^{29}\text{-Alk}_3\text{-COOH} \qquad \text{(Ib-2)}$$

wherein each symbol is as defined above, for acylation; and the compound (Ic) or a salt thereof can be produced by reacting a compound represented by the formula

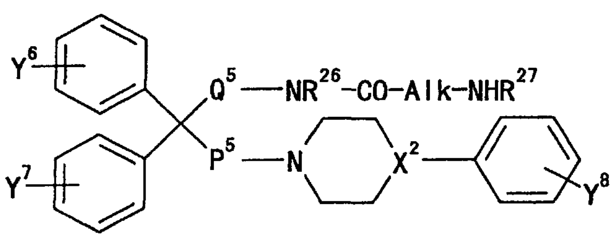

(Ic-1)

wherein each symbol is as defined above, or a salt thereof, with,

(1) when $Alk_2$ is $C_{1-6}$ alkylene group optionally having substituents, a reactive derivative of an organic acid represented by the formula

$$R^{31}\text{-Alk}_3\text{-NR}^{30}\text{-Alk}_2\text{-COOH} \qquad \text{(Ic-2)}$$

wherein each symbol is as defined above, for acylation, or
(2) when $Alk_2$ is a bond, by reacting with a reactive derivative represented by the formula

$$R^{31}\text{-Alk}_3\text{-NR}^{30}\text{-CO-X or } R^{31}\text{-Alk}_3\text{-NCO} \qquad (Ic\text{-}3)$$

wherein X is a leaving group and other symbols are as defined above.

The compounds (Ia-1), (Ia-3), (Ib-1) and (Ic-1) and salts thereof can be produced according to a method known *per se*, such as a method described in, for example, JP-A-8-253447, JP-A-10-81665, JP-A-11-71350 and the like or a similar method.

As the reactive derivative of an organic acid of the formula (Ia-2), (Ib-2) or (Ic-2), acid anhydride, acid halide (e.g., acid chloride, acid bromide and the like), active ester and the like of compound (Ia-2), (Ib-2) or (Ic-2) are used, with preference given to active ester.

As the reactive derivative represented by the formula (Ia-4), aralkyloxycarbonyl halide (e.g., aralkyloxycarbonyl chloride, aralkyloxycarbonyl bromide and the like), carbonate containing aralkyloxy group or an equivalent thereto (e.g., aralkyl phenyl carbonate, aralkyl p-nitrophenyl carbonate, N-((aralkyloxy)carbonyloxy)succinimide, 1-((aralkyloxy)carbonyl)imidazole and the like) and the like are used. Of these, aralkyloxycarbonylchloride and aralkyl p-nitrophenyl carbonate, wherein a leaving group X is chlorine or p-nitrophenyloxy group, are preferable.

As the reactive derivative represented by the formula (Ic-3), carbamic acid halide (e.g., carbamic acid chloride, carbamic acid bromide and the like) having aryl group or aralkyl group at nitrogen, carbamate or equivalent thereto (e.g., phenyl carbamate, p-nitrophenyl carbamate, N-((amino)carbonyloxy)succinimide, 1-((amino)carbonyl)imidazole and the like), isocyanate and the like are used. Of these, isocyanate is preferable.

The acylation and ureide reaction can be carried out according to a known method [e.g., method described in ORGANIC FUNCTIONAL GROUP PREPARATIONS 2nd ed., ACADEMIC PRESS, INC.].

For example, 1 to 5 equivalents, preferably 1 to 3 equivalents, of a reactive derivative of an organic acid represented by the formula (Ia-2), (Ib-2) or (Ic-2), or a reactive derivative represented by (Ia-4) or (Ic-3) and compound (Ia-1), (Ia-3), (Ib-1), (Ic-1) or a salt thereof are reacted in an inert solvent at a reaction temperature of from about -20°C to about 50°C (preferably from about 0°C to at room temperature), for a reaction time of about 5 min to about 100 h.

As the inert solvent, for example, ether solvent, halogen solvent, aromatic solvent, acetonitrile, N,N-dimethylformamide (DMF), acetone, methyl ethyl ketone, dimethyl sulfoxide (DMSO), water and the like can be used alone or in combination. Of these, acetonitrile, tetrahydrofuran, dichloromethane, chloroform and the like are preferable. In addition, by coexistence of 1 to 10 equivalents, preferably 1 to 3 equivalents, of a base may afford more smooth progress of the reaction.

As the base, both inorganic base and organic base are effective. Examples of the inorganic base include hydroxide, hydride, carbonate, hydrogencarbonate, salt with organic acid and the like of alkali metal and alkaline earth metal. Of these, potassium carbonate, sodium carbonate, sodium hydroxide, potassium hydroxide, sodium hydrogencarbonate and potassium hydrogencarbonate are preferable. As the organic base, pyridine, 2,6-lutidine, triethylamine, N,N-diisopropylethylamine and the like are mentioned. Of these, tertiary amines such as triethylamine, N,N-diisopropylethylamine and the like are preferable.

In the case of acylation of carboxylic acid with active ester, moreover, it can be produced by conducting reaction in the presence of 1 to 1.5 equivalents of carboxylic acid and a dehydrative condensation agent (1 to 1.5 equivalents) such as dicyclohexylcarbodiimide (DCC), 1-ethyl-3-(3-dimethylaminopropyl)carbodiimide hydrochloride (WSC) and the like, in an inert solvent (e.g., halogen solvent, acetonitrile, tetrahydrofuran) at about 0°C to at room temperature, for about 0.5 to about 24 h. In this case, the co-presence of 1 to 1.5 equivalents of an activation agent of carboxylic acid, such as N-hydroxysuccinimide (HOSu), 1-hydroxybenztriazole (HOBt), N-hydroxy-5-norbornene-2,3-dicarboxyimide (HONB) and the like, may afford more smooth progress of the reaction.

A compound represented by the formula

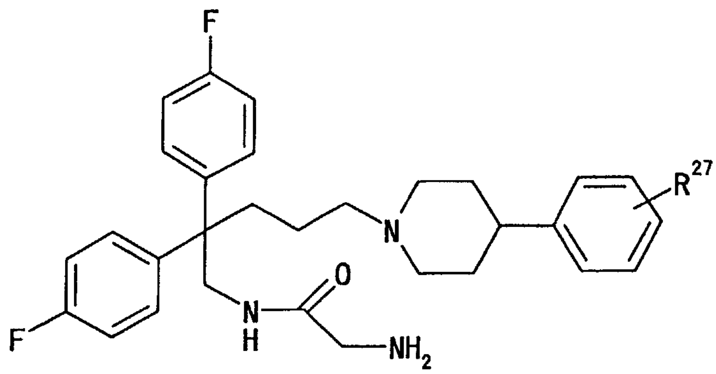

wherein $R^{27}$ is hydrogen atom, halogen atom (e.g., fluorine atom, chlorine atom, bromine atom and the like), optionally halogenated $C_{1-6}$ alkyl group (e.g., methyl, ethyl, propyl m trifluoromethyl and the like) or optionally halogenated $C_{1-6}$ alkoxy group (e.g., methoxy, ethoxy, propoxy, trifluoromethoxy and the like) or a salt thereof is a novel synthetic intermediate for the production of compound (Ib) or a salt thereof. This synthetic intermediate can be produced according to a method known *per se,* such as a method described in, for example, JP-A-8-253447, JP-A-10-81665, JP-A-11-71350 and the like or a similar method.

Inasmuch as the compound (I) or a pharmaceutically acceptable salt thereof has a superior MCH receptor antagonistic action, it is useful as an agent for the prophylaxis or therapy of diseases caused by MCH. In addition, the compound of the present invention shows low toxicity, and superior oral absorption performance and transfer into the brain.

Accordingly, a melanin-concentrating hormone antagonist (hereinafter sometimes to be briefly referred to as MCH antagonist) containing the compound (I) or a pharmaceutically acceptable salt thereof is safely administered as an agent for the prophylaxis or therapy of the diseases caused by MCH to mammals (e.g., rat, mouse, guinea pig, rabbit, sheep, horse, pig, cow, monkey, human and the like).

The diseases caused by MCH include, for example, obesity [e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity and the like], hyperphagia, emotional disorder, sexual dysfunction and the like.

The compound (I) or a pharmaceutically acceptable salt thereof is useful as an agent for the prophylaxis or treatment of life-style related diseases such as diabetes, diabetic complications (e.g., diabetic retinopathy, diabetic neuropathy, diabetic nephropathy and the like), arteriosclerosis, gonarthritis and the like.

Furthermore, the compound or a pharmaceutically acceptable salt thereof is useful as an agent for suppressing food intake.

The MCH antagonist and a pharmaceutical composition of the present invention can be concurrently used with diet therapy (e.g., diet therapy for diabetes and the like), or an exercise therapy.

The MCH antagonist and a pharmaceutical composition of the present invention can be produced by formulating compound (I), (Ia), (Ib), (Ic) or a pharmaceutically acceptable salt thereof as it is or along with a pharmacologically acceptable carrier according to a method known *per se.*

As the pharmacologically acceptable carrier, various organic or inorganic carrier substance conventionally used as a material for preparation, such as excipient, lubricant, binder, disintegrant for solid preparation; solvent, dissolution aids, suspending agent, isotonicity agent, buffer, soothing agent and the like for liquid preparation are mentioned. In formulating a preparation, additives such as preservative, antioxidant, coloring agent, sweetening agent, absorbent, moistening agent and the like can be added as necessary.

Examples of the excipient include lactose, sucrose, D-mannitol, starch, cornstarch, crystalline cellulose, light silicic anhydride and the like.

Examples of the lubricant include magnesium stearate, calcium stearate, talc, colloidal silica and the like.

Examples of the binder include crystalline cellulose, saccharose, D-mannitol, dextrin, hydroxypropyl cellulose, hydroxypropylmethyl cellulose, polyvinylpyrrolidone, starch, sucrose, gelatin, methyl cellulose, carboxymethyl cellulose sodium and the like.

Examples of the disintegrant include starch, carboxymethyl cellulose, carboxymethyl cellulose calcium, crosscarmellose sodium, carboxymethyl starch sodium, low substituted hydroxypropyl cellulose (L-HPC) and the like.

Examples of the solvent include water for injection, alcohol, propylene glycol, macrogol, sesame oil, corn oil and the like.

Examples of the dissolution aids include polyethylene glycol, propylene glycol, D-mannitol, benzyl benzoate, ethanol, tris-aminomethane, cholesterol, triethanolamine, sodium carbonate, sodium citrate and the like.
Examples of the suspending agent include surfactant such as stearyl triethanolamine, sodium lauryl sulfate, lauryl aminopropionic acid, lecithin, benzalkonium chloride, benzethonium chloride, glyceryl monostearate and the like; hydrophilic polymers such as polyvinyl alcohol, polyvinylpyrrolidone, carboxymethyl cellulose sodium, methyl cellulose, hydroxymethylcellulose, hydroxyethyl cellulose, hydroxypropyl cellulose and the like, and the like.
Examples of the isotonicity agent include glucose, D-sorbitol, sodium chloride, glycerine, D-mannitol and the like.
Examples of the buffer include buffers such as phosphate, acetate, carbonate, citrate and the like and the like.
Examples of the soothing agent include benzyl alcohol and the like.
Examples of the preservative include p-oxybenzoic acid esters, chlorobutanol, benzyl alcohol, phenethyl alcohol, dehydroacetic acid, sorbic acid and the like.
Examples of the antioxidant include sulfite, ascorbic acid and the like.
Examples of the dosage form of the MCH antagonist and a pharmaceutical composition of the present invention include oral preparations such as tablet (inclusive of sugar-coated tablet and firm coated tablet), powder, granule, capsule (inclusive of soft capsule), liquid and the like; parenteral preparations such as injection (e.g., subcutaneous injection, intravenous injection, intramuscular injection, intraperitoneal injection and the like), external preparation (e.g., transnasal administration preparation, percutaneous preparation, ointment and the like), suppository (e.g., rectal suppository, pessary and the like), sustained-release preparation (e.g., sustained release microcapsule and the like), pellet, drops and the like; and the like, and can be safely administered orally or parenterally (e.g., topical, rectal, intravenous administration and the like).
The content of compound (I) or a pharmaceutically acceptable salt thereof in the MCH antagonist of the present invention, and the content of compound (Ia), (Ib), (Ic) or a pharmaceutically acceptable salt thereof in the pharmaceutical composition of the present invention is, for example, about 0.1 to 100 wt% of the whole amount of the MCH antagonist or the pharmaceutical composition, respectively.
The dose of the MCH antagonist and the pharmaceutical composition of the present invention is appropriately determined according to the administration subject, administration route, disease and the like.
For example, when the MCH antagonist or the pharmaceutical composition of the present invention is orally administered to adult patients (body weight about 60 kg) with obesity, the daily dose in terms of compound (I), (Ia), (Ib), (Ic) or a pharmaceutically acceptable salt thereof as an active ingredient is about 0.1 to about 500 mg, preferably about 1 to about 100 mg, more preferably about 5 to about 100 mg, which dose is administered once or divided in several times a day.
With the aim of, for example, "enhancement of treatment effect against obesity", "reduction of the amount of MCH antagonist to be used" and the like, the MCH antagonist and the pharmaceutical composition of the present invention may be used along with a combination drug, which does not exert an adverse influence on the MCH antagonist and the pharmaceutical composition of the present invention. As such combination drug, for example, "antidiabetic agent", "diabetic complication treatment agent", "anti-obesity agent other than MCH antagonist", "hypertension treatment agent", "hyperlipidemia treatment agent", "arthritis treatment agent", "anti-anxiety agent", "antidepressant" and the like are mentioned. These combination drugs may be used in an appropriate combination of two or more thereof.
As the above-mentioned "antidiabetic agent", for example, insulin sensitizer, insulin secretagogue, biguanide agent, insulin, $\alpha$-glucosidase inhibitor, $\beta$3 adrenergic receptor agonist and the like are mentioned.
As the insulin sensitizer, for example, pioglitazone or a salt thereof (preferably hydrochloride), troglitazone, rosiglitazone or a salt thereof (preferably maleate), JTT-501, GI-262570, MCC-555, YM-440, DRF-2593, BM-13-1258, KRP-297, R-119702, CS-011 and the like are mentioned.
As the insulin secretagogue, for example, sulfonylurea agent is mentioned. Examples of the sulfonylurea agent include tolbutamide, chlorpropamide, tolazamide, acetohexamide, glyclopyramide and ammonium salt thereof, glibenclamide, gliclazide, glimepiride and the like.
In addition to the above, insulin secretagogue includes, for example, repaglinide, nateglinide, mitiglinide (KAD-1229), JTT-608 and the like.
As the biguanide agent, for example, metformin, buformin, phenformin and the like are mentioned.
As the insulin, for example, animal insulin extracted from pancreas of cow and swine; semi-synthetic human insulin enzymatically synthesized from insulin extracted from pancreas of swine; human insulin genetically synthesized using Escherichia coli or yeast; and the like are mentioned. As insulin, insulin zinc containing 0.45 to 0.9 (w/w)% of zinc; protamine insulin zinc produced from zinc chloride, protamine sulfate and insulin, and the like can be used. Moreover, insulin can be a fragment or derivative thereof (e.g., INS-1 and the like).
While insulin includes various types such as very rapid acting type, short-acting type, biphasic type, intermediate-acting type, extended type and the like, which can be determined depending on the disease state of patients.
As the $\alpha$-glucosidase inhibitor, for example, acarbose, voglibose, miglitol, emiglitate and the like are mentioned.

As the β3 adrenergic receptor agonist, for example, AJ-9677, BMS-196085, SB-226552, AZ40140 and the like are mentioned.

In addition to the above, the "antidiabetic agent" includes, for example, ergoset, pramlintide, leptin, BAY-27-9955 and the like.

As the above-mentioned "diabetes complication treatment agent", for example, aldose reductase inhibitor, glycation inhibitor, protein kinase C inhibitor and the like are mentioned.

As the aldose reductase inhibitor, for example, tolrestat; epalrestat; imirestat; zenarestat; SNK-860; zopolrestat; ARI-509; AS-3201 and the like are mentioned.

As the glycation inhibitor, for example, pimagedine and the like are mentioned.

As the protein kinase C inhibitor, for example, NGF, LY-333531 and the like are mentioned.

In addition to the above, the "diabetes complication treatment agent" includes, for example, alprostadil, tiapride hydrochloride, cilostazol, mexiletine hydrochloride, ethyl icosapentate, memantine, pimagedline (ALT-711) and the like.

As the above-mentioned "anti-obesity agent other than MCH antagonist", for example, lipase inhibitor, anorexiant and the like are mentioned.

As the lipase inhibitor, for example, orlistat and the like are mentioned.

As the anorexiant, for example, mazindol, dexfenfluramine, fluoxetine, sibutramine, biamine and the like are mentioned.

In addition to the above, the "anti-obesity agent other than MCH antagonist" includes, for example, lipstatin and the like.

As the above-mentioned "hypertension treatment agent", for example, angiotensin converting enzyme inhibitor, calcium antagonist, potassium channel opener, angiotensin II antagonist and the like are mentioned.

As the angiotensin converting enzyme inhibitor, for example, captoril, enalapril, alacepril, delapril (HCl), lisinopril, imidapril, benazepril, cilazapril, temocapril, trandolapril, manidipine (HCl) and the like are mentioned.

As the calcium antagonist, for example, nifedipine, amlodipine, efonidipine, nicardipine and the like are mentioned.

As the potassium channel opener, for example, levcromakalim, L-27152, AL 0671, NIP-121 and the like are mentioned.

As the angiotensin II antagonist, for example, losartan, candesartan cilexetil, valsartan, irbesartan, CS-866, E4177 and the like are mentioned.

As the above-mentioned "hyperlipidemia treatment agent", for example, HMG-CoA reductase inhibitor, fibrate compound and the like are mentioned.

As the HMG-CoA reductase inhibitor, for example, pravastatin, simvatatin, lovastatin, atorvastatin, fluvastatin, lipantil, cerivastatin, itavastatin, ZD-4522 or salts thereof (e.g., sodium salt and the like) and the like are mentioned.

As the fibrate compound, for example, bezafibrate, clinofibrate, clofibrate, simfibrate and the like are mentioned.

As the above-mentioned "arthritis treatment agent", for example, ibuprofen and the like are mentioned.

As the above-mentioned "anti-anxiety", for example, chlordiazepoxide, diazepam, oxazolam, medazepam, cloxazolam, bromazepam, lorazepam, alprazolam, fludiazepam and the like are mentioned.

As the above-mentioned "antidepressant", for example, fluoxetine, fluvoxamine, imipramine, paroxetine, sertraline and the like are mentioned.

The time of administration of the aforementioned combination drug is not limited. The MCH antagonist or a pharmaceutical composition and a combination drug may be simultaneously administered to an administration subject or administered in a staggered manner. The dose of the combination drug can be determined according to the dose clinically employed, and can be determined as appropriate depending on the administration subject, administration route, disease, combination and the like.

The mode of administration of the combination drug is not particularly limited, and may be any as long as the MCH antagonist or pharmaceutical composition and combination drug are combined on administration. Such administration mode is exemplified by (1) administration of a single preparation obtained by simultaneously formulating the MCH antagonist or pharmaceutical composition and combination drug(s), (2) simultaneous administration by the same administration route of two kinds of preparations obtained by separately formulating the MCH antagonist or pharmaceutical composition and combination drug(s), (3) staggered administration by the same administration route of two kinds of preparations obtained by separately formulating the MCH antagonist or pharmaceutical composition and combination drug, (4) simultaneous administration by different administration routes of two kinds of preparations obtained by separately formulating the MCH antagonist or pharmaceutical composition and combination drug, (5) staggered administration by different administration routes of two kinds of preparations obtained by separately formulating the MCH antagonist or pharmaceutical composition and combination drug (e.g., administration of MCH antagonist or pharmaceutical composition and combination drug in this order, and administration in the reversed order) and the like.

The admixing ratio of the MCH antagonist or pharmaceutical composition and combination drug can be appropriately determined depending on the administration subject, administration route, disease and the like.

The present invention is explained in detail in the following by referring to Reference Examples, Examples, Formulation Examples and Experimental Examples. The present invention is not limited by these examples, and may be modified as long as it does not deviate from the range of the present invention.

In the following Reference Example and Example, "room temperature" means 0 to 30°C and other definitions are as follows.

| | |
|---|---|
| s | : singlet |
| d | doublet |
| t | : triplet |
| q | quartet |
| m | : multiplet |
| br | : broad |
| brs | : broad singlet |
| ABq | : AB quartet |
| dd | : double doublet |
| J | : coupling constant |
| Hz | : Hertz |
| $CDCl_3$ | : deuterated chloroform |
| THF | : tetrahydrofuran |
| DMF | : N,N-dimethylformamide |
| DMSO | dimethyl sulfoxide |
| $^1$H-NMR : | proton nuclear magnetic resonance (free compound was used for measurement) |

In the present specification and drawings, when base, amino acid and the like are shown using abbreviations, they are based on abbreviations according to IUPAC-IUB Commission on Biochemical Nomenclature and conventional abbreviations employed in this field. Examples thereof are given in the following. When optical isomer is present due to amino acid, it is an L form unless particularly indicated.

| | |
|---|---|
| DNA : | deoxyribonucleic acid |
| cDNA : | complementary deoxyribonucleic acid |
| A : | adenine |
| T : | thymine |
| G : | guanine |
| C : | cytosine |
| RNA : | ribonucleic acid |
| mRNA | : messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP : | adenosine triphosphate |
| EDTA : | ethylenediamine tetraacetic acid |
| SDS : | sodium dodecylsulfate |
| EIA : | enzyme immunoassay |
| Gly : | glycine |
| Ala : | alanine |
| Val : | valine |
| Leu : | leucine |
| Ile : | isoleucine |
| Ser : | serine |
| Thr : | threonine |
| Cys : | cysteine |
| Met : | methionine |
| Glu : | glutamic acid |
| Asp : | aspartic acid |
| Lys : | lysin |

Arg : arginine
His : histidine
Phe : phenylalanine
Tyr : thyrosin
Trp : tryptophan
Pro : proline
Asn : asparagine
Gln : glutamine
pGl : pyroglutamic acid
Me : methyl group
Et : ethyl group
Bu : butyl group
Ph : phenyl group
TC : thiazolidine-4(R)-carboxamide group

The substituent, protecting group and reagent frequently appear in the present specification are shown using the following symbols.

Tos : p-toluenesulfonyl
CHO : formyl
Bzl : benzyl
$Cl_2Bzl$ : 2,6-dichlorobenzyl
Bom : benzyloxymethyl
Z : benzyloxycarbonyl
Cl-Z : 2-chlorobenzyloxycarbonyl
Br-Z 2-bromobenzyloxycarbonyl
Boc : t-butoxycarbonyl
DNP : dinitrophenol
Trt trityl
Bum : t-butoxymethyl
Fmoc : N-9-fluorenylmethoxycarbonyl
HOBt : 1-hydroxybenztriazole
HOOBt : 3,4-dihydro-3-hydroxy-4-oxo-1,2,3-benzotriazine
HONB : N-hydroxy-5-norbornene-2,3-dicarboxyimide
DCC : N,N'-dicyclohexylcarbodiimide
WSC : 1-ethyl-3-(3-dimethylaminopropyl)carbodiimide hydrochloride
DMAP : 4-dimethylaminopyridine
IPE : diisopropyl ether
THF : tetrahydrofuran
DMF : N,N-dimethylformamide

The sequence numbers in the Sequence Listing in the present specification show the following sequences.

[SEQ ID NO:1]

Synthetic DNA used for screening cDNA encoding rat SLC-1.

SEQ ID NO:2]

Synthetic DNA used for screening cDNA encoding rat SLC-1.

[SEQ ID NO:3]

Full length amino acid sequence of rat SLC-1.

[SEQ ID NO:4]

Full length base sequence of rat SLC-1 cDNA comprising Sal I recognition sequence added on the 5' side

and Spe I recognition sequence added on the 3' side.

[SEQ ID NO:5]

Riboprobe used for the determination of expression amount of SLC-1 mRNA in each clone of rat SLC-1 expression CHO cells.

[SEQ ID NO:6]

Synthetic DNA used for obtaining cDNA encoding human SLC-1.

[SEQ ID NO:7]

Primer used to make cDNA encoding human SLC-1 double-stranded.

[SEQ ID NO:8]

Full length base sequence of cDNA encoding human SLC-1.

[SEQ ID NO:9]

Full length amino acid sequence of human SLC-1.

[SEQ ID NO:10]

Synthetic DNA used for screening cDNA encoding human SLC-1(S).

[SEQ ID NO:11]

Synthetic DNA used for screening cDNA encoding human SLC-1(S).

[SEQ ID NO:12]

Synthetic DNA used for screening cDNA encoding human SLC-1(L).

[SEQ ID NO:13]

Synthetic DNA used for screening cDNA encoding human SLC-1(L).

[SEQ ID NO:14]

Full length base sequence of human SLC-1(S) cDNA comprising Sal I recognition sequence added on the 5' side and Spe I recognition sequence added on the 3' side.

[SEQ ID NO:15]

Full length base sequence of human SLC-1(L) cDNA comprising Sal I recognition sequence added on the 5' side and Spe I recognition sequence added on the 3' side.

[SEQ ID NO:16]

Riboprobe used for the determination of expression amount of SLC-1 mRNA in each clone of human SLC-1 (S) expression CHO cells and human SLC-1(L) expression CHO cells.
The transformant *Escherichia coli* DH10B/phSLC1L8 with a plasmid containing DNA encoding the base sequence obtained in Reference Example 6D, which is depicted in SEQ:No. 9 has been deposited at the National Institute of Bioscience and Human-Technology (NIBH) since February 1,1999 under deposit No. FERM BP-6632 and at the Institute for Fermentation, Osaka (IFO) under deposit No. IFO 16254 since January 21, 1999.

**Examples**

The following Reference Examples IA-IVA can be produced according to JP-A-8-253447.

Reference Example IA-1: 1-(5-amino-4,4-diphenylpentyl)-4-phenylpiperidine 1-(5-formamino-4,4-diphenylpentyl)-4-phenylpiperidine

Reference Example IA-2: 3,4-dihydro-6-methoxy-1'- (5-amino-4,4-diphenylpentyl)spiro[naphthalene-2(1H),2'-piperidine]

Reference Example IA-3: 1- [5-amino-4-(4-methoxyphenyl)-4-phenylpentyl]-4-phenylpiperidine

Reference Example IA-4: 1- [5-amino-4,4-bis(4-chlorophenyl)-pentyl]-4-(4-fluorophenyl)piperazine

Reference Example IA-5: 3,4-dihydro-6-methoxy-1'-(6-amino-4,4-diphenylhexyl)spiro[naphthalene-2(1H),2'-piperidine]

Reference Example IA-6: 3,4-dihydro-6,7-dimethoxy-1'-(7-amino-4,4-diphenylheptyl)spiro[naphthalene-2(1H),2'-piperidine]

Reference Example IIA-1: 1-(N,N-dimethylamino)-4,4-diphenyl-5-(formylamino)pentane

Reference Example IIA-2: 1-(N-benzyl-N-methylamino)-4,4-diphenyl-5-(formylamino)pentane hydrochloride

Reference Example IIA-3: 4,4-diphenyl-5-formylamino-1-(morpholino)pentane hydrochloride

Reference Example IIA-4: 4,4-diphenyl-5-formylamino-1-(2,3,4,5-tetrahydro-1H-3-benzazepin-3-yl)pentane hydrochloride

Reference Example IIA-5: 4,4-diphenyl-5-formylamino-1-(4-phenylpiperidino)pentane hydrochloride

Reference Example IIA-6: 1-[4-(4-fluorophenyl)piperazin-1-yl]-5-formylamino-4,4-diphenylpentane dihydrochloride

Reference Example IIA-7: 3,4-dihydro-6-methoxy-1'-(5-formylamino-4,4-diphenylpentyl)spiro[naphthalene-2 (1H) ,2'-piperidine] dihydrochloride

Reference Example IIA-8: 1-benzylamino-4,4-diphenyl-5-(tosylamino)pentane hydrochloride

Reference Example IIA-9: 1-(N-benzyl-N-methylamino)-4,4-diphenyl-5-(tosylamino)pentane hydrochloride

Reference Example IIA-10: 4,4-diphenyl-1-(3-nitrobenzylamino)-5-(tosylamino)pentane hydrochloride

Reference Example IIA-11: 1-(3-aminobenzylamino)-4,4-diphenyl-5-(tosylamino)pentane

Reference Example IIA-12: 4,4-diphenyl-1-[3-(methoxycarbonyl)-benzylamino]-5-(tosylamino)pentane hydrochloride

Reference Example IIA-13: 4,4-diphenyl-1-(2-picolylamino)-5-(tosylamino)pentane dihydrochloride

Reference Example IIA-14: 4,4-diphenyl-1-(1-hexamethyleneimino)-5-(tosylamino)pentane hydrochloride

Reference Example IIA-15: 4,4-diphenyl-1-(4-phenylpiperazin-1-yl)-5-(tosylamino)pentane

Reference Example IIA-16: 4,4-diphenyl-1-[4-(2-methoxyphenyl)-piperazin-1-yl]-5-(tosylamino)pentane hydrochloride

Reference Example IIA-17: 4,4-diphenyl-5-mesylamino-1-(4-phenylpiperidino)pentane hydrochloride

Reference Example IIA-18: 5-benzenesulfonylamino-4,4-diphenyl-1-(4-phenylpiperidino)pentane

Reference Example IIA-19: 4,4-diphenyl-1-(4-phenylpiperidino)-5-(2,4,6-trimethylbenzenesulfonylamino)pentane

Reference Example IIA-20: 4,4-diphenyl-1-(4-phenylpiperidino)-5-(2,4,6-triisopropylbenzenesulfonylamino)pentane

Reference Example IIA-21: 4,4-diphenyl-5-(1-naphthylsulfonylamino)-1-(4-phenylpiperidino)pentane

Reference Example IIA-22: 4,4-diphenyl-5-(2-naphthylsulfonylamino)-1-(4-phenylpiperidino)pentane

Reference Example IIA-23: 3,4-dihydro-6-methoxy-1'-(5-acetylamino-4,4-diphenylpentyl)spiro[naphthalene-2 (1H),2'-piperidine] dihydrochloride

Reference Example IIA-24: 3,4-dihydro-6-methoxy-1'-(5-tosylamino-4,4-diphenylpentyl)spiro[naphthalene-2(1H), 2'-piperidine] dihydrochloride

Reference Example IIA-25: 4-(4-chlorophenyl)-5-formylamino-4-phenyl-1-(4-phenylpiperidino)pentane hydrochloride

Reference Example IIA-26: 4-(4-chlorophenyl)-5-formylamino-4-phenyl-1-(4-phenylpiperazin-1-yl)pentane dihydrochloride

Reference Example IIA-27: 4-(4-chlorophenyl)-1-[4-(4-fluorophenyl)piperazin-1-yl]-5-formylamino-4-phenylpentane 2 hydrochloride

Reference Example IIA-28: 4-(4-chlorophenyl)-1-[4-(diphenylmethyl)piperazin-1-yl]-5-formylamino-4-phenylpentane

Reference Example IIA-29: 5-formylamino-4-(4-methoxyphenyl)-4-phenyl-1-(4-phenylpiperidino)pentane hydrochloride

Reference Example IIA-30: 4-(4-methoxyphenyl)-5-(1-naphthylsulfonylamino)-4-phenyl-1-(4-phenylpiperidino) pentane hydrochloride

Reference Example IIA-31: 4,4-bis(4-chlorophenyl)-1-[4-(4-fluorophenyl)piperazin-1-yl]-5-(formylamino)pentane dihydrochloride

Reference Example IIA-32: 4,4-bis(4-chlorophenyl)-1- [4-(4-fluorophenyl)piperazin-1-yl]-5-(mesylamino)pentane dihydrochloride

Reference Example IIA-33: 4,4-bis(4-chlorophenyl)-1-[4-(4-fluorophenyl)piperazin-1-yl]-5-(tosylamino)pentane dihydrochloride

Reference Example IIA-34: 1-[4-(4-fluorophenyl)piperazin-1-yl]-6-formylamino-5,5-diphenylhexane dihydrochloride

Reference Example IIA-35: 1-[4-(4-fluorophenyl)piperazin-1-yl]-6-formylamino-4,4-diphenylhexane dihydrochloride

Reference Example IIA-36: 4,4-diphenyl-1-(4-phenylpiperidino]-6-(tosylamino)hexane hydrochloride

Reference Example IIA-37: 3,4-dihydro-6-methoxy-1'-(6-acetylamino-4,4-diphenylhexyl)spiro[naphthalene-2(1H), 2'-piperidine] dihydrochloride

Reference Example IIA-38: 3,4-dihydro-6-methoxy-1'-(6-tosylamino-4,4-diphenylhexyl)spiro[naphthalene-2(1H), 2'-piperidine] hydrochloride

Reference Example IIA-39: 3,4-dihydro-6-methoxy-1'- (6-benzylamino-4,4-diphenylhexyl)spiro[naphthalene-2 (1H),2'-piperidine] dihydrochloride

Reference Example IIA-40: 7-acetylamino-4,4-diphenyl-1-[3-(methoxycarbonyl)benzylamino]heptane dihydrochloride

Reference Example IIA-41: 7-acetylamino-4,4-diphenyl-1-(β-phenethylamino)heptane dihydrochloride

Reference Example IIA-42: 7-acetylamino-1-[2-(6,7-dimethoxy-1,2,3,4-tetrahydronaphthylamino)]-4,4-diphenylheptane dihydrochloride

Reference Example IIA-43: 7-acetylamino-1-{N-benzyl-N-[2-(6,7-dimethoxy-1,2,3,4-tetrahydronaphthyl)]amino}-4,4-diphenylheptane dihydrochloride

Reference Example IIA-44: 1'-(7-acetylamino-4,4-diphenylheptyl)-3,4-dihydro-8-methoxyspiro[naphthalene-2 (1H),2'-piperidine] dihydrochloride

Reference Example IIA-45: 1'- (7-acetylamino-4,4-diphenylheptyl)-3,4-dihydro-6-methoxyspiro [naphthalene-2 (1H),2'-piperidine] dihydrochloride

Reference Example IIA-46: 1'-(7-acetylamino-4,4-diphenylheptyl)-3,4-dihydro-6,7-dimethoxyspiro[naphthalene-2 (1H),2'-piperidine] dihydrochloride

Reference Example IIA-47: 1'- [7- (cyclohexylacetyl)amino-4,4-diphenylheptyl]-3,4-dihydro-6,7-dimethoxyspiro [naphthalene-2(1H),2'-piperidine] hydrochloride

Reference Example IIA-48: 3,4-dihydro-6,7-dimethoxy-1'-[4,4-diphenyl-7-(phenylacetylamino)heptyl]spiro[naphthalene-2(1H),2'-piperidine] hydrochloride

Reference Example IIA-49: 3,4-dihydro-6,7-dimethoxy-1'-{4,4-diphenyl-7-[(2-fluorophenylacetyl)amino]heptyl}-spiro[naphthalene-2(1H),2'-piperidine] hydrochloride

Reference Example IIA-50: 3,4-dihydro-6,7-dimethoxy-1'-{4,4-diphenyl-7-[(4-fluorophenylacetyl)amino]heptyl}-spiro[naphthalene-2(1H),2'-piperidine] hydrochloride

Reference Example IIA-51: 3,4-dihydro-6,7-dimethoxy-1'-{4,4-diphenyl-7-[(4-chlorophenylacetyl)amino]heptyl}-spiro[naphthalene-2(1H),2'-piperidine] hydrochloride

Reference Example IIA-52: 3,4-dihydro-6,7-dimethoxy-1'-{4,4-diphenyl-7-[(3-nitrophenylacetyl)amino]heptyl)-spiro[naphthalene-2(1H),2'-piperidine] hydrochloride

Reference Example IIA-53: 3,4-dihydro-6,7-dimethoxy-1'-{4,4-diphenyl-7-[(4-nitrophenylacetyl)amino]heptyl}-spiro[naphthalene-2(1H),2'-piperidine] hydrochloride

Reference Example IIA-54: 3,4-dihydro-6,7-dimethoxy-1'-{4,4-diphenyl-7-[(4-methylphenylacetyl)amino]heptyl)-spiro[naphthalene-2(1H),2'-piperidine] hydrochloride

Reference Example IIA-55: 3,4-dihydro-6,7-dimethoxy-1'-{4,4-diphenyl-7-[(4-trifluoromethylphenylacetyl)amino] heptyl}-spiro[naphthalene-2(1H),2'-piperidine] hydrochloride

Reference Example IIA-56: 3,4-dihydro-6,7-dimethoxy-1'-{4,4-diphenyl-7-[(2-methoxyphenylacetyl)amino]heptyl}-spiro[naphthalene-2(1H),2'-piperidine] hydrochloride

Reference Example IIA-57: 3,4-dihydro-6,7-dimethoxy-1'-{4,4-diphenyl-7-[(3-methoxyphenylacetyl)amino]heptyl}-spiro[naphthalene-2(1H),2'-piperidine] hydrochloride

Reference Example IIA-58: 3,4-dihydro-6,7-dimethoxy-1'-{4,4-diphenyl-7-[(4-methoxyphenylacetyl)amino]heptyl}-spiro[naphthalene-2(1H),2'-piperidine] hydrochloride

Reference Example IIA-59: 3,4-dihydro-6,7-dimethoxy-1'-{7-[(3,4-dimethoxyphenylacetyl)amino]-4,4-diphenylheptyl}-spiro[naphthalene-2(1H),2'-piperidine] hydrochloride

Reference Example IIA-60: 3,4-dihydro-6,7-dimethoxy-1'-{4,4-diphenyl-7-[(3,4-methylenedioxyphenylacetyl)amino]heptyl}-spiro[naphthalene-2(1H),2'-piperidine] hydrochloride

Reference Example IIA-61: 3,4-dihydro-6,7-dimethoxy-1'-{4,4-diphenyl-7-[(phenoxyacetyl)amino]heptyl}spiro [naphthalene-2(1H),2'-piperidine] hydrochloride
Reference Example IIA-62: 3,4-dihydro-6,7-dimethoxy-1'-{4,4-diphenyl-7-[(2-thienylacetyl) amino]heptyl}spiro [naphthalene-2(1H),2'-piperidine] hydrochloride
Reference Example IIA-63: 3,4-dihydro-6,7-dimethoxy-1'-{4,4-diphenyl-7-[(3-thienylacetyl)amino]heptyl}spiro [naphthalene-2(1H),2'-piperidine] hydrochloride
Reference Example IIA-64: 3,4-dihydro-6,7-dimethoxy-1'-{4,4-diphenyl-7-[(3-phenylpropionyl)amino]heptyl}spiro [naphthalene-2(1H),2'-piperidine] hydrochloride
Reference Example IIA-65: 3,4-dihydro-6,7-dimethoxy-1'-{4,4-diphenyl-7-{[3-(4-methoxyphenyl)propionyl]amino} heptyl}-spiro[naphthalene-2(1H),2'-piperidine] hydrochloride
Reference Example IIA-66: 3,4-dihydro-6,7-dimethoxy-1'-{4,4-diphenyl-7-[(1-naphthylacetyl)amino]heptyl}spiro [naphthalene-2(1H),2'-piperidine] hydrochloride
Reference Example IIA-67: 3,4-dihydro-6,7-dimethoxy-1'-{4,4-diphenyl-7-[(2-naphthylacetyl) amino]heptyl}spiro [naphthalene-2(1H),2'-piperidine] hydrochloride
Reference Example IIA-68: 3,4-dihydro-6,7-dimethoxy-1'-{4,4-bis(4-fluorophenyl)-7-[(4-methoxyphenylacetyl) amino]heptyl}-spiro[naphthalene-2(1H),2'-piperidine] hydrochloride
Reference Example IIIA-1:

(1) 4,4-diphenyl-5-hydroxy-6-heptenenitrile
(2) 7-(6,7-dimethoxy-1,2,3,4-tetrahydronaphthalene-2-spiro-2'-piperidin-1'-yl)-4,4-diphenyl-5-heptenenitrile hydrochloride
(3) 1-(7-amino-4,4-diphenyl-2-heptenyl)-6',7'-dimethoxy-1',2',3',4'-tetrahydronaphthalene-2'-spiro-2-piperidine

Reference Example IIIA-2: N-(7-(6,7-dimethoxy-1,2,3,4-tetrahydronaphthalene-2-spiro-2'-piperidine-1'-yl)-4,4-diphenyl-5-heptenyl)-3-(4-methoxyphenyl)propionamide hydrochloride
Reference Example IVA-1: 4,4-diphenyl-1-[(6-methoxy-1,2,3,4-tetrahydro-2-naphthyl)amino]-7-{[3-(4-methoxyphenyl)-propionyl]amino}heptane hydrochloride
Reference Example IVA-2: 4,4-diphenyl-1-[3-(4-methoxyphenyl)piperidino]-7-{[3-(4-methoxyphenyl)-propionyl] amino}heptane hydrochloride
Reference Example IVA-3: 4,4-diphenyl-1-(4-phenylpiperidino)-7-[(3-phenylpropionyl)amino]heptane hydrochloride
Reference Example IVA-4: 4,4-diphenyl-1-[4-(3-methoxyphenyl)-piperidino]-7-[(3-phenylpropionyl)amino]heptane hydrochloride
Reference Example IVA-5: 4,4-diphenyl-1-[4-(4-methoxyphenyl)piperidino]-7-{[3-(4-methoxyphenyl)propionyl]-amino}heptane hydrochloride
Reference Example IVA-6: 4,4-diphenyl-7-{[3-(4-methoxyphenyl)-propionyl]amino}-1-[2,3,4,5-tetrahydro-3(1H)-benzazepin-3-yl]heptane hydrochloride
Reference Example IVA-7: 1-[7-acetyl-2,3,4,5-tetrahydro-3(1H)-benzazepin-3-yl]-4,4-diphenyl-7-{[3-(4-methoxyphenyl)-propionyl]amino}heptane hydrochloride
Reference Example IVA-8: 4,4-diphenyl-1-(7,8-dimethoxy-2,3,4,5-tetrahydro-3(1H)-benzazepin-3-yl)-7-{[3-(4-methoxyphenyl)-propionyl]amino)heptane hydrochloride
Reference Example IVA-9: 1-(8,9-dimethoxy-6,6-dimethyl-1,2,3,4,5,6-hexahydro-3-benzazocin-3-yl)-4,4-diphenyl-7-{[3-(4-methoxyphenyl)propionyl]amino}heptane hydrochloride
Reference Example IVA-10: 4,4-diphenyl-7-{[3-(4-methoxyphenyl)-propionyl]amino}-1-(cis-1,2,3,4,4a,9,10,10a-octahydrobenzo [f] quinolin-1-yl)heptane hydrochloride
Reference Example IVA-11: 1-(3-aza-6-methyl-1,1a,2,3,4,4a-hexahydro-9-fluorenon-3-yl)-4,4-diphenyl-7-{[3-(4-methoxyphenyl)propionyl]amino}heptane hydrochloride
Reference Example IVA-12: 3,4-dihydro-1'-(4,4-diphenyl-7-{(3-(4-methoxyphenyl)propionyl]amino}heptyl}spiro [naphthalene-2(1H),2'-pyrrolidine] hydrochloride
Reference Example IVA-13: 3,4-dihydro-6-methoxy-1'-{4,4-diphenyl-7-{[3-(4-methoxyphenyl)propionyl]amino} heptyl}-spiro[naphthalene-2(1H),2'-piperidine] dihydrochloride
Reference Example IVA-14: 6-ethoxy-3,4-dihydro-1'-{4,4-diphenyl-7-{[3-(4-methoxyphenyl)propionyl]amino}heptyl}-spiro[naphthalene-2(1H),2'-piperidine] hydrochloride
Reference Example IVA-15: 3,4-dihydro-6,7-dimethoxy-1'-{4,4-diphenyl-7-{[3-(4-dimethylaminophenyl)propionyl] amino}heptyl}-spiro[naphthalene-2(1H),2'-piperidine] hydrochloride
Reference Example IVA-16: 3,4-dihydro-6,7-dimethoxy-1'-{4,4-diphenyl-7-{[3-(4-fluorophenyl)propionyl]amino} heptyl}-spiro[naphthalene-2(1H),2'-piperidine] hydrochloride

Reference Example IVA-17: 3,4-dihydro-6,7-dimethoxy-1'-{7-{[3-(4-chlorophenyl)propionyl]amino)-4,4-diphenylheptyl}-spiro[naphthalene-2(1H),2'-piperidine] hydrochloride
Reference Example IVA-18: 3,4-dihydro-6,7-dimethoxy-1'-{7-{[3-(3,5-difluorophenyl)propionyl]amino)-4,4-diphenylheptyl}-spiro[naphthalene-2(1H),2'-piperidine] hydrochloride
Reference Example IVA-19: 3,4-dihydro-6,7-dimethoxy-1'-{4,4-diphenyl-7-{[3-(4-pyridyl)propionyl]amino}heptyl}-spiro[naphthalene-2(1H),2'-piperidine] dihydrochloride
Reference Example IVA-20: 3,4-dihydro-6,7-dimethoxy-1'-{4,4-diphenyl-7-([2-(5-methoxyindane)carbonyl]amino}heptyl}-spiro[naphthalene-2(1H),2'-piperidine] hydrochloride
Reference Example IVA-21: 3,4-dihydro-6,7-dimethoxy-1'-(4,4-diphenyl-7-{[3-(3,4-methylenedioxyphenyl)propionyl]amino}-heptyl)spiro [naphthalene-2(1H),2'-piperidine] hydrochloride
Reference Example IVA-22: 3,4-dihydro-6,7-dimethoxy-1'-(4,4-diphenyl-7-{[3-(4-methoxyphenyl)propionyl]amino}heptyl}-spiro [naphthalene-2(1H),2'-piperidine]-1-one hydrochloride
Reference Example IVA-23: 3,4-dihydro-6-methoxy-5-nitro-1'-{4,4-diphenyl-7-{[3-(4-methoxyphenyl)propionyl]amino}heptyl}-spiro[naphthalene-2(1H),2'-piperidine] hydrochloride
Reference Example IVA-24: 3,4-dihydro-6-methoxy-7-nitro-1'-{4,4-diphenyl-7-{[3-(4-methoxyphenyl)propionyl]amino}heptyl}-spiro[naphthalene-2(1H),2'-piperidine] hydrochloride
Reference Example IVA-25: 7-amino-3,4-dihydro-6-methoxy-1'-{4,4-diphenyl-7-{[3-(4-methoxyphenyl)propionyl]amino}heptyl}-spiro [naphthalene-2(1H),2'-piperidine] dihydrochloride
Reference Example IVA-26: 7-acetylamino-3,4-dihydro-6-methoxy-1'-{4,4-diphenyl-7-{[3-(4-methoxyphenyl)propionyl]amino}-heptyl}spiro[naphthalene-2(1H),2'-piperidine] hydrochloride
Reference Example IVA-27: 7-acetyl-3,4-dihydro-6-methoxy-1'-{4,4-diphenyl-7-{[3-(4-methoxyphenyl)propionyl]amino}heptyl}-spiro[naphthalene-2(1H),2'-piperidine] hydrochloride
Reference Example IVA-28: 3,4-dihydro-6,7-methylenedioxy-1'-{4,4-diphenyl-7-{[3-(4-methoxyphenyl)propionyl]amino}heptyl}-spiro [naphthalene-2(1H) ,2'-piperidine] hydrochloride
Reference Example IVA-29: 6,7-diethoxy-3,4-dihydro-1'-{4,4-diphenyl-7-{[3-(4-methoxyphenyl)propionyl] amino}heptyl}-spiro [naphthalene-2(1H),2'-piperidine] hydrochloride
Reference Example IVA-30: 3,4-dihydro-1'-{4,4-diphenyl-7-{[3-(4-methoxyphenyl)propionyl]amino}heptyl}spiro [naphthalene-2(1H),2'-hexamethylenimine] hydrochloride
Reference Example IVA-31: (+)-3,4-dihydro-6-methoxy-1'-{4,4-diphenyl-7-[(4-methoxyphenylacetyl)amino]heptyl}-spiro[naphthalene-2(1H),2'-piperidine] hydrochloride
Reference Example IVA-32: (-)-3,4-dihydro-6-methoxy-1'-{4,4-diphenyl-7-[(4-methoxyphenylacetyl)amino]heptyl}-spiro[naphthalene-2(1H),2'-piperidine] hydrochloride
Reference Example IVA-33: (-)-3,4-dihydro-6,7-dimethoxy-1'-{4,4-diphenyl-7-[(4-methoxyphenylacetyl)amino]heptyl}-spiro[naphthalene-2(1H),2'-piperidine]-1-one hydrochloride
Reference Example IVA-34: (-)-3,4-dihydro-6,7-dimethoxy-1'-{4,4-diphenyl-7-[(4-fluorophenylacetyl)amino]heptyl}-spiro [naphthalene-2 (1H),2'-piperidine] hydrochloride
Reference Example IVA-35: (+)-3,4-dihydro-6,7-dimethoxy-1'-{4,4-diphenyl-7-[(4-fluorophenylacetyl)amino]heptyl}-spiro[naphthalene-2(1H),2'-piperidine] hydrochloride
Reference Example IVA-36: (-)-3,4-dihydro-6,7-dimethoxy-1'-{4,4-diphenyl-7-{[3-(4-fluorophenyl)propionyl]amino}heptyl}-spiro[naphthalene-2(1H),2'-piperidine] hydrochloride
Reference Example IVA-37: (+)-3,4-dihydro-6,7-dimethoxy-1'-{4,4-diphenyl-7-{[3-(4-fluorophenyl)propionyl]amino}heptyl}-spiro[naphthalene-2(1H),2'-piperidine] hydrochloride
Reference Example IVA-38: (+)-3,4-dihydro-6,7-dimethoxy-1'-{7-{[3-(4-chlorophenyl)propionyl]amino}-4,4-diphenylheptyl}-spiro[naphthalene-2(1H),2'-piperidine] hydrochloride
Reference Example IVA-39: (-)-3,4-dihydro-6,7-dimethoxy-1'-{7-{[3-(4-chlorophenyl)propionyl]amino}-4,4-diphenylheptyl}-spiro[naphthalene-2(1H),2'-piperidine] hydrochloride
Reference Example IVA-40: (-)-3,4-dihydro-6,7-dimethoxy-1'-{4,4-diphenyl-7-{[3-(4-methoxyphenyl)propionyl]amino}heptyl}-spiro[naphthalene-2(1H),2'-piperidine] hydrochloride
Reference Example IVA-41: (+)-3,4-dihydro-6,7-dimethoxy-1'-{4,4-diphenyl-7-{[3-(4-methoxyphenyl)propionyl]amino}heptyl}-spiro[naphthalene-2(1H),2'-piperidine] hydrochloride
Reference Example IVA-42: 3,4-dihydro-4'-{4,4-diphenyl-7-{[3-(4-methoxyphenyl)propionyl]amino}heptyl}spiro [naphthalene-2(1H),3'-morpholine] hydrochloride
Reference Example IVA-43: 3,4-dihydro-7-methoxy-4'-{4,4-diphenyl-7-[3-(4-methoxyphenyl)propionylamino]heptyl}-spiro [naphthalene-2(1H),3'-morpholine] hydrochloride
Reference Example IVA-44: 3,4-dihydro-6,7-dimethoxy-4'-{4,4-diphenyl-7-[3-(4-methoxyphenyl) propionylamino]heptyl}-spiro[naphthalene-2(1H),3'-morpholine] hydrochloride
Reference Example IVA-45: 3,4-dihydro-6,7-dimethoxy-4'-methyl-1'-{4,4-diphenyl-7-[3-(4-methoxyphenyl)propionylamino]heptyl}-spiro[naphthalene-2(1H),2'-piperazine] dihydrochloride

Reference Examples 1B-40B can be produced according to JP-A-10-81665.

Reference Example 1B-1: 5-[4-(4-chlorophenyl)-4-hydroxypiperidino]-1-formylamino-2,2-diphenylpentane hydrochloride
Reference Example 1B-2: 5-[4-(4-fluorophenyl)piperazin-1-yl]-1-formylamino-2,2-diphenylpentane dihydrochloride
Reference Example 1B-3: 1-formylamino-5-(4-hydroxy-4-phenylpiperidino)-2,2-diphenylpentane hydrochloride
Reference Example 1B-4: 5-[4-(4-trifluoromethylphenyl)-4-hydroxypiperidino]-1-formylamino-2,2-diphenylpentane hydrochloride
Reference Example 1B-5: 5-[4-[3,5-bis(trifluoromethyl)phenyl]-4-hydroxypiperidino]-1-formylamino-2,2-diphenylpentane hydrochloride
Reference Example 1B-6: 5-[4-(3,5-dichlorophenyl)-4-hydroxypiperidino]-1-formylamino-2,2-diphenylpentane hydrochloride
Reference Example 1B-7: 5-[4-(4-chlorophenyl)-1,2,3,6-tetrahydropyridin-1-yl]-1-formylamino-2,2-diphenylpentane hydrochloride
Reference Example 1B-8: 1-formylamino-2,2-diphenyl-5-(4-phenylpiperidino)pentane
Reference Example 1B-9: 5-[4-(4-chlorophenyl)piperidino]-1-formylamino-2,2-diphenylpentane hydrochloride
Reference Example 1B-10: 7-[4-(4-chlorophenyl)-4-hydroxypiperidino]-1-formylamino-4,4-diphenylheptane hydrochloride
Reference Example 2B-1: 5-[4-(4-fluorophenyl)-4-hydroxypiperidino]-1-formylamino-2,2-diphenylpentane hydrochloride
Reference Example 2B-2: 1-formylamino-5-[4-hydroxy-4-(4-methoxyphenyl)piperidino]-2,2-diphenylpentane hydrochloride
Reference Example 2B-3: 1-formylamino-5- [4-hydroxy-4-(2-pyridyl)piperidino]-2,2-diphenylpentane dihydrochloride
Reference Example 3B-1: 1-acetylamino-5-[4-(4-chlorophenyl) -4-hydroxypiperidino]-2,2-diphenylpentane hydrochloride
Reference Example 3B-2: 1-acetoacetylamino-5-[4-(4-chlorophenyl)-4-hydroxypiperidino]-2,2-diphenylpentane hydrochloride
Reference Example 3B-3: ethyl N- [5- [4- (4-chlorophenyl) -4-hydroxypiperidino]-2,2-diphenylpentyl]succinamate hydrochloride
Reference Example 3B-4: N- [5- [4- (4-chlorophenyl) -4-hydroxypiperidino]-2,2-diphenylpentyl]succinamic acid
Reference Example 3B-5: 1-[5-[4-(4-chlorophenyl) -4-hydroxypiperidino]-2,2-diphenylpentyl]-3-ethylurea
Reference Example 3B-6: N-[5-[4-(4-chlorophenyl)-4-hydroxypiperidino]-2,2-diphenylpentyl]methanesulfonamide hydrochloride
Reference Example 3B-7: phenyl N-[5-[4-(4-chlorophenyl)-4-hydroxypiperidino]-2,2-diphenylpentyl]carbamate
Reference Example 3B-8: 1-acetylamino-5-[4-(4-chlorophenyl)-4-hydroxypiperidino]-2-phenyl-2-(2-pyridyl)pentane dihydrochloride
Reference Example 3B-9: ethyl N-[5-[4-(4-chlorophenyl)-4-hydroxypiperidino]-2,2-diphenylpentyl]oxamidate hydrochloride
Reference Example 3B-10: ethyl N-[5-[4-(4-chlorophenyl)-4-hydroxypiperidino]-2,2-diphenylpentyl]malonamate hydrochloride
Reference Example 3B-11: ethyl N-[5-[4-(4-chlorophenyl)-4-hydroxypiperidino]-2,2-diphenylpentyl]glutarmate
Reference Example 3B-12: ethyl N-[5-[4-(4-chlorophenyl)-4-hydroxypiperidino]-2-phenyl-2-(2-pyridyl)pentyl]succinamate dihydrochloride
Reference Example 4B-1: 1-[5-[4-(4-chlorophenyl)-4-hydroxypiperidino]-2,2-diphenylpentyl]-3-pentamethyleneurea hydrochloride
Reference Example 4B-2: 1-[5-[4-(4-chlorophenyl)-4-hydroxypiperidino]-2,2-diphenylpentyl]-3-(3-hydroxypropyl) urea hydrochloride
Reference Example 4B-3: 1-[5- [4-(4-chlorophenyl)-4-hydroxypiperidino]-2,2-diphenylpentyl]-3-(4-hydroxybutyl) urea hydrochloride
Reference Example 4B-4: ethyl 3-[3-[5-[4-(4-chlorophenyl)-4-hydroxypiperidino]-2,2-diphenylpentyl]ureido]propionate
Reference Example 4B-5: 1-[5-[4-(4-chlorophenyl)-4-hydroxypiperidino]-2,2-diphenylpentyl]-3-(2-dimethylaminoethyl)urea
Reference Example 4B-6: 1-[5-[4-(4-chlorophenyl)-4-hydroxypiperidino]-2,2-diphenylpentyl]-3-(3-diethylaminopropyl)urea
Reference Example 4B-7: 1-[5-[4-(4-chlorophenyl)-4-hydroxypiperidino]-2,2-diphenylpentyl]-3-[3-(2-pyrrolidone-1-yl)propyl]urea
Reference Example 4B-8: 1-[5-[4-(4-chlorophenyl)-4-hydroxypiperidino]-2,2-diphenylpentyl]-3-(2-piperidinoethyl) urea
Reference Example 4B-9: 2-[3-[5-[4-(4-chlorophenyl)-4-hydroxypiperidino]-2,2-diphenylpentyl]ureido]ethanesulfonamide hydrochloride
Reference Example 4B-10: 2-[3-[5- [4-(4-chlorophenyl)-4-hydroxypiperidino]-2,2-diphenylpentyl]ureido] ethanesulfonic acid
Reference Example 5B-1: N-[5-[4-(4-chlorophenyl)-4-hydroxypiperidino]-2,2-diphenylpentyl]succinamic acid
Reference Example 5B-2: N-[5-[4-(4-chlorophenyl)-4-hydroxypiperidino]-2,2-diphenylpentyl]oxamic acid
Reference Example 5B-3: N-[5-[4-(4-chlorophenyl)-4-hydroxypiperidino]-2,2-diphenylpentyl]malonamic acid
Reference Example 5B-4: N-[5-[4-(4-chlorophenyl)-4-hydroxypiperidino]-2,2-diphenylpentyl]glutaramic acid
Reference Example 5B-5: N-[5-[4-(4-chlorophenyl)-4-hydroxypiperidino]-2-phenyl-2-(2-pyridyl)pentyl]succinamic acid
Reference Example 6B-1: N-[5-[4-(4-chlorophenyl)-4-hydroxypiperidino]-2,2-diphenylpentyl]glycine ethyl ester dihydrochloride
Reference Example 6B-2: ethyl N-[5-[4-(4-chlorophenyl)-4-hydroxypiperidino]-2,2-diphenylpentyl]-4-aminobutyrate dihydrochloride
Reference Example 7B-1: N-[5-[4-(4-chlorophenyl)-4-hydroxypiperidino]-2,2-diphenylpentyl]glycine
Reference Example 7B-2: N-[5-[4-(4-chlorophenyl)-4-hydroxypiperidino]-2,2-diphenylpentyl]-4-aminobutyric acid
Reference Example 8B-1: N-[5-[4-(4-chlorophenyl)-4-hydroxypiperidino] -2,2-diphenylpentyl]-3-(3-hydroxypyrrolidin-1-yl)propanamide
Reference Example 8B-2: 5-[4-(4-chlorophenyl)-4-hydroxypiperidino]-2,2-diphenyl-1-(3-pyrrolidin-1-yl-propionylamino)pentane
Reference Example 8B-3: 5-[4-(4-chlorophenyl)-4-hydroxypiperidino]-1-[3-(dimethylamino)propionylamino]-2,2-diphenylpentane
Reference Example 9B: N-[5-[4-(4-chlorophenyl)-4-hydroxypiperidino]-2,2-diphenylpentyl]-3-(t-butoxycarbonyl)-aminopropanamide
Reference Example 10B: N-[5-[4-(4-chlorophenyl)-4-hydroxypiperidino]-2,2-diphenylpentyl]-3-aminopropanamide dihydrochloride
Reference Example 11B: N-[5-[4-(4-chlorophenyl)-4-hydroxypiperidino]-2,2-diphenylpentyl]-3-(acetylamino)-propanamide
Reference Example 12B: N-[5-[4-(4-chlorophenyl)-4-hydroxypiperidino]-2,2-diphenylpentyl]-3-(propionylamino) propanamide
Reference Example 13B: 1-[4,4-diphenyl-5-(phenyloxycarbonylamino)pentanoyl]-4-(4-chlorophenyl)-4-hydroxypiperidine
Reference Example 14B: 1-[5- [4-(4-chlorophenyl)-4-hydroxypiperidino]-2,2-diphenyl-5-oxopentyl]-3-[3-(hydroxy)-propyl]urea
Reference Example 15B: 1-[5-[4-(4-chlorophenyl)-4-hydroxypiperidino]-2,2-diphenyl-5-oxopentyl]-3-[3-(dimethylamino)ethyl)urea
Reference Example 16B: 1-(5-acetylamino-4,4-diphenylpentanoyl)-4-(4-chlorophenyl)-4-hydroxypiperidine
Reference Example 17B: ethyl N-[5-[4-(4-chlorophenyl)-4-hydroxypiperidino]-2,2-diphenyl-5-oxopentyl]succinamate
Reference Example 18B: N- [5- [4-(4-chlorophenyl)-4-hydroxypiperidino]-2,2-diphenyl-5-oxopentyl]succinamic acid
Reference Example 19B: 1-[5- [4-(4-chlorophenyl)-4-hydroxypiperidino]-2,2-diphenyl-5-oxopentyl]-3- [3-(2-oxo-1-pyrrolidino)propyl]urea
Reference Example 20B: 5-[3-(4-chlorophenyl)-3-hydroxypyrrolidin-1-yl]-2,2-diphenyl-1-formylpentanamine
Reference Example 21B: 1-[5-[4-(4-chlorophenyl)-3-hydroxypiperidino]-2,2-diphenylpentyl]-3-[3-(hydroxy)-propyl] urea
Reference Example 22B: 1-formylamino-[5-[4-hydroxy-4-(4-chlorophenyl)hexamethylenimin-1-yl]-2,2-diphenylpentane hydrochloride
Reference Example 23B: 5- [4-(4-chlorophenyl)-4-hydroxypiperidino]-1-formylamino-2-phenyl-2-(2-thienyl)pentane hydrochloride
Reference Example 24B: 2,2-bis(4-chlorophenyl)-5-[4-(4-chlorophenyl)-4-hydroxypiperidino]-1-formylaminopentane hydrochloride
Reference Example 25B: ethyl N- [2,2-bis(4-chlorophenyl)-5-[4-(4-chlorophenyl)-4-hydroxypiperidino]]pentylsuccinamate hydrochloride
Reference Example 26B: N- [2,2-bis(4-chlorophenyl)-5-[4-(4-chlorophenyl)-4-hydroxypiperidino]]pentylsuccinamic acid
Reference Example 27B-1: 1-[5-[4-(4-chlorophenyl)-4-hydroxypiperidino]-2,2-diphenylpentyl]-3-[(1-ethoxycarbonyl)piperidin-4-yl]urea
Reference Example 27B-2: 1-[5-[4-(4-chlorophenyl)-4-hydroxypiperidino]-2,2-diphenylpentyl]-3-[2-(1-pyrrolidino) ethyl]urea
Reference Example 27B-3: 1-[5-[4-(4-chlorophenyl)-4-hydroxypiperidino]-2,2-diphenylpentyl]-3-[2-(diethylamino) ethyl]urea
Reference Example 27B-4: 1-[5- [4-(4-chlorophenyl)-4-hydroxypiperidino]-2,2-diphenylpentyl]-3-(3-aminopropyl)-3-methylurea
Reference Example 27B-5: 1-[5-[4-(4-chlorophenyl)-4-hydroxypiperidino]-2,2-diphenylpentyl]-3-(5-hydroxypentyl) urea
Reference Example 27B-6: 1-[5-[4-(4-chlorophenyl)-4-hydroxypiperidino]-2,2-diphenylpentyl]-3-[2-(dimethylamino)-ethyl]-3-methylurea
Reference Example 27B-7: 1-[5-[4-(4-chlorophenyl)-4-hydroxypiperidino]-2,2-diphenylpentyl]-3-[2-(methylamino) ethyl]-3-methylurea
Reference Example 27B-8: 1-[5-[4-(4-chlorophenyl)-4-hydroxypiperidino]-2,2-diphenylpentyl]-3-(2-hydroxyethyl)-3-methylurea
Reference Example 27B-9: 1-[5-[4-(4-chlorophenyl)-4-hydroxypiperidino]-2,2-diphenylpentyl]-3-[2-(acetylamino)-ethyl]urea
Reference Example 27B-10: ethyl 4-[5-[4-(4-chlorophenyl)-4-hydroxypiperidino]-2,2-diphenylpentyl]ureidobutyrate
Reference Example 27B-11: 1-[5-[4-(4-chlorophenyl)-4-hydroxypiperidino]-2,2-diphenylpentyl]-3-(3-hydroxypropyl)urea
Reference Example 27B-12: 1-[5-[4-(4-chlorophenyl)-4-hydroxypiperidino]-2,2-diphenylpentyl]-3-(1-benzylpiperidin-4-yl)urea
Reference Example 27B-13: N- [5-[4-(4-chlorophenyl)-4-hydroxypiperidino]-2,2-diphenylpentyl]-4-methylpiperazine-1-carboxamide
Reference Example 27B-14: 1N-[5-[4-(4-chlorophenyl)-4-hydroxypiperidino]-2,2-diphenylpentyl]-4-benzylpiperazine-1-carboxamide
Reference Example 27B-15: lN-[5-[4-(4-chlorophenyl)-4-hydroxypiperidino]-2,2-diphenylpentyl]-1,2,4,5-tetrahydro-3-benzazepine-3-carboxamide
Reference Example 27B-16: 1N-[5-[4-(4-chlorophenyl)-4-hydroxypiperidino]-2,2-diphenylpentyl]-3-(trifluoroacetylamino)pyrrolidine-1-carboxamide
Reference Example 27B-17: 1N-[5-[4-(4-chlorophenyl)-4-hydroxypiperidino]-2,2-diphenylpentyl]-4-(t-butoxycarboxamide)piperidine-1-carboxamide
Reference Example 27B-18: ethyl [4-[3-[5-[4-(4-chlorophenyl)-4-hydroxypiperidino]-2,2-diphenylpentyl]ureido]piperidino]acetate
Reference Example 27B-19: 1-[5-[4-(4-chlorophenyl)-4-hydroxypiperidino]-2,2-diphenylpentyl]-3-[1-(trifluoroacetyl)-piperidin-4-yl]urea
Reference Example 27B-20: 1N- [5- [4- (4-chlorophenyl) -4-hydroxypiperidino]-2,2-diphenylpentyl]-4-formyl-1-piperazinecarboxamide
Reference Example 27B-21: 1N- [5- [4-(4-chlorophenyl)-4-hydroxypiperidino]-2,2-diphenylpentyl]-4-(3-hydroxypropyl)-1-piperazinecarboxamide
Reference Example 27B-22: 1N-[5- [4-(4-chlorophenyl)-4-hydroxypiperidino]-2,2-diphenylpentyl]-4-(ethoxycarbonyl)-1-piperazinecarboxamide
Reference Example 27B-23: 1N-[5-[4-(4-chlorophenyl)-4-hydroxypiperidino]-2,2-diphenylpentyl]-4-(morpholinocarbonylmethyl)-1-piperazinecarboxamide
Reference Example 28B-1: 3-[3-[5-[4-(4-chlorophenyl)-4-hydroxypiperidino]-2,2-diphenylpentyl]ureido] propionic acid
Reference Example 28B-2: 4-[3-[5-[4-(4-chlorophenyl)-4-hydroxypiperidino]-2,2-diphenylpentyl]ureido]butyric acid
Reference Example 29B: 1N-[5-[4-(4-chlorophenyl)-4-hydroxypiperidino]-2,2-diphenylpentyl]vinylsulfonamide
Reference Example 30B: 1N-[5-[4-(4-chlorophenyl)-4-hydroxypiperidino]-2,2-diphenylpentyl]-2-(pyrrolidino)-ethylsulfonamide
Reference Example 31B: 1-[5-[4-(4-chlorophenyl)-4-hydroxypiperidino]-2,2-diphenylpentyl]-3-[3-(carbamoyloxy)-propyl]urea
Reference Example 32B: 1-[5-[4-(4-chlorophenyl)-4-hydroxypiperidino]-2,2-diphenylpentyl]-3-(piperidin-4-yl)urea
Reference Example 33B-1: ethyl 4-[4-[5-[4-(4-chlorophenyl)-4-hydroxypiperidino]-2,2-diphenylpentyl]aminocarbonylamino]-piperidino-4-oxobutyrate

Reference Example 33B-2: N-ethyl-4-[5-[4-(4-chlorophenyl)-4-hydroxypiperidino]-2,2-diphenylpentyl]aminocarbonylamino-1-piperidinecarboxamide

Reference Example 33B-3: 1-[5-[4-(4-chlorophenyl)-4-hydroxypiperidino]-2,2-diphenylpentyl]-3-(1-acetylpiperidin-4-yl) urea

Reference Example 33B-4: N-ethoxycarbonylmethyl-4-[5-[4-(4-chlorophenyl)-4-hydroxypiperidino]-2,2-diphenylpentyl]-aminocarbonylamino-1-piperidinecarboxamide

Reference Example 33B-5: ethyl 3-[4-[5-[4-(4-chlorophenyl)-4-hydroxypiperidino]-2,2-diphenylpentyl]aminocarbonylamino]-piperidino-3-oxopropionate

Reference Example 34B-1: 1-[5-[4-(4-chlorophenyl)-4-hydroxypiperidino]-2,2-diphenylpentyl]-3-(1-ethylpiperidin-4-yl) urea

Reference Example 34B-2: 1-[5-[4-(4-chlorophenyl)-4-hydroxypiperidino]-2,2-diphenylpentyl]-3-[1-(2-hydroxyethyl)-piperidin-4-yl]urea

Reference Example 34B-3: ethyl 3-[4-[5-[4-(4-chlorophenyl)-4-hydroxypiperidino]-2,2-diphenylpentyl]aminocarbonylamino-piperidino]propionate

Reference Example 34B-4: 1-[5-[4-(4-chlorophenyl)-4-hydroxypiperidino]-2,2-diphenylpentyl]-3-[1-(3-hydroxypropyl)-piperidin-4-yl]urea

Reference Example 35B: 1-[(piperidin-4-yl)carboxamide]-5-[4-(4-chlorophenyl)-4-hydroxypiperidino]-2,2-diphenylpentane dihydrochloride

Reference Example 36B-1: 1-[(N-ethylpiperidin-4-yl)carboxamide]-5-[4-(4-chlorophenyl)-4-hydroxypiperidino]-2,2-diphenylpentane dihydrochloride

Reference Example 36B-2: 1-[[N-(ethoxycarbonylmethyl)piperidin-4-yl]carboxamide]-5-[4-(4-chlorophenyl)-4-hydroxypiperidino]-2,2-diphenylpentane dihydrochloride

Reference Example 36B-3: 1-[[N-(2-morpholinoethyl)piperidin-4-yl]carboxamide]-5-[4-(4-chlorophenyl)-4-hydroxypiperidino]-2,2-diphenylpentane 3 hydrochloride

Reference Example 36B-4: 1-[[N-(2-dimethylaminoethyl)piperidin-4-yl]carboxamide]-5-[4-(4-chlorophenyl)-4-hydroxypiperidino]-2,2-diphenylpentane 3 hydrochloride

Reference Example 37B-1: 1- [[(N-ethylcarbamoyl)piperidin-4-yl]carboxamide]-5-[4-(4-chlorophenyl)-4-hydroxypiperidino] -2,2-diphenylpentane hydrochloride

Reference Example 37B-2: 1-[[(N-methylcarbamoyl)piperidin-4-yl] carboxamide]-5- [4-(4-chlorophenyl)-4-hydroxypiperidino]-2,2-diphenylpentane hydrochloride

Reference Example 37B-3: 1-[[(N-phenylcarbamoyl)piperidin-4-yl]carboxamide]-5-[4-(4-chlorophenyl)-4-hydroxypiperidino]-2,2-diphenylpentane hydrochloride

Reference Example 37B-4: 1-[[(N-(4-chlorobenzoyl)piperidin-4-yl]carboxamide]-5-[4-(4-chlorophenyl)-4-hydroxypiperidino]-2,2-diphenylpentane hydrochloride

Reference Example 37B-5: 1-[[N-(ethoxycarbonylacetyl)piperidin-4-yl]carboxamide]-5-[4-(4-chlorophenyl)-4-hydroxypiperidino]-2,2-diphenylpentane hydrochloride

Reference Example 37B-6: 1-[[N-(3-methoxycarbonylpropionyl)-piperidin-4-yl]carboxamide]-5-[4-(4-chlorophenyl)-4-hydroxypiperidino]-2,2-diphenylpentane hydrochloride

Reference Example 37B-7: 1-[[N-(nicotinoyl)piperidin-4-yl]carboxamide]-5-[4-(4-chlorophenyl)-4-hydroxypiperidino]-2,2-diphenylpentane dihydrochloride

Reference Example 37B-8: 1-[[N-(4-dimethylaminobutyryl)-piperidin-4-yl]carboxamide]-5-[4-(4-chlorophenyl)-4-hydroxypiperidino]-2,2-diphenylpentane dihydrochloride

Reference Example 38B: 1-[(N-propylpiperidin-4-yl)carboxamide]-5-[4-(4-chlorophenyl)-4-hydroxypiperidino]-2,2-diphenylpentane dihydrochloride

Reference Example 39B: 1-[[N-3-pyridylacetyl)piperidin-4-yl] carboxamide] -5- [4- (4-chiorophenyl) -4-hydroxypiperidino] -2,2-diphenylpentane dihydrochloride

Reference Example 40B: 1-[[N-ethylcarbamoyl)piperidin-4-yl] carboxamide] -5- [4- (4-chlorophenyl} -4-hydroxypiperidino] -2,2-diphenylpentane dihydrochloride

Reference Examples 1C-15C can be produced according to JP-A-11-71350.

Reference Example 1C: 1-(tert-butoxycarbonyl)piperidin-4-yl N-(5-(4-(4-chlorophenyl)-4-hydroxypiperidino)-2,2-diphenylpentyl)carbamate

Reference Example 2C: piperidin-4-yl N-(5-(4-(4-chlorophenyl)-4-hydroxypiperidino)-2,2-diphenylpentyl)carbamate

Reference Example 3C: 1-(N-ethylcarbamoyl)piperidin-4-yl N-(5-(4-(4-chlorophenyl)-4-hydroxypiperidino)-2,2-diphenylpentyl)-carbamate

Reference Example 4C: 1-(nicotinoyl)piperidin-4-yl N-(5-(4-(4-chlorophenyl)-4-hydroxypiperidino)-2,2-diphenylpentyl)carbamate

Reference Example 5C-1: 1-(5-(4-(4-chlorophenyl)-4-hydroxypiperidino) -2,2-diphenylpentyl) -3- (1- (2-chlorethyloxycarbonyl)piperidin-4-yl)urea

Reference Example 5C-2: 1-(5-(4-(4-chlorophenyl)-4-hydroxypiperidino)-2,2-diphenylpentyl)-3-(1-(acetoxyacetyl)-piperidin-4-yl)urea

Reference Example 5C-3: 1-(5-(4-(4-chlorophenyl)-4-hydroxypiperidino)-2,2-diphenylpentyl)-3-(1-(nicotinoyl)-piperidin-4-yl)urea

Reference Example 5C-4: 1-(5-(4-(4-chlorophenyl)-4-hydroxypiperidino)-2,2-diphenylpentyl)-3- (1- (isonicotinoyl)-piperidin-4-yl)urea

Reference Example 5C-5: 1-(5-(4-(4-chlorophenyl)-4-hydroxypiperidino) -2 ,2-diphenylpentyl) -3- (1- (benzoyl) piperidin-4-yl)urea

Reference Example 6C: 1-(5-(4-(4-chlorophenyl)-4-hydroxypiperidino)-2,2-diphenylpentyl)-3-(1-(2-hydroxyacetyl)-piperidin-4-yl)urea

Reference Example 7C: 1-(5-(4-(4-chlorophenyl) -4-hydroxypiperidino)-2,2-diphenylpentyl) -3-(1-(2-pyrrolidin-1-yl)ethyloxycarbonyl)piperidin-4-yl)urea

Reference Example 8C-1: N-(5-(4-(4-chlorophenyl)-4-hydroxypiperidino)-2,2-diphenylpentyl) nicotinamide dihydrochloride

Reference Example 8C-2: 2-chloroethyl (5-(4-(4-chlorophenyl)-4-hydroxypiperidino)-2,2-diphenylpentylamino) carbamate

Reference Example 9C: 1-(5-(4-(4-chlorophenyl)-4-hydroxypiperidino)-2,2-diphenylpentyl) -4,5-dihydro-2-oxazolone

Reference Example 10C-1: 2-(1-(t-butoxycarbonyl)piperidin-4-yl)-N-(5-(4-(4-chlorophenyl)-4-hydroxypiperidino)-2,2-diphenylpentyl)acetamide

Reference Example 10C-2: 2-(1-(t-butoxycarbonyl)piperidin-4-ylidene)-N-(5-(4-(4-chlorophenyl)-4-hydroxypiperidino)-2,2-diphenylpentyl) acetamide

Reference Example 11C-1: N-(5-(4-(4-chlorophenyl)-4-hydroxypiperidino)-2,2-diphenylpentyl) -2-(piperidin-4-yl) acetamide dihydrochloride

Reference Example 11C-2: N-(5-(4-(4-chlorophenyl)-4-hydroxypiperidino)-2,2-diphenylpentyl) -2-(piperidine-4-ylidene) acetamide dihydrochloride

Reference Example 12C-1: N- (5- (4- (4-chlorophenyl) -4-hydroxypiperidino)-2-phenyl-2-(2-pyridyl)pentyl)-1-(ethoxycarbonyl)piperidine-4-carboxamide dihydrochloride

Reference Example 12C-2: N-(5-(4-(4-chlorophenyl)-4-hydroxypiperidino)-2-phenyl-2-(2-pyridyl)pentyl)-1-(isonicotinoyl)piperidine-4-carboxamide 3 hydrochloride

Reference Example 12C-3: N-(5-(4-(4-chlorophenyl)-4-hydroxypiperidino)-2,2-bis(4-fluorophenyl)pentyl)-1-(isonicotinoyl)piperidine-4-carboxamide dihydrochloride

Reference Example 12C-4: N-(5-(4-(4-chlorophenyl)-4-hydroxypiperidino)-2-(4-fluorophenyl)-2-phenylpentyl)-1-(isonicotinoyl)piperidine-4-carboxamide dihydrochloride

Reference Example 13C-1: 1-(5-(4-(4-chlorophenyl)-4-hydroxypiperidino) -2-phenyl-2- (2-pyridyl) pentyl) -3- (3-hydroxypropyl)urea dihydrochloride

Reference Example 13C-2: 1-(5-(4-(4-chlorophenyl)-4-hydroxypiperidino)-2-phenyl-2-(2-pyridyl)pentyl)-3-(1-(nicotinoyl)piperidin-4-yl)urea 3 hydrochloride

Reference Example 13C-3: 1-(5-(4-(4-chlorophenyl)-4-hydroxypiperidino)-2-phenyl-2-(2-pyridyl)pentyl)-3-(1-(isonicotinoyl)piperidin-4-yl)urea 3 hydrochloride

Reference Example 13C-4: 1-(5-[4-(4-chlorophenyl)-4-hydroxypiperidino]-2,2-bis(4-fluorophenyl)pentyl)-3-(1-(isonicotinoyl)piperidin-4-yl)urea dihydrochloride

Reference Example 13C-5: 1- (5-[4- (4-chlorophenyl) -4-hydroxypiperidino]-2,2-bis(4-fluorophenyl)pentyl)-3-(3-hydroxypropyl)urea

Reference Example 13C-6: 1-(5- [4-(4-chlorophenyl)-4-hydroxypiperidino]-2,2-bis(4-fluorophenyl)pentyl)-3-(1-(nicotinoyl)piperidin-4-yl)urea

Reference Example 13C-7: 1- (5-[4-(4-chlorophenyl)-4-hydroxypiperidino]-2-(4-fluorophenyl)-2-phenylpentyl)-3-(1-(isonicotinoyl)piperidin-4-yl)urea dihydrochloride

Reference Example 13C-8: 1-(5- [4-(4-chlorophenyl)-4-hydroxypiperidino]-2-(4-fluorophenyl)-2-phenylpentyl)-3-(3-hydroxypropyl)urea hydrochloride

Reference Example 13C-9: 1-(5-[4-(4-chlorophenyl)-4-hydroxypiperidino]-2-(4-fluorophenyl)-2-phenylpentyl)-3-(1-(nicotinoyl)piperidin-4-yl)urea dihydrochloride

Reference Example 14C: N- (5- (4- (4-chlorophenyl) -4-hydroxypiperidino)-2,2-bis(4-fluorophenyl)pentyl)acetamide hydrochloride

Reference Example 15C: N-(5-[4-(4-chlorophenyl)-4-hydroxypiperidino]-2-(4-fluorophenyl)-2-phenylpentyl)acetamide hydrochloride

Reference Example 1D

benzyl 2-((5-hydroxy-2,2-diphenylpentyl)amino)-2-oxoethylcarbamate

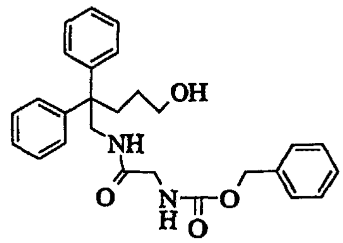

To a solution of 5-amino-4,4-diphenylpentanol (15.8 g) in acetonitrile (100 ml) were added 2-(((benzyloxy) carbonyl)-amino)acetic acid(13 g) and WSC(14 g). The mixture was stirred at room temperature overnight. The reaction mixture was concentrated and ethyl acetate and water were added to the residue. The organic layer was separated, washed with saturated aqueous sodium hydrogen carbonate, dried and concentrated. The residue was recrystallized from IPE/ethyl acetate to give the title compound (21 g).
melting point: 122-123°C.

Reference Example 2D

tert-butyl 2-((5-hydroxy-2,2-diphenylpentyl)amino)-2-oxoethylcarbamate

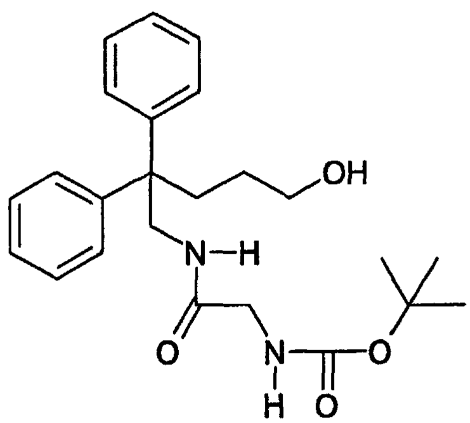

To a solution of 5-amino-4,4-diphenylpentanol (4 g) in acetonitrile (30 ml) were added 2-(((tert-butoxy)carbonyl)-amino)acetic acid (3.5 g), WSC(4 g) and triethylamine(5 ml). The mixture was stirred at room temperature overnight. The reaction mixture was concentrated and ethyl acetate and water were added to the residue. The organic layer was separated, washed with saturated aqueous sodium hydrogen carbonate, dried and concentrated. The residue was purified by silica gel column chromatography (eluent: ethyl acetate) to give the title compound (4 g).
oil:
$^1$H-NMR($CDCl_3$)δ: 1.2-1.6 (2H, m), 1.43 (9H, s) , 1.802.3 (2H, m), 3.4-3.6 (2H, m), 3.69 (2H, d), 4.04 (2H, d), 5.0 (1H, br), 5.70 (1H, br), 7.1-7.4 (10H, m).

Reference Example 3D

2,2-diphenyl-5-(4-phenylpiperidino)pentanenitrile

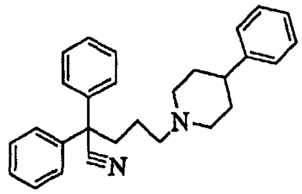

To a solution of 5-bromo-2,2-diphenylpentanenitrile (9.5 g) in acetonitrile (100 ml) were added potassium carbonate (6 g) and 4-phenylpiperidine (4.8 g). The reaction mixture was stirred at 60°C overnight and concentrated. The residue was recrystallized from IPE/ethyl acetate to give the title compound (11 g).
melting point: 88-89°C.

Reference Example 4D

7-oxo-4,4-diphenyl-7-(4-phenylpiperidino)heptanenitrile

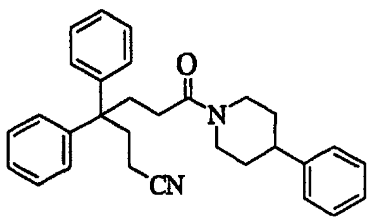

To a solution of 6-cyano-4,4-diphenylhexanoic acid (5.87 g) in dichloromethane (60 ml) was added thionyl chloride (3.57 g) under ice-cooling and the mixture was stirred at room temperature for 1 h. To the reaction mixture was added a solution of phenylpiperidine (4.8 g) and triethylamine (5 g) in dichloromethane (20 ml) by small portions and the mixture was stirred at room temperature for another 1h. The reaction mixture was washed with 1N hydrochloric acid and saturated brine and dried over anhydrous sodium sulfate. Concentration under reduced pressure gave the residue, which was subject to silica gel column chromatography with elution of hexane-ethyl acetate (4:1-1:1) to give the title compound (7.5 g).
oil:
$^1$H-NMR($CDCl_3$) δ: 1.19-1.95 (4H, m), 1.96-2.08 (4H, m), 2.20-2.79 (6H, m), 2.91 (1H, dt, J=2.6, 18.0 Hz), 3.38-3.52 (1H, m), 4.66-4.80 (1H, m), 7.10-7.38 (15H, m).

Reference Example 5D

2,2-diphenyl-5-(4-phenylpiperidino)pentylamine

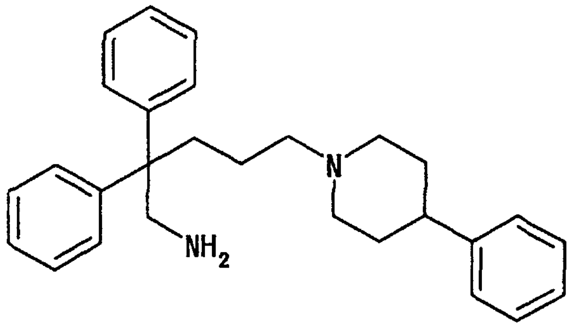

2,2-diphenyl-5-(4-phenylpiperidino)pentanenitrile(10.0 g) produced in Reference Example 3D was added to a suspension of lithium aluminum hydride (4.8 g) and aluminum chloride (16.9 g) in THF (350 ml) under ice-cooling and the mixture was stirred at room temperature for 3 h. To the reaction mixture was added 1N aqueous sodium hydroxide solution (400 ml) by small portions under ice-cooling and the mixture was stirred for 5 min. Ether (500 ml) was added and the mixture was filtered through Celite. The organic layer of the filtrate was washed with saturated brine and dried over anhydrous sodium sulfate. Concentration under reduced pressure gave the residue, which was subject to silica gel column chromatography with elution of ethyl acetate-methanol (5:1)-ethyl acetate-methanol-saturated aqueous ammonia (50:10:1) to give the title compound (8.1 g).
oil:
$^1$H-NMR($CDCl_3$)δ: 1.25-1.36 (4H, m), 1.69-2.03 (6H, m), 2.10-2.18 (2H, m), 2.28-2.48 (3H, m), 2.90 (2H, d, J=11.4 Hz), 3.33 (2H, s), 7.12-7.32 (15H, m).

Reference Example 6D

benzyl 2-((5-(4-methylbenzenesulfonyloxy)-2,2-diphenylpentyl)-amino)-2-oxoethylcarbamate

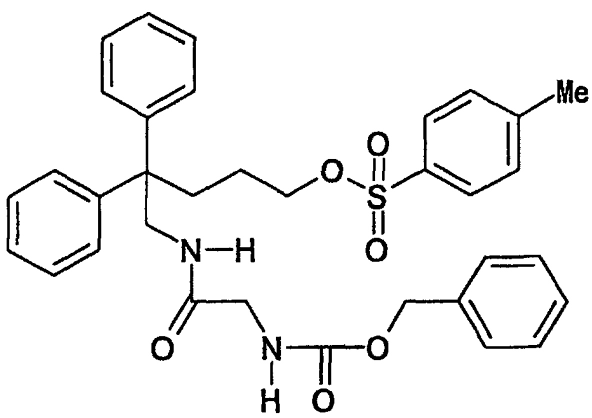

To a solution of the compound (5.50 g) obtained in Reference Example 1D in acetonitrile (150 ml) were added triethylamine (2.76 ml), 4-dimethylaminopyridine (0.16 g) and p-toluenesulfonyl chloride (3.79 g). The reaction mixture was stirred at room temperature overnight and concentrated under reduced pressure. The residue was dissolved in ethyl acetate and the mixture was washed with water, dried and concentrated. The resulting residue was crystallized

from ethyl acetate-hexane to give the title compound (6.28 g).
melting point: 143-144°C

Reference Example 7D

benzyl 2-((5-iodo-2,2-diphenylpentyl)amino)-2-oxoethylcarbamate

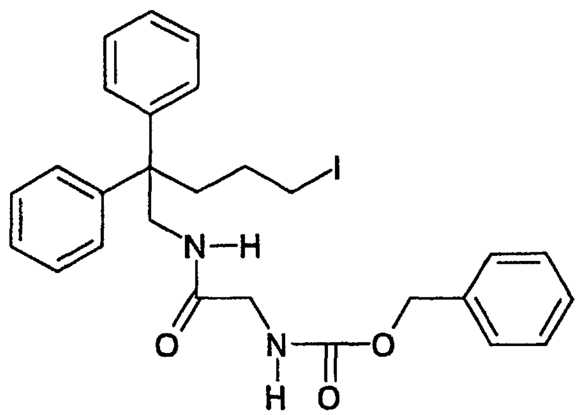

To a solution of the compound (6.25 g) obtained in Reference Example 6D in acetone (100 ml) was added sodium iodide (15.0 g) under ice-cooling. The reaction mixture was stirred at room temperature for 2 days and concentrated under reduced pressure. The residue was dissolved in ethyl acetate and the mixture was washed with water, dried and concentrated. The resulting residue was subject to silica gel column chromatography. By purification with hexane-ethyl acetate (1:1), the title compound (5.93 g) was obtained.
oil.
$^1$H-NMR($CDCl_3$)δ: 1.51-1.63 (2H, m) , 2.12-2.19 (2H, m) , 3.10 (2H, t, J=6.4Hz), 3.73 (2H, d, J=5.6Hz), 4.00 (2H, d, J=6.5Hz), 5.10 (2H, s), 5.23 (1H, s), 5.51 (1H, s), 7.15-7.40 (15H, m).

Reference Example 8D

benzyl 2-((4-hydroxy-2,2-diphenylbutyl)amino)-2-oxoethylcarbamate

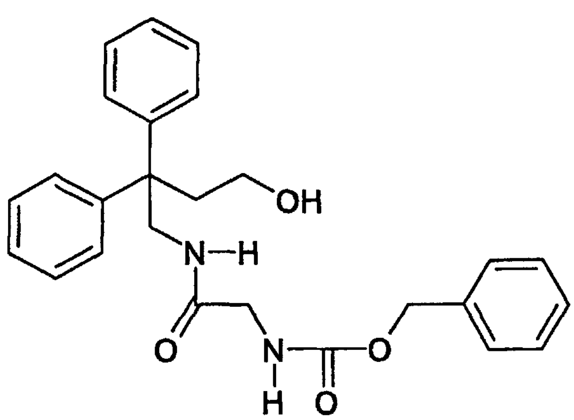

In the same manner as in Reference Example 1D, the compound was synthesized from 4-amino-3,3-diphenylbutanol. amorphous powder.
$^1$H-NMR($CDCl_3$)δ: 1.26-1.34 (2H, m), 1.71 (1H, s) , 2.09-2.14 (2H, m), 3.53 (2H, t, J=5.8 Hz), 3.72 (2H, d, J=5.8 Hz),

4.00 (2H, d, J=5.5 Hz), 5.08 (2H, s), 5.30 (1H, s), 5.56-5.59 (1H, m), 7.13-7.37 (15H, m).

Reference Example 9D

benzyl 2-((4-(4-methylbenzenesulfonyloxy)-2,2-diphenylbutyl)-amino)-2-oxoethylcarbamate

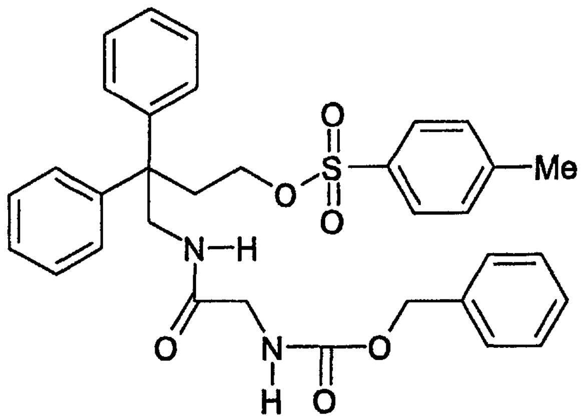

In the same manner as in Reference Example 6D, the compound was synthesized from the compound obtained in Reference Example 8D.
oil.
$^1$H-NMR($CDCl_3$) δ: 1.34-1.42 (2H, m) , 2.01-2.09 (2H, m) , 2.43 (3H, s), 3.73 (2H, d, J=5.6 Hz), 3.90-3.96 (4H, m), 5.08 (2H, s), 5.29 (1H, s), 5.48 (1H, s), 7.09 (4H, d, J=7.0 Hz), 7.19-7.36 (13H, m), 7.73 (2H, d, J=8.2 Hz).

Reference Example 10D

benzyl 2-((4-iodo-2,2-diphenylbutyl)amino)-2-oxoethylcarbamate

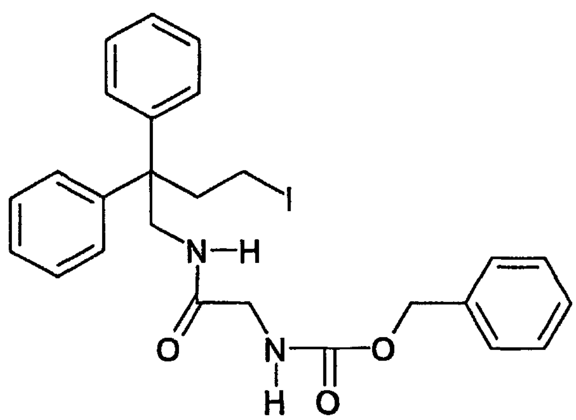

In the same manner as in Reference Example 7D, the compound was synthesized from the compound obtained in Reference Example 9D.
amorphous powder.
$^1$H-NMR($CDCl_3$)δ: 2.52-2.70 (2H, m), 3.37-3.59 (2H, m), 3.92-4.10 (4H, m), 4.18 (2H, s), 5.13 (2H, s), 5.78 (1H, s), 7.15-7.36 (15H, m).

Reference Example 11D

tert-butyl 2-((2,2-bis(4-chlorophenyl)-5-hydroxypentyl)amino)-2-oxoethylcarbamate

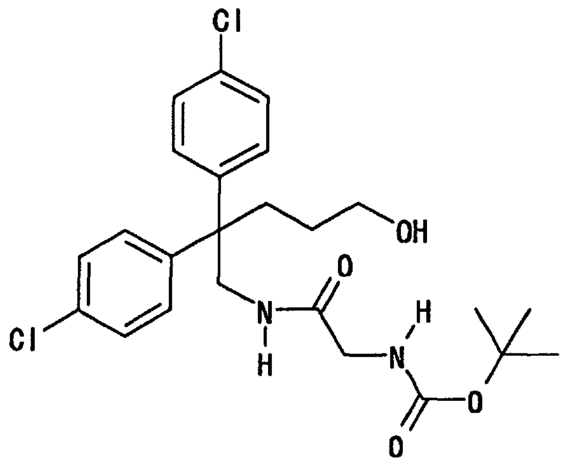

In the same manner as in Reference Example 2D, the compound was synthesized from 5-amino-4,4-bis (4-chlorophenyl)-1-pentanol.
amorphous powder.
$^1$H-NMR($CDCl_3$)δ: 1.23-1.35 (2H, m), 1.41 (9H, s), 1.85 (1H, t, J=5.6 Hz), 2.05-2.14 (2H, m), 3.55 (2H, q, J=5.6 Hz), 3.68 (2H, d, J=6.0 Hz), 3.96 (2H, d, J=6.2 Hz), 4.98 (1H, br), 5.75 (1H, br), 7.13 (4H, d, J=8.7 Hz), 7.29 (4H, d, J=8.7Hz).

Reference Example 12D

tert-butyl 2-((2,2-bis(4-chlorophenyl)-5-(4-methylbenzenesulfonyloxy)pentyl)amino)-2-oxoethylcarbamate

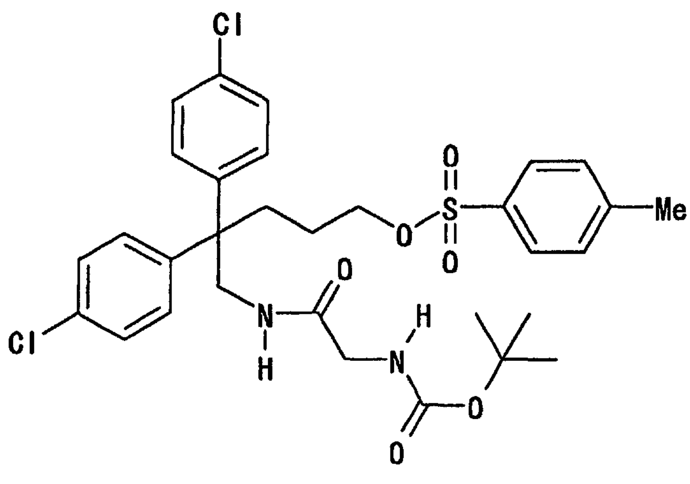

In the same manner as in Reference Example 6D, the compound was synthesized from the compound obtained in Reference Example 11D.
amorphous powder.
$^1$H-NMR($CDCl_3$)δ: 1.30-1.38 (2H, m), 1.40 (9H, s), 2.01-2.09 (2H, m) , 2.45 (3H, s), 3.68 (2H, d, J=6.1 Hz), 3.88-3.96 (4H, m), 4.94 (1H, br), 5.65 (1H, br), 7.02-7.12 (5H, m), 7.24-7.35 (5H, m), 7.74 (2H, d, J=8.3 Hz).

Reference Example 13D

tert-butyl 2-((2,2-bis (4-chlorophenyl)-5-iodopentyl)amino)-2-oxoethylcarbamate

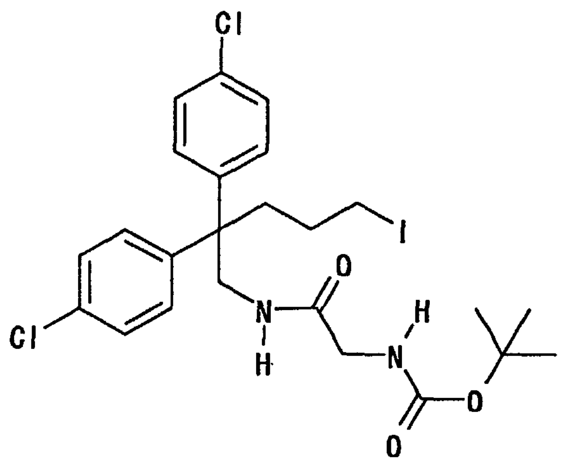

In the same manner as in Reference Example 7D, the compound was synthesized from the compound obtained in Reference Example 12D.
amorphous powder.
$^1$H-NMR($CDCl_3$)δ: 1.41 (9H, s) , 1.47-1.56 (2H, m), 2.04-2.17 (2H, m), 3.10 (2H, t, J=6.4 Hz), 3.68 (2H, d, J=6.2 Hz), 3.94 (2H, d, J=6.2 Hz) , 4.89 (1H, br) , 5.68 (1H, br) , 7.06-7.13 (4H, m) , 7.25-7.33 (4H, m).

Reference Example 14D

bis(4-fluorophenyl)acetonitrile

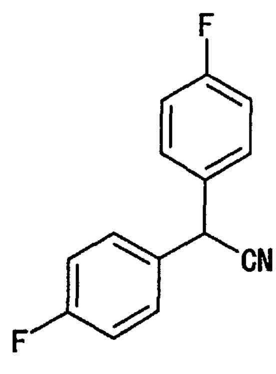

Thionyl chloride (50 ml) was added to bis(4-fluorophenyl)methanol (24.2 g) at 0°C and after stirring for 30 min, the mixture was poured into 2N hydrochloric acid (500 ml). The mixture was extracted with ethyl acetate and the organic layer was dried over calcium chloride and concentrated under reduced pressure. The resulting residue was dissolved in dichloromethane (200 ml) and after addition of trimethylsilylcyanide (16.4 ml), titanium tetrachloride (13.4 ml) was added dropwise at 0°C. The mixture was stirred for 50 min. Methanol (5 ml) was added to the reaction mixture and the mixture was poured into saturated aqueous sodium hydrogen carbonate. The mixture was extracted with ethyl acetate and washed with saturated brine. The organic layer was dried over anhydrous magnesium sulfate and concentrated under reduced pressure to give the title compound (21.8 g).
oil.

$^1$H-NMR($CDCl_3$)δ: 5.11 (1H, s), 7.03-7.11 (4H, m), 7.26-7.33 (4H, m).

Reference Example 15D

ethyl 4,4-bis(4-fluorophenyl)-4-cyanobutyrate

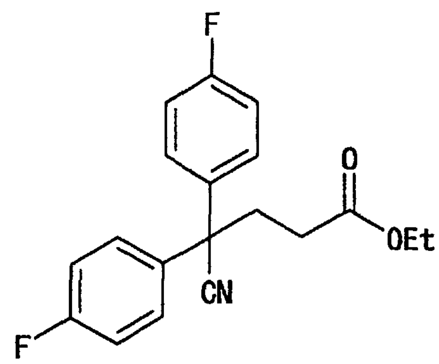

A mixture of bis(4-fluorophenyl)acetonitrile (20.4 g) obtained in Reference Example 14D, ethanol(150 ml), 1,8-diazabicyclo[5.4.0]-7-undecene (2.63 ml) and ethyl acrylate (12.5 ml) was heated under reflux for 18 h. After cooling, the reaction mixture was concentrated under reduced pressure. 2N Hydrochloric acid (200 ml) was added and the mixture was extracted with ethyl acetate. The organic layer was washed with saturated brine, dried over anhydrous magnesium sulfate and concentrated under reduced pressure. The resulting residue was subject to silica gel column chromatography with elution of hexane-ethyl acetate (5:1) to give the title compound (28.8 g).
oil.
$^1$H-NMR($CDCl_3$) δ: 1.25 (3H, t, J=7.2 Hz), 2.42-2.47 (2H, m), 2.70-2.75 (2H, m), 4.13 (2H, q, J=7.1 Hz), 7.06-7.13 (4H, m), 7.34-7.39 (4H, m) .

Reference Example 16D

2,2-bis(4-fluorophenyl)-5-hydroxypentylamine

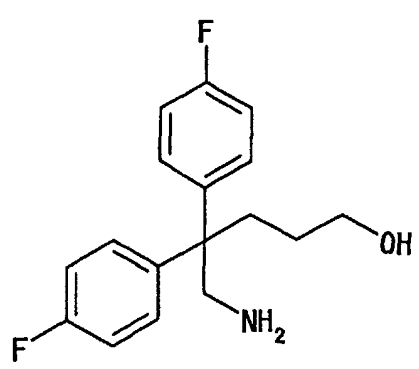

To a mixture of ethyl 4,4-bis(4-fluorophenyl)-4-cyanobutyrate (34 g) obtained in Reference Example 15D and THF (150 ml) was added lithium aluminum hydride (15.7 g) and the mixture was heated under reflux for 16 h. After cooling, methanol (50 ml) was added by small portions and the mixture was extracted with ethyl acetate. The organic layer was washed with saturated brine, dried over anhydrous magnesium sulfate and concentrated under reduced pressure. The resulting residue was subject to silica gel column chromatography with elution of ethyl acetate to give the title compound (19.7 g).

oil.
$^1$H-NMR ($CDCl_3$) δ: 1.24-1.33 (3H, m), 2.18-2.24 (2H, m) , 3.32 (2H, s), 3.61 (2H, t, J= 6.2Hz), 6.96-7.04 (4H, m), 7.11-7.19 (4H, m).

Reference Example 17D

tert-butyl 2-((2,2-bis(4-fluorophenyl)-5-hydroxypentyl)amino)-2-oxoethylcarbamate

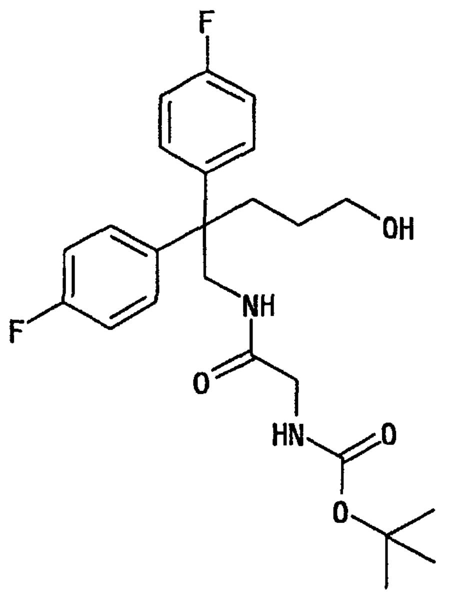

In the same manner as in Reference Example 2D, the compound was synthesized from the compound obtained in Reference Example 16D.
oil.
$^1$H-NMR($CDCl_3$)δ: 1.25-1.36 (2H, m) , 1.42 (9H, s), 2.09-2.14 (2H, m) , 3.57 (2H, t, J= 5.9Hz), 3.69 (2H, d, J= 6.1Hz), 3.98 (2H, d, J= 6.2Hz), 5.02 (1H, s), 5.76 (1H, s), 6.98-7.05 (4H, m), 7.11-7.17 (4H, m) .

Reference Example 18D

tert-butyl 2-((2,2-bis(4-fluorophenyl)-5-(4-methylbenzenesulfonyloxy)pentyl)amino)-2-oxoethylcarbamate

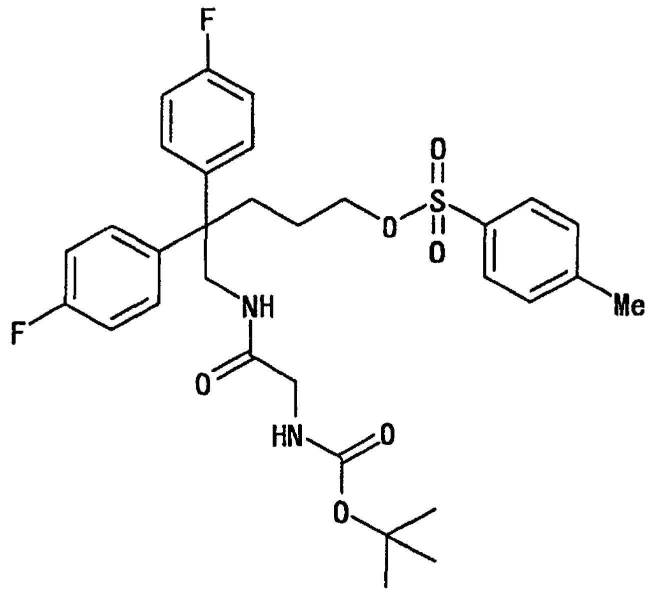

In the same manner as in Reference Example 6D, the compound was synthesized from the compound obtained in Reference Example 17D.
oil.
$^1$H-NMR($CDCl_3$)δ: 1.25-1.40 (11H, m), 2.03-2.08 (2H, m), 2.45 (3H, s), 3.67 (2H, d, J= 6.1Hz), 3.89-3.95 (4H, m), 4.94 (1H, s), 5.61-5.65 (1H, m), 6.96-7.11 (8H, m), 7.33 (2H, d, J= 8.2Hz), 7.74 (2H, d, J= 8.3Hz).

Reference Example 19D

tert-butyl 2-((2,2-bis(4-fluorophenyl)-5-iodopentyl)amino)-2-oxoethylcarbamate

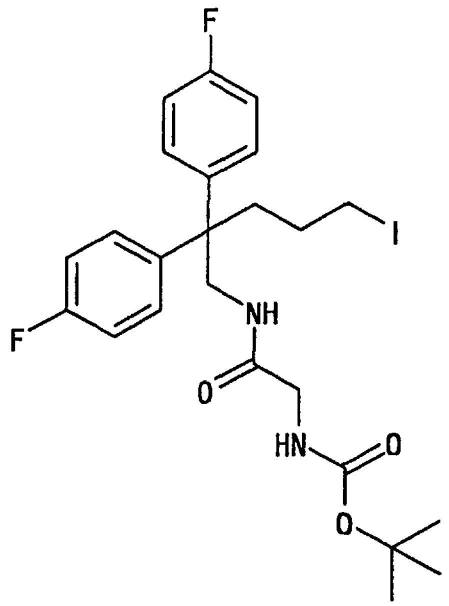

In the same manner as in Reference Example 7D, the compound was synthesized from the compound obtained in Reference Example 18D.
oil.
$^1$H-NMR($CDCl_3$) δ: 1.41 (9H, s), 1.47-1.57 (2H, m) , 2.08-2.14 (2H, m), 2.39-3.23 (1H, m), 3.82-3.92 (7H, m) , 5.77-5.81 (1H, m), 6.84-7.06 (6H, m), 7.19-7.27 (6H, m).

**Example 1**

benzyl 2-((2,2-diphenyl-5-(4-phenylpiperidino)pentyl)amino)-2-oxoethylcarbamate hydrochloride

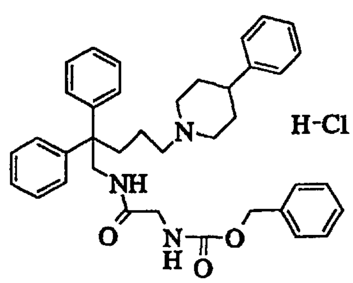

To a solution of triphenylphosphine (520 mg) in acetonitrile (10 ml) was added bromine (320 mg) under ice-cooling. A solution of benzyl 2-((5-hydroxy-2,2-diphenylpentyl)amino)-2-oxoethylcarbamate (0.88 g) in acetonitrile (10 ml) was then added dropwise to the reaction mixture. The mixture was stirred at room temperature for 1 h and concentrated. The residue was dissolved in ethyl acetate and the mixture was washed with water, dried and concentrated. The residue was purified by silica gel column chromatography (eluent; IPE: ethyl acetate=1:1) to give a bromide. To a solution of the bromide in acetonitrile (20 ml) were added 4-phenylpiperidine (320 mg) and potassium carbonate (300 mg). The reaction mixture was stirred at 40°C overnight, poured into water and extracted with ethyl acetate. The organic

layer was washed with water, dried and concentrated. The residue was purified by alumina column chromatography (eluent: ethyl acetate) and converted to hydrochloride. Recrystallization from ethyl acetate/ethanol gave the title compound (0.56g). melting point: 167-168°C.

**Example 2**

tert-butyl 2-((2,2-diphenyl-5-(4-phenylpiperidino)-pentyl)amino)-2-oxoethylcarbamate

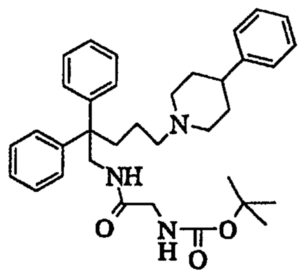

In the same manner as in Example 1, the compound was synthesized from tert-butyl 2-((5-hydroxy-2,2-diphenylpentyl)-amino)-2-oxoethylcarbamate.
melting point: 145-146°C

**Example 3**

4,4-diphenyl-7-(4-phenylpiperidino)heptylamine dihydrochloride

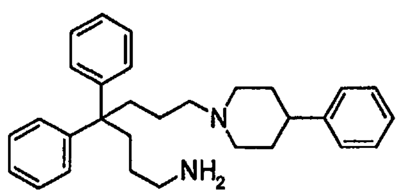

2HCl

To a solution of 7-oxo-4,4-diphenyl-7-(4-phenylpiperidino)heptanenitrile (2.2 g) in THF (20 ml) was added a suspension of lithium aluminum hydride (760 mg) in THF (40 ml) under ice-cooling and the mixture was stirred at 60°C for 14 h. After completion of the reaction, 1N aqueous sodium hydroxide solution was added dropwise slowly and the precipitated crystals were filtered off. The filtrate was concentrated. The resulting residue was dissolved in ethyl acetate and the organic layer was washed with saturated brine, dried and concentrated. The residue was converted to hydrochloride and recrystallized from dichloromethane-IPE to give the title compound (2.0 g).
melting point: 155-159°C.

**Example 4**

N-(4,4-diphenyl-7-(4-phenylpiperidino)heptyl)-4-methylbenzenesulfonamide hydrochloride

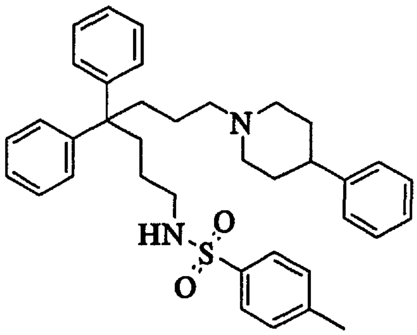

HCl

To a solution of 4,4-diphenyl-7-(4-phenylpiperidino)-heptylamine (500 mg) in dichloromethane (15 ml) were added triethylamine (3 ml), p-tosyl chloride (209 mg, 1.1 mmol) and DMAP (catalytic amount) under ice-cooling and the mixture was stirred at room temperature for 1 h. After completion of the reaction, the solvent was evaporated off under reduced pressure and the resulting residue was subject to silica gel column chromatography. The resultant was purified with hexane-ethyl acetate (1:1), made to be a hydrochloride and recrystallized from chloroform-IPE to give the title compound (420 mg).
melting point: 132-134°C

**Example 5**

N-(4,4-diphenyl-7-(4-phenylpiperidino)heptyl)acetamide hydrochloride

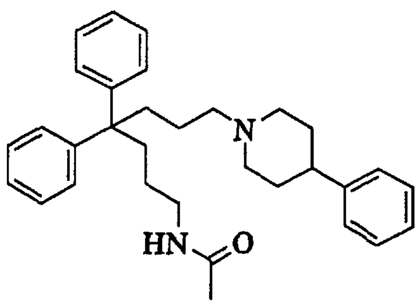

HCl

To a solution of 4,4-diphenyl-7-(4-phenylpiperidino)-heptylamine (400 mg) in dichloromethane (15 ml) were added triethylamine (3 ml) and acetic anhydride (102 mg, 1 mmol) under ice-cooling and the mixture was stirred at room temperature for 12 h. After completion of the reaction, the solvent was evaporated off under reduced pressure and the resulting residue was subject to silica gel column chromatography. The residue was purified by elution with ethyl acetate-methanol (1:0-10:1) and recrystallized from ethyl acetate-IPE to give the title compound (150 mg).
melting point: 80-85°C

**Example 6**

N-benzyl-N-(4,4-diphenyl-7-(4-phenylpiperidino)heptyl)amine dihydrochloride

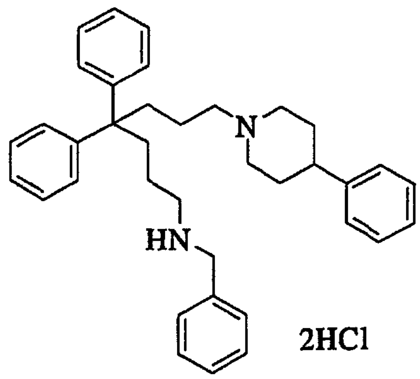

2HCl

To a solution of 4,4-diphenyl-7-(4-phenylpiperidino)-heptylamine (426 mg), benzaldehyde (106 mg) and p-tosylic acid hydrate (catalytic amount) in benzene (5 ml) was added anhydrous magnesium sulfate (1 g) and the mixture was stirred at 50°C for 1 h. The precipitate was filtered off and the filtrate was concentrated under reduced pressure. The resulting residue was dissolved in methanol (5 ml) and sodium borohydride (38 mg) was added. The mixture was stirred at room temperature for 5 min. After completion of the reaction, the mixture was concentrated under reduced pressure and the resulting residue was subject to silica gel column chromatography. The residue was purified by elution with ethyl acetate-methanol (1:0-20:1), made to be a hydrochloride and recrystallized from chloroform-IPE to give the title compound (350 mg).
melting point: 223-226°C

**Example 7**

N-(4,4-diphenyl-7-(4-phenylpiperidino)heptyl)-N-(3-methoxybenzyl)amine dihydrochloride

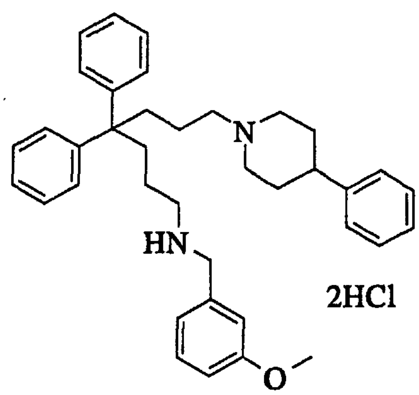

2HCl

The compound was synthesized in the same manner as in Example 6.
Recrystallization solvent: chloroform-IPE.
melting point: 215-217°C.

**Example 8**

N-(4,4-diphenyl-7-(4-phenylpiperidino)heptyl)-N-(2-methoxybenzyl)amine dihydrochloride

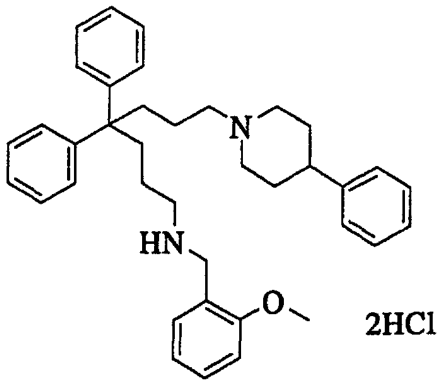

The compound was synthesized in the same manner as in Example 6.
Recrystallization solvent: chloroform-IPE.
melting point: 100-108°C.

**Example 9**

N-(4,4-diphenyl-7-(4-phenylpiperidino)heptyl)-N-(2-fluorobenzyl)amine dihydrochloride

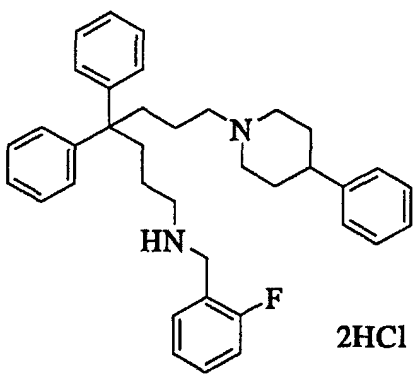

The compound was synthesized in the same manner as in Example 6.
Recrystallization solvent: chloroform-IPE
melting point: 198-200°C.

**Example 10**

N-(4,4-diphenyl-7-(4-phenylpiperidino)heptyl)-2-thiophenecarboxamide hydrochloride

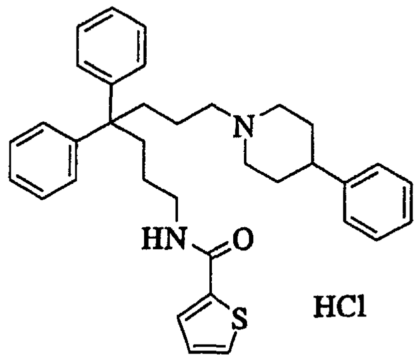

To a solution of 4,4-diphenyl-7-(4-phenylpiperidino)-heptylamine (426 mg) in ethyl acetate (10 ml) was added a saturated aqueous sodium carbonate solution (10 ml) and 2-thiophenecarbonyl chloride (146 mg) was added while vigorously stirring the mixture. After 30 min, the organic layer was separated, washed with saturated brine, dried and concentrated. The residue was applied to silica gel column chromatography, eluted with hexane-ethyl acetate (1:1), converted to hydrochloride and recrystallized from chloroform-IPE to give the title compound (0.5 g).
melting point: 125-130°C.

**Example 11**

N-(4,4-diphenyl-7-(4-phenylpiperidino)heptyl)-2-phenylacetamide hydrochloride

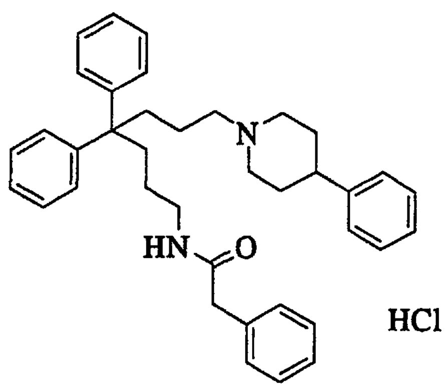

The compound was synthesized in the same manner as in Example 10.
Recrystallization solvent: chloroform-IPE.
melting point: 103-110°C

**Example 12**

N-(4,4-diphenyl-7-(4-phenylpiperidino)heptyl)-N-(2-thienylmethyl)amine dihydrochloride

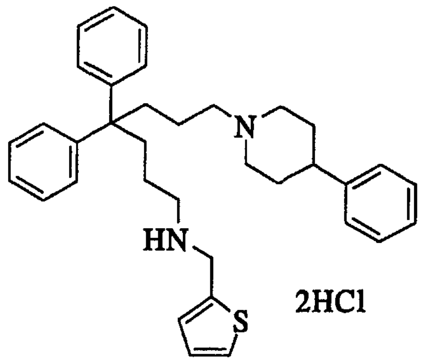

2HCl

To a solution of a free form (300 mg) of N-(4,4-diphenyl-7-(4-phenylpiperidino)heptyl)-2-thiophenecarboxamide in THF (5 ml) was added a suspension of lithium aluminum hydride (114 mg) in THF (5 ml) under ice-cooling, and the mixture was heated under reflux for 12 h. After the completion of the reaction, a 1N aqueous sodium hydroxide solution was dropwise added gradually. The precipitated crystals were filtered off and the filtrate was concentrated. The residue was dissolved in ethyl acetate, and the organic layer was washed with saturated brine, dried and concentrated to give the title compound (300 mg) as an amorphous. A part of the obtained compound was converted to hydrochloride, and recrystallized from chloroform-IPE.
melting point: 120-125°C.

**Example 13**

N-benzyl-N-(4,4-diphenyl-7-(4-phenylpiperidino)heptyl)-N-methylamine dihydrochloride

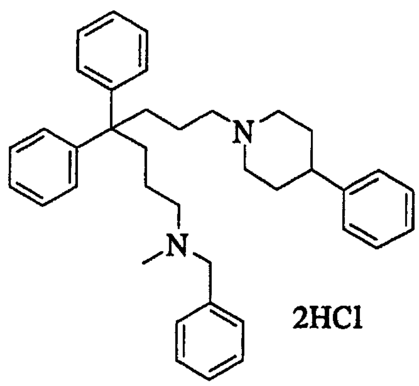

2HCl

To a solution of N-benzyl-N-(4,4-diphenyl-7-(4-phenylpiperidino)heptyl)amine (175 mg) in acetonitrile (5 ml)-37% formalin (0.3 ml) were added sodium cyanoborohydride (31 mg) and acetic acid (0.5 ml) and the mixture was stirred at room temperature for 1 h. After the completion of the reaction, the mixture was concentrated under reduced pressure, and the resulting residue was applied to silica gel column chromatography, purified by elution with ethyl acetate, converted to hydrochloride and recrystallized from chloroform-IPE to give the title compound (130 mg).
melting point: 115-120°C.

**Example 14**

N-(2,2-diphenyl-5-(4-phenylpiperidino)pentyl)-6-phenylhexanamide

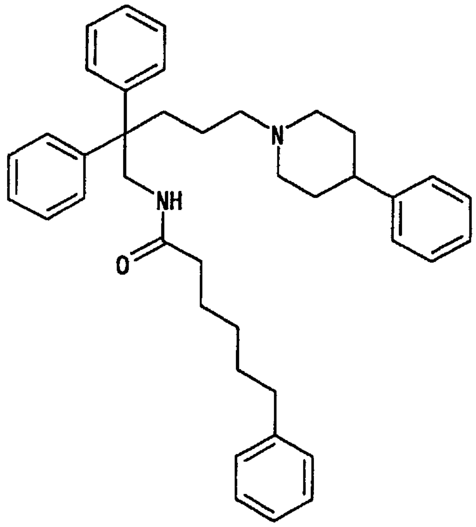

To a solution of 6-phenylhexanoic acid (80 mg) in THF (5 ml) were added oxalyl chloride (63 mg) and one drop of DMF under ice-cooling, and the mixture was stirred at room temperature for 2 h and concentrated under reduced pressure. This residue was added to a solution of 2,2-diphenyl-5-(4-phenylpiperidino)pentanamine (110 mg) synthesized in Reference Example 5D and triethylamine (56 mg) in THF (10 ml) under ice-cooling, and the mixture was stirred for 1 h. To the reaction mixture was added saturated aqueous sodium hydrogen carbonate (100 ml), and the mixture was extracted with ethyl acetate (100 ml), and after washing with saturated brine, dried over anhydrous sodium sulfate. The residue was concentrated under reduced pressure and the resulting residue was applied to silica gel column chromatography and eluted with ethyl acetate-hexane (2:1)-ethyl acetate to give the title compound (110 mg). amorphous powder.
$^1$H-NMR($CDCl_3$)δ: 1.25-1.38 (5H, m), 1.45-1.68 (5H, m), 1.72-1.78 (2H, m), 1.82-2.03 (6H, m), 2.28 (2H, t, J=7.3 Hz), 2.25-2.45 (1H, m), 2.52 (2H, t, J=7.6 Hz), 2.88 (2H, d, J=11.4 Hz), 3.99 (2H, d, J=5.9 Hz), 4.96 (1H, t, J=5.6 Hz), 7.12-7.34 (20H, m) .

**Example 15**

4-benzyloxy-N-(2,2-diphenyl-5-(4-phenylpiperidino)pentyl)-butylamide

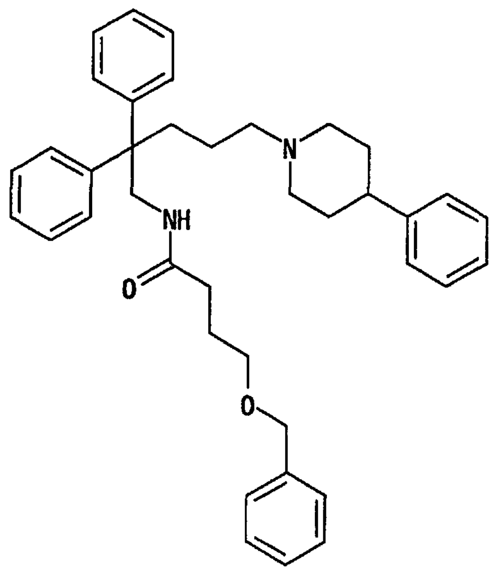

The compound was synthesized in the same manner as in Example 14.
amorphous powder.
$^1$H-NMR ($CDCl_3$)δ: 1.27-1.38 (2H, m), 1.68-2.44 (15H, m), 2.87-2.92 (2H, m), 3.41 (2H, t, J=6.0Hz), 3.99 (2H, d, J=5.9Hz), 4.39 (2H, s) , 5.14 (1H, t, J=5.7Hz), 7.15-7.31 (20H, m).

**Example 16**

4-benzoyl-N-(2,2-diphenyl-5-(4-phenylpiperidino)pentyl)-butylamide

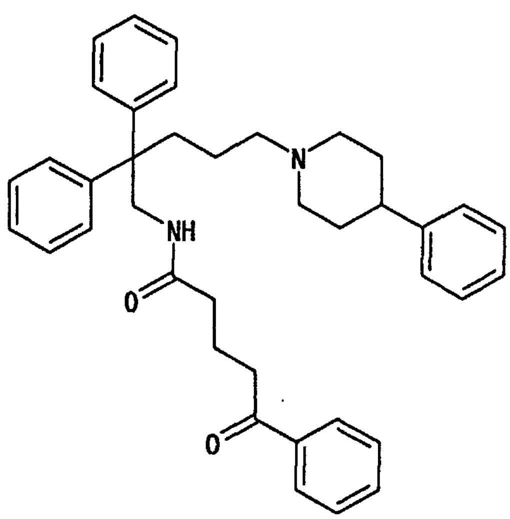

The compound was synthesized in the same manner as in Example 14.
Recrystallization solvent: ethyl acetate-hexane.

melting point: 101-102°C.

**Example 17**

N-(2,2-diphenyl-5-(4-phenylpiperidino)pentyl)-4-phenylbenzamide

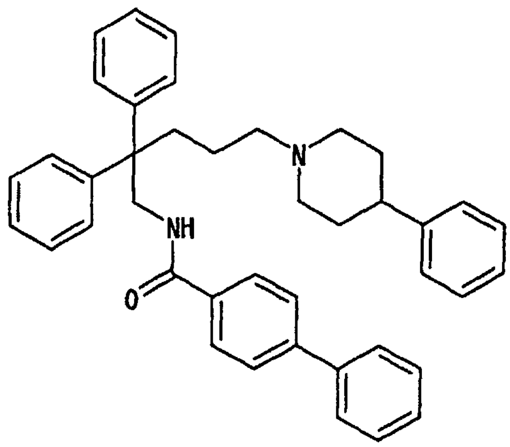

To a solution of 2,2-diphenyl-5-(4-phenylpiperidino)-pentanamine (150 mg) synthesized in Reference Example 5D and triethylamine (57 mg) in THF (5 ml) was added 4-biphenylcarbonyl chloride (98 mg) under ice-cooling, and the mixture was stirred for 1.5 h. To the reaction mixture was added saturated aqueous sodium hydrogen carbonate (100 ml). The mixture was extracted with ethyl acetate (100 ml), and after washing with saturated brine, dried over anhydrous sodium sulfate and concentrated under reduced pressure. The resulting residue was applied to silica gel column chromatography and eluted with ethyl acetate-ethyl acetate-methanol (20:1) to give the title compound (110 mg).
Recrystallization solvent: ether-hexane.
melting point: 155-156°C

**Example 18**

N-(2,2-diphenyl-5-(4-phenylpiperidino)pentyl)-3-ethoxycarbonylpropanamide

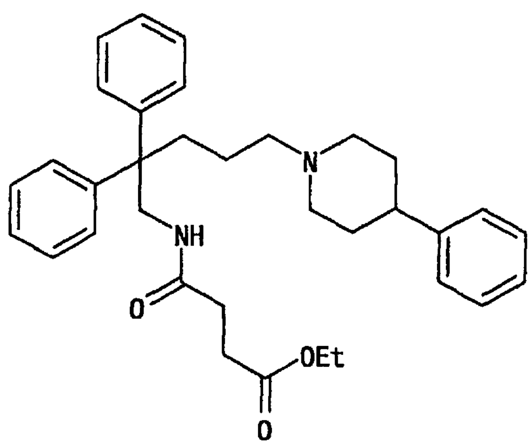

The compound was synthesized in the same manner as in Example 17.

amorphous powder.
$^1$H-NMR ($CDCl_3$)δ: 1.19-1.31 (5H, m), 1.68-2.60 (15H, m), 2.85-2.91 (2H, m), 3.99-4.02 (2H, m), 4.09 (2H, q, J=7.1Hz), 7.17-7.33 (15H, m).

**Example 19**

2-acetoxy-N-(2,2-diphenyl-5-(4-phenylpiperidino)pentyl)-acetamide

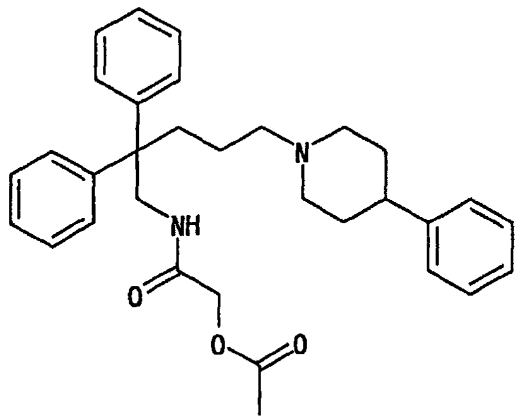

The compound was synthesized in the same manner as in Example 17.
Recrystallization solvent: diethyl ether-hexane.
melting point: 106-107°C.

**Example 20**

benzyl (1S)-2-((2,2-diphenyl-5-(4-phenylpiperidino)pentyl)-amino)-1-methyl-2-oxoethylcarbamate

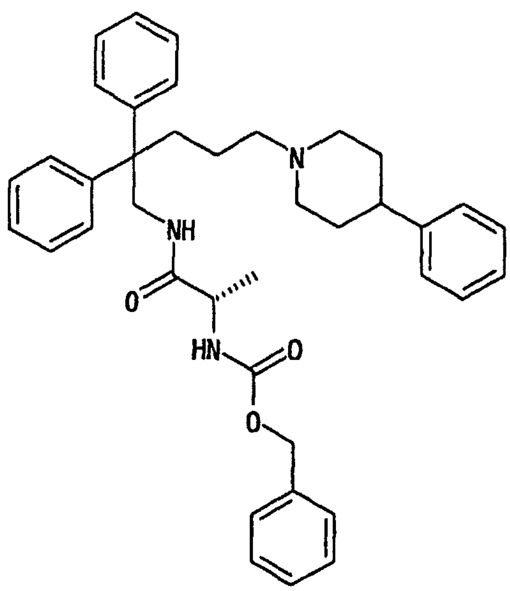

To a solution of 2,2-diphenyl-5-(4-phenylpiperidino)-pentanamine (110 mg) synthesized in Reference Example 5D, Z-L-alanine (68 mg) and HOBt (41 mg) in acetonitrile (10 ml) was added WSC (58 mg) at -20°C, and the mixture

was stirred at room temperature for 18 h. Water (100 ml) was added to the reaction mixture. The mixture was extracted with ethyl acetate (100 ml), and after washing with saturated brine, dried over anhydrous sodium sulfate and concentrated under reduced pressure. The resulting residue was applied to silica gel column chromatography and eluted with ethyl acetate-methanol (100:1-25:1) to give the title compound (120 mg).
amorphous powder.
$^1$H-NMR ($CDCl_3$)δ: 1.39 (3H, d, J=7.3Hz), 1.59 (4H, s) , 1.96 (2H, br), 2.58-2.92 (7H, m), 3.53-3.59 (1H, m), 3.71-3.82 (2H, m), 4.11-4.25 (2H, m), 5.07 (2H, s), 5.89 (1H, br), 6.30 (1H, br), 7.12-7.40 (20H, m).

**Example 21**

benzyl (1S)-1-(((2,2-diphenyl-5-(4-phenylpiperidino)pentyl)-amino)carbonyl)-3-methylbutylcarbamate

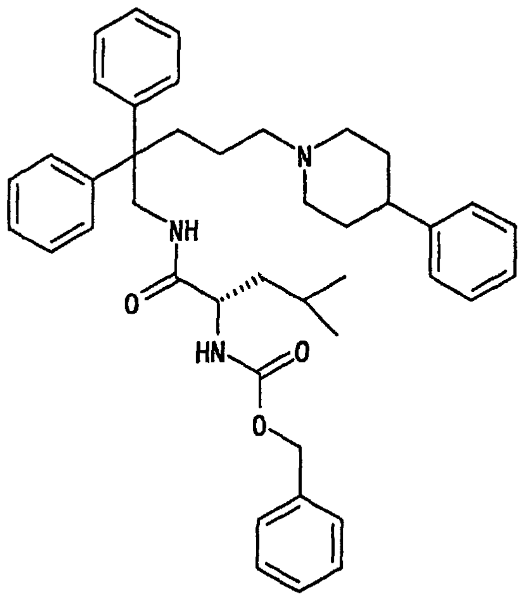

The compound was synthesized in the same manner as in Example 20.
amorphous powder.
$^1$H-NMR ($CDCl_3$) δ: 0.84 (6H, d, J=6.2Hz), 1.21-2.09 (12H, m), 2.22-2.45 (4H, m), 2.84-2.90 (2H, m) , 3.87-4.03 (2H, m), 4.08-4.17 (1H, m), 5.05 (2H, s), 5.12 (1H, d, J=8.4Hz), 5.56 (1H, brs), 7.14-7.35 (20H, m).

**Example 22**

benzyl (1S)-1-benzyl-((2,2-diphenyl-5-(4-phenylpiperidino)-pentyl)amino)-2-oxoethylcarbamate

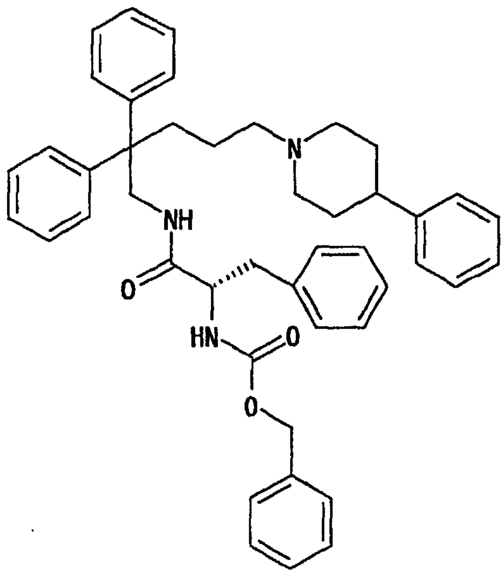

The compound was synthesized in the same manner as in Example 20.
amorphous powder.
$^1$H-NMR ($CDCl_3$)δ: 1.21-1.28 (3H, m), 1.81-2.03 (6H, m), 2.34-2.47 (3H, m), 2.99-3.02 (4H, m), 3.90-3.92 (2H, m), 4.06-4.30 (2H, m), 5.00 (2H, s), 5.42 (1H, br), 5.53 (1H, br), 7.04-7.32 (25H, m).

**Example 23**

benzyl (1S)-2-((2,2-diphenyl-5-(4-phenylpiperidino)pentyl)-amino)-1-hydroxymethyl-2-oxoethylcarbamate

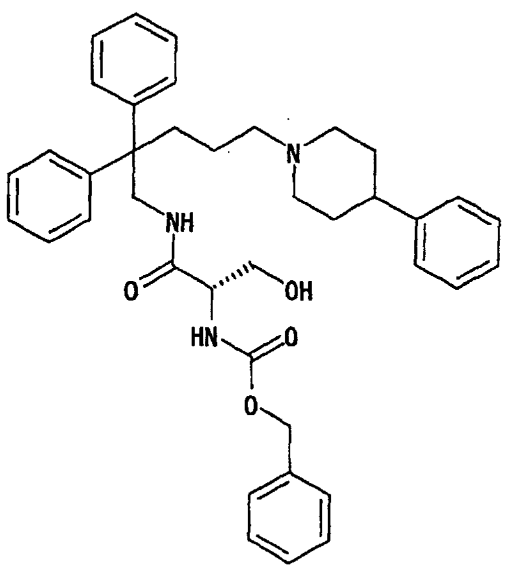

The compound was synthesized in the same manner as in Example 20.

amorphous powder.
$^1$H-NMR ($CDCl_3$)δ: 1.21-1.46 (2H, m), 1.82-2.30 (9H, m), 2.50-2.56 (3H, m), 3.15-3.32 (2H, m), 3.66 (1H, dd, J=11.1Hz, 4.7Hz), 3.84 (1H, dd, J=13.0Hz, 5.0Hz), 4.06-4.17 (3H, m), 5.02 (2H, s), 5.98 (1H, br), 6.02 (1H, br), 7.14-7.32 (20H, m).

## Example 24

benzyl (1R)-2-((2,2-diphenyl-5-(4-phenylpiperidino)pentyl)-amino)-1-methyl-2-oxoethylcarbamate

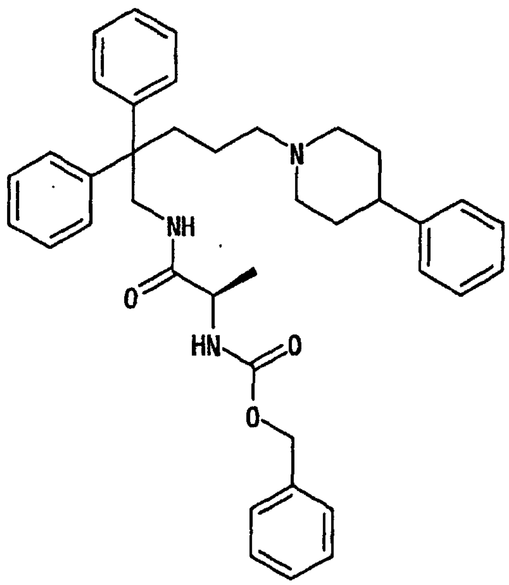

The compound was synthesized in the same manner as in Example 20.
amorphous powder.
$^1$H-NMR ($CDCl_3$)δ: 1.21-1.32 (6H, m), 1.71-2.09 (6H, m), 2.24-2.52 (3H, m), 2.87-2.93 (2H, m), 3.83-4.16 (4H, m), 5.05 (2H, s), 5.30 (1H, d, J=6.2Hz), 5.55 (1H, t, J=5.1Hz), 7.14-7.34 (20H, m).

**Example 25**

(2S)-1-benzyloxycarbonyl-2-(((2,2-diphenyl-5-(4-phenylpiperidino)pentyl)amino)carbonyl)pyrrolidine

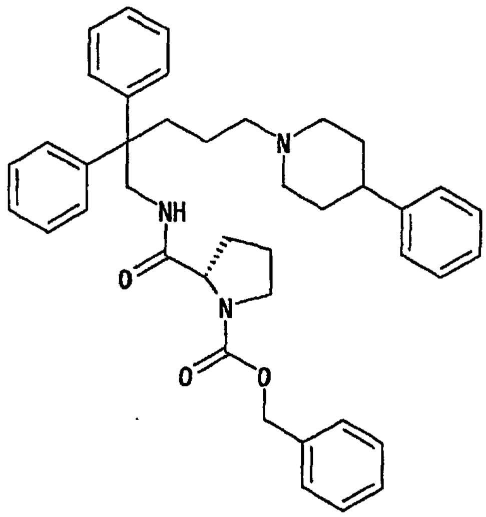

The compound was synthesized in the same manner as in Example 20.
amorphous powder.
$^1$H-NMR ($CDCl_3$)δ: 1.21-1.28 (2H, m), 1.61-2.47 (13H, m), 2.85-2.90 (2H, m), 3.18-3.34 (2H, m), 3.86-4.25 (3H, m), 5.07 (2H, brs), 5.54, 6.07 (1H, 2br), 7.16-7.34 (20H, m).

**Example 26**

N-(2,2-diphenyl-5-(4-phenylpiperidino)pentyl)-2-thiophenecarboxamide

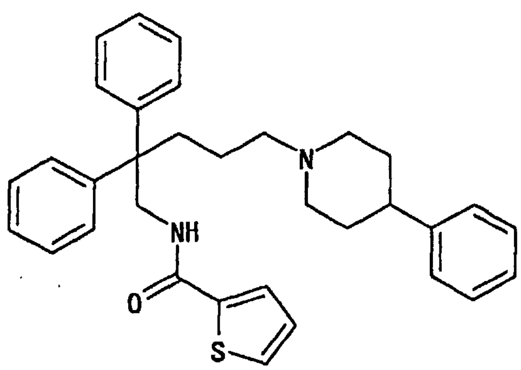

To a solution of 2,2-diphenyl-5-(4-phenylpiperidino)-pentanamine (510 mg) synthesized in Reference Example 5D and 2-thiophenecarboxylic acid (176 mg) in acetonitrile (20 ml) was added WSC (265 mg) under ice-cooling, and the mixture was stirred at room temperature for 16 h. Water (200 ml) was added to the reaction mixture. The mixture was extracted with ethyl acetate (200 ml), and after washing with saturated brine, dried over anhydrous sodium sulfate, and concentrated under reduced pressure. The resulting residue was applied to silica gel column chromatography and eluted with ethyl acetate-ethyl acetate-methanol (20:1) to give the title compound (510 mg). Recrystallization solvent: diethyl ether.
melting point: 80-81°C.

**Example 27**

4-benzyloxy-N-(2,2-diphenyl-5-(4-phenylpiperidino)pentyl)-benzamide

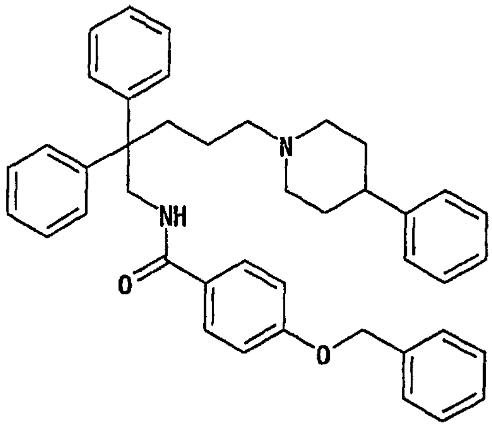

The compound was synthesized in the same manner as in Example 26.
Recrystallization solvent: ethyl acetate-hexane.
melting point: 148-149°C.

**Example 28**

N-(2,2-diphenyl-5-(4-phenylpiperidino)pentyl)-4-phenoxybenzamide hydrochloride

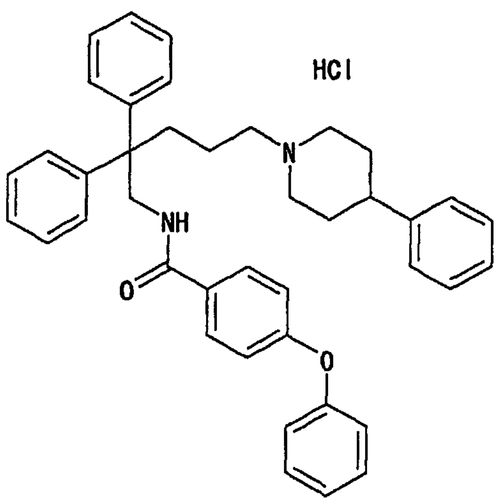

The compound was synthesized in the same manner as in Example 26 and converted to hydrochloride.
Recrystallization solvent: ethyl acetate-diethyl ether.
melting point: 199-204°C.

**Example 29**

5-bromo-N-(2,2-diphenyl-5-(4-phenylpiperidino)pentyl)-2-furancarboxamide

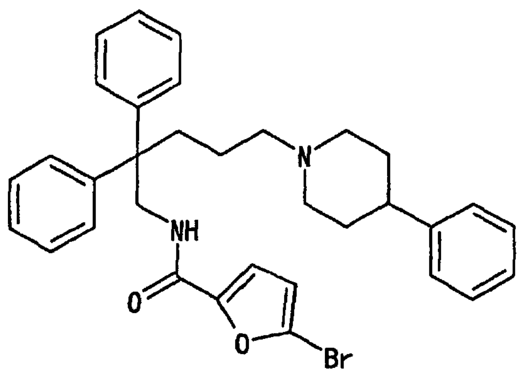

The compound was synthesized in the same manner as in Example 26. Recrystallization solvent: diethyl ether-hexane. melting point: 129-130°C.

**Example 30**

5-bromo-N-(2,2-diphenyl-5-(4-phenylpiperidino)pentyl)-2-thiophenecarboxamide

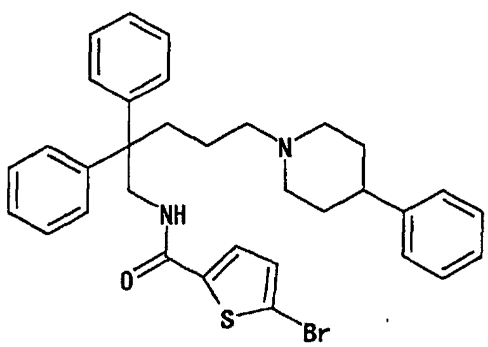

The compound was synthesized in the same manner as in Example 26. Recrystallization solvent: ethyl acetate-hexane. melting point: 147-148°C.

**Example 31**

N-(2,2-diphenyl-5-(4-phenylpiperidino)pentyl)-2-pyrrolecarboxamide

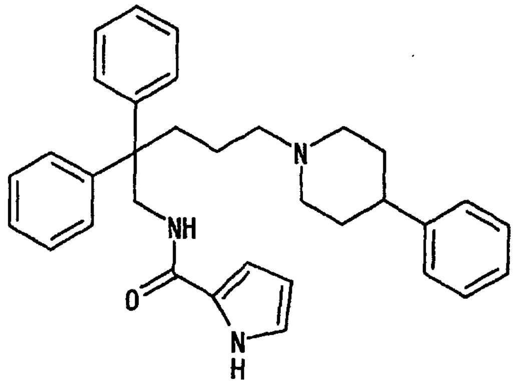

The compound was synthesized in the same manner as in Example 26.
Recrystallization solvent: diethyl ether-hexane.
melting point: 155-156°C.

**Example 32**

3-bromo-N-(2,2-diphenyl-5-(4-phenylpiperidino)pentyl)benzamide

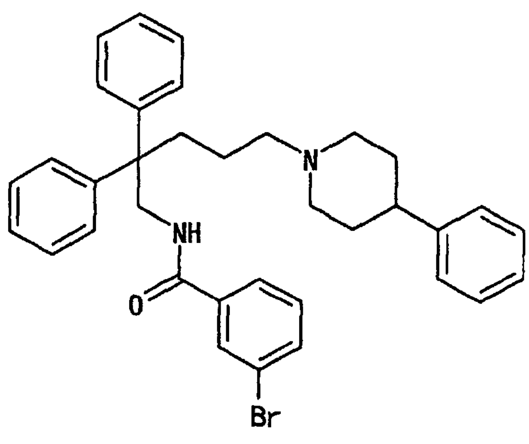

The compound was synthesized in the same manner as in Example 26.
Recrystallization solvent: diethyl ether-hexane.
melting point: 103-104°C.

**Example 33**

N-(2,2-diphenyl-5-(4-phenylpiperidino)pentyl)-4-biphenylacetamide

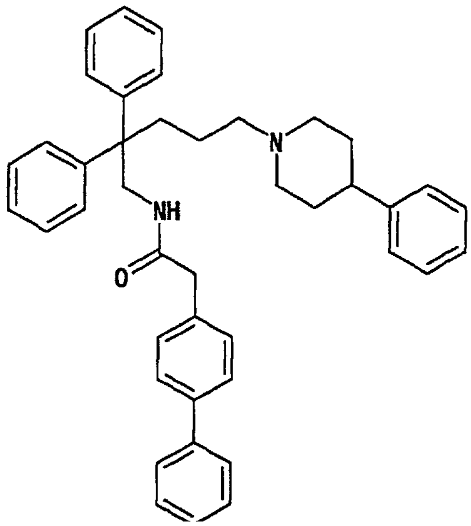

The compound was synthesized in the same manner as in Example 26.
Recrystallization solvent: ethyl acetate-hexane.
melting point: 86-87°C.

**Example 34**

3-benzyloxy-N-(2,2-diphenyl-5-(4-phenylpiperidino)pentyl)-benzamide

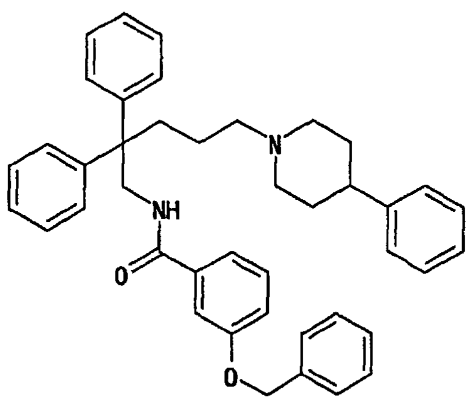

The compound was synthesized in the same manner as in Example 26.
Recrystallization solvent: ethyl acetate-diethyl ether.
melting point: 122-123°C.

**Example 35**

2-benzoylamino-N-(2,2-diphenyl-5-(4-phenylpiperidino)-pentyl)thiazole-4-carboxamide

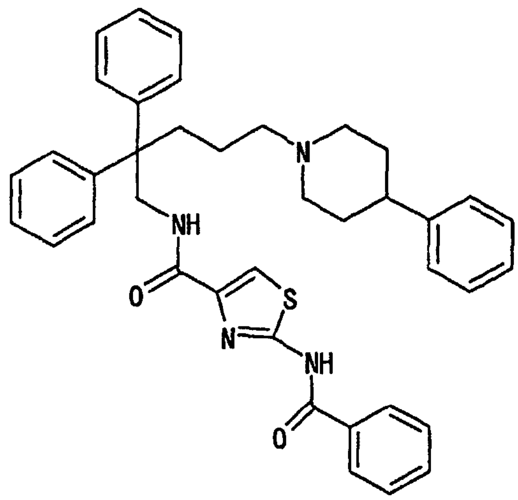

The compound was synthesized in the same manner as in Example 26.
Recrystallization solvent: ethyl acetate-hexane.
melting point: 181-182°C.

**Example 36**

4-bromo-N-(2,2-diphenyl-5-(4-phenylpiperidino)pentyl)benzamide

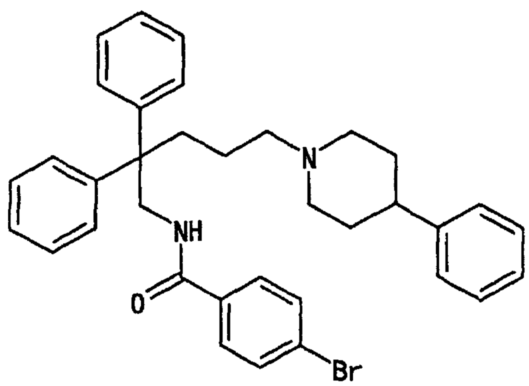

The compound was synthesized in the same manner as in Example 17.
Recrystallization solvent: ethyl acetate-hexane.
melting point: 140-141°C.

**Example 37**

4-benzoylamino-N-(2,2-diphenyl-5-(4-phenylpiperidino)-pentyl)benzamide

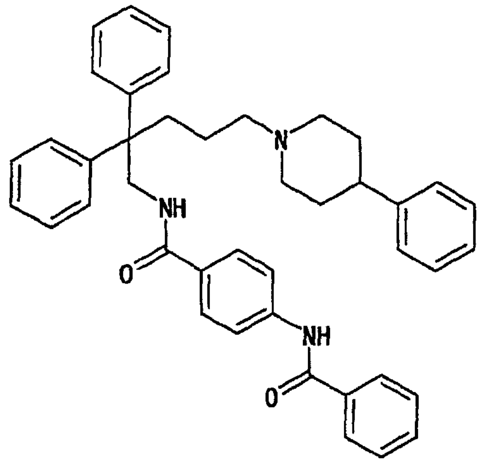

The compound was synthesized in the same manner as in Example 26.
amorphous powder.
$^1$H-NMR ($CDCl_3$)δ: 1.26-1.43 (3H, m), 1.63-1.92 (5H, m), 2.13-2.41 (5H, m), 2.84-2.91 (2H, m) , 4.18 (2H, d, J=5.8Hz), 5.67 (1H, brs), 7.14-7.39 (15H, m), 7.46-7.58 (5H, m), 7.67 (2H, d, J=8.8Hz), 7.84-7.89 (3H, m).

**Example 38**

N-(2,2-diphenyl-5-(4-phenylpiperidino)pentyl)-4-(phenylaminocarbonyl)benzamide

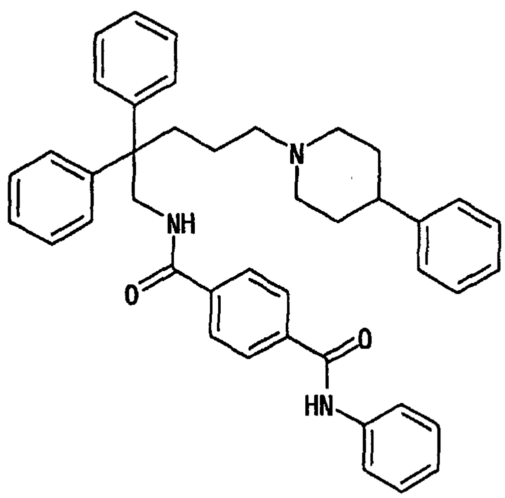

The compound was synthesized in the same manner as in Example 26.
Recrystallization solvent: ethyl acetate-diethyl ether.
melting point: 134-135°C.

**Example 39**

benzyl 3-((2,2-diphenyl-5-(4-phenylpiperidino)pentyl)amino)-3-oxopropylcarbamate

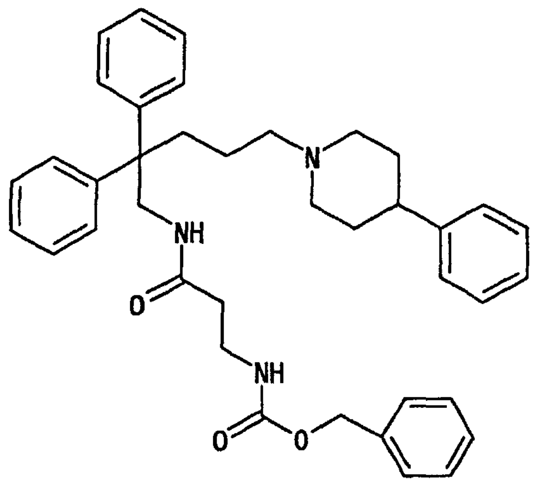

The compound was synthesized in the same manner as in Example 26.
amorphous powder.
$^{1}$H-NMR ($CDCl_3$) δ: 1.21-1.33 (2H, m) , 1.67-2.09 (8H, m) , 2.20-2.61 (5H, m), 2.89-2.94 (2H, m), 3.42 (2H, q, J=5.9Hz), 4.00 (2H, d, J=6.1Hz), 5.08 (1H, br), 5.11 (2H, s), 6.05 (1H, br), 7.13-7.37 (20H, m).

**Example 40**

benzyl 4-((2,2-diphenyl-5-(4-phenylpiperidino)pentyl)amino)-4-oxobutylcarbamate

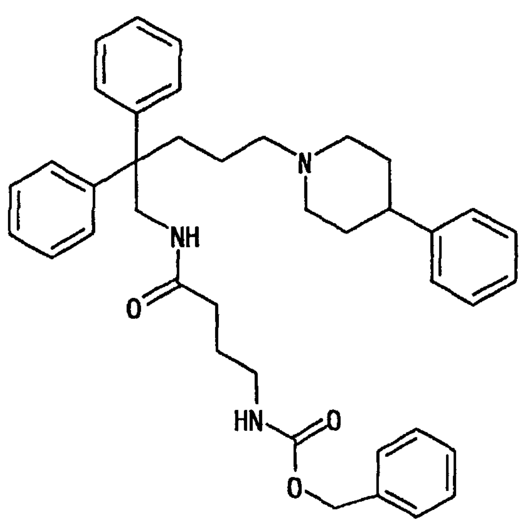

The compound was synthesized in the same manner as in Example 26.
amorphous powder.
$^{1}$H-NMR ($CDCl_3$)δ: 1.21-1.38 (2H, m), 1.67-2.12 (12H, m), 2.24-2.52 (3H, m), 2.86-2.91 (2H, m), 3.10 (2H, q, J=6.4Hz), 4.00 (2H, d, J=5.9Hz) , 5.05 (2H, s) , 5.14 (1H, br) , 5.30 (1H, br), 7.17-7.32 (20H, m).

**Example 41**

benzyl N-(2-((2,2-diphenyl-5-(4-phenylpiperidino)pentyl)amino)-2-oxoethyl)-N-methyl)carbamate hydrochloride

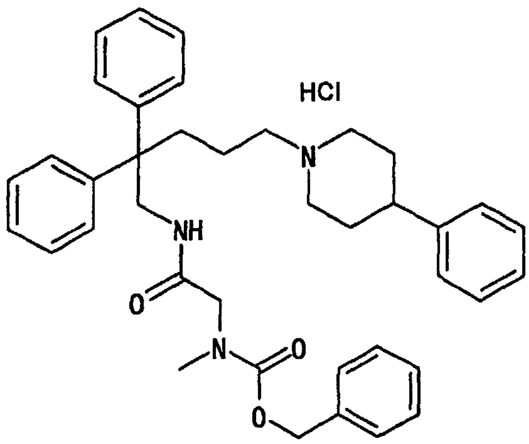

The compound was synthesized in the same manner as in Example 26.
amorphous powder.
$^1$H-NMR ($CDCl_3$)δ: 1.53-1.68 (3H, m), 2.17-2.28 (2H, m), 2.65 (4H, brs), 2.89 (5H, br s), 3.49-3.55 (2H, m), 3.88-4.00 (4H, m), 5.08 (2H, s), 5.66 (1H, t, J=5.7Hz), 7.15-7.35 (20H, m), 12.02 (1H, br).

**Example 42**

1-benzyl-3-(2,2-diphenyl-5-(4-phenylpiperidino)pentyl)urea

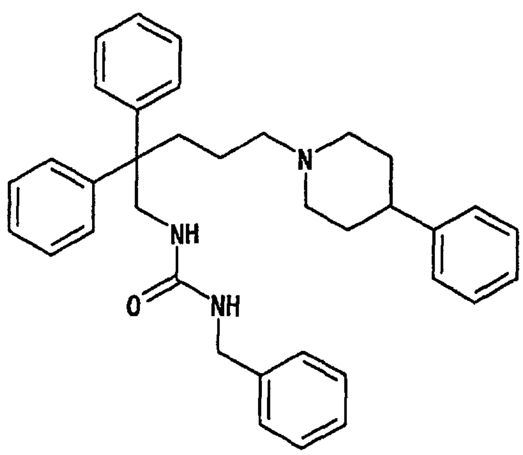

A solution of 2,2-diphenyl-5-(4-phenylpiperidino)-pentanamine (110 mg) synthesized in Reference Example 5D and benzyl isocynate (41 mg) in pyridine (5 ml) was stirred at room temperature for 3 h and concentrated under reduced pressure. This residue was partitioned between water (100 ml) and ethyl acetate (100 ml). The organic layer was washed with saturated brine, dried over anhydrous sodium sulfate and concentrated under reduced pressure. The resulting residue was recrystallized from ethyl acetate-hexane to give the title compound (114 mg).
melting point: 156-157°C.

**Example 43**

N-(2-((2,2-diphenyl-5-(4-phenylpiperidino)pentyl)amino)-2-oxoethyl)pyrrole-2-carboxamide

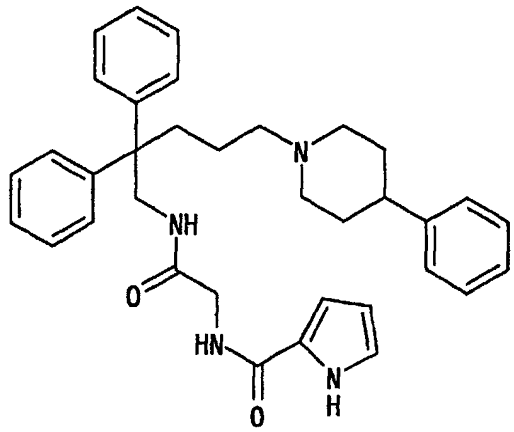

To a solution of tert-butyl 2-((2,2-diphenyl-5-(4-phenylpiperidino)pentyl)amino)-2-oxoethylcarbamate (4.0 g) synthesized in Example 2 in ethyl acetate (60 ml) was added 4N hydrogen chloride-ethyl acetate (150 ml) and the mixture was stirred at room temperature for 1 h. The reaction mixture was concentrated and reprecipitated with methanol-ethyl acetate to give diamine as hydrochloride (4.3 g). To a solution of amine hydrochloride (200 mg), pyrrole-2-carboxylic acid (46 mg) and triethylamine (77 mg) in acetonitrile (15 ml) was added WSC (80 mg) under ice-cooling, and the mixture was stirred at room temperature for 19 h. Water (200 ml) was added to the reaction mixture and the mixture extracted with ethyl acetate (200 ml), and after washing with saturated brine, dried over anhydrous sodium sulfate and concentrated under reduced pressure. The resulting residue was applied to silica gel column chromatography, eluted with ethyl acetate-ethyl acetate-methanol (5:1), and recrystallized from ethyl acetate-hexane to give the title compound (97 mg).
melting point: 158-159°C.

**Example 44**

N-(2-((2,2-diphenyl-5-(4-phenylpiperidino)pentyl)amino)-2-oxoethyl)indole-2-carboxamide

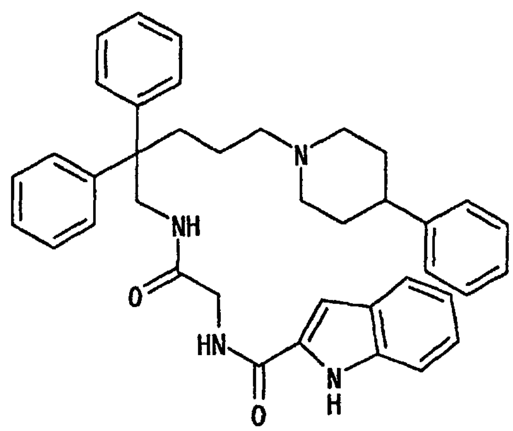

The compound was synthesized in the same manner as in Example 43.
Recrystallization solvent: ethyl acetate-hexane.

melting point: 184-185°C.

**Example 45**

N-(2-((2,2-diphenyl-5-(4-phenylpiperidino)pentyl)amino)-2-oxoethyl)-1-methylindole-2-carboxamide

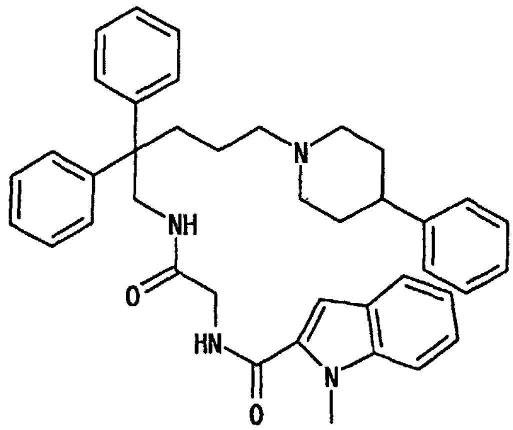

The compound was synthesized in the same manner as in Example 43.
amorphous powder.
$^1$H-NMR ($CDCl_3$)δ: 1.22-1.38 (2H, m), 1.76-2.48 (11H, m), 2.94-2.99 (2H, m), 3.97 (3H, s), 3.98-4.06 (4H, m), 5.76 (1H, brs), 6.93 (1H, s), 7.11-7.41 (19H, m), 7.63-7.67 (1H, m).

**Example 46**

N-(2-((2,2-diphenyl-5-(4-phenylpiperidino)pentyl)amino)-2-oxoethyl)benzofuran-2-carboxamide

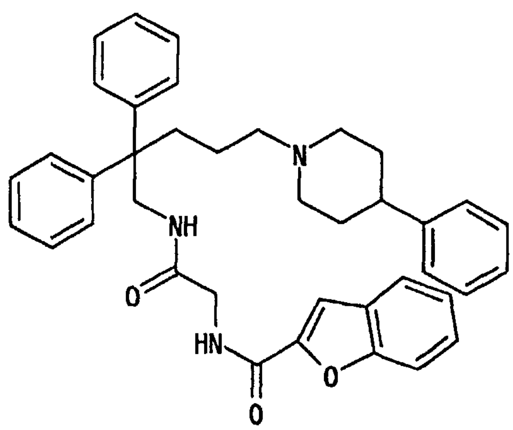

The compound was synthesized in the same manner as in Example 43.
amorphous powder.
$^1$H-NMR ($CDCl_3$)δ: 1.26-1.34 (2H, m), 1.80-2.47 (11H, m), 2.94-2.99 (2H, m), 4.01-4.07 (4H, m), 5.61 (1H, brs), 7.06-7.55 (20H, m), 7.68-7.72 (1H, m).

**Example 47**

5-chloro-N-(2-((2,2-diphenyl-5-(4-phenylpiperidino)pentyl)-amino)-2-oxoethyl)indole-2-carboxamide

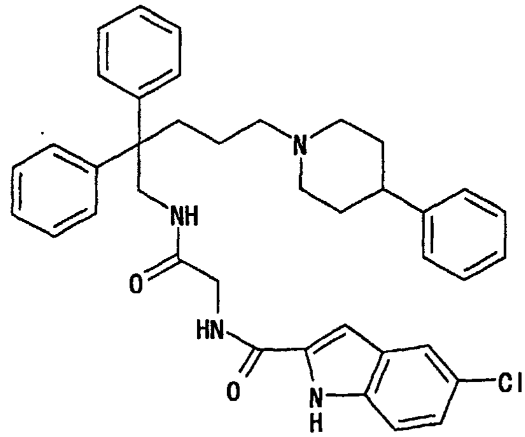

The compound was synthesized in the same manner as in Example 43.
amorphous powder.
$^1$H-NMR ($CDCl_3$) δ: 1.22-1.29 (2H, m), 1.73-2.35 (13H, m), 2.94-2.98 (2H, m) , 3.98-4.17 (4H, m), 5.80 (1H, brs), 6.92 (1H, s), 7.06-7.63 (19H, m), 9.92 (1H, s).

**Example 48**

N-(2-((2,2-diphenyl-5-(4-phenylpiperidino)pentyl)amino)-2-oxoethyl)naphthalene-2-carboxamide

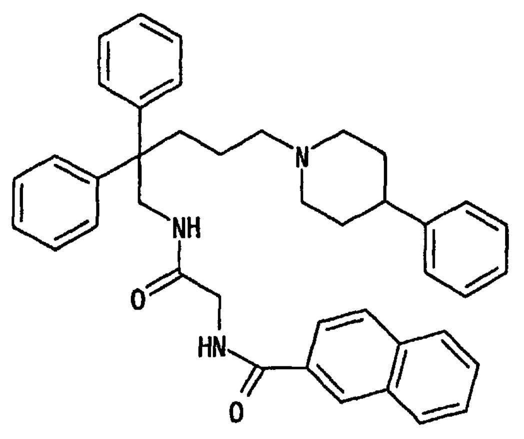

The compound was synthesized in the same manner as in Example 43.
amorphous powder.
$^1$H-NMR ($CDCl_3$)δ: 1.25-1.33 (2H, m), 1.75-2.19 (9H, m), 2.30-2.38 (3H, m), 2.94-2.99 (2H, m) , 3.98-4.05 (4H, m), 5.92 (1H, brs), 7.09-7.26 (15H, m), 7.53-7.59 (3H, m), 7.86-7.88 (4H, m), 8.32 (1H, s).

**Example 49**

N-(2-((2,2-diphenyl-5-(4-phenylpiperidino)pentyl)amino)-2-oxoethyl)-1-hydroxynaphthalene-2-carboxamide

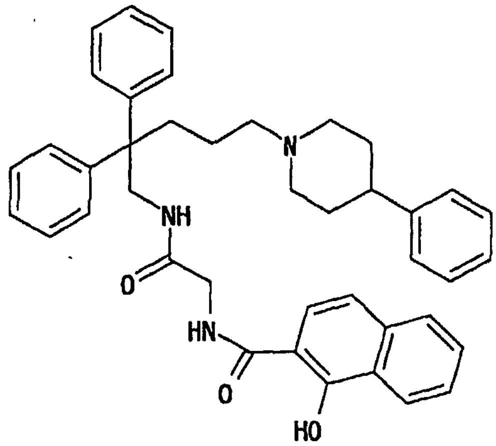

The compound was synthesized in the same manner as in Example 43.
amorphous powder.
$^1$H-NMR ($CDCl_3$)δ: 1.25-1.33 (2H, m), 1.75-2.11 (10H, m) , 2.38-2.46 (3H, m), 3.11-3.15 (2H, m), 4.00-4.05 (4H, m), 5.89 (1H, brs), 7.08-7.24 (18H, m), 7.45-7.72 (3H, m), 8.39-8.43 (1H, m) .

**Example 50**

N-(2-((2,2-diphenyl-5-(4-phenylpiperidino)pentyl)amino)-2-oxoethyl)-3-hydroxynaphthalene-2-carboxamide

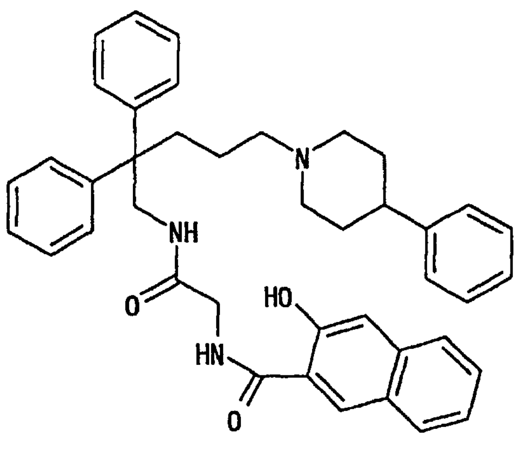

The compound was synthesized in the same manner as in Example 43.
amorphous powder.
$^1$H-NMR ($CDCl_3$) δ: 1.26-1.34 (2H, m), 1.85-2.21 (10H, m), 2.42-2.49 (3H, m), 3.12-3.19 (2H, m), 4.01-4.06 (4H, m), 5.80 (1H, brs), 7.08-7.35 (18H, m), 7.48-7.53 (1H, m), 7.68 (1H, d, J=8.1Hz), 7.84 (1H, d, J=8.4Hz).

**Example 51**

N-(2-((2-((2,2-Biphenyl-5-(4-phenylpiperidino)pentyl)amino)-2-oxoethyl)amino)-2-oxoethyl)-2,2,2-trifluoro-N-phenylacetamide

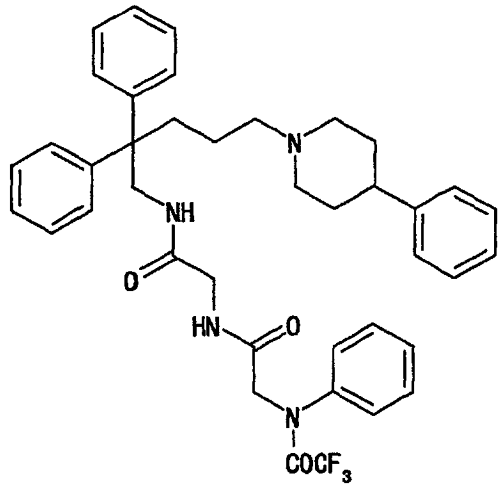

The compound was synthesized in the same manner as in Example 43.
Recrystallization solvent: ethyl acetate-hexane.
melting point: 145-146°C.

**Example 52**

N-(2-((2,2-diphenyl-5-(4-phenylpiperidino)pentyl)amino)-2-oxoethyl)indole-3-carboxamide

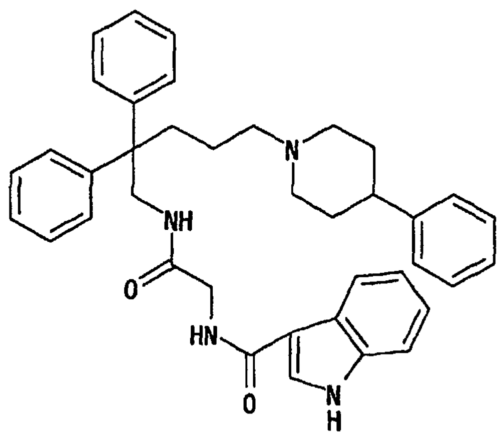

The compound was synthesized in the same manner as in Example 43.
Recrystallization solvent: ethyl acetate-hexane.
melting point: 153-154°C.

**Example 53**

N-(2-((2,2-diphenyl-5-(4-phenylpiperidino)pentyl)amino)-2-oxoethyl)-4-biphenylcarboxamide

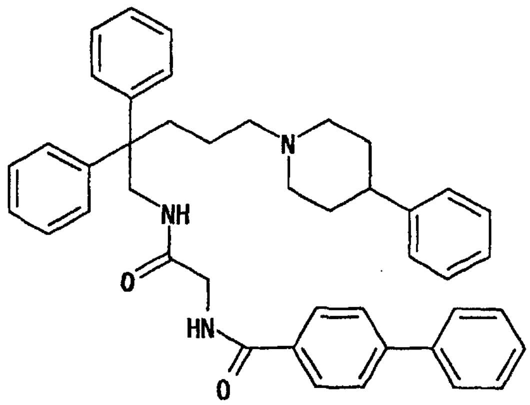

In the same manner as in Example 43, diamine hydrochloride was synthesized. To a solution of diamine hydrochloride (120 mg) and triethylamine (80 mg) in THF (10 ml) was added 4-biphenylcarbonyl chloride (54 mg) under ice-cooling, and the mixture was stirred for 2 h. Saturated aqueous sodium hydrogen carbonate (100 ml) was added and the reaction mixture was extracted with ethyl acetate (100 ml), and after washing with saturated brine, dried over anhydrous sodium sulfate and concentrated under reduced pressure. The resulting residue was applied to silica gel column chromatography and eluted with ethyl acetate-ethyl acetate-methanol (10:1) to give the title compound (100 mg).
amorphous powder.
$^1$H-NMR ($CDCl_3$)δ: 1.22-1.48 (2H, m), 1.81-2.36 (9H, m), 2.42-2.68 (3H, m), 3.18-3.22 (2H, m), 4.01-4.13 (4H, m), 5.81 (1H, brs), 7.10-7.29 (20H, m), 7.40-7.51 (1H, m), 7.62-7.70 (2H, m), 7.93 (1H, d, J=8.3Hz).

**Example 54**

N-(2-((2,2-diphenyl-5-(4-phenylpiperidino)pentyl)amino)-2-oxoethyl)benzamide

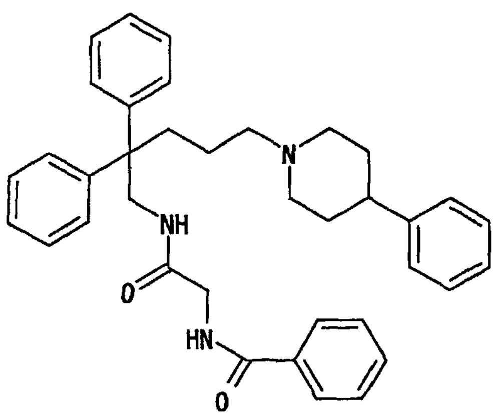

The compound was synthesized in the same manner as in Example 53.
amorphous powder.
$^1$H-NMR ($CDCl_3$)δ: 1.24-1.55 (2H, m), 1.82-1.89 (2H, m), 2.02-2.31 (9H, m), 2.54-2.60 (3H, m), 3.25-3.30 (2H, m), 3.98-4.09 (2H, m) , 5.89-5.92 (1H, m) , 7.07-7.54 (18H, m), 7.79-7.83 (1H, m), 8.04-8.18 (1H, m).

**Example 55**

N-(2-((2,2-diphenyl-5-(4-phenylpiperidino)pentyl)amino)-2-oxoethyl)-3-phenylpropanamide

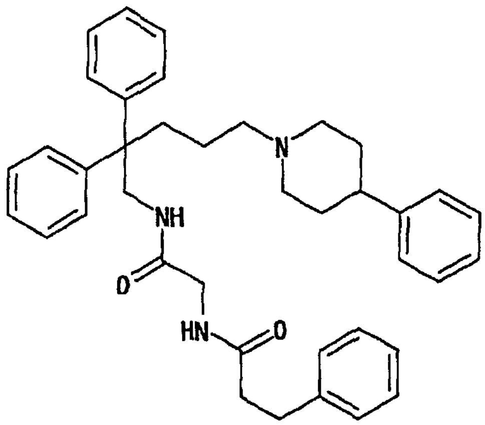

The compound was synthesized in the same manner as in Example 53.
amorphous powder.
$^1$H-NMR ($CDCl_3$)δ: 1.21-1.38 (2H, m), 1.78-2.17 (9H, m), 2.39-2.53 (4H, m), 2.83-3.03 (4H, m), 3.73 (2H, d, J=5.3Hz), 3.99 (2H, d, J=5.9Hz), 5.67-5.73 (1H, m), 6.69-6.74 (1H, m), 7.13-7.31 (20H, m).

**Example 56**

4-nitrobenzyl 2-((2,2-diphenyl-5-(4-phenylpiperidino)-pentyl)amino)-2-oxoethylcarbamate

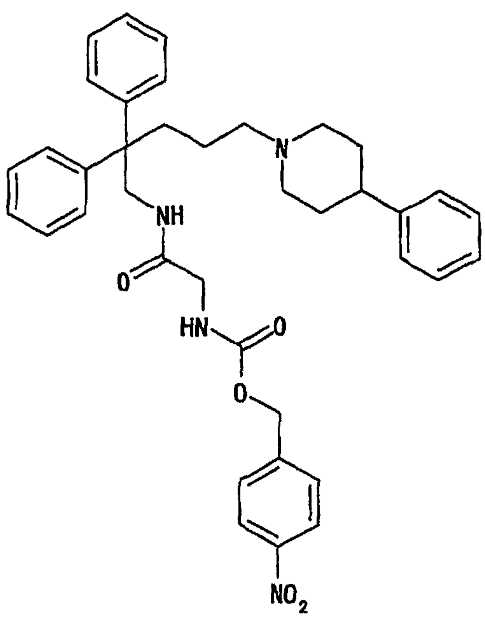

The compound was synthesized in the same manner as in Example 53.
amorphous powder.
$^1$H-NMR ($CDCl_3$)δ: 1.21-1.33 (2H, m) , 1.78-2.15 (8H, m), 2.29-2.49 (3H, m), 2.92-2.97 (2H, m) , 3.73 (2H, d, J=5.5Hz),

4.01 (2H, d, J=5.9Hz), 5.14 (2H, s), 5.53 (1H, s), 5.79 (1H, s), 7.13-7.32 (15H, m), 7.45 (2H, d, J=8.5Hz), 8.18 (2H, d, J=8.7Hz).

**Example 57**

4-chlorobenzyl 2-((2,2-diphenyl-5-(4-phenylpiperidino)-pentyl)amino)-2-oxoethylcarbamate

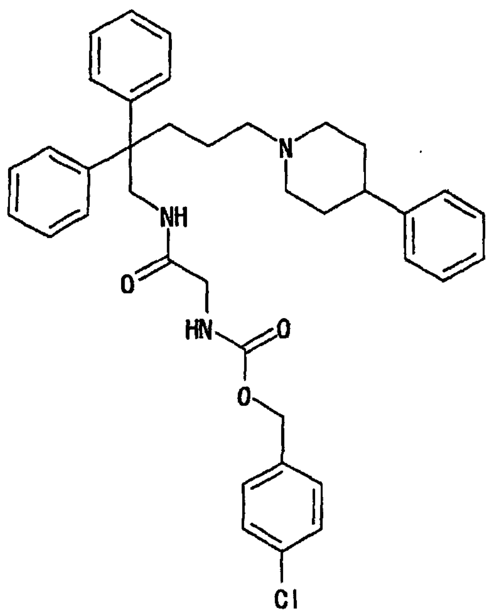

In the same manner as in Example 43, diamine hydrochloride was synthesized. To a solution of diamine hydrochloride (150 mg) and triethylamine (115 mg) in THF (6 ml) was added O-4-chlorobenzyl-O'-4-nitrophenylcarbonate (175 mg) and the mixture was stirred at room temperature for 18 h. Saturated aqueous sodium hydrogen carbonate (100 ml) was added to the reaction mixture, and the mixture was extracted with ethyl acetate (100 ml), and after washing with saturated brine, dried over anhydrous sodium sulfate and concentrated under reduced pressure. The resulting residue was applied to silica gel column chromatography, eluted with ethyl acetate-ethyl acetate-methanol (10:1) and recrystallized from ethyl acetate-hexane to give the title compound (124 mg).
melting point: 130-131°C.

**Example 58**

3-chlorobenzyl 2-((2,2-diphenyl-5-(4-phenylpiperidino)pentyl)-amino)-2-oxoethylcarbamate

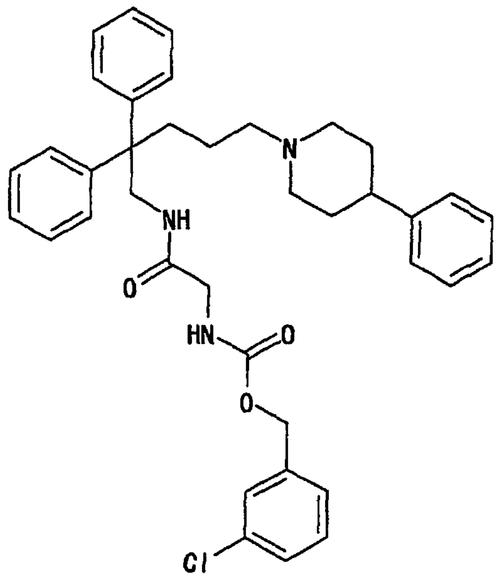

The compound was synthesized in the same manner as in Example 57.
Recrystallization solvent: diethyl ether.
melting point: 125-126°C.

**Example 59**

2-anilino-N-(2-((2,2-diphenyl-5-(4-phenylpiperidino)pentyl)-amino)-2-oxoethyl)acetamide

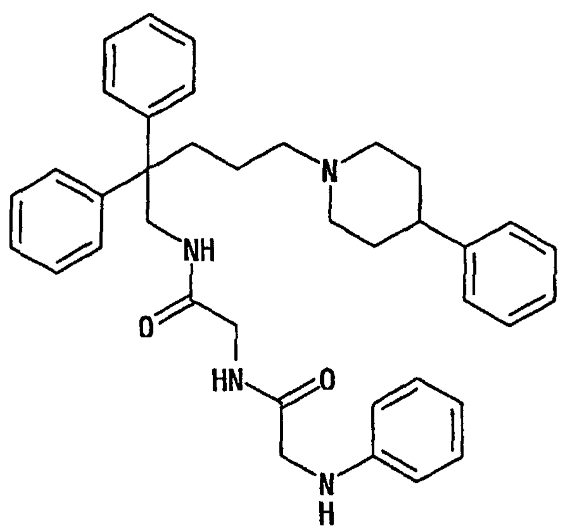

To a solution of N-(2-((2-((2,2-diphenyl-5-(4-phenylpiperidino)pentyl)amino)-2-oxoethyl)amino)-2-oxoethyl)-2,2,2-trifluoro-N-phenylacetamide (150 mg) synthesized in Example 51 in THF (3 ml) was added 5N aqueous sodium

hydroxide solution (3 ml) and the mixture was stirred at room temperature for 3 h. After the completion of the reaction, water was added to the reaction mixture, and the mixture was extracted with ethyl acetate and washed with saturated brine. The organic layer was dried over anhydrous magnesium sulfate and concentrated under reduced pressure. The resulting residue was applied to silica gel column chromatography and eluted with ethyl acetate-methanol-aqueous ammonia (50:10:1) to give the title compound (100 mg).
amorphous powder.
$^1$H-NMR ($CDCl_3$)δ: 1.22-1.30 (2H, m) , 1.71-2.13 (8H, m) , 2.29 (2H, t, J=7.2Hz), 2.35-2.45 (1H, m), 2.91 (2H, t, J=10.9Hz), 3.70 (2H, d, J=5.7Hz), 3.77 (2H, d, J=5.6Hz), 3.90 (2H, d, J=5.7Hz), 4.23 (1H, t, J=5.7Hz), 5.53 (1H, t, J=5.7Hz), 6.50 (2H, d, J=7.6Hz), 6.78 (1H, t, J=7.4Hz), 7.16-7.35 (18H, m).

**Example 60**

2-chlorobenzyl 2-((2,2-diphenyl-5-(4-phenylpiperidino)-pentyl)amino)-2-oxoethylcarbamate

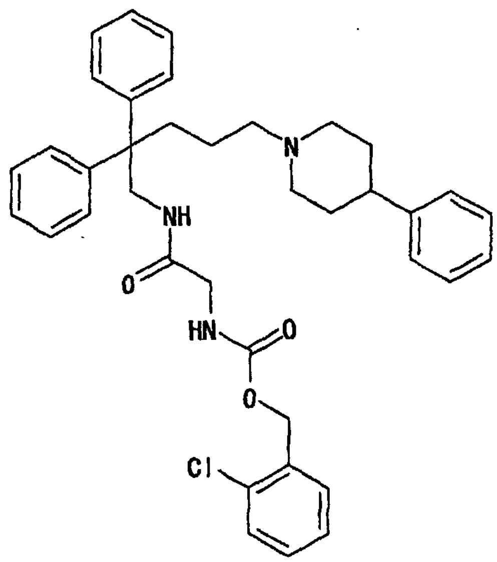

The compound was synthesized in the same manner as in Example 57.
Recrystallization solvent: diethyl ether.
melting point: 83-84°C.

**Example 61**

4-methoxybenzyl 2-((2,2-diphenyl-5-(4-phenylpiperidino)-pentyl)amino)-2-oxoethylcarbamate

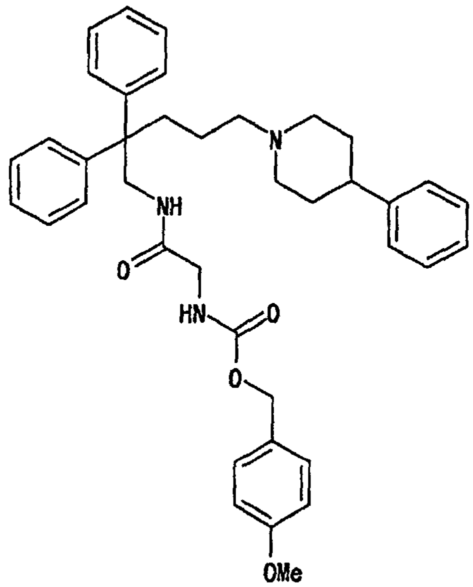

The compound was synthesized in the same manner as in Example 57.
Recrystallization solvent: ethyl acetate-diethyl ether-hexane.
melting point: 115-116°C.

**Example 62**

3-methoxybenzyl 2-((2,2-diphenyl-5-(4-phenylpiperidino)pentyl)-amino)-2-oxoethylcarbamate

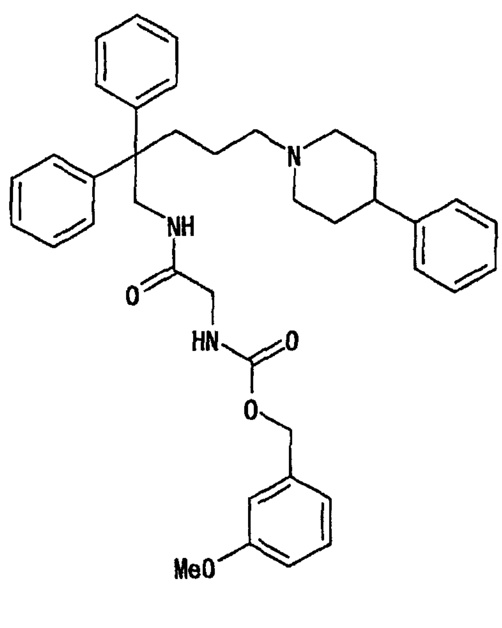

The compound was synthesized in the same manner as in Example 57.
Recrystallization solvent: ethyl acetate-diethyl ether-hexane.
melting point: 96-97°C.

**Example 63**

2-methoxybenzyl 2-((2,2-diphenyl-5-(4-phenylpiperidino)-pentyl)amino)-2-oxoethylcarbamate

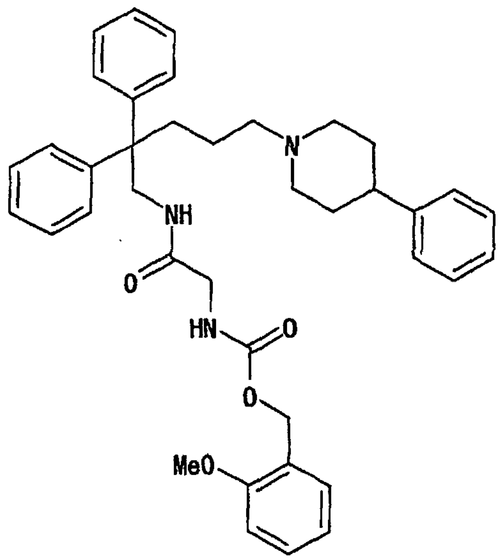

The compound was synthesized in the same manner as in Example 57.
amorphous powder.
$^1$H-NMR ($CDCl_3$)δ: 1.21-1.38 (2H, m), 1.70-2.11 (8H, m) , 2.26-2.52 (3H, m), 2.85-2.94 (2H, m), 3.71 (2H, d, J=5.8Hz). 3.83 (3H, m), 3.97 (2H, d, J=5.9Hz), 5.14 (2H, s), 5.41 (1H, brs), 5.62 (1H, brs), 6.87-6.98 (2H, m), 7.14-7.35 (17H, m) .

**Example 64**

benzyl (1S)-1-benzyl-2-((2-((2,2-diphenyl-5-(4-phenylpiperidino)pentyl)amino)-2-oxoethyl)amino)-2-oxoethylcarbamate

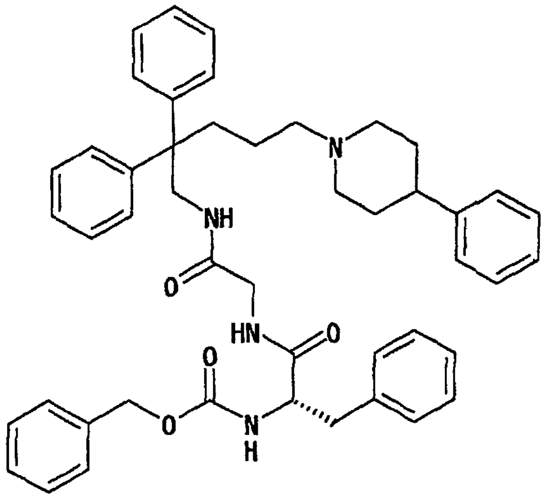

In the same manner as in Example 43, diamine hydrochloride was synthesized. To a solution of diamine hydrochloride (200 mg) , Z-L-phenylalanine (125 mg) and HOBt (79 mg) in acetonitrile (15 ml) was added WSC (80 mg) at -20°C, and the mixture was stirred at room temperature for 3 days. Water (100 ml) was added to the reaction mixture. The mixture was extracted with ethyl acetate (100 ml), and after washing with saturated brine, dried over anhydrous sodium sulfate and concentrated under reduced pressure. The resulting residue was applied to silica gel column chromatography and eluted with ethyl acetate-ethyl acetate-methanol (10:1) to give the title compound (123 mg).
amorphous powder.
$^1$H-NMR ($CDCl_3$)δ: 1.28-1.45 (2H, m) , 1.81-2.41 (8H, m) , 2.42-2.69 (2H, m), 2.83-3.31 (4H, m), 3.60-4.12 (5H, m), 4.40-4.59 (1H, m) , 5.02 (2H, s), 5.64 (1H, d, J=8.0Hz), 5.84 (1H, brs), 7.07-7.28 (25H, m), 7.58 (1H, brs).

**Example 65**

2-(((benzylamino)carbonyl)amino)-N-(2,2-diphenyl-5-(4-phenylpiperidino)pentyl)acetamide

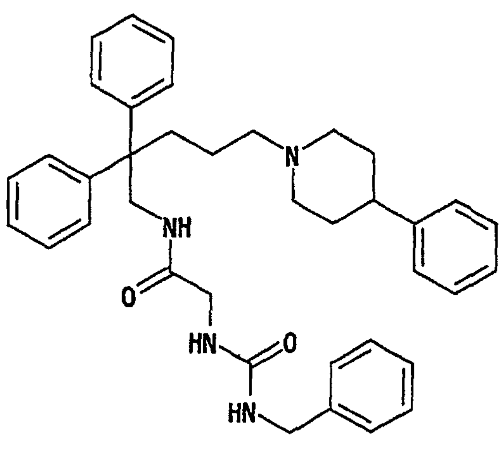

In the same manner as in Example 43, diamine hydrochloride was synthesized. A solution of diamine hydrochloride (150 mg) and benzyl isocyanate (42 mg) in pyridine (5 ml) was stirred at room temperature for 3 h. This reaction mixture was partitioned between saturated aqueous sodium hydrogen carbonate (100 ml) and ethyl acetate (100 ml). The organic layer was washed with saturated brine, dried over anhydrous sodium sulfate and concentrated under reduced pressure. The resulting residue was recrystallized from ethyl acetate-ethyl acetate-diethyl ether to give the title compound (130 mg).
melting point: 149-152°C.

**Example 66**

N-(2,2-diphenyl-5-(4-phenylpiperidino)pentyl)-5-hydroxy-5-phenylpentanamide

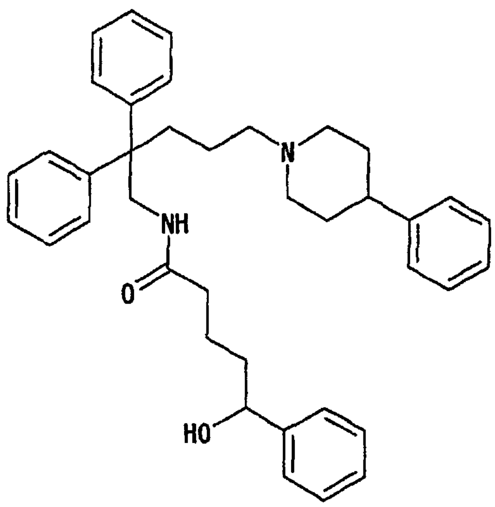

To a solution of 4-benzoyl-N-(2,2-diphenyl-5-(4-phenylpiperidino)pentyl)butylamide(100 mg) synthesized in Example 16 in a mixture of THF (2 ml) and methanol (2 ml) was added sodium borohydride (10 mg) under ice-cooling, and the mixture was stirred under ice-cooling for 30 min and at room temperature for 1 h. The reaction mixture was partitioned between water (100 ml) and ethyl acetate (100 ml). The organic layer was washed with saturated brine, dried over anhydrous sodium sulfate and concentrated under reduced pressure. The resulting residue was applied to silica gel column chromatography and eluted with ethyl acetate-methanol (20:1-10:1) to give the title compound (70 mg).
amorphous powder.
$^1$H-NMR ($CDCl_3$)δ: 1.25-1.36 (2H, m) , 1.61-1.77 (7H, m), 1.93-2.12 (6H, m), 2.31-2.51 (3H, m), 2.93-2.99 (2H, m), 3.56 (2H, s), 3.99 (2H, d, J=5.2Hz), 4.59-4.67 (1H, m), 5.16 (1H, s), 7.14-7.32 (20H, m).

**Example 67**

N-benzyl-N'-(2,2-diphenyl-5-(4-phenylpiperidino)-pentyl)succinamide

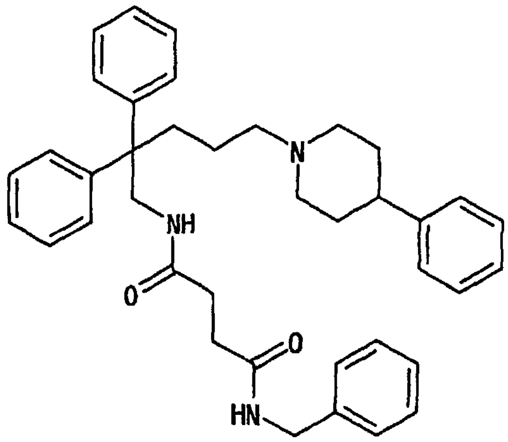

To a solution of N-(2,2-diphenyl-5-(4-phenylpiperidino)-pentyl)-3-ethoxycarbonylpropionylamide (100 mg) synthesized in Example 18 in THF (1 ml) was added 2N aqueous sodium hydroxide solution (1 ml) and the mixture was stirred at room temperature for 1 h. The reaction mixture was diluted with water (100 ml), neutralized with 2N hydrochloric acid (1 ml) and extracted with ethyl acetate. The organic layer was washed with saturated brine, dried over anhydrous sodium sulfate and concentrated under reduced pressure to give carboxylic acid. To a solution of the obtained carboxylic acid and benzylamine (23 mg) and HOBt (30 mg) in acetonitrile (5 ml) was added WSC (42 mg) at -20°C and the mixture was stirred for 16 h at room temperature. To the reaction mixture was added saturated aqueous ammonium chloride solution (100 ml). The mixture was extracted with ethyl acetate (100 ml), and after washing with saturated brine, dried over anhydrous sodium sulfate and concentrated under reduced pressure. The resulting residue was applied to silica gel column chromatography and eluted with ethyl acetate-ethyl acetate-methanol (10:1-20:3) to give the title compound (100 mg).
amorphous powder.
$^1$H-NMR ($CDCl_3$)δ: 1.21-1.35 (2H, m) , 1.72-2.15 (9H, m) , 2.27-2.50 (6H, m), 2.89-2.94 (2H, m), 3.96 (2H, d, J=6.1Hz), 4.37 (2H, d, J=5.9Hz), 5.41-5.47 (1H, m), 6.51-6.58 (1H, m), 7.14-7.33 (20H, m) .

**Example 68**

N-(2,2-diphenyl-5-(4-phenylpiperidino)pentyl)-3-((phenylacetyl)amino)propanamide

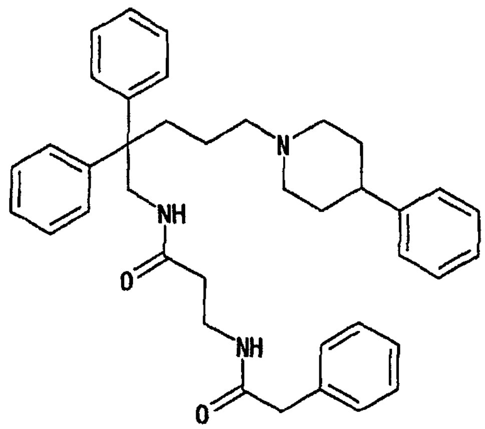

To a solution of benzyl 3-((2,2-diphenyl-5-(4-phenylpiperidino)pentyl) amino)-3-oxopropylcarbamate (370 mg) synthesized in Example 39 in ethanol (5 ml) was added 10% palladium carbon (37 mg) and the mixture was stirred at room temperature under a hydrogen atmosphere for 2 h and at 70°C for 10 h. The reaction mixture was filtered and the filtrate was concentrated under reduced pressure. The resulting residue was applied to silica gel column chromatography and eluted with ethyl acetate-methanol (5:1)-ethyl acetate-methanol-saturated aqueous ammonia (50:10:1) to give diamine. To a solution of diamine and triethylamine (74 mg) in THF (10 ml) was added phenylacetyl chloride (82 mg) under ice-cooling, and the mixture was stirred for 3 h. To the reaction mixture was added saturated aqueous sodium hydrogen carbonate (200 ml). The mixture was extracted with ethyl acetate (200 ml), and after washing with saturated brine, dried over anhydrous sodium sulfate and concentrated under reduced pressure. The resulting residue was applied to silica gel column chromatography, eluted with ethyl acetate-ethyl acetate-methanol (10:1) and recrystallized from ethyl acetate-hexane to give the title compound (68 mg).
melting point: 149-150°C.

**Example 69**

N-(4-((2,2-diphenyl-5-(4-phenylpiperidino)pentyl)amino)-4-oxobutyl)benzamide

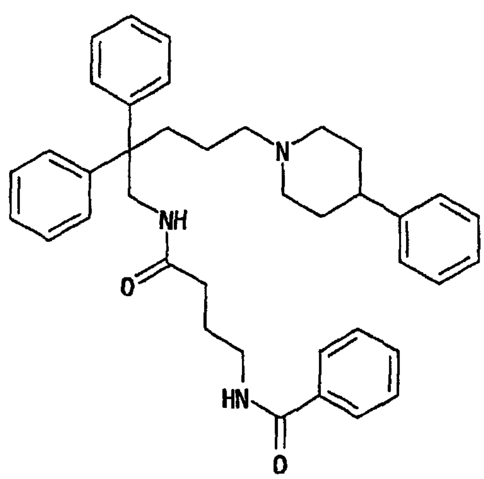

The compound was synthesized in the same manner as in Example 67 from benzyl 4-((2,2-diphenyl-5-(4-phenylpiperidino)-pentyl)amino)-4-oxobutylcarbamate synthesized in Example 40. Recrystallization solvent: ethyl acetate-hexane
melting point: 144-145°C.

**Example 70**

benzyl 2-((2,2-diphenyl-5-(4-phenylpiperidino)pentyl)amino)-2-oxoethylcarbonate

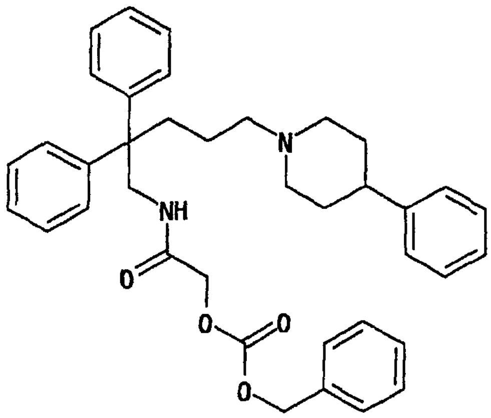

To a solution of 2-acetoxy-N-(2,2-diphenyl-5-(4-phenylpiperidino)pentyl)acetamide (130 mg) synthesized in Example 19 in THF (5 ml) was added 2N aqueous sodium hydroxide solution (5 ml) and the mixture was stirred at room temperature for 1 h. The reaction mixture was diluted with water (100 ml) and extracted with ethyl acetate. The organic layer was washed with saturated brine, dried over anhydrous sodium sulfate and concentrated under reduced pressure. To a solution of this residue, triethylamine (272 mg) and DMAP (2 mg) in THF (5 ml) was added carbobenzoxy chloride (448 mg) under ice-cooling, and the mixture was stirred at room temperature for 20 h. To the reaction mixture was added saturated aqueous sodium hydrogen carbonate (100 ml). The mixture was extracted with ethyl acetate (100 ml), and after washing with saturated brine, dried over anhydrous sodium sulfate and concentrated under reduced pressure. The resulting residue was applied to silica gel column chromatography and eluted with ethyl acetate-ethyl acetate-methanol (20:1-10:1) to give the title compound (70 mg). amorphous powder.
$^1$H-NMR ($CDCl_3$)δ: 1.19-1.37 (2H, m), 1.66-2.08 (6H, m), 2.23-2.43 (3H, m), 2.84-2.89 (2H, m), 3.99 (2H, d, J=5.9Hz), 4.52 (2H, s), 5.13 (2H, s), 5.77 (1H, brs), 7.14-7.39 (20H, m).

**Example 71**

N-(2,2-diphenyl-5-(4-phenylpiperidino)pentyl)-5-phenyl-2-thiophenecarboxamide

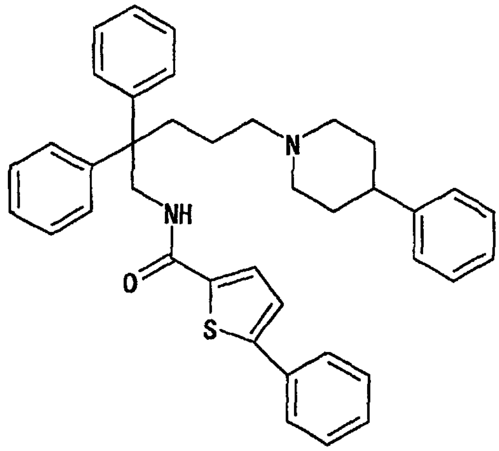

To a solution of 5-bromo-N-(2,2-diphenyl-5-(4-phenylpiperidino)pentyl)-2-thiophenecarboxamide (140 mg) synthesized in Example 30, phenyl borate (33 mg) and 2N aqueous sodium hydrogencarbonate solution (2 ml) in dimethoxyethane (10 ml) was added tetrakis(triphenylphosphine)palladium(0) (28 mg) and the mixture was heated under reflux under a nitrogen atmosphere for 6 h. After cooling, the reaction mixture was diluted with water (200 ml) and extracted with ethyl acetate (200 ml). The organic layer was washed with saturated brine, dried over anhydrous sodium sulfate and concentrated under reduced pressure. The residue was applied to silica gel column chromatography, eluted with ethyl acetate-hexane (2:1) and recrystallized to give the title compound (66 mg).
melting point: 137-138°C.

**Example 72**

N-(2,2-diphenyl-5-(4-phenylpiperidino)pentyl)-5-phenyl-2-furancarboxamide

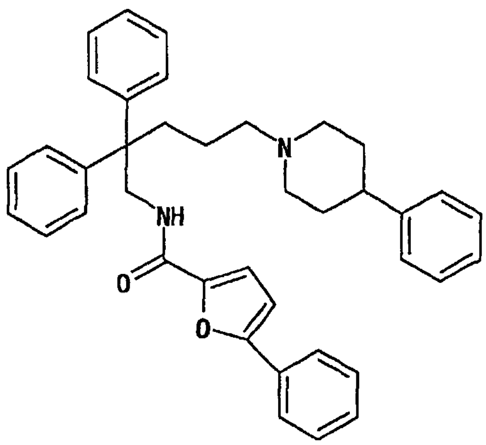

The compound was synthesized in the same manner as in Example 70 from 5-bromo-N-(2,2-diphenyl-5-(4-phenylpiperidino)-pentyl)-2-furancarboxamide synthesized in Example 29.
Recrystallization solvent: diethyl ether-hexane

melting point: 131-132°C.

**Example 73**

N-(2,2-diphenyl-5-(4-phenylpiperidino)pentyl)-3-phenylbenzamide

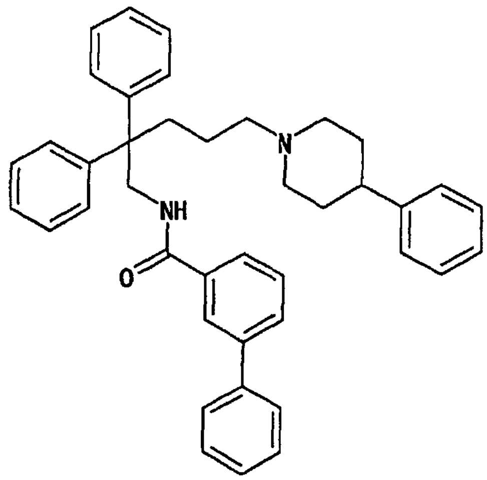

The compound was synthesized in the same manner as in Example 70 from 3-bromo-N-(2,2-diphenyl-5-(4-phenylpiperidino)-pentyl)benzamide synthesized in Example 32.
Recrystallization solvent: diethyl ether-hexane
melting point: 140-141°C.

**Example 74**

(2S)-2-amino-N-(2-((2,2-diphenyl-5-(4-phenylpiperidino)pentyl)-amino)-2-oxoethyl)-3-phenylpropanamide

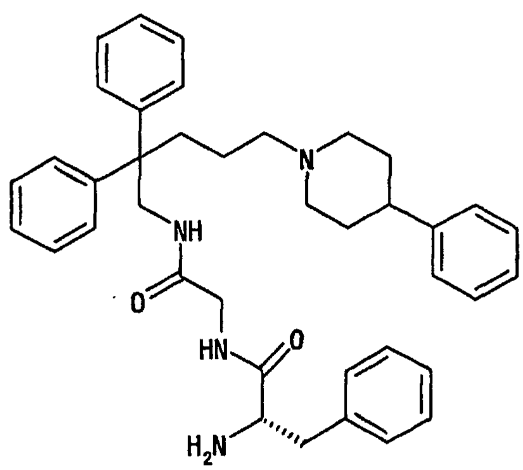

To a solution of benzyl (1S)-1-benzyl-2-((2-((2,2-diphenyl-5-(4-phenylpiperidino)pentyl)amino)-2-oxoethyl) amino)-2-oxoethylcarbamate (110 mg) synthesized in Example 64 in ethanol (5 ml) was added 10% palladium carbon (11 mg) and the mixture was stirred at room temperature under a hydrogen atmosphere for 20 h. The reaction mixture was filtered and the filtrate was concentrated under reduced pressure. The resulting residue was applied to silica gel

column chromatography and eluted with ethyl acetate-ethyl acetate-methanol (20:3)-ethyl acetate-methanol-saturated aqueous ammonia (50:10:1) to give the title compound (40 mg).
amorphous powder.
$^1$H-NMR ($CDCl_3$) δ: 1.21-1.42 (2H, m) , 1.70-2.19 (9H, m), 2.25-2.58 (3H, m), 2.60-3.29 (4H, m), 3.45-4.37 (6H, m), 5.58-5.81 (2H, m), 7.02-7.27 (20H, m), 7.87 (1H, brs).

**Example 75**

benzyl 2-(N-(2,2-diphenyl-5-(4-phenylpiperidino)pentyl)-N-methylamino)-2-oxoethylcarbamate

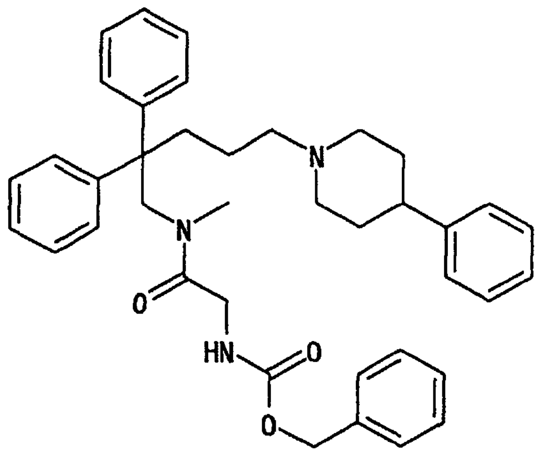

To a solution of 1-formamino-2,2-diphenyl-5-(4-phenylpiperidino)pentane (420 mg) synthesized in Reference Example 1B-8 in THF (15 ml) was added lithium aluminum hydride (112 mg) under ice-cooling, and the mixture was stirred at room temperature for 2 h and at 60°C for 1 h. The reaction mixture was cooled and water (5 ml) and then 2N aqueous sodium hydroxide solution (3 ml) were added by small portions under ice-cooling. Ether (300 ml) was added and the mixture was filtered through Celite. The organic layer of the filtrate was washed with saturated brine, dried over anhydrous sodium sulfate and concentrated under reduced pressure. The resulting residue was applied to silica gel column chromatography and eluted with ethyl acetate-methanol (5:1)-ethyl acetate-methanol-saturated aqueous ammonia (50:10:1). To a solution of this and Z-glycine (84 mg) in acetonitrile (10 ml) was added WSC (76 mg) under ice-cooling, and the mixture was stirred at room temperature for 16 h. Water (200 ml) was added to the reaction mixture. The mixture was extracted with ethyl acetate (200 ml), and after washing with saturated brine, dried over anhydrous sodium sulfate and concentrated under reduced pressure. The resulting residue was applied to silica gel column chromatography and eluted with ethyl acetate-ethyl acetate-methanol (20:1) to give the title compound (130 mg).
amorphous powder.
$^1$H-NMR ($CDCl_3$)δ: 1.22-1.39 (2H, m), 1.65-2.05 (9H, m), 2.23-2.51 (3H, m) , 2.75-2.96 (2H, m), 3.88-3.90 (2H, m), 4.06-4.18 (2H, m), 5.11 (2H, s), 5.85 (1H, s), 7.20-7.35 (20H, m).

**Example 76**

benzyl 2-((5-(4-(3-fluorophenyl)piperidino)-2,2-diphenylpentyl)amino)-2-oxoethylcarbamate hydrochloride

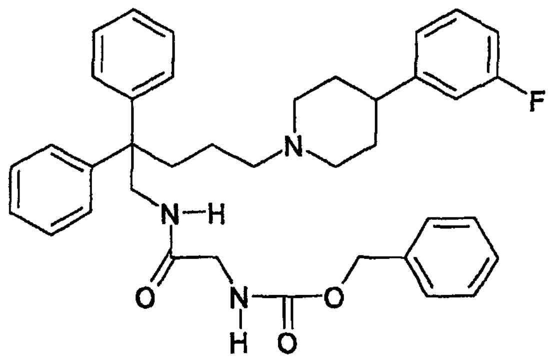

To a solution (10 ml) of the compound (0.31 g) obtained in Reference Example 7D in tetrahydrofuran was added 4-(3-fluorophenyl)piperidine (178 mg). The reaction mixture was stirred overnight at room temperature and the reaction mixture was concentrated under reduced pressure. The residue was dissolved in ethyl acetate, washed with water, dried and concentrated. The resulting residue was purified by silica gel column chromatography using methanol-ethyl acetate (0:100-5:95). To a solution (10 ml) of the obtained free amine in ethanol was added 1M ethereal hydrogen chloride (2.0 ml) under ice-cooling and the mixture was stirred at the same temperature for 15 min. The reaction mixture was concentrated under reduced pressure, and the residue was crystallized from ethyl acetate-hexane to give the title compound (0.29 g).
amorphous powder.
$^1$H-NMR($CDCl_3$)δ: 1.23-1.33 (2H, m) , 1.43-1.47 (2H, m) , 1.97-2.01 (2H, m) , 2.57-2.90 (7H, m), 3.84-4.13 (6H, m), 5.02 (2H, s) , 5.86 (1H, s), 6.73 (1H, s), 6.90-7.61 (19H, m), 11.37 (1H, brs).

**Example 77**

benzyl 2-((5-(4-(2-fluorophenyl)piperidino)-2,2-diphenylpentyl)amino)-2-oxoethylcarbamate hydrochloride

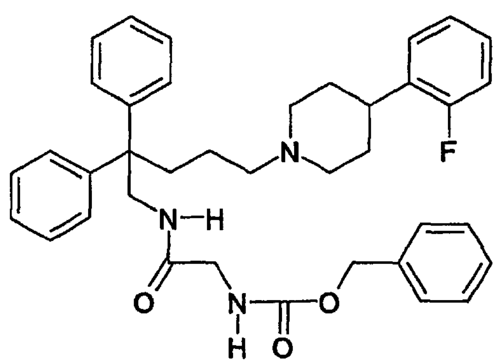

The compound was synthesized in the same manner as in Example 76 from the compound obtained in Reference Example 7D.
amorphous powder.
$^1$H-NMR($CDCl_3$)δ: 1.25 (2H, brs), 1.47 (2H, s), 1.96-2.00 (2H, m) , 2.53-3.11 (7H, m), 3.81-3.98 (6H, m), 5.01 (2H, s) , 5.86 (1H, s), 6.85 (1H, s), 6.98-7.43 (19H, m), 11.30 (1H, brs).

**Example 78**

benzyl 2-((5-(4-(4-fluorophenyl)piperidino)-2,2-diphenylpentyl)amino)-2-oxoethylcarbamate hydrochloride

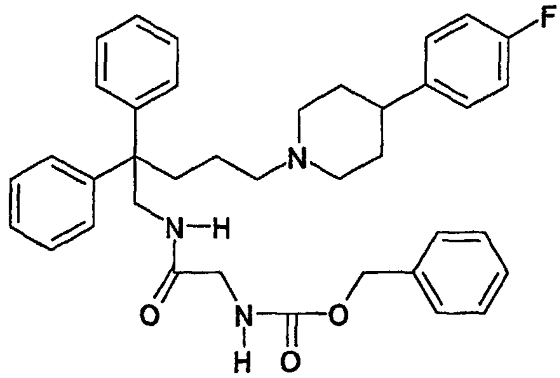

The compound was synthesized in the same manner as in Example 76 from the compound obtained in Reference Example 7D.
amorphous powder.
$^1$H-NMR($CDCl_3$) δ: 1.46 (2H, brs), 1.69 (2H, brs), 1.93-1.97 (2H, m), 2.54-2.89 (7H, m), 3.78-3.98 (6H, m), 5.02 (2H, s), 5.86 (1H, s), 6.83 (1H, s), 6.96-7.38 (19H, m), 11.31 (1H, brs).

**Example 79**

benzyl 2-((5-(4-(3-methoxyphenyl)piperidino)-2,2-diphenylpentyl)amino)-2-oxoethylcarbamate hydrochloride

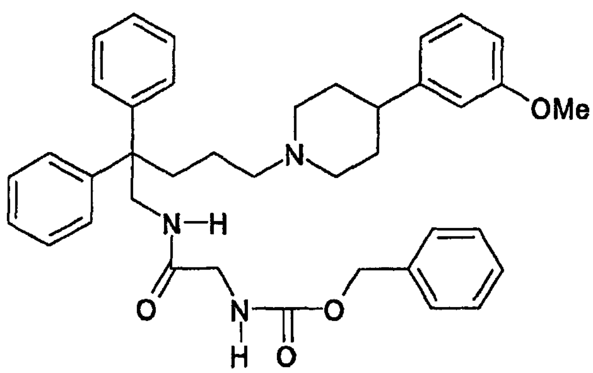

The compound was synthesized in the same manner as in Example 76 from the compound obtained in Reference Example 7D.
amorphous powder.
$^1$H-NMR($CDCl_3$)δ: 1.50 (2H, brs), 1.64 (2H, s) , 1.98-2.02 (2H, m) , 2.57-2.85 (5H, m), 2.91 (2H, brs), 5.03 (2H, s), 5.89 (1H, s), 6.74-6.92 (4H, m), 7.18-7.37 (18H, m), 11.33 (1H, brs).

**Example 80**

benzyl 2-((5-(4-(2-methoxyphenyl)piperidino)-2,2-diphenylpentyl)amino)-2-oxoethylcarbamate hydrochloride

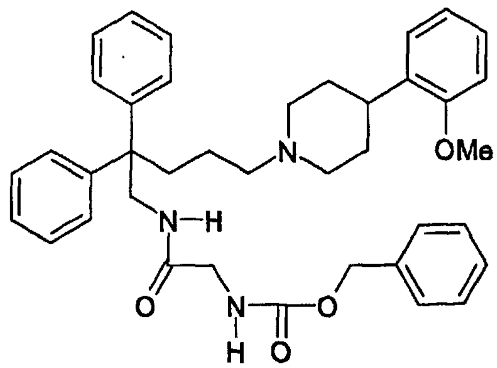

The compound was synthesized in the same manner as in Example 76 from the compound obtained in Reference Example 7D.
amorphous powder.
$^1$H-NMR($CDCl_3$)δ: 1.26 (2H, brs), 1.62 (2H, s), 1.97-1.99 (2H, brs), 2.57-2.65 (4H, m) , 2.89-2.90 (2H, m) , 3.11-3.16 (1H, m) , 3.81 (3H, s), 3.89-3.98 (4H, m), 5.01 (2H, s), 5.91 (1H, s), 6.83-6.97 (3H, m), 7.16-7.38 (19H, m), 11.23 (1H, brs).

**Example 81**

benzyl 2-((5-(4-(4-methoxyphenyl)piperidino)-2,2-diphenylpentyl)amino)-2-oxoethylcarbamate hydrochloride

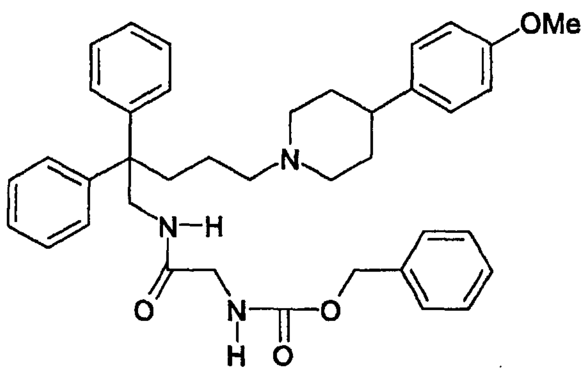

The compound was synthesized in the same manner as in Example 76 from the compound obtained in Reference Example 7D.
Recrystallization solvent: ethyl acetate
melting point: 119-120°C
$^1$H-NMR($CDCl_3$)δ: 1.49 (2H, brs), 1.67 (2H, s), 1.99-2.06 (2H, m) , 2.58-2.80 (5H, m), 2.90 (2H, brs), 3.80 (3H, s), 3.91-4.03 (4H, m), 5.03 (2H, s), 5.91 (1H, s), 6.85-6.90 (3H, m), 7.12-7.63 (19H, m), 11.31 (1H, brs).

**Example 82**

benzyl 2-((2,2-diphenyl-5-(4-(3-trifluoromethylphenyl)-piperidino)pentyl)amino)-2-oxoethylcarbamate hydrochloride

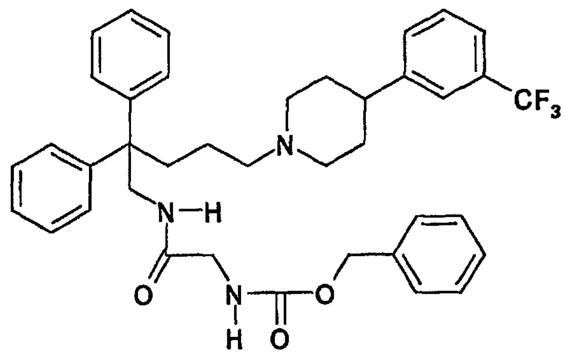

The compound was synthesized in the same manner as in Example 76 from the compound obtained in Reference Example 7D.
amorphous powder.
$^1$H-NMR($CDCl_3$)δ: 1.54 (2H, brs), 1.65 (2H, s), 2.04 (2H, brs), 2.48 (2H, brs), 2.74-3.00 (5H, m), 3.90-4.00 (4H, m), 5.00 (2H, s), 5.88 (1H, brs), 7.18-7.32 (18H, m), 7.45-7.58 (3H, m), 9.98 (1H, brs).

**Example 83**

benzyl 2-((5-(4-methyl-4-phenylpiperidino)-2,2-diphenylpentyl)amino)-2-oxoethylcarbamate hydrochloride

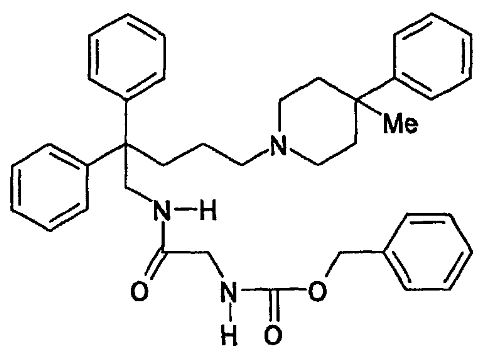

The compound was synthesized in the same manner as in Example 76 from the compound obtained in Reference Example 7D.
amorphous powder.
$^1$H-NMR($CDCl_3$) δ: 1.26-1.36 (4H, m) , 1.62 (4H, s), 2.45-2.95 (8H, m) , 3.60 (2H, brs), 3.93-3.95 (4H, m) , 5.04 (2H, s), 5.99 (1H, s), 6.78-7.61 (20H, m), 10.95 (1H, brs).

**Example 84**

benzyl 2-((2,2-diphenyl-5-piperidinopentyl)amino)-2-oxoethylcarbamate hydrochloride

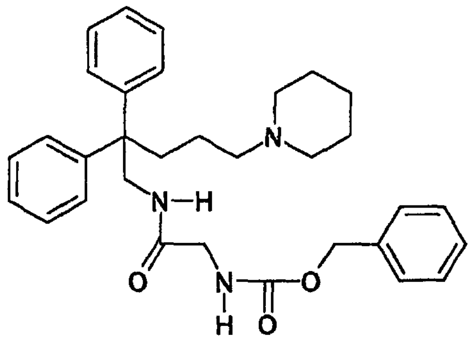

The compound was synthesized in the same manner as in Example 76 from the compound obtained in Reference Example 7D.
amorphous powder.
$^1$H-NMR($CDCl_3$)δ: 1.33-1.44 (2H, m) , 1.67 (3H, s), 1.79-1.89 (2H, m), 2.33-2.54 (5H, m), 2.85-2.87 (2H, m) , 3.65-3.68 (2H, m) , 3.92-3.99 (4H, m), 5.05 (2H, s), 5.91 (1H, s), 6.91-6.93 (1H, s) , 7.18-7.41 (15H, m), 10.99 (1H, brs).

**Example 85**

benzyl 2-((5-(4-benzylpiperidino)-2,2-diphenylpentyl)amino)-2-oxoethylcarbamate hydrochloride

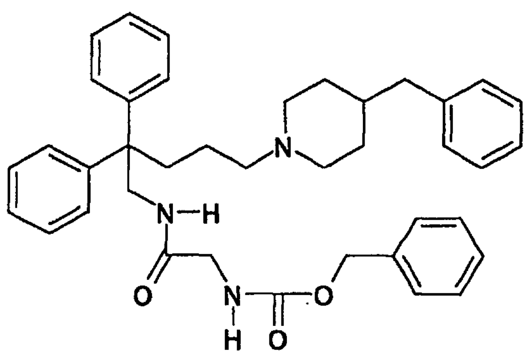

The compound was synthesized in the same manner as in Example 76 from the compound obtained in Reference Example 7D.
amorphous powder.
$^1$H-NMR($CDCl_3$)δ: 1.44 (2H, s) , 1.71-1.83 (3H, m) , 2.15-2.28 (2H, m) , 2.35-2.64 (6H, m), 2.83-2.84 (2H, m), 3.69-3.72 (2H, m) , 3.89-3.98 (4H, m), 5.06 (2H, s), 5.90 (1H, s), 6.90 (1H, s), 7.09-7.63 (21H, m), 11.10 (1H, brs).

**Example 86**

benzyl 2-((5-(4-(3-fluorobenzyl)piperidino)-2,2-diphenylpentyl)amino)-2-oxoethylcarbamate hydrochloride

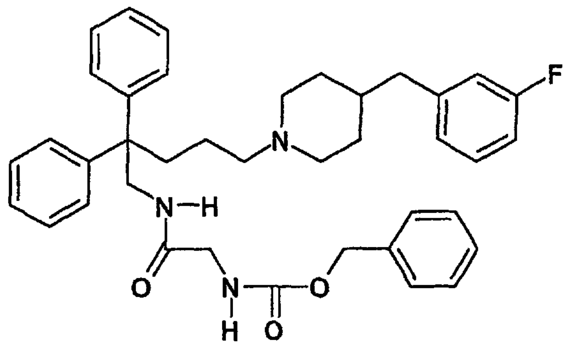

The compound was synthesized in the same manner as in Example 76 from the compound obtained in Reference Example 7D.
amorphous powder.
$^1$H-NMR($CDCl_3$) δ: 1.42 (2H, s), 1.63-1.80 (4H, m) , 2.15-2.27 (2H, m), 2.38-2.62 (6H, m), 2.81 (2H, s), 3.69-3.72 (2H, m), 3.91-3.96 (4H, m), 5.04 (2H, s), 5.86 (1H, s), 6.78-6.93 (4H, m), 7.16-7.61 (17H, m), 11.10 (1H, brs).

**Example 87**

benzyl 2-((5-(4-(2-fluorobenzyl)piperidino)-2,2-diphenylpentyl)amino)-2-oxoethylcarbamate hydrochloride

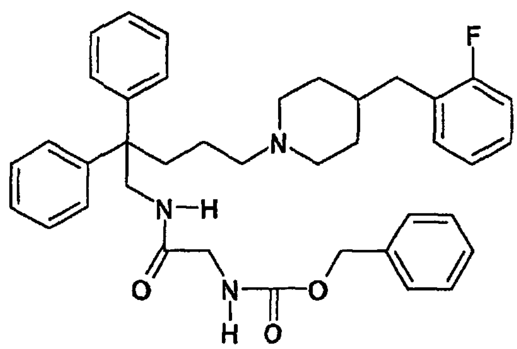

The compound was synthesized in the same manner as in Example 76 from the compound obtained in Reference Example 7D.
amorphous powder.
$^1$H-NMR ($CDCl_3$) δ: 1.41 (2H, s), 1.76-1.80 (4H, m) , 2.14-2.27 (2H, m), 2.36-2.66 (4H, m), 2.81 (2H, s), 3.66-3.70 (2H, m), 3.91-3.96 (4H, m), 5.04 (2H, s), 5.89 (1H, s), 7.00-7.35 (23H, m) , 11.07 (1H, brs).

**Example 88**

benzyl 2-((5-(4-(4-fluorobenzyl)piperidino)-2,2-diphenylpentyl)amino)-2-oxoethylcarbamate hydrochloride

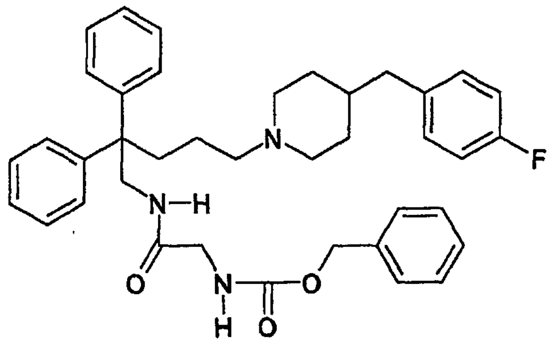

The compound was synthesized in the same manner as in Example 76 from the compound obtained in Reference Example 7D.
amorphous powder.
$^1$H-NMR($CDCl_3$) δ: 1.42 (2H, s), 1.63-1.79 (4H, m) , 2.13-2.25 (2H, m) , 2.33-2.59 (6H, m), 2.81 (2H, s), 3.68-3.90 (2H, m), 3.90-3.96 (4H, m), 5.04 (2H, s), 5.87 (1H, s), 6.93-7.35 (19H, m), 11.10 (1H, brs).

**Example 89**

benzyl 2-((5-(4-(2,4-difluorobenzyl) piperidino)-2,2-diphenylpentyl) amino)-2-oxoethylcarbamate hydrochloride

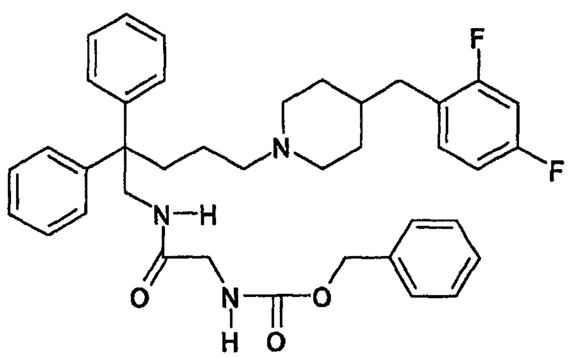

The compound was synthesized in the same manner as in Example 76 from the compound obtained in Reference Example 7D.
amorphous powder.
$^1$H-NMR($CDCl_3$) δ: 1.42-1.44 (2H, m), 1.69-1.80 (4H, m) , 2.16-2.29 (2H, m) , 2.37-2.64 (6H, m), 2.84 (2H, S), 3.69-3.72 (2H, m) , 3.92-4.01 (4H, m), 5.06 (2H, s), 5.89 (1H, s), 6.75-6.87 (4H, m) , 7.05-7.37 (14H, m) , 11.11 (1H, brs).

**Example 90**

benzyl 2-((5-(4-(4-cyanobenzyl)piperidino) -2,2-diphenylpentyl)amino)-2-oxoethylcarbamate hydrochloride

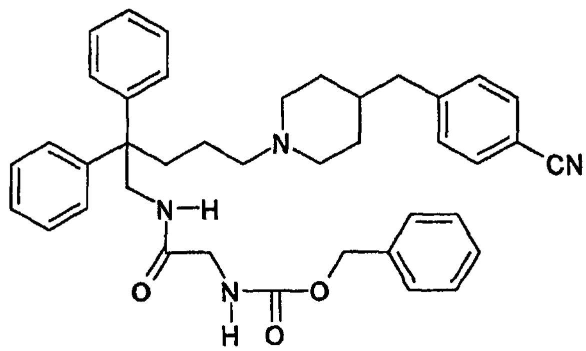

The compound was synthesized in the same manner as in Example 76 from the compound obtained in Reference Example 7D.
amorphous powder.
$^1$H-NMR($CDCl_3$)δ: 1.44 (2H, s), 1.66-1.79 (4H, m), 2.21-2.53 (6H, m), 2.67-2.70 (2H, m) , 2.84 (2H, s), 3.72-3.75 (2H, m), 3.92-3.98 (4H, m), 5.06 (2H, s), 5.85 (1H, s), 6.86 (1H, s), 7.18-7.37 (17H, m), 7.58-7.63 (2H, m), 11.16 (1H, brs).

**Example 91**

benzyl 2-((5-(4-anilinopiperidino)-2,2-diphenylpentyl)amino)-2-oxoethylcarbamate hydrochloride

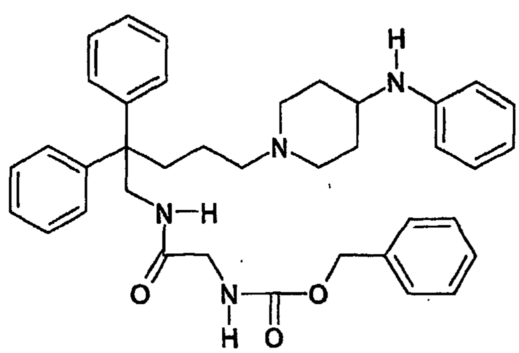

The compound was synthesized in the same manner as in Example 76 from the compound obtained in Reference Example 7D.
Recrystallization solvent: ethyl acetate
melting point: 134-136°C
$^1$H-NMR($CDCl_3$)δ: 1.40 (2H, s), 2.26-3.04 (10H, m) , 3.53-3.94 (8H, m) , 4.99 (2H, s), 5.88 (1H, s), 6.63 (1H, s), 6.91 (1H, s), 7.15-7.44 (19H, m), 10.30 (1H, brs), 10.87 (1H, brs).

**Example 92**

benzyl 2-((2,2-diphenyl-5-(1,2,3,4-tetrahydroisoquinolin-2-yl)pentyl)amino)-2-oxoethylcarbamate hydrochloride

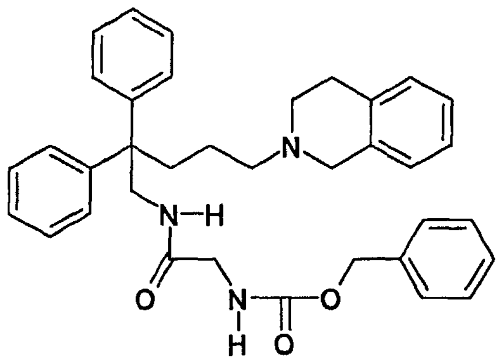

The compound was synthesized in the same manner as in Example 76 from the compound obtained in Reference Example 7D.
amorphous powder.
$^1$H-NMR($CDCl_3$) δ: 1.26-1.68 (4H, m) , 2.52-2.65 (2H, m) , 2.99 (3H, s), 3.36 (2H, brs), 3.66 (1H, brs), 3.87-4.00 (4H, m), 4.67-4.76 (1H, m), 5.00 (2H, s), 5.90 (1H, s), 6.84 (1H, s), 7.13-7.37 (19H, m), 11.97 (1H, brs).

**Example 93**

benzyl 2-((5-(isoindolin-2-yl)-2,2-diphenylpentyl)amino)-2-oxoethylcarbamate hydrochloride

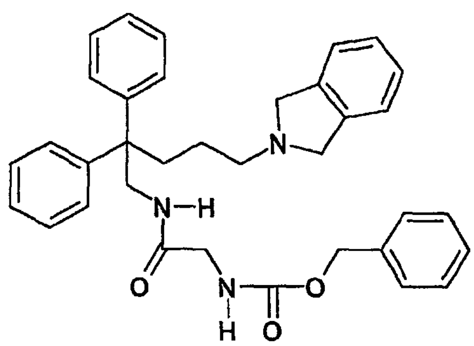

The compound was synthesized in the same manner as in Example 76 from the compound obtained in Reference Example 7D.
Recrystallization solvent: ethyl acetate-hexane
melting point: 121-123°C
$^1$H-NMR($CDCl_3$)δ: 1.53-1.62 (6H, m), 2.58-2.64 (2H, m), 3.12-3.13 (2H, m) , 3.92-4.01 (4H, m) , 4.16-4.22 (2H, m), 5.00 (2H, s), 5.11-5.18 (2H, m), 5.86 (1H, s), 6.77 (1H, s), 7.17-7.35 (15H, m), 12.39 (1H, brs).

**Example 94**

benzyl 2-((5-(N-methyl-N-phenethylamino)-2,2-diphenylpentyl)amino)-2-oxoethylcarbamate hydrochloride

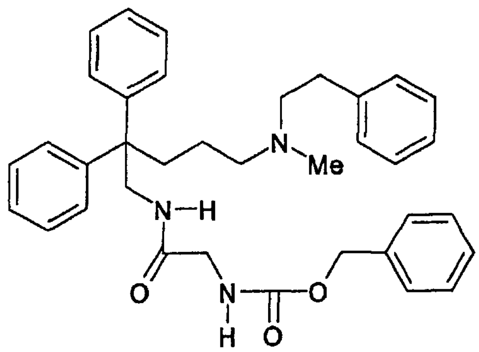

The compound was synthesized in the same manner as in Example 76 from the compound obtained in Reference Example 7D.
amorphous powder.
$^1$H-NMR($CDCl_3$)δ: 1.36 (1H, brs), 1.68 (2H, s), 2.16-2.20 (1H, m), 2.81-2.84 (4H, m), 3.06-3.33 (5H, m), 3.75-4.04 (3H, m), 4.21-4.28 (1H, m), 4.98-5.08 (2H, m), 5.88 (1H, s), 6.81 (1H, s), 7.20-7.63 (20H, m), 11.52 (1H, brs).

**Example 95**

benzyl 2-((5-(N-benzyl-N-methylamino)-2,2-diphenylpentyl)-amino)-2-oxoethylcarbamate hydrochloride

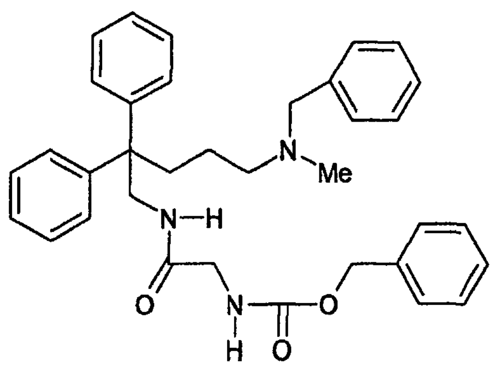

The compound was synthesized in the same manner as in Example 76 from the compound obtained in Reference Example 7D.
amorphous powder.
$^1$H-NMR($CDCl_3$)δ: 1.29-1.36 (1H, m) , 1.59-1.70 (1H, m) , 2.05-2.13 (1H, m), 2.68-2.89 (6H, m), 3.74-4.06 (4H, m), 4.21-4.27 (1H, m), 4.39-4.44 (1H, m), 5.04 (2H, s), 5.94 (1H, s), 6.88 (1H, s), 7.18-7.55 (20H, m), 11.55 (1H, brs).

**Example 96**

benzyl 2-((2,2-diphenyl-4-(4-phenylpiperidino)butyl)amino)-2-oxoethylcarbamate hydrochloride

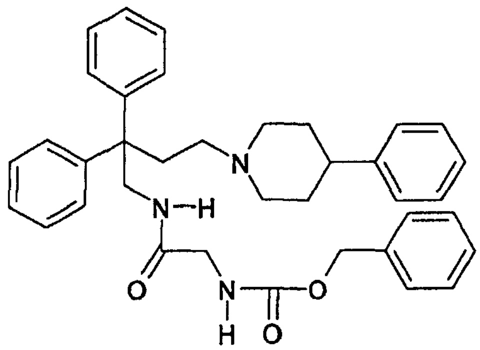

The compound was synthesized in the same manner as in Example 76 from the compound obtained in Reference Example 7D.
amorphous powder.
$^1$H-NMR($CDCl_3$)δ: 1.61 (1H, s), 1.94-2.04 (2H, m) , 2.61 (4H, s), 2.81-2.89 (4H, m), 3.69 (2H, s), 3.93-4.05 (4H, m), 5.08 (2H, s), 6.12 (1H, s), 6.43 (1H, s), 7.13-7.35 (20H, m), 11.87 (1H, brs).

**Example 97**

N-(2-((2,2-diphenyl-5-(4-phenylpiperidino)pentyl)amino)-2-oxoethyl)benzothiophene-2-carboxamide

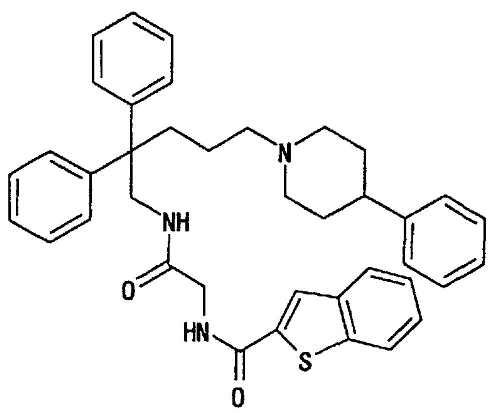

The compound was synthesized in the same manner as in Example 43.
Recrystallization solvent: diethyl ether-hexane.
melting point: 110-113°C.

**Example 98**

N-(2-((2,2-diphenyl-5-(4-phenylpiperidino)pentyl)amino)-2-oxoethyl)indole-4-carboxamide

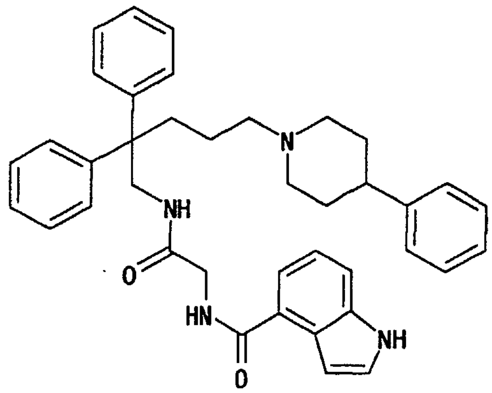

The compound was synthesized in the same manner as in Example 43.
Recrystallization solvent: ethyl acetate-diethyl ether.
melting point: 215-218°C.

**Example 99**

N-(2-((2,2-diphenyl-5-(4-phenylpiperidino)pentyl)amino)-2-oxoethyl)indole-5-carboxamide

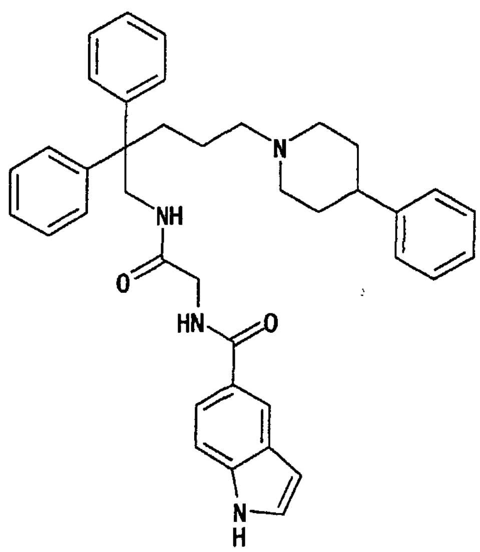

The compound was synthesized in the same manner as in Example 43.
Recrystallization solvent: ethyl acetate-diethyl ether.
melting point: 156-160°C.

**Example 100**

N-(2-((2,2-diphenyl-5-(4-phenylpiperidino)pentyl)amino)-2-oxoethyl)indole-6-carboxamide

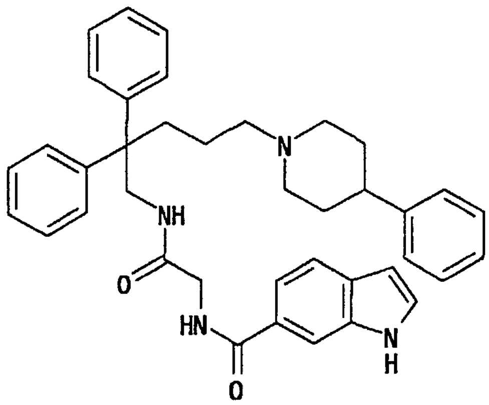

The compound was synthesized in the same manner as in Example 43.
Recrystallization solvent: ethyl acetate-diethyl ether.
melting point: 149-152°C.

**Example 101**

N-(2-((2,2-diphenyl-5-(4-phenylpiperidino)pentyl)amino)-2-oxoethyl)indole-7-carboxamide

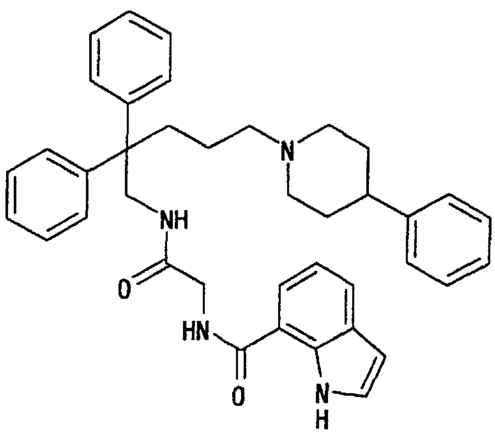

The compound was synthesized in the same manner as in Example 43.
Recrystallization solvent: ethyl acetate-diethyl ether.
melting point: 166-168°C.

**Example 102**

tert-butyl 2-((2,2-bis(4-chlorophenyl)-5-(4-phenylpiperidino)pentyl)amino)-2-oxoethylcarbamate

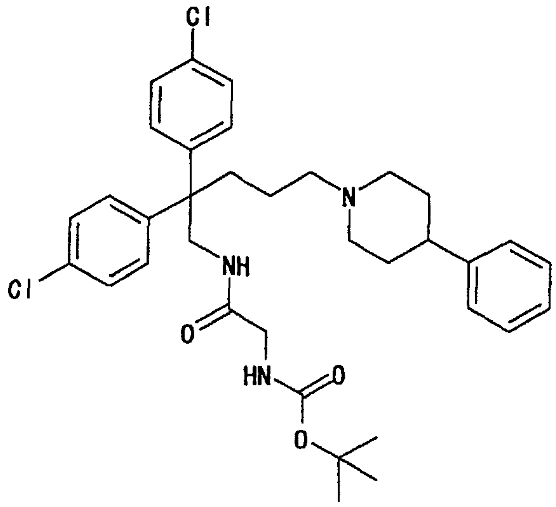

The compound was synthesized in the same manner as in Example 76 from the compound obtained in Reference Example 13D.
amorphous powder.
$^1$H-NMR($CDCl_3$) δ: 1.21-1.28 (2H, m) , 1.40 (9H, s), 1.68-2.02 (8H, m), 2.24-2.31 (2H, m) , 2.37-2.53 (1H, m), 2.86-2.91 (2H, m) , 3.63 (2H, d, J=5.9Hz), 3.93 (2H, d, J=5.9Hz), 5.25 (1H, t, J=5.5Hz), 5.89 (1H, br), 7.08-7.29 (13H, m).

**Example 103**

3-chlorobenzyl 2-((2,2-bis(4-chlorophenyl)-5-(4-phenylpiperidino)pentyl)amino)-2-oxoethylcarbamate

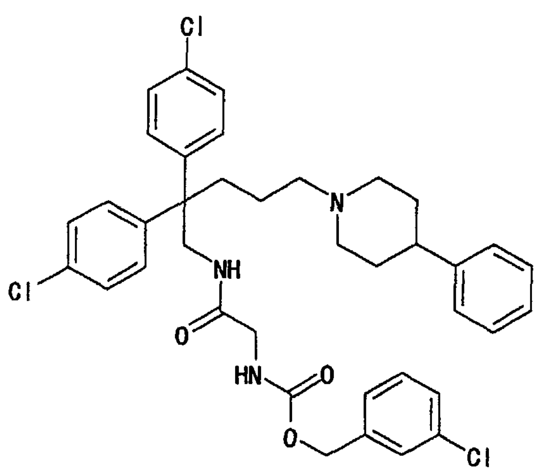

The compound was synthesized in the same manner as in Example 57 from the compound obtained in Example 102.
amorphous powder.
$^1$H-NMR($CDCl_3$)δ: 1.22-1.29 (2H, m), 1.71-2.03 (8H, m), 2.29 (2H, t, J=7.0Hz), 2.37-2.53 (1H, m), 2.89-2.94 (2H, m),

3.71 (2H, d, J=5.9Hz), 3.93 (2H, d, J=6.0Hz), 5.03 (2H, s), 5.56 (1H, br), 5.67 (1H, br), 7.05-7.33 (17H, m).

**Example 104**

N-(2-((2,2-bis(4-chlorophenyl)-5-(4-phenylpiperidino)pentyl)-amino)-2-oxoethyl)indole-2-carboxamide

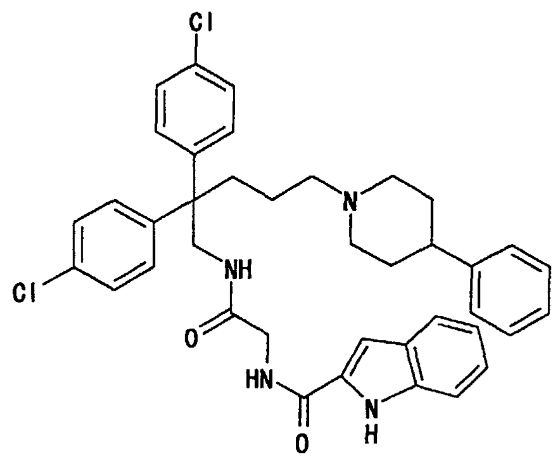

The compound was synthesized in the same manner as in Example 43 from the compound obtained in Example 102.
Recrystallization solvent: ethyl acetate-diethyl ether.
melting point: 143-145°C.

**Example 105**

N-(2-((2,2-bis(4-chlorophenyl)-5-(4-phenylpiperidino)pentyl)-amino)-2-oxoethyl)-5-chloroindole-2-carboxamide

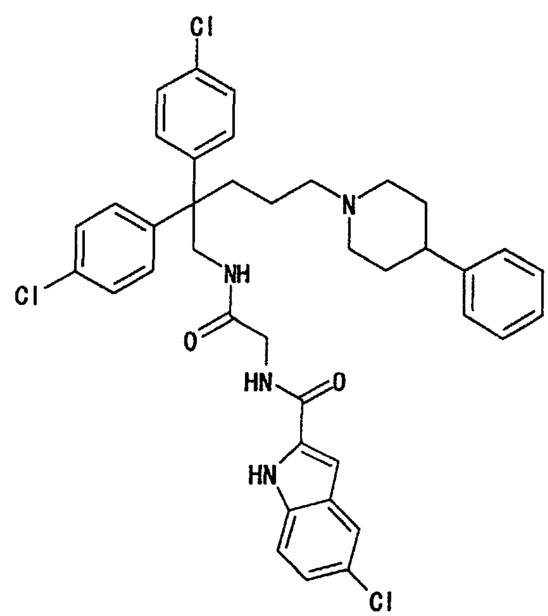

The compound was synthesized in the same manner as in Example 43 from the compound obtained in Example 102.
Recrystallization solvent: ethyl acetate-diethyl ether.
melting point: 159-161°C.

**Example 106**

N-(2-((2,2-bis(4-chlorophenyl)-5-(4-phenylpiperidino)-pentyl)amino)-2-oxoethyl)-1-methylindole-2-carboxamide

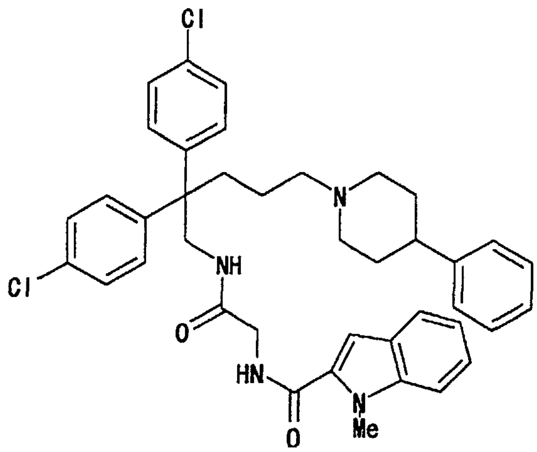

The compound was synthesized in the same manner as in Example 43 from the compound obtained in Example 102.
Recrystallization solvent: diethyl ether-hexane.
melting point: 107-110°C.

**Example 107**

N-(2-((2,2-bis(4-chlorophenyl)-5-(4-phenylpiperidino)-pentyl)amino)-2-oxoethyl)-5-fluoroindole-2-carboxamide

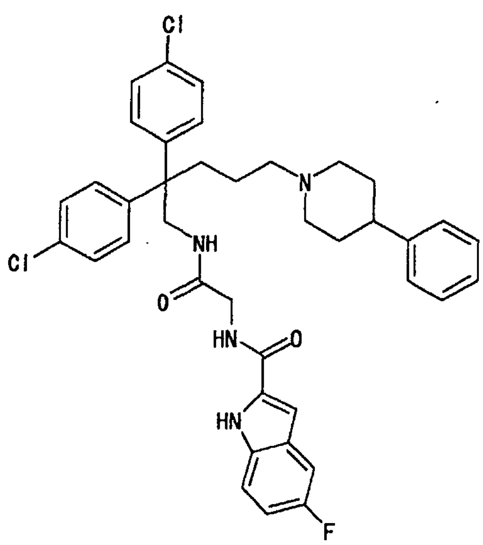

The compound was synthesized in the same manner as in Example 43 from the compound obtained in Example 102.
Recrystallization solvent: ethyl acetate-diethyl ether-hexane.
melting point: 133-135°C.

**Example 108**

N-(2-((2,2-bis(4-chlorophenyl)-5-(4-phenylpiperidino)pentyl)-amino)-2-oxoethyl)-5-methoxyindole-2-carboxamide

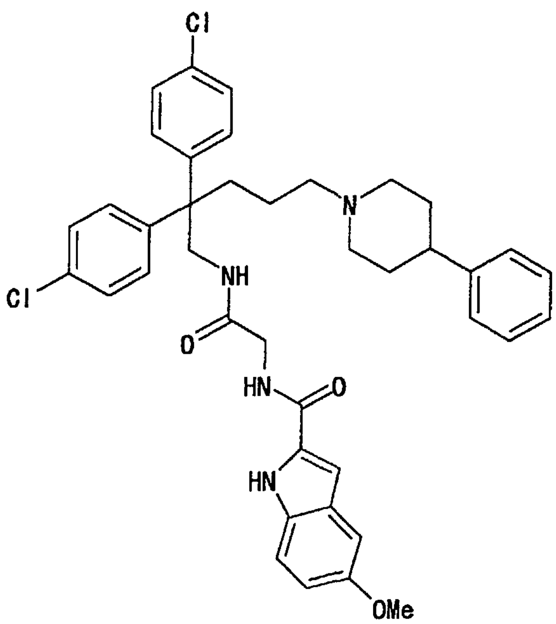

The compound was synthesized in the same manner as in Example 43 from the compound obtained in Example 102.
Recrystallization solvent: ethyl acetate-hexane.
melting point: 124-127°C.

**Example 109**

N-(2-((2,2-bis(4-chlorophenyl)-5-(4-phenylpiperidino)-pentyl)amino)-2-oxoethyl)-5-hydroxyindole-2-carboxamide

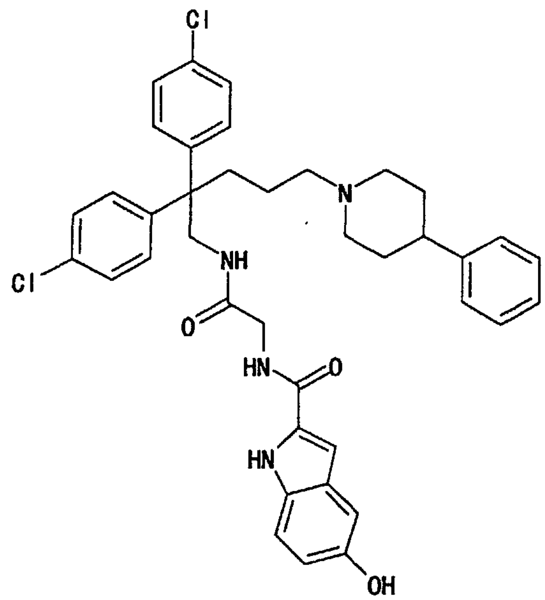

The compound was synthesized in the same manner as in Example 43 from the compound obtained in Example 102.
Recrystallization solvent: ethyl acetate-hexane.
melting point: 154-157°C.

**Example 110**

N-(2-((2,2-bis(4-chlorophenyl)-5-(4-phenylpiperidino)pentyl)-amino)-2-oxoethyl)-2-phenoxyacetamide

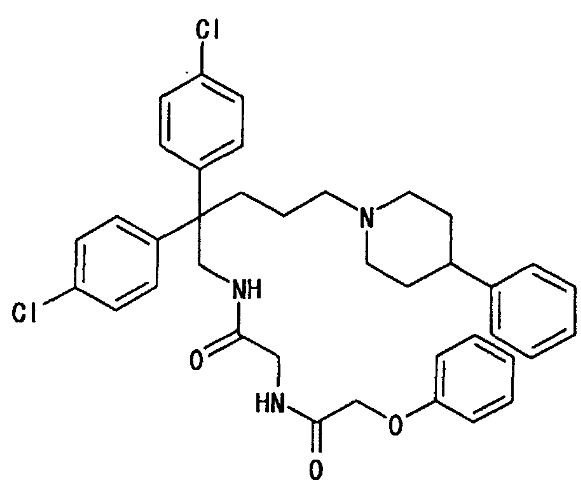

The compound was synthesized in the same manner as in Example 43 from the compound obtained in Example 102.

Recrystallization solvent: diethyl ether-hexane.
melting point: 143-145°C.

**Example 111**

benzyl 2-((5-(4-phenoxypiperidino)-2,2-diphenylpentyl)amino)-2-oxoethylcarbamate

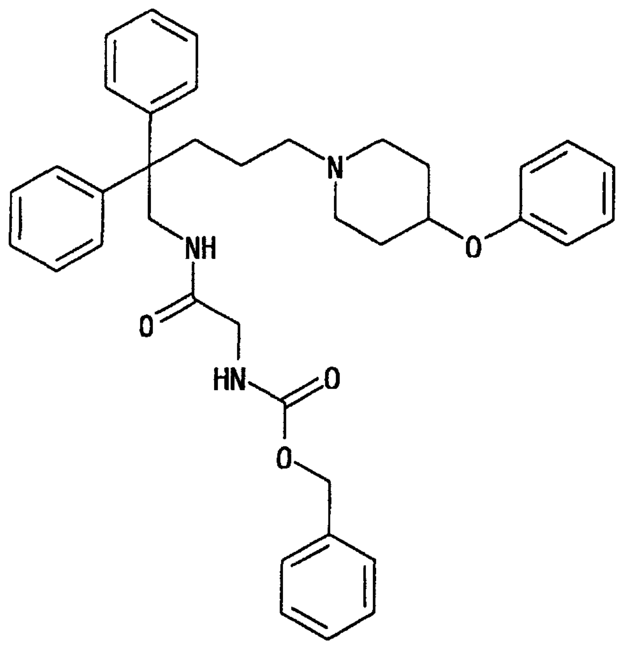

The compound was synthesized in the same manner as in Example 76 from the compound obtained in Reference Example 7D.
amorphous powder.
$^1$H-NMR ($CDCl_3$)δ: 1.24 (1H, m) , 1.45 (2H, brs), 1.78 (4H, s), 2.07-2.25 (2H, m), 2.53-2.91 (7H, m), 3.56-3.59 (1H, m), 3.82-3.97 (4H, m), 4.59-4.61 (1H, m), 5.03 (2H, s), 5.83-5.89 (1H, m), 6.83-7.01 (4H, m), 7.17-7.34 (16H, m).

**Example 112**

benzyl 2-((2,2-diphenyl-5-(4-piperidinopiperidino)pentyl)-amino)-2-oxoethylcarbamate

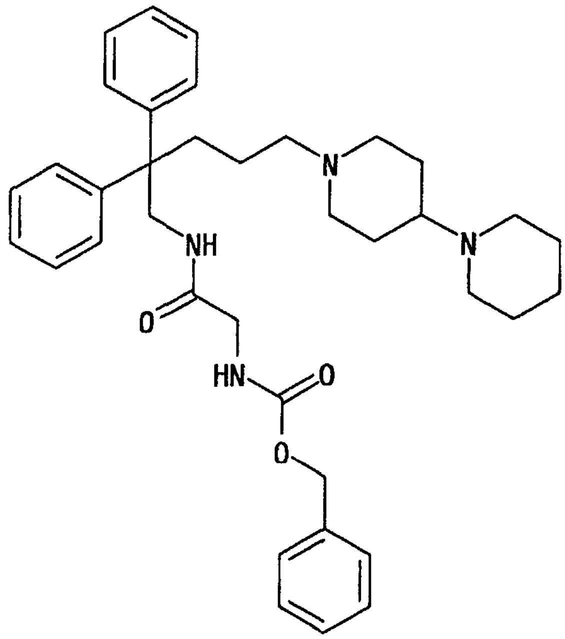

The compound was synthesized in the same manner as in Example 76 from the compound obtained in Reference Example 7D.
amorphous powder.
$^1$H-NMR ($CDCl_3$)δ: 1.28-1.48 (2H, m) , 1.81-1.87 (4H, m) , 2.04-2.41 (10H, m), 2.62-2.94 (5H, m), 3.11-3.18 (2H, m), 3.40-3.49 (2H, m) , 3.77-3.96 (6H, m), 5.04 (2H, m), 5.78 (1H, s), 6.80 (1H, s), 7.18-7.34 (15H, m).

**Example 113**

benzyl 2-((5-(4-(2-fluorophenyl)-1,2,5,6-tetrahydropyridin-1-yl)-2,2-diphenylpentyl)amino)-2-oxoethylcarbamate

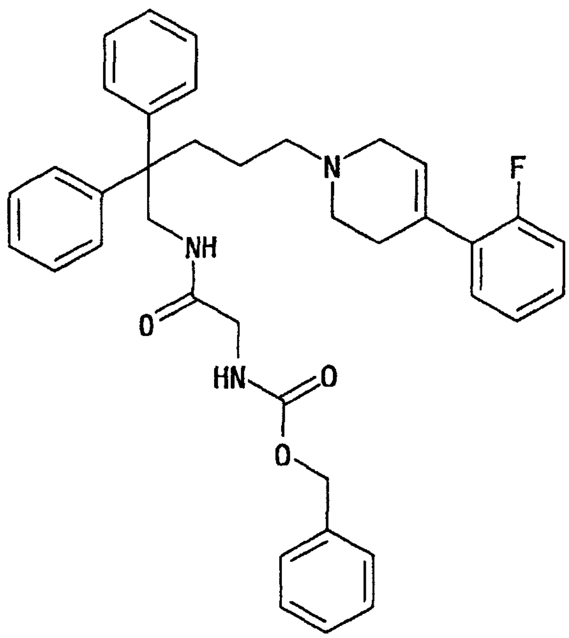

The compound was synthesized in the same manner as in Example 76 from the compound obtained in Reference Example 7D.
amorphous powder.
$^1$H-NMR ($CDCl_3$)δ: 1.63 (4H, s) , 2.53-2.81 (4H, m) , 3.06-3.23 (4H, m), 3.82-3.98 (4H, m) , 4.23-4.29 (1H, m) , 5.06 (2H, s) , 5.89 (2H, s) , 6.84-7.71 (19H, m).

**Example 114**

tert-butyl 2-((5-(4-(2-fluorophenyl)piperidino)-2,2-diphenylpentyl)amino)-2-oxoethylcarbamate

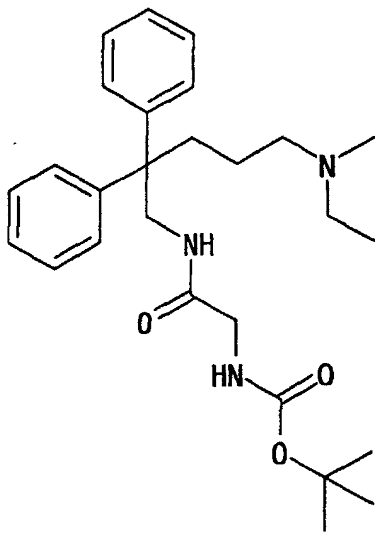

The compound was synthesized in the same manner as in Example 1 from tert-butyl 2-((5-hydroxy-2,2-diphenylpentyl)-amino)-2-oxoethylcarbamate.
amorphous powder.
$^1$H-NMR ($CDCl_3$) δ: 1.39 (9H, s), 1.66-1.76 (5H, m) , 1.90-2.30 (7H, m) , 2.68-2.89 (3H, m), 3.63-3.67 (2H, m), 3.97-4.01 (2H, m) , 4.92 (1H, s), 5.59 (1H, s), 7.06-7.42 (14H, m).

**Example 115**

N- (2-((5-(4-(2-fluorophenyl)piperidino)-2,2-diphenylpentyl)-amino)-2-oxoethyl) indole-2-carboxamide

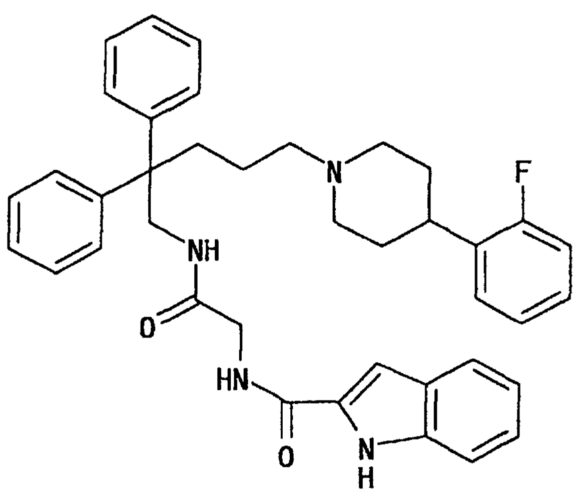

The compound was synthesized in the same manner as in Example 43 from the compound obtained in Example 114.
amorphous powder.
$^1$H-NMR ($CDCl_3$) δ: 1.45-1.49 (2H, m) , 1.65 (5H, s), 2.05-2.10 (1H, m) , 2.57-2.96 (6H, m), 3.91-4.02 (4H, m), 4.16-4.19

(2H, m) , 4.00-4.02 (1H, m), 5.91 (1H, s), 6.95-7.46 (17H, m), 7.60-7.62 (1H, m), 9.04 (1H, s), 7.79-7.82 (1H, m), 9.04 (1H, s) .

**Example 116**

N-(2-((5-(4-(2-fluorophenyl)piperidino)-2,2-diphenylpentyl)-amino)-2-oxoethyl)-1-methylindole-2-carboxamide

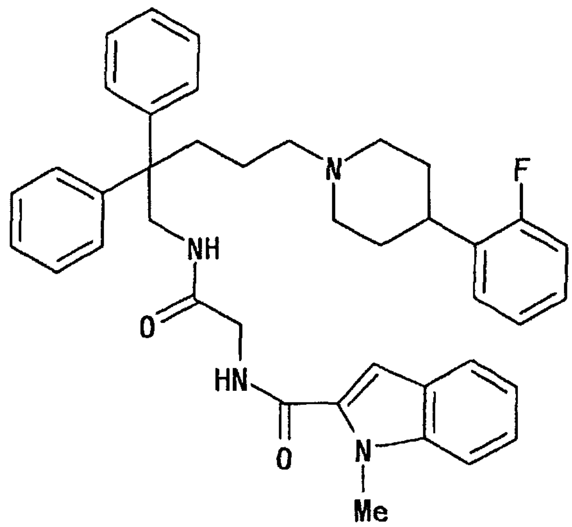

The compound was synthesized in the same manner as in Example 43 from the compound obtained in Example 114.
amorphous powder.
$^1$H-NMR ($CDCl_3$) δ: 1.25-1.81 (5H, m) , 2.03-2.07 (1H, m), 2.34-3.33 (7H, m), 3.90-4.00 (7H, m), 4.16-4.19 (2H, m) , 5.89 (1H, s), 6.91-7.47 (17H, m), 7.58-7.66 (1H, m), 7.77-7.82 (1H, m).

**Example 117**

5-chloro-N-(2-((5-(4-(2-fluorophenyl) piperidino)-2,2-diphenylpentyl)amino)-2-oxoethyl) -1-methylindole-2-carboxamide

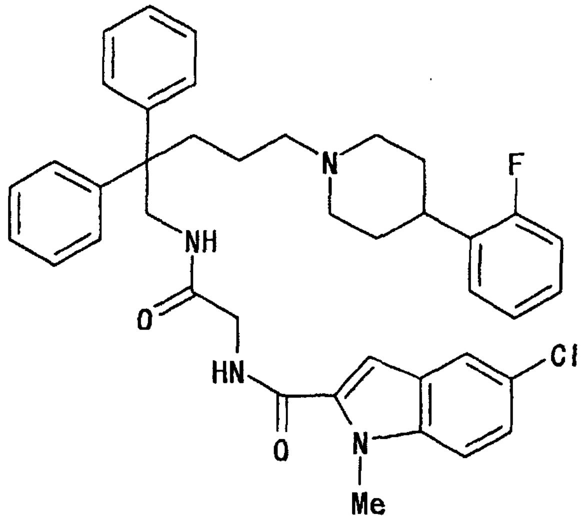

The compound was synthesized in the same manner as in Example 43 from the compound obtained in Example 114.
amorphous powder.
$^1$H-NMR ($CDCl_3$)δ: 1.27-1.83 (7H, m) , 2.06-2.10 (1H, m) , 2.39-3.17 (7H, m), 3.73-4.19 (7H, m), 5.86 (1H, s) , 6.94-7.78 (19H, m) .

**Example 118**

5-chloro-N-(2-((5-(4-(2-fluorophenyl)piperidino)-2,2-diphenylpentyl)amino)-2-oxoethyl)indole-2-carboxamide

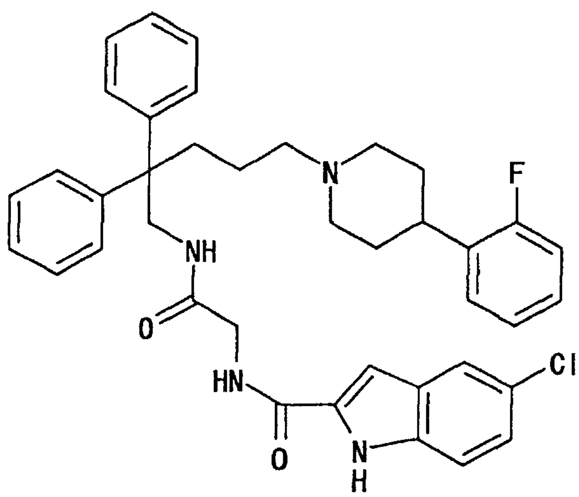

The compound was synthesized in the same manner as in Example 43 from the compound obtained in Example 114.

amorphous powder.
$^1$H-NMR ($CDCl_3$) δ: 1.33-1.61 (7H, m) , 2.07-2.10 (1H, m), 2.38-3.15 (6H, m), 3.83-4.15 (5H, m), 5.81 (1H, m), 6.91-7.73 (19H, m), 9.12 (1H, s).

**Example 119**

tert-butyl 2-((2,2-bis(4-chlorophenyl)-5-(4-(2-fluorophenyl)-piperidino)pentyl)amino)-2-oxoethylcarbamate

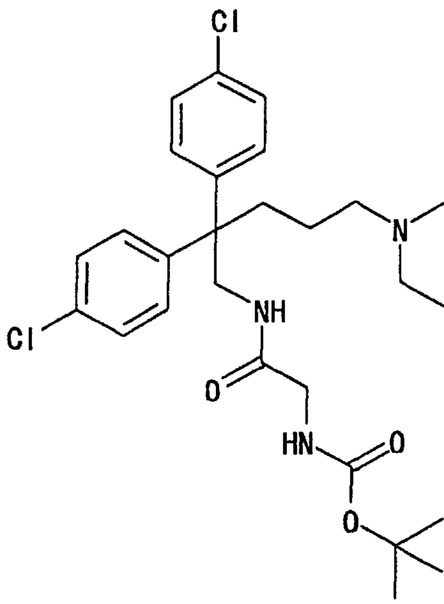

The compound was synthesized in the same manner as in Example 76 from the compound obtained in Reference Example 13D.
amorphous powder.
$^1$H-NMR ($CDCl_3$)δ: 1.26-1.39 (11H, m), 1.85-2.56 (10H, m), 2.91-3.30 (3H, m), 3.73-3.94 (4H, m), 5.82 (1H, s), 6.97-7.43 (13H, m) .

**Example 120**

N-(2-((2,2-bis(4-chlorophenyl)-5-(4-(2-fluorophenyl)-piperidino)pentyl)amino)-2-oxoethyl)-5-fluoroindole-2-carboxamide

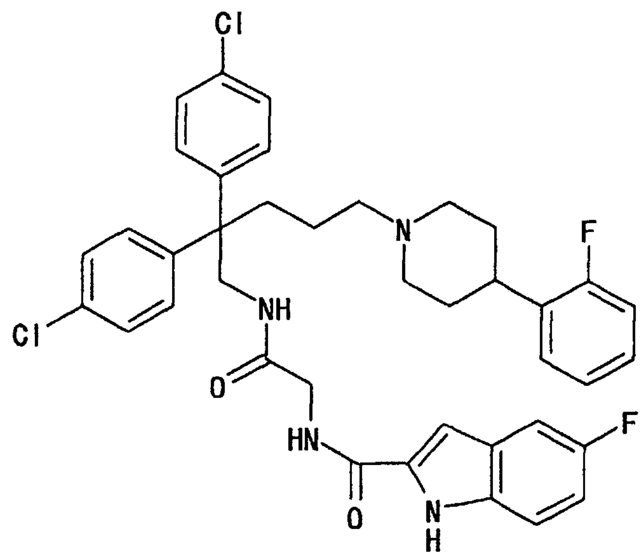

The compound was synthesized in the same manner as in Example 43 from the compound obtained in Example 119.
amorphous powder.
$^1$H-NMR ($CDCl_3$)δ: 1.14-1.84 (6H, m), 1.96-3.19 (8H, m), 3.65-4.15 (5H, m), 5.81 (1H, s), 6.90-7.65 (17H, m), 9.17 (1H, s).

**Example 121**

N-(2-((2,2-bis(4-chlorophenyl)-5-(4-(2-fluorophenyl)-piperidino)pentyl)amino)-2-oxoethyl)-5-methoxyindole-2-carboxamide

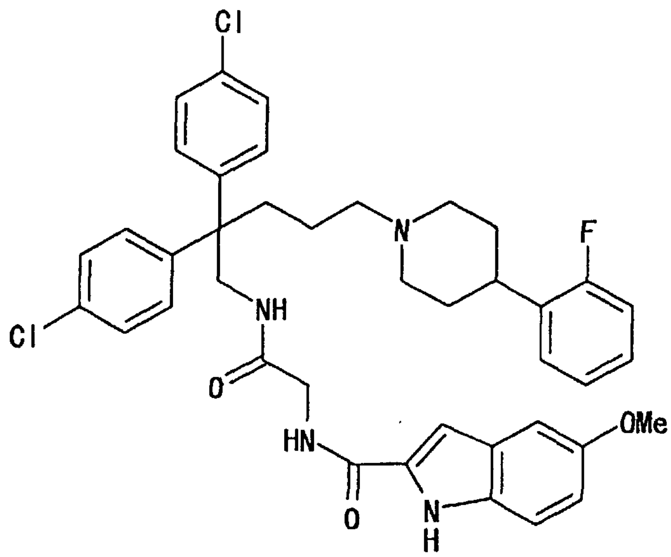

The compound was synthesized in the same manner as in Example 43 from the compound obtained in Example 119.
amorphous powder.
$^1$H-NMR ($CDCl_3$) δ: 1.23-1.93 (6H, m), 2.07-3.14 (8H, m), 3.63-4.23 (8H, m), 5.84 (1H, s), 6.89-7.72 (17H, m) , 9.04 (1H, s).

**Example 122**

N-(2-((2,2-bis(4-chlorophenyl)-5-(4-(2-fluorophenyl)-piperidino)pentyl)amino)-2-oxoethyl)indole-2-carboxamide

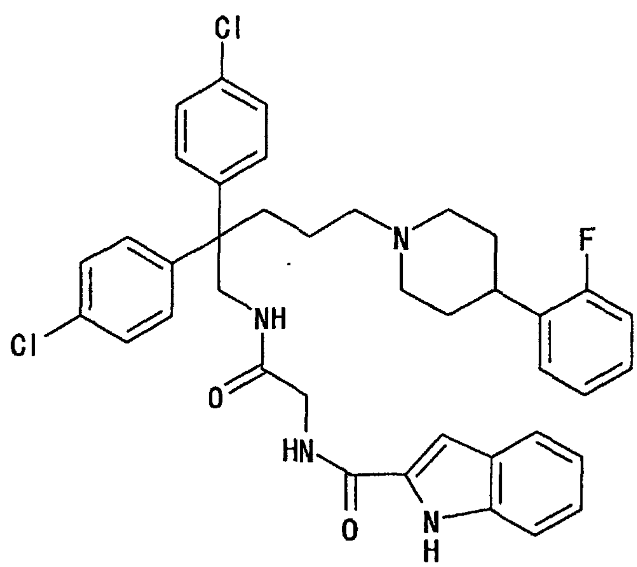

The compound was synthesized in the same manner as in Example 43 from the compound obtained in Example 119.
amorphous powder
$^1$H-NMR ($CDCl_3$) δ: 1.32-1.93 (6H, m), 2.04-3.10 (8H, m), 3.63-4.23 (8H, m), 5.84 (1H, s), 6.89-7.72 (17H, m) , 9.04 (1H, s).

**Example 123**

tert-butyl 2-((2,2-bis(4-fluorophenyl)-5-(4-phenylpiperidino)pentyl)amino)-2-oxoethylcarbamate

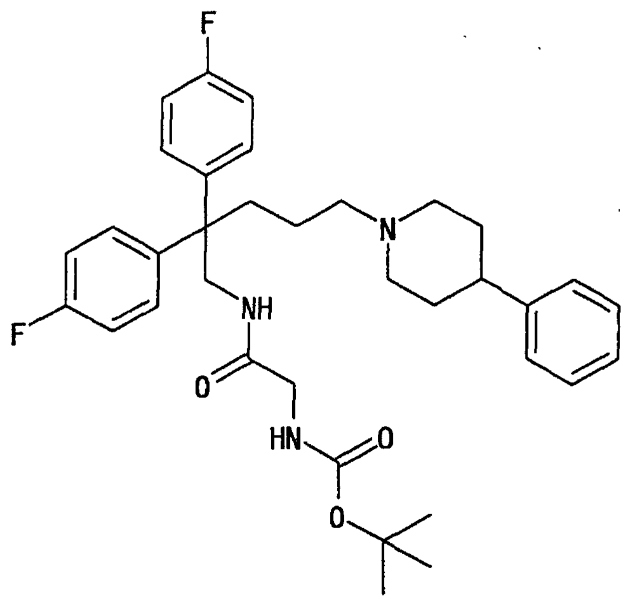

The compound was synthesized in the same manner as in Example 76 from the compound obtained in Reference Example 19D.
amorphous powder.
$^1$H-NMR ($CDCl_3$) δ: 1.19-1.34 (2H, m) , 1.40 (9H, s), 1.66-1.80 (4H, m) , 1.89-2.05 (4H, m) , 2.25-2.30 (2H, m), 2.39-2.50 (1H, m) , 2.86-2.90 (2H, m), 3.66 (2H, d, J =6.0Hz), 3.94 (2H, d, J =6.0Hz), 4.91 (1H, s), 5.67-5.71 (1H, s), 6.97-7.02 (3H, m) , 7.12-7.31 (10H, m).

**Example 124**

N-(2-((2,2-bis(4-fluorophenyl)-5-(4-phenylpiperidino)pentyl)-amino)-2-oxoethyl)indole-2-carboxamide

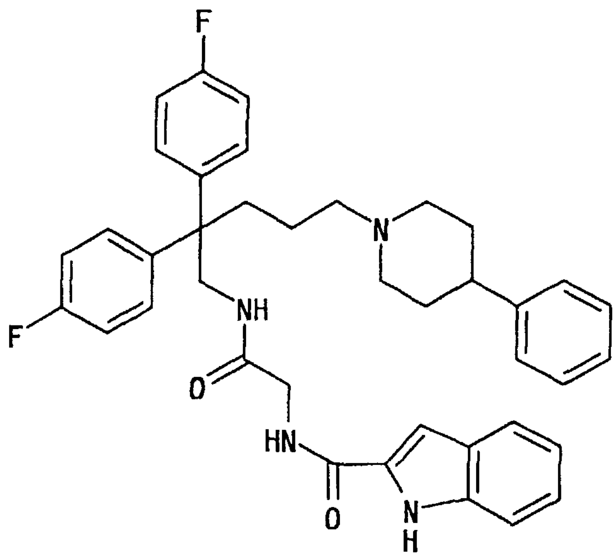

The compound was synthesized in the same manner as in Example 43 from the compound obtained in Example 123.
amorphous powder.
$^1$H-NMR ($CDCl_3$)δ: 1.24-1.27 (2H, m) , 1.77-2.09 (8H, m) , 2.29-2.48 (3H, m), 2.93-2.96 (2H, m), 3.96-4.02 (4H, m), 5.85-5.88 (1H, m), 6.82-6.91 (4H, m) , 6.96 (1H, s), 7.07-7.35 (12H, m), 7.45 (1H, d, J=8.1Hz), 7.68 (1H, d, J=8.0Hz), 9.40 (1H, s).

**Example 125**

N-(2-((2,2-bis(4-fluorophenyl)-5-(4-phenylpiperidino)pentyl)-amino)-2-oxoethyl)-5-chloroindole-2-carboxamide

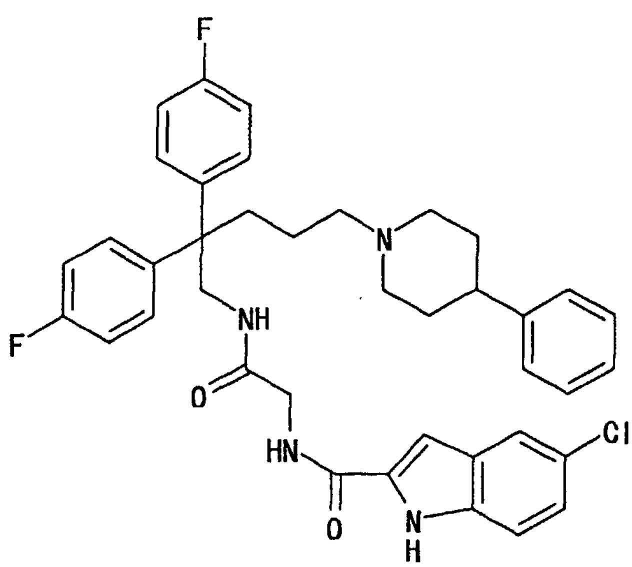

The compound was synthesized in the same manner as in Example 43 from the compound obtained in Example 123.
amorphous powder.
$^{1}$H-NMR ($CDCl_3$)δ: 1.25-1.33 (2H, m), 1.81-2.07 (8H, m) , 2.19-2.46 (3H, m), 2.98-3.01 (2H, m), 3.96-4.02 (4H, m), 6.83-6.91 (5H, m), 7.03-7.31 (11H, m), 7.38 (1H, d, J=8.8Hz), 7.65 (1H, s), 9.44 (1H, s).

**Example 126**

tert-butyl 2-((2,2-bis(4-fluorophenyl)-5-(4-(2-methoxyphenyl)-piperidino)pentyl)amino)-2-oxoethylcarbamate

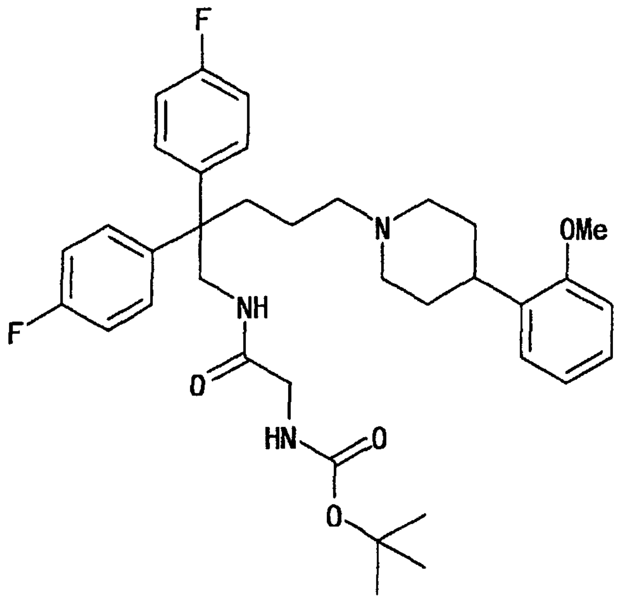

The compound was synthesized in the same manner as in Example 76 from the compound obtained in Reference Example 19D.
amorphous powder.
$^{1}$H-NMR ($CDCl_3$)δ: 1.35 (9H, s), 1.45-1.47 (2H, m) , 2.00-2.04 (2H, m), 2.39-3.23 (1H, m) , 3.82-3.92 (7H, m), 5.77-5.81 (1H, m) , 6.84-7.06 (6H, m), 7.19-7.27 (6H, m).

**Example 127**

N-(2-((2,2-bis(4-fluorophenyl)-5-(4-(2-methoxyphenyl)-piperidino)pentyl)amino)-2-oxoethyl)indole-2-carboxamide

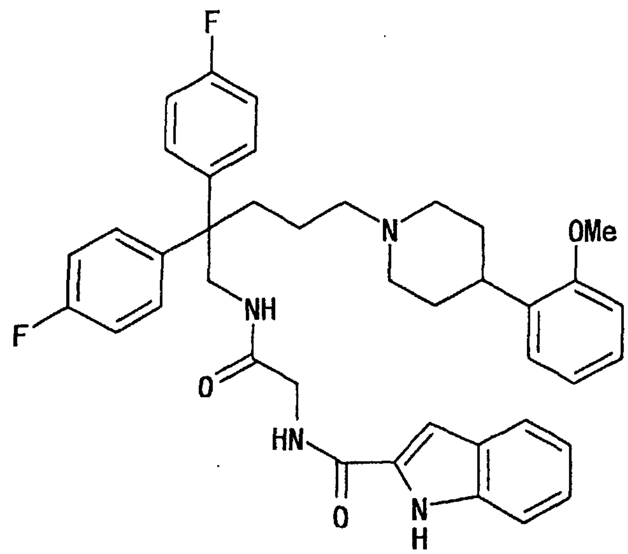

The compound was synthesized in the same manner as in Example 43 from the compound obtained in Example 126.
amorphous powder.
$^{1}$H-NMR ($CDCl_3$) δ: 1.23-1.28 (2H, m), 1.76-2.36 (10H, m), 2.89-2.99 (3H, m), 3.79 (3H, s), 3.96-4.02 (4H, m), 5.89-5.92 (1H, m), 6.81-7.35 (16H, m) , 7.45 (1H, d, J=8.3Hz), 7.68 (1H, d, J=8.0Hz), 9.46 (1H, s) .

**Example 128**

N-(2-((2,2-bis(4-fluorophenyl)-5-(4-(2-methoxyphenyl)-piperidino)pentyl)amino)-2-oxoethyl)-5-chloroindole-2-carboxamide

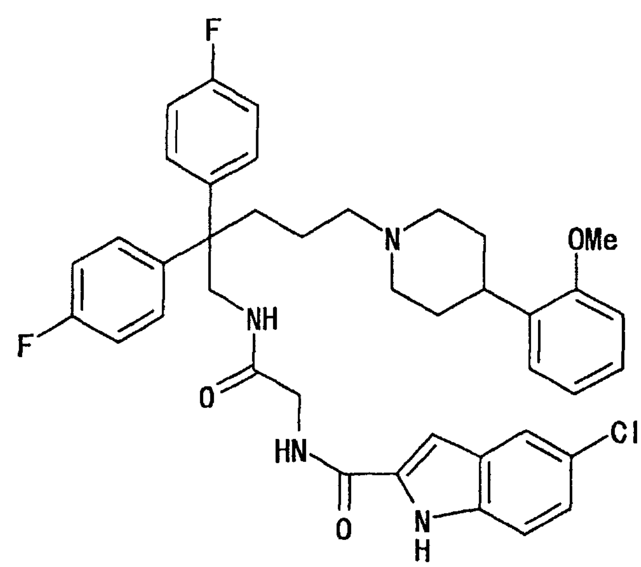

The compound was synthesized in the same manner as in Example 43 from the compound obtained in Example 126.
amorphous powder.
$^1$H-NMR ($CDCl_3$)δ: 1.26-1.30 (2H, m), 1.78-1.87 (3H, m), 2.02-2.14 (5H, m) , 2.35-2.71 (3H, m), 2.88-3.03 (3H, m), 3.81 (3H, s), 3.98-4.03 (3H, m), 5.92-5.96 (1H, m), 6.83-6.95 (7H, m) , 7.06-7.21 (6H, m), 7.38 (2H, d, J =8.8Hz), 7.52 (1H, s), 7.63-7.66 (1H, s), 9.77 (1H, s).

**Example 129**

tert-butyl 2-((2,2-bis(4-fluorophenyl)-5-(4-(2-fluorophenyl)-piperidino)pentyl)amino)-2-oxoethylcarbamate

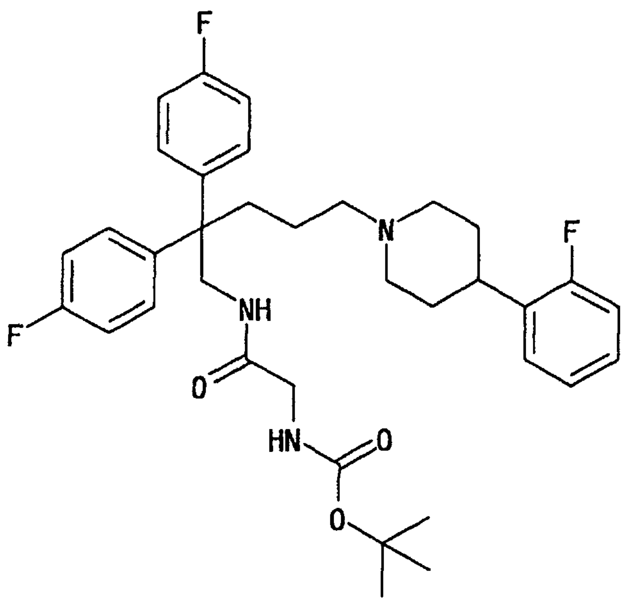

The compound was synthesized in the same manner as in Example 76 from the compound obtained in Reference Example 19D.
amorphous powder.
$^1$H-NMR ($CDCl_3$)δ: 1.19-1.31 (2H, m) , 1.40 (9H, s) , 1.61-1.80 (4H, m), 1.89-2.05 (4H, m), 2.25-2.30 (2H, m), 2.40-2.50 (1H, m) , 2.86-2.90 (2H, m), 3.66 (2H, d, J=6.0Hz), 3.94 (2H, d, J=6.0Hz), 4.93 (1H, s), 5.68-5.71 (1H, s), 6.96-7.03 (4H, m), 7.11-7.31 (9H, m).

**Example 130**

N-(2-((2,2-bis(4-fluorophenyl)-5-(4-(2-fluorophenyl)-piperidino)pentyl)amino)-2-oxoethyl)indole-2-carboxamide

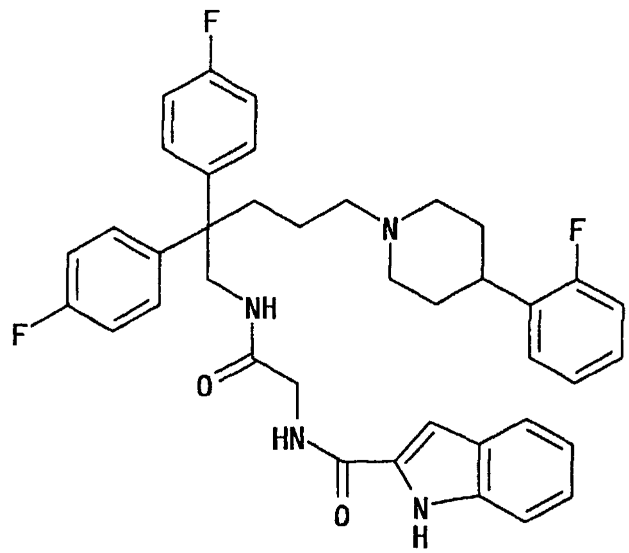

The compound was synthesized in the same manner as in Example 43 from the compound obtained in Example 129.
amorphous powder.
$^1$H-NMR ($CDCl_3$)δ: 1.23-1.28 (2H, m) , 1.70-2.37 (10H, m) , 2.79-2.86 (1H, m), 2.98-3.01 (2H, m) , 3.93-4.02 (4H, m), 5.88-5.92 (1H, m) , 6.77-6.86 (4H, m), 6.95-7.47 (14H, m), 7.68 (1H, d, J=8.0Hz), 9.49 (1H, s).

**Example 131**

N-(2-((2,2-bis(4-fluorophenyl)-5-(4-(2-fluorophenyl)-piperidino)pentyl)amino)-2-oxoethyl)-5-chloroindole-2-carboxamide

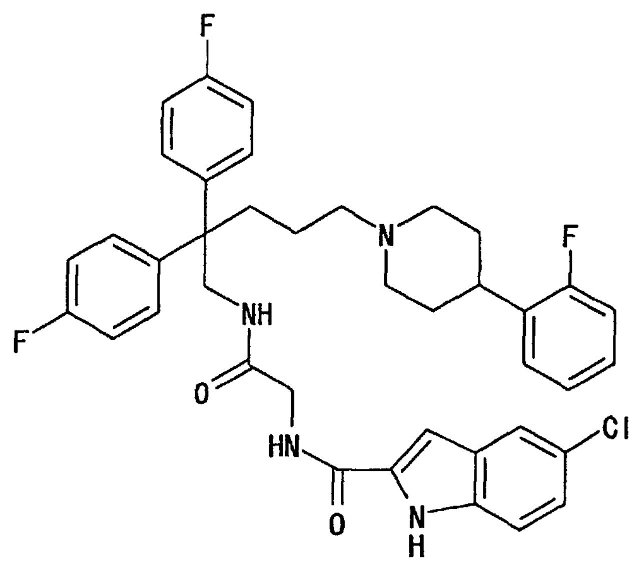

The compound was synthesized in the same manner as in Example 43 from the compound obtained in Example 129.
amorphous powder.
$^1$H-NMR ($CDCl_3$)δ: 1.23-1.33 (2H, m), 1.67-2.37 (10H, m), 2.80-2.87 (1H, m), 3.02-3.05 (2H, m), 3.93-4.02 (4H, m), 5.84-5.88(1H, m), 6.73-6.94 (4H, m), 6.97-7.47 (12H, m), 7.64 (1H, s), 9.69 (1H, s).

The following compounds can be synthesized in completely the same manner.

N-(2-((2,2-bis(4-methylphenyl)-5-(4-phenylpiperidino)-pentyl)amino)-2-oxoethyl)indole-2-carboxamide.
N-(2-((2,2-bis(4-methylphenyl)-5-(4-phenylpiperidino)-pentyl)amino)-2-oxoethyl)-5-chloroindole-2-carboxamide.
N- (2-((2,2-bis(4-methylphenyl)-5-(4-(2-methoxyphenyl)-piperidino)pentyl)amino)-2-oxoethyl)indole-2-carboxamide.
N-(2-((2,2-bis(4-methylphenyl)-5-(4-(2-methoxyphenyl)-piperidino)pentyl)amino)-2-oxoethyl)-5-chloroindole-2-carboxamide.
N-(2-((2,2-bis(4-methylphenyl)-5-(4-(2-fluorophenyl)-piperidino)pentyl)amino)-2-oxoethyl)indole-2-carboxamide.
N-(2-((2,2-bis(4-methylphenyl)-5-(4-(2-fluorophenyl)-piperidino)pentyl)amino)-2-oxoethyl)-5-chloroindole-2-carboxamide.

**Formulation Example 1A**

| | |
|---|---|
| (1) compound of Reference Example IIA-45 | 10.0 g |
| (2) lactose | 60.0 g |
| (3) cornstarch | 35.0 g |
| (4) gelatin | 3.0 g |
| (5) magnesium stearate | 2.0 g |

Using 10 wt% aqueous gelatin solution (30 ml)(3.0 g as gelatin), a mixture of the compound (10.0 g) obtained Reference Example IIA-45, lactose (60.0 g) and cornstarch (35.0 g) was granulated by passing through a 1 mm mesh sieve. The granules were dried at 40°C and passed through the sieve again. The obtained granules were mixed with magnesium stearate (2.0 g) and compressed. The obtained core tablets were coated with a sugar coating of aqueous suspension containing sucrose, titanium dioxide, talc and gum arabic. The coated tablets were glazed with bee wax to give 1000 coated tablets.

**Formulation Example 2A**

| | |
|---|---|
| (1) compound of Reference Example IIA-45 | 10.0 g |
| (2) lactose | 70.0 g |
| (3) cornstarch | 50.0 g |
| (4) soluble starch | 7.0 g |
| (5) magnesium stearate | 3.0 g |

Using an aqueous solution (70 ml) of soluble starch (7.0 g as soluble starch), the compound (10.0 g) obtained in Reference Example IIA-45 and magnesium stearate (3.0 g) were granulated, dried and mixed with lactose (70.0 g) and cornstarch (50.0 g). The mixture was compressed to give 1000 tablets.

**Formulation Example 1B**

| | |
|---|---|
| (1) compound of Reference Example 4B-2 | 10.0 g |
| (2) lactose | 60.0 g |
| (3) cornstarch | 35.0 g |

(continued)

| | |
|---|---|
| (4) gelatin | 3.0 g |
| (5) magnesium stearate | 2.0 g |

Using a 10 wt% aqueous gelatin solution (30 ml)(3.0 g as gelatin), a mixture of the compound (10.0 g) obtained Reference Example 4B-2, lactose (60.0 g) and cornstarch (35.0 g) was granulated by passing through a 1 mm mesh sieve. The granules were dried at 40°C and passed through the sieve again. The obtained granules were mixed with magnesium stearate (2.0 g) and compressed. The obtained core tablets were coated with a sugar coating of aqueous suspension containing sucrose, titanium dioxide, talc and gum arabic. The coated tablets were glazed with bee wax to give 1000 coated tablets.

**Formulation Example 2B**

| | |
|---|---|
| (1) compound of Reference Example 4B-2 | 10.0 g |
| (2) lactose | 70.0 g |
| (3) cornstarch | 50.0 g |
| (4) soluble starch | 7.0 g |
| (5) magnesium stearate | 2.0 g |

Using an aqueous solution (70 ml) of soluble starch (7.0 g as soluble starch), the compound (10.0 g) obtained in Reference Example 4B-2 and magnesium stearate (3.0 g) were granulated, dried and mixed with lactose (70.0 g) and cornstarch (50.0 g). The mixture was compressed to give 1000 tablets.

**Formulation Example 1C**

| | |
|---|---|
| (1) compound of Reference Example 5C-3 | 10.0 g |
| (2) lactose | 60.0 g |
| (3) cornstarch | 35.0 g |
| (4) gelatin | 3.0 g |
| (5) magnesium stearate | 2.0 g |

Using a 10 wt% aqueous gelatin solution (30 ml)(3.0 g as gelatin), a mixture of the compound (10.0 g) obtained Reference Example 5C-3, lactose (60.0 g) and cornstarch (35.0 g) was granulated by passing through a 1 mm mesh sieve. The granules were dried at 40°C and passed through the sieve again. The obtained granules were mixed with magnesium stearate (2.0 g) and compressed. The obtained core tablets were coated with a sugar coating of aqueous suspension containing sucrose, titanium dioxide, talc and gum arabic. The coated tablets were glazed with bee wax to give 1000 coated tablets.

**Formulation Example 2C**

| | |
|---|---|
| (1) compound of Reference Example 5C-3 | 10.0 g |
| (2) lactose | 70.0 g |
| (3) cornstarch | 50.0 g |
| (4) soluble starch | 7.0 g |
| (5) magnesium stearate | 2.0 g |

Using an aqueous solution (70 ml) of soluble starch (7.0 g as soluble starch), the compound (10.0 g) obtained in Reference Example 5C-3 and magnesium stearate (3.0 g) were granulated, dried and mixed with lactose (70.0 g)

and cornstarch (50.0 g). The mixture was compressed to give 1000 tablets.

**Formulation Example 1D**

| | |
|---|---|
| (1) compound of Example 1 | 10.0 g |
| (2) lactose | 60.0 g |
| (3) cornstarch | 35.0 g |
| (4) gelatin | 3.0 g |
| (5) magnesium stearate | 2.0 g |

Using a 10 wt% aqueous gelatin solution (30 ml) (3.0 g as gelatin), a mixture of the compound (10.0 g) obtained Example 1, lactose (60.0 g) and cornstarch (35.0 g) was granulated by passing through a 1 mm mesh sieve. The granules were dried at 40°C and passed through the sieve again. The obtained granules were mixed with magnesium stearate (2.0 g) and compressed. The obtained core tablets were coated with a sugar coating of aqueous suspension containing sucrose, titanium dioxide, talc and gum arabic. The coated tablets were glazed with bee wax to give 1000 coated tablets.

**Formulation Example 2D**

| | |
|---|---|
| (1) compound of Example 1 | 10.0 g |
| (2) lactose | 70.0 g |
| (3) cornstarch | 50.0 g |
| (4) soluble starch | 7.0 g |
| (5) magnesium stearate | 2.0 g |

Using an aqueous solution (70 ml) of soluble starch (7.0 g as soluble starch), the compound (10.0 g) obtained in Example 1 and magnesium stearate (3.0 g) were granulated, dried and mixed with lactose (70.0 g) and cornstarch (50.0 g). The mixture was compressed to give 1000 tablets.

**Reference Example 1E** Amplification of rat SLC-1 receptor cDNA by PCR using cDNA derived from rat brain

Using poly $(A)^+$ RNA derived from rat brain (Clontech) as a template and a random primer, reverse-transcription reaction was carried out. For the reverse-transcription reaction, a reagent of TaKaRa RNA PCR ver. 2 kit was used. Using this reverse-transcription product as a template and synthetic DNA primers of SEQ Nos:1 and 2, amplification was performed by the PCR method. The synthetic DNA primers were constructed such that the gene in the region to be translated into the receptor protein could be amplified, during which restriction enzyme recognizing sequences of restriction enzyme Sal I and restriction enzyme Spe I were added to the 5' side and 3' side, respectively, so that a base sequence recognized by the restriction enzyme Sal I would be added to the 5' side of the gene and a base sequence recognized by the restriction enzyme Spe I would be added to the 3' side of the gene. The composition of the reaction mixture was cDNA template 5 μl, each synthetic DNA primer 0.4 μM, 0.25 mM dNTPs, pfu (Stratagene) DNA polymerase 0.5 μl and buffer annexed to the enzyme, with the total reaction volume of 50 μl. For amplification cycle, Thermal Cycler (Perkins Elmer) was used. After heating at 94°C for 60 seconds, a cycle of heating at 94°C for 60 seconds, at 60°C for 30 seconds, and at 72°C for 150 seconds was repeated 35 times, and the mixture was finally reacted at 72°C for 10 minutes. The amplified product was confirmed by ethidium bromide staining after 0.8% agarose gel electrophoresis.

**Reference Example 2E** Subcloning of PCR product to plasmid vector and confirmation of amplified cDNA sequence by decoding base sequence of insert cDNA

The reaction product after PCR conducted in Reference Example 1E was separated using 0.8% low melting point agarose gel and the band was excised with a razor, and subjected to minimization, phenol extraction, phenol·chloroform extraction and ethanol precipitation to recover DNA. According to the direction of PCR-Script™ Amp SK(+) cloning kit (Stratagene), the recovered DNA was subcloned to plasmid vector pCR-Script Amp $SK(^+)$. This was introduced into Escherichia coli XL-1 Blue (Stratagene) to allow transformation, after which clones containing cDNA insert fragment were selected in an LB agar medium containing ampicillin and X-gal, separated using a sterile toothpick for white clones to give transformant E. coli XL-1 Blue/rat SLC-1. The respective clones were cultured overnight in an LB medium containing ampicillin, and using QIA prep8 mini prep (QIAGEN), plasmid DNAs were prepared. A part of the prepared DNAs was cleaved with restriction enzymes Sal I and Spe I to confirm the size of the inserted receptor cDNA fragment. The reaction for determination of the base sequence was carried out using DyeDeoxy Terminator Cycle Sequence Kit (Perkins Elmer), and decoded using a fluorescence automatic DNA sequencer. The sequences of the obtained three clones were analyzed and confirmed to be identical with the gene sequence consisting of a cDNA sequence (Lakaye, B. et al. Biochim. Biophys. Acta, Vol. 1401, pp. 216-220 (1998), accession No. AF08650) encoding rat SLC-1 protein (SEQ No:3) whose full length sequence had been reported, a Sal I recognizing sequence added on the 5' side and a Spe I recognizing sequence added on the 3' side (SEQ No:4).

**Reference Example 3E** Preparation of rat SLC-1 expression CHO cell

From a clone of E. coli transformed with a plasmid incorporating a gene encoding a full length amino acid sequence of rat brain derived SLC-1 and having a Sal I recognizing sequence added on the 5' side and a Spe I recognizing sequence added on the 3' side, whose sequence was confirmed in Reference Example 2E, a plasmid was prepared using Plasmid Midi Kit (QIAGEN) and cleaved with restriction enzymes Sal I and Spe I to excise an insert. The insert DNA was recovered by excising, after electrophoresis, from agarose gel with a razor and applying minimization, phenol extraction, phenol·chloroform extraction and ethanol precipitation. This insert DNA was added to animal cell expression vector plasmid pAKKO-111H (vector plasmid identical with pAKKO1.11H described in Hinuma, S. et al. Biochim. Biophys. Acta, Vol. 1219, pp. 251-259 (1994)) cleaved with Sal I and Spe I and ligated using T4 ligase (Takara Shuzo Co.) to construct protein expression plasmid pAKKO-SLC-1.

E. coli DH5 (TOYOBO) transformed with pAKKO-SLC-1 was cultured and plasmid DNA of pAKKO-SLC-1 was prepared using Plasmid Midi Kit (QIAGEN). This was introduced into CHO dhfr$^-$ cell using CellPhect Transfection Kit (Amersham Pharmacia Biotech) and in accordance with the attached protocol. DNA (10 μg) was prepared into a coprecipitation suspension with calcium phosphate and added into a 10 cm dish inoculated with 5 x $10^5$ or 1 x $10^6$ CHO dhfr$^-$ cells 24 hours before. The cells were cultured in an MEMα medium containing 10% fetal bovine serum for one day, passaged and cultured in a nucleic acid-free MEMα medium (selection medium) containing 10% dialyzed fetal bovine serum. 56 clones of transformed cell colonies, which were SLC-1 expression CHO cells grown in the selection medium, were selected.

**Reference Example 4E** Selection of CHO/SLC-1 cell line with high expression amount of full length rat SLC-1 receptor protein mRNA

The expression amount of full length rat SLC-1 receptor protein mRNA by 56 clones of CHO/SLC-1 cell line established in Reference Example 3E was measured as in the following using Cytostar T Plate (Amersham Pharmacia Biotech) and in accordance with the attached protocol. Each clone of the CHO/SLC-1 cell line was inoculated to each well of Cytostar T Plate at 2.5 x $10^4$, cultured for 24 hours and fixed with 10% formalin. After 0.25% Triton X-100 was added to each well to enhance permeability of the cells, riboprobe of $^{35}S$ labeled SEQ No:5 was added for hybridization. RNase A (20 mg/ml) was added to each well to digest free riboprobe. The plate was washed thoroughly and the radioactivity of the hybridized riboprobe was measured on Topcounter. The cell strain having high radioactivity showed higher expression amount of mRNA. Of the three clones showing high mRNA expression amount, particularly clone No. 44 was used mainly.

**Reference Example 5E** Isolation of plasmid containing human SLC-1 cDNA

According to the manual attached to Genetrapper cDNA positive selection system (GIBCOBRL) and using phage F1 endonuclease, nick was inserted into cDNA derived from human fetal brain library (SUPERSCRIPT™ cDNA Library; GIBCOBRL) and digested with Escherichia coli exonuclease III to prepare a single strand cDNA derived from human fetal brain library.

Using Terminal Deoxynucleotidyl Transferase, biotin-14-dCTP was added to the 3' terminal of the synthetic oligonucleotide (corresponding to 1434-1451 of accession No. U71092) of SEQ No:6 prepared based on the report of Kolakowski Jr. et al. (Kolakowski Jr., et al (1996) FEBS Lett. Vol. 398, pp. 253-258), whereby biotinylated oligonucleotide was prepared. The composition of the reaction mixture and reaction time followed the manual.

Single strand cDNA library derived from human fetal brain 4 μg was kept at 95°C for 1 min and rapidly cooled on ice. Biotinylated oligonucleotide (20 ng) was added and the mixture was hybridized in the accompanying hybridization buffer at 37°C for 1 hr. Streptavidin beads were added and single strand cDNA derived from human fetal brain

hybridized to biotinylated oligonucleotide was isolated using MAGNA-SEP Magnetic Particle Separator (GIBCOBRL). Using synthetic oligonucleotide (50 ng, corresponding to 1011-1028 of accession No. U71092) of SEQ No:7 prepared according to the report of Kolakowski Jr. et al. (Kolakowski Jr., et al (1996) FEBS Lett. Vol. 398, pp. 253-258) as a primer, a complementary chain was synthesized according to the manual to give a double strand plasmid.

**Reference Example 6E** Determination of base sequence of plasmid containing isolated human SLC-1 cDNA.

The plasmid obtained in Reference Example 5E was introduced into ELECTROMAX™ DH10B™ Cells by electroporation method to allow transformation, after which clones containing cDNA insert fragment were selected in an LB agar medium containing ampicillin and X-gal and separated using a sterile toothpick for white clones to give transformant E. coli DH10B/hSLC-1. The respective clones were cultured overnight in an LB medium containing ampicillin, and using QIA prep8 mini prep (QIAGEN), the plasmid DNA was purified. The reaction for determination of the base sequence was carried out using DyeDeoxy Terminator Cycle Sequence Kit (Perkins Elmer), and decoded using a fluorescence automatic DNA sequencer. As a result, the sequence depicted in SEQ No:8 was obtained. The amino acid sequence (SEQ No:9) encoded by the obtained base sequence was different from the human SLC-1 amino acid sequence as the sequence deduced from rat SLC-1 based on the human chromosomal DNA sequence (accession number:Z86090) containing the sequence of human SLC-1, in a report by Lakaye et al. (Lakaye, B. et al. (1998) Biochem. Biophys. Acta, vol. 1401, pp. 216-220) in that the presence of the initiating codon ATG on mRNA was indicated at 69 and 64 amino acids further upstream of the deduced sequence. A transformant Escherichia coli DH10B/phSLC1L8 obtained using a plasmid containing DNA encoding this sequence was deposited at IFO and NIBH.

**Reference Example 7E** Amplification of human SLC-1 cDNA by PCR using cDNA derived from human fetal brain

Using, as a template, the plasmid containing human SLC-1DNA sequence cloned by the gene-trap method, synthetic DNA primers of SEQ Nos:10 and 11 and synthetic DNA primers of SEQ Nos:12 and 13, amplification was conducted by the PCR method. The amplified DNA of the former was named human SLC-1(S) and the amplified DNA of the latter was named human SLC-1(L). The synthetic DNA primers were constructed such that the gene of the region to be translated into the receptor protein was amplified, during which restriction enzyme recognizing sequences of restriction enzyme Sal I and restriction enzyme Spe I were added to the 5' side and 3' side, respectively, so that a base sequence recognized by the restriction enzyme Sal I would be added to the 5' side of the gene and a base sequence recognized by the restriction enzyme Spe I would be added to the 3' side of the gene. The composition of the reaction mixture for human SLC-1(S) amplification was plasmid template containing human SLC-1 DNA sequence 5 μl, each synthetic DNA primer 0.4 μM, 0.2 mM dNTPs, pfuDNA polymerase 0.5 μl and buffer annexed to the enzyme, with the total reaction volume of 50 μl. For amplification cycle, Thermal Cycler (Perkins Elmer) was used. After heating at 94°C for 60 seconds, a cycle of heating at 94°C for 60 seconds, at 57°C for 60 seconds, and at 72°C for 150 seconds was repeated 25 times, and the mixture was finally incubated at 72°C for 10 minutes. The composition of the reaction mixture for human SLC-1(L) amplification was plasmid template containing human SLC-1 DNA sequence 5 μl, each synthetic DNA primer 0.4 μM, 0.2 mM dNTPs, pfuDNA polymerase 0.5 μl and buffer annexed to the enzyme, with the total reaction volume of 50 μl. For amplification cycle, Thermal Cycler (Perkins Elmer) was used. After heating at 94°C for 60 seconds, a cycle of heating at 94°C for 60 seconds, at 60°C for 60 seconds, and at 72°C for 3 min was repeated 25 times, and the mixture was finally incubated at 72°C for 10 minutes. The amplified product was confirmed by ethidium bromide staining after 0.8% agarose gel electrophoresis.

**Reference Example 8E** Subcloning of PCR product to plasmid vector and confirmation of amplified cDNA sequence by decoding base sequence of insert cDNA

The reaction product after PCR conducted in Reference Example 7E was separated using 0.8% low melting point agarose gel and the band region was excised with a razor, and subjected to minimization, phenol extraction, phenol·chloroform extraction and ethanol precipitation to recover DNA. According to the direction of PCR-Script™ Amp SK(+) cloning kit (Stratagene), the recovered DNA was subcloned to plasmid vector pCR-Script Amp SK($^+$). This was introduced into Escherichia coli DH5α competent cell (TOYOBO) to allow transformation, after which clones containing cDNA insert fragment were selected in an LB agar medium containing ampicillin and X-gal, separated using a sterile toothpick for white clones to give transformant E. coli DH5α/hSLC-1(S) of human SLC-1(S) and transformant E. coli DH5α/hSLC-1(L) of human SLC-1(L). The respective clones were cultured overnight in an LB medium containing ampicillin, and using QIA prep8 mini prep (QIAGEN), the plasmid DNA was prepared. A part of the prepared DNA was cleaved with restriction enzymes Sal I and Spe I to confirm the size of the inserted receptor cDNA fragment. The reaction for determination of the base sequence was carried out using DyeDeoxy Terminator Cycle Sequence Kit (Perkins Elmer), and decoded using a fluorescence automatic DNA sequencer. The sequences of the obtained clones

were respectively identical with the DNA sequence (SEQ No:14) to be amplified using synthetic DNA primers of SEQ Nos:10 and 11 and DNA sequence (SEQ No:15) to be amplified using synthetic DNA primers of SEQ Nos: 12 and 13, with human SLC-1 gene as a template.

**Reference Example 9E** Preparation of human SLC-1(S) expression CHO cell and human SLC-1(L) expression CHO cell

From a clone of E. coli transformed with a plasmid incorporating human SLC-1(S) and human SLC-1(L), whose sequences were confirmed in Reference Example 8E, a plasmid was prepared using Plasmid Midi Kit (QIAGEN) and cleaved with restriction enzymes Sal I and Spe I to excise an insert. The insert DNA was recovered by cutting out, after electrophoresis, from agarose gel with a razor and applying minimization, phenol extraction, phenol·chloroform extraction and ethanol precipitation. This insert DNA was added to animal cell expression vector plasmid pAKKO-111H (vector plasmid identical with pAKKO1.11H described in Hinuma, S. et al. Biochim. Biophys. Acta, Vol. 1219, pp. 251-259 (1994)) cleaved with Sal I and Spe I and ligated using T4 ligase (Takara Shuzo Co.) to respectively construct protein expression plasmids pAKKO-hSLC-1 (S) and PAKKO-hSLC-1(L).

E. coli DH5α (TOYOBO) transformed with pAKKO-hSLC-1(S) and pAKKO-hSLC-1(L) was cultured and, using Plasmid Midi Kit (QIAGEN), plasmid DNAs of pAKKO-hSLC-1(S) and pAKKO-hSLC-1(L) were prepared. These were introduced into CHO dhfr⁻ cells using CellPhect Transfection Kit (Amersham Pharmacia Biotech) in accordance with the attached protocol. DNA (10 μg) was prepared into a coprecipitation suspension with calcium phosphate and added into a 10 cm dish inoculated with 5 x $10^5$ or 1 x $10^6$ CHO dhfr⁻ cells 24 hours before. The cells were cultured in an MEMα medium containing 10% fetal bovine serum for one day, passaged and cultured in a nucleic acid-free MEMα, medium (selection medium) containing 10% dialyzed fetal bovine serum. 56 clones of transformed cell colonies, which were human SLC-1(S) gene introduced CHO cells, and 61 clones of transformed cell colonies, which were human SLC-1(L) gene introduced CHO cells, grew in the selection medium and were selected.

**Reference Example 10E** Selection of gene introduced cell line with high expression amount of human SLC-1(S) mRNA and human SLC-1(L) mRNA

The expression amount of mRNA of 56 clone of CHO/hSLC-1(S) cell line and 61 clones of CHO/hSLC-1(L) cell line established in Reference Example 9E was measured as in the following using Cytostar T Plate (Amersham Pharmacia Biotech) and in accordance with the attached protocol.

Each clone of the CHO/hSLC-1(S) cell line and CHO/hSLC-1(L) cell line was inoculated to each well of Cytostar T Plate at 2.5 x $10^4$, cultured for 24 hours and fixed with 10% formalin. After adding 0.25% Triton X-100 to each well to enhance permeability of the cells, riboprobe of $^{35}S$ labeled SEQ No:16 was added for hybridization. RNase A (20 mg/ml) was added to each well to digest free riboprobe. The plate was washed thoroughly and the radioactivity of the hybridized riboprobe was measured on Topcounter. The cell strain having high radioactivity showed higher expression amount of mRNA. Of the 7 clones showing high mRNA expression amount, particularly clone No. 57 was used mainly.

**Experimental Example 1** Determination of antagonistic activity using GTPνS binding assay of test compound

Using human SLC-1 expression CHO cell clone 57 obtained in Reference Example 10E and rat SLC-1 expression CHO cell clone 44 obtained in Reference Example 4E, membrane fractions were prepared by the following method. In phosphate buffered saline (pH 7.4) supplemented with 5 mM EDTA (ethylenediamine teraacetic acid) were suspended human and rat SLC-1 expression CHO cells (1x$10^8$) and centrifuged. Homogenate buffer (10 ml, 10 mM $NaHCO_3$, 5 mM EDTA, pH 7.5) was added to the pellets of the cells and, using Polytron Homogeniser, the mixture was homogenated. The supernatant obtained after centrifugation at 400×g for 15 min was further centrifuged at 100,000×g for 1 hr to give precipitate of the membrane fraction. The precipitate was suspended in 2 ml of an assay buffer [50 mM Tris-HCl (pH 7.5), 1 mM EDTA, 0.1% BSA (bovine serum albumin), 10 mM $MgCl_2$, 100 mM NaCl, 1 mM GDP (guanosine 5'-diphosphate), 0.25 mM PMSF (phenylmethylsulfonylfluoride), 1 mg/ml pepstatin, 20 mg/ml leupeptin, 10 mg/ml phosphoramidon] and centrifuged at 100,000×g for 1 hr. The membrane fraction recovered as precipitate was suspended again in 20 ml of an assay buffer, and after dispensing, preserved at -80°C and used upon thawing each time when in use.

The antagonistic activity of the test compound was determined as follows. The SLC-1 expression CHO cell membrane fraction (171 μl) diluted with an assay buffer was dispensed to a polypropylene 96 well plate and 3x$10^{-10}$ M MCH (2 ml) diluted with DMSO solution, test compound solution (2 ml) diluted to various concentrations and [$^{35}S$] -Guanosine-5'-(ν-thio) triphosphate (25 ml, Daiichi Pure Chemicals Co., Ltd.) were respectively added (cell membrane final concentration: 20 mg/ml, [$^{35}S$]-Guanosine 5'-(ν-thio)triphosphate final concentration: 0.33 nM). The reaction mixture was reacted at 25°C for 1 hr with stirring, suction filtered with a glass filter (GF-C) and washed 3 times with a wash solution (300 ml, 50 mM Tris-HCl buffer, pH 7.5). Liquid Scintillator (50 ml) was added to the glass filter and the residual radioactivity was determined by a liquid scintillation counter.

Binding inhibition (%) = (radioactivity upon addition of compound and MCH - radioactivity upon addition of DMSO solution)/(radioactivity upon addition of MCH - radioactivity upon addition of DMSO solution) x 100

From the binding inhibition (%), $IC_{50}$ of the compound was calculated. The results are shown in Table 1.

Table 1

| Compound | Inhibitory activity($IC_{50}$:nM) |
|---|---|
| Reference Example IIA-5 | 100 |
| Example 1 | 5 |

**Industrial Applicability**

The compound (I) and a salt thereof or a prodrug thereof of the present invention have a superior MCH antagonistic action and are useful as an agent for the prophylaxis and treatment of the diseases (e.g., obesity and the like) caused by melanin-concentrating hormone.

## Claims

1. A melanin-concentrating hormone antagonist containing a compound of the formula

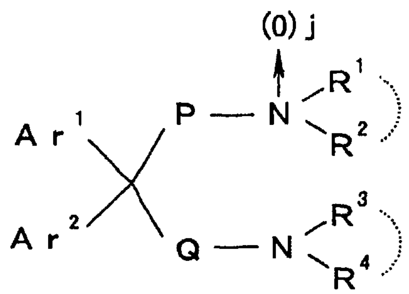

wherein

| | |
|---|---|
| $Ar^1$ and $Ar^2$ | are each an aromatic group optionally having substituents, |
| P and Q | are each a divalent aliphatic hydrocarbon group which optionally contains ether oxygen or ether sulfur in a carbon chain and which optionally has substituents, |
| $R^1$ and $R^3$ | are each (i) a hydrogen atom, (ii) an acyl group or (iii) a hydrocarbon group optionally having substituents, |
| $R^2$ and $R^4$ | are each (i) a hydrogen atom, (ii) an alkyl group optionally having substituents or (iii) an alkylcarbonyl group optionally having substituents, |
| $R^1$ and $R^2$ or $R^3$ and $R^4$ | optionally form, together with the adjacent nitrogen atom, a monocyclic or fused nitro- |

gen-containing heterocyclic group optionally having substituents, and
j is 0 or 1,

or a salt thereof or a prodrug thereof.

**2.** The antagonist of claim 1, wherein $Ar^1$ and $Ar^2$ are each (i) a $C_{6-14}$ aryl group or (ii) a 5 to 14-membered monocyclic or fused aromatic heterocyclic group containing, besides carbon atom, 1 to 4 heteroatom(s) selected from the group consisting of nitrogen atom, sulfur atom and oxygen atom, which optionally has 1 to 5 substituent(s) selected from the group (group Aa) consisting of

(a) a halogen atom,
(b) a $C_{1-3}$ alkylenedioxy group,
(c) a nitro group,
(d) a cyano group,
(e) an optionally halogenated $C_{1-6}$ alkyl group,
(f) an optionally halogenated $C_{3-6}$ cycloalkyl group,
(g) an optionally halogenated $C_{1-6}$ alkoxy group,
(h) an optionally halogenated $C_{1-6}$ alkylthio group,
(i) a hydroxy group,
(j) an amino group,
(k) a mono-$C_{1-6}$ alkylamino group,
(l) a di-$C_{1-6}$ alkylamino group,
(m) an optionally halogenated $C_{1-6}$ alkyl-carbonylamino group,
(n) a formyl group,
(o) a $C_{1-6}$ alkyl-carbonyl group optionally substituted by halogen atom or $C_{1-6}$ alkoxy-carbonyl group,
(p) a $C_{1-6}$ alkyl-carbonyloxy group,
(q) a carboxyl group,
(r) a $C_{1-6}$ alkoxy-carbonyl group,
(s) a carbamoyl group,
(t) a mono-$C_{1-6}$ alkyl-carbamoyl group optionally substituted by $C_{1-6}$ alkoxy-carbonyl group,
(u) a di-$C_{1-6}$ alkyl-carbamoyl group optionally substituted by $C_{1-6}$ alkoxy-carbonyl group,
(v) a sulfo group,
(w) a $C_{1-6}$ alkylsulfonyl group,
(x) a $C_{1-6}$ alkylsulfinyl group,
(y) a $C_{6-10}$ aryl group optionally having 1 to 4 substituent(s) selected from above-mentioned (a) to (x),
(z) a $C_{6-10}$ aryloxy group optionally having 1 to 4 substituent(s) selected from the above-mentioned (a) to (x),

(aa) an optionally halogenated $C_{6-10}$ aryl-carbonyl group,
(ab) an optionally halogenated 5 or 6-membered heterocyclic ring-carbonyl group,
(ac) a $C_{1-6}$ alkoxy-carbonylamino group,
(ad) a $C_{6-10}$ aryl-carbonylamino group and
(ae) a $C_{7-16}$ aralkyloxy-carbonyl group,

P and Q are each a divalent $C_{1-6}$ aliphatic hydrocarbon group which optionally contains ether oxygen or ether sulfur in a carbon chain and which is optionally substituted by oxo group or thioxo group;
$R^1$ and $R^3$ are each (i) hydrogen atom, (ii) acyl group represented by $-CO-R^a$, $-CONR^aR^b$, $-SO-R^a$, $-SO_2-R^a$, $-CONR^aR^b$, $-COO-R^a$, $-(C{=}S)O-R^a$, $-(C{=}S)NR^aR^b$, $-SONR^aR^b$, $-SO_2NR^aR^b$, $-SO-O-R^a$ or $-SO_2-O-R^a$, wherein $R^a$ is (A) hydrogen atom; (B) carboxyl group; (C) (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group, which optionally has 1 to 5 substituent(s) selected from the group (group Ba) consisting of

(a) a halogen atom,
(b) a $C_{1-3}$ alkylenedioxy group,
(c) a nitro group,
(d) a cyano group,
(e) an optionally halogenated $C_{1-6}$ alkyl group,
(f) an optionally halogenated $C_{3-6}$ cycloalkyl group,
(g) an optionally halogenated $C_{1-6}$ alkoxy group,

(h) an optionally halogenated $C_{1-6}$ alkylthio group,
(i) a hydroxy group,
(j) an amino group,
(k) a mono-$C_{1-6}$ alkylamino group,
(l) a di-$C_{1-6}$ alkylamino group,
(m) a $C_{1-6}$ alkyl-carbonylamino group,
(n) a formyl group,
(o) a $C_{1-6}$ alkyl-carbonyl group,
(p) a $C_{1-6}$ alkyl-carbonyloxy group,
(q) a carboxyl group,
(r) a $C_{1-6}$ alkoxy-carbonyl group,
(s) a carbamoyl group,
(t) a mono-$C_{1-6}$ alkyl-carbamoyl group,
(u) a di-$C_{1-6}$ alkyl-carbamoyl group,
(v) a sulfo group,
(w) a $C_{1-6}$ alkylsulfonyl group,
(x) a $C_{1-6}$ alkylsulfinyl group,
(y) a $C_{6-10}$ aryl group optionally having 1 to 4 substituent(s) selected from the aforementioned (a) to (x),
(z) a $C_{6-10}$ aryloxy group optionally having 1 to 4 substituent(s) selected from the aforementioned (a) to (x),
(zz) a 5 to 7-membered heterocyclic group optionally having 1 to 4 substituent(s) selected from the aforementioned (a) to (x),

(aa) a di-$C_{1-6}$ alkyl-carbonylamino group,
(ab) a sulfamoyl group,
(ac) a $C_{1-6}$ alkoxy-carbonylamino group,
(ad) a $C_{7-16}$ aralkyloxy-carbonylamino group,
(ae) a $C_{7-16}$ aralkyloxy group,
(af) a $C_{6-10}$ aryl-carbonyl group,
(ag) a $C_{1-6}$ alkyl-carbonyloxy group,
(ah) a $C_{6-10}$ aryl-carbonylamino group,
(ai) a $C_{6-10}$ aryl-carbamoyl group,
(aj) a $C_{7-16}$ aralkylaminocarbonyl group,
(ak) a $C_{7-16}$ aralkylcarbonylamino group and
(al) a $C_{7-16}$ aralkyloxy-carbonyloxy group;

(D) a 5 to 10-membered heterocyclic group containing, besides carbon atom, 1 to 4 heteroatom(s) selected from the group consisting of nitrogen atom, oxygen atom and sulfur atom, which optionally has 1 to 5 substituent(s) selected from the group consisting of

(a) substituent selected from group Aa,
(b) (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group, which optionally has 1 to 5 substituent(s) selected from group Ba,
(c) oxo group and
(d) thioxo group; or

(E) a $C_{1-6}$ alkoxy-carbonyl group;
$R^b$ is a hydrogen atom or a $C_{1-6}$ alkyl group, or
(iii) (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group optionally having 1 to 5 substituent(s) selected from group Ba;
$R^2$ and $R^4$ are each (i) a hydrogen atom, (ii) $C_{1-6}$ alkyl group optionally having substituents selected from group Ba or (iii) $C_{1-6}$ alkyl-carbonyl group optionally having substituents selected from group Ba;
$R^1$ and $R^2$ or $R^3$ and $R^4$ may form, together with the adjacent nitrogen atom, a group of

(i) the formula

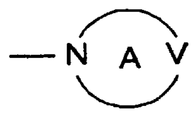

wherein ring A is a 4 to 8-membered ring optionally substituted by hydroxy or oxo, V is a group represented by the formula >O, >C=O, >C(W)-$W^a$ or >N-W (W is (a) hydrogen atom, (b) (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group, which optionally has 1 to 5 substituent(s) selected from group Ba, or (c) 5 to 10-membered heterocyclic group containing, besides carbon atom, 1 to 4 heteroatom(s) selected from nitrogen, oxygen and sulfur, which optionally has 1 to 5 substituent(s) selected from group Aa, $W^a$ is hydrogen atom, hydroxy group or $C_{1-6}$ alkyl group),
(ii) the formula

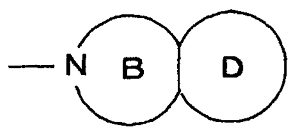

wherein ring B is monocyclic or bicyclic 4 to 12-membered ring optionally substituted by 1 or 2 oxo group(s) or 1 to 5 $C_{1-6}$ alkyl group(s), ring D is a 4 to 12-membered aromatic ring optionally having 1 to 5 substituent (s) selected from group Aa or
(iii) the formula

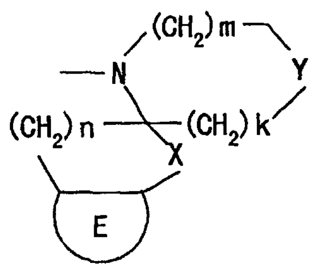

wherein ring E is a 4 to 12-membered aromatic ring optionally having 1 to 5 substituent(s) selected from group Aa;

X is $-CH_2-$, -CO- or -CH(OH)-;
Y is $-CH_2-$, -O- or $-NW^b-$ ($W^b$ is (a) hydrogen atom or (b) $C_{1-6}$ alkyl group optionally having substituents selected from group Ba) ;
k and m are each an integer of 0 to 4, and k+m is an integer of 1 to 4;
n is an integer of 1 to 3.

**3.** The antagonist of claim 1, wherein $Ar^1$ and $Ar^2$ are each (i) a $C_{6-14}$ aryl group or (ii) a 5 to 14-membered monocyclic or fused aromatic heterocyclic group containing, besides carbon atom, 1 to 4 heteroatom(s) selected from the group consisting of nitrogen atom, sulfur atom and oxygen atom, which optionally has 1 to 5 substituent(s) selected from the group (group A) consisting of

(a) a halogen atom,
(b) a $C_{1-3}$ alkylenedioxy group,
(c) a nitro group,
(d) a cyano group,
(e) an optionally halogenated $C_{1-6}$ alkyl group,
(f) an optionally halogenated $C_{3-6}$ cycloalkyl group,
(g) an optionally halogenated $C_{1-6}$ alkoxy group,

(h) an optionally halogenated $C_{1-6}$ alkylthio group,
(i) a hydroxy group,
(j) an amino group,
(k) a mono-$C_{1-6}$ alkylamino group,
(l) a di-$C_{1-6}$ alkylamino group,
(m) a $C_{1-6}$ alkyl-carbonylamino group,
(n) a formyl group,
(o) a $C_{1-6}$ alkyl-carbonyl group,
(p) a $C_{1-6}$ alkyl-carbonyloxy group,
(q) a carboxyl group,
(r) a $C_{1-6}$ alkoxy-carbonyl group,
(s) a carbamoyl group,
(t) a mono-$C_{1-6}$ alkylcarbamoyl group,
(u) a di-$C_{1-6}$ alkylcarbamoyl group,
(v) a sulfo group,
(w) a $C_{1-6}$ alkylsulfonyl group,
(x) a $C_{1-6}$ alkylsulfinyl group,
(y) a $C_{6-10}$ aryl group optionally having 1 to 4 substituent(s) selected from the above-mentioned (a) to (x) and
(z) a $C_{6-10}$ aryloxy group optionally having 1 to 4 substituent(s) selected from the above-mentioned (a) to (x),

P and Q are each a $C_{1-6}$ aliphatic hydrocarbon group which optionally contains ether oxygen or ether sulfur in a carbon chain and which is optionally substituted by oxo group or thioxo group,
$R^1$ and $R^3$ are each (i) hydrogen atom, (ii) an acyl group represented by $-CO-R^a$, $-CONR^aR^b$, $-SO-R^a$, $-SO_2-R^a$, $-CONR^aR^b$, $-COO-R^{a,}$ $-(C=S)O-R^a$ or $-(C=S)NR^aR^b$ wherein $R^a$ is

(a) hydrogen atom,
(b) carboxyl group,
(c) a (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group, which optionally has 1 to 5 substituent(s) selected from the group (group B) consisting of

(a) a halogen atom,
(b) a $C_{1-3}$ alkylenedioxy group,
(c) a nitro group,
(d) a cyano group,
(e) an optionally halogenated $C_{1-6}$ alkyl group,
(f) an optionally halogenated $C_{3-6}$ cycloalkyl group,
(g) an optionally halogenated $C_{1-6}$ alkoxy group,
(h) an optionally halogenated $C_{1-6}$ alkylthio group,
(i) a hydroxy group,
(j) an amino group,
(k) a mono-$C_{1-6}$ alkylamino group,
(l) a di-$C_{1-6}$ alkylamino group,
(m) a $C_{1-6}$ alkyl-carbonylamino group,
(n) a formyl group,
(o) a $C_{1-6}$ alkyl-carbonyl group,
(p) a $C_{1-6}$ alkyl-carbonyloxy group,
(q) a carboxyl group,
(r) a $C_{1-6}$ alkoxy-carbonyl group,
(s) a carbamoyl group,
(t) a mono-$C_{1-6}$ alkylcarbamoyl group,
(u) a di-$C_{1-6}$ alkylcarbamoyl group,
(v) a sulfo group,
(w) a $C_{1-6}$ alkylsulfonyl group,
(x) a $C_{1-6}$ alkylsulfinyl group,
(y) a $C_{6-10}$ aryl group optionally having 1 to 4 substituent (s) selected from the aforementioned (a) to (x),
(z) a $C_{6-10}$ aryloxy group optionally having 1 to 4 substituent(s) selected from the aforementioned (a) to (x) and

(zz) a 5 to 7-membered heterocyclic group optionally having 1 to 4 substituent(s) selected from the aforementioned (a) to (x), or

(d) a 5 to 10-membered heterocyclic group containing, besides carbon atom, 1 to 4 heteroatom(s) selected from the group consisting of nitrogen atom, oxygen atom and sulfur atom, which optionally has 1 to 5 substituent(s) selected from the group (group C) consisting of

(a) a halogen atom,
(b) a $C_{1-3}$ alkylenedioxy group,
(c) a nitro group,
(d) a cyano group,
(e) a $C_{1-6}$ alkyl group optionally having substituents selected from the group consisting of (aa) a halogen atom, (bb) $C_{1-3}$ alkylenedioxy group, (cc) nitro group, (dd) cyano group, (ee) an optionally halogenated $C_{1-6}$ alkyl group, (ff) an optionally halogenated $C_{3-6}$ cycloalkyl group, (gg) an optionally halogenated $C_{1-6}$ alkoxy group, (hh) an optionally halogenated $C_{1-6}$ alkylthio group, (ii) a hydroxy group, (jj) amino group, (kk) a mono-$C_{1-6}$ alkylamino group, (ll) a di-$C_{1-6}$ alkylamino group, (mm) $C_{1-6}$ alkyl-carbonylamino group, (nn) a formyl group, (oo) $C_{1-6}$ alkyl-carbonyl group, (pp) $C_{1-6}$ alkyl-carbonyloxy group, (qq) carboxyl group, (rr) $C_{1-6}$ alkoxy-carbonyl group, (ss) carbamoyl group, (tt) a mono-$C_{1-6}$ alkylcarbamoyl group, (uu) a di-$C_{1-6}$ alkylcarbamoyl group, (vv) a sulfo group, (ww) $C_{1-6}$ alkylsulfonyl group, (xx) $C_{1-6}$ alkylsulfinyl group, (yy) $C_{6-10}$ aryl group optionally having 1 to 4 substituent(s) selected from the aforementioned (aa) to (xx), (zz) $C_{6-10}$ aryloxy group optionally having 1 to 4 substituent(s) selected from the aforementioned (aa) to (xx) and (zzz) 5 to 7-membered heterocyclic group optionally having 1 to 4 substituent(s) selected from the aforementioned (aa) to (xx),
(f) an optionally halogenated $C_{3-6}$ cycloalkyl group,
(g) an optionally halogenated $C_{1-6}$ alkoxy group,
(h) an optionally halogenated $C_{1-6}$ alkylthio group,
(i) a hydroxy group,
(j) an amino group,
(k) a mono-$C_{1-6}$ alkylamino group,
(l) a di-$C_{1-6}$ alkylamino group,
(m) an optionally halogenated $C_{1-6}$ alkyl-carbonylamino group,
(n) a formyl group,
(o) a $C_{1-6}$ alkyl-carbonyl group,
(p) a $C_{1-6}$ alkyl-carbonyloxy group,
(q) a carboxyl group,
(r) a $C_{1-6}$ alkoxy-carbonyl group,
(s) a carbamoyl group,
(t) a mono-$C_{1-6}$ alkylcarbamoyl group,
(u) a di-$C_{1-6}$ alkylcarbamoyl group,
(v) a sulfo group,
(w) a $C_{1-6}$ alkylsulfonyl group,
(x) a $C_{1-6}$ alkylsulfinyl group,
(y) a $C_{6-10}$ aryl group optionally having 1 to 4 substituent (s) selected from the aforementioned (a) to (x) and
(z) a $C_{6-10}$ aryloxy group optionally having 1 to 4 substituent(s) selected from the aforementioned (a) to (x), and

$R^b$ is a hydrogen atom or a $C_{1-6}$ alkyl group) or
(iii) (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group, which optionally has 1 to 5 substituent(s) selected from group B,
$R^2$ and $R^4$ are each (i) hydrogen atom, (ii) $C_{1-6}$ alkyl optionally having substituents selected from group B or (iii) $C_{1-6}$ alkyl-carbonyl group optionally having substituents selected from group B,
$R^1$ and $R^2$ or $R^3$ and $R^4$ form, together with the adjacent nitrogen atom, a nitrogen-containing heterocyclic group represented by

(i) the formula

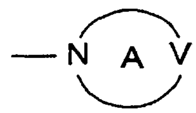

wherein ring A is a 4 to 8-membered ring optionally substituted by hydroxy or oxo, V is a group represented by the formula >O, >C=O, >C-(W)$W^a$ or >N-W (W is (a) hydrogen atom, (b) (1) $C_{1-6}$ alkyl group, (2) $C_{2-6}$ alkenyl group, (3) $C_{2-6}$ alkynyl group, (4) $C_{3-6}$ cycloalkyl group, (5) $C_{6-14}$ aryl group or (6) $C_{7-16}$ aralkyl group, which optionally has 1 to 5 substituent(s) selected from group B, or (c) 5 to 10-membered heterocyclic group containing, besides carbon atom, 1 to 4 heteroatom(s) selected from nitrogen, oxygen and sulfur, which optionally has 1 to 5 substituent(s) selected from group A, $W^a$ is hydrogen atom or hydroxy group),
(ii) the formula

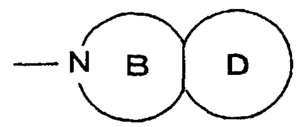

wherein ring B is monocyclic or bicyclic 4 to 12-membered ring optionally substituted by oxo group or 1 to 5 $C_{1-6}$ alkyl group(s), ring D is a 4 to 12-membered aromatic ring optionally having 1 to 5 substituent(s) selected from group A or
(iii) the formula

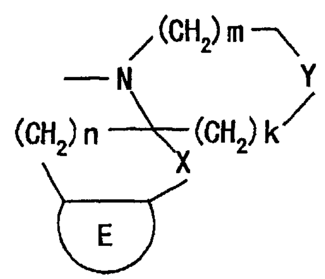

wherein ring E is a 5 to 10-membered aromatic ring optionally having 1 to 5 substituent(s) selected from group A
X is $-CH_2-$, -CO- or -CH(OH)-,
Y is $-CH_2-$, -O- or $-NW^b-$ ($W^b$ is (a) hydrogen atom or (b) $C_{1-6}$ alkyl group optionally having substituents selected from group B) ;
k+m is an integer of 1 to 4; and
n is an integer of 1 to 3.

**4.** The antagonist of claim 1, wherein $Ar^1$ and $Ar^2$ are each (i) a phenyl group optionally substituted by halogen atom or $C_{1-6}$ alkoxy group or (ii) a 5 or 6-membered heterocyclic group containing, besides carbon atom, 1 to 3 heteroatom(s) selected from nitrogen atom, oxygen atom and sulfur atom.

**5.** The antagonist of claim 1, wherein P and Q are each a $C_{1-6}$ alkylene group.

**6.** The antagonist of claim 1, wherein j is 0.

**7.** The antagonist of claim 1, wherein
$R^1$ is (i) $C_{1-6}$ alkyl group optionally having a 5 or 6-membered nitrogen-containing heterocyclic group, (ii) $C_{7-16}$ aralkyl group optionally having nitro, amino or $C_{1-6}$ alkoxy-carbonyl or (iii) cyclohexyl group fused with benzene ring optionally having $C_{1-6}$ alkoxy;
$R^2$ is (i) hydrogen atom, (ii) $C_{1-6}$ alkyl group or (iii) $C_{7-16}$ aralkyl group; or $R^1$ and $R^2$ form, together with the adjacent nitrogen atom, a nitrogen-containing heterocyclic group represented by

(i) the formula

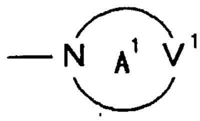

wherein ring $A^1$ is a 4 to 8-membered ring optionally substituted by hydroxy or oxo, $V^1$ is a group represented by the formula >O, >C($W^1$)-$W^{a1}$ or >N-$W^1$ ($W^1$ is (a) hydrogen atom, (b) $C_{6-14}$ aryl group optionally having 1 or 2 substituent (s) 5 selected from the group consisting of a halogen atom, optionally halogenated $C_{1-6}$ alkyl group and optionally halogenated $C_{1-6}$ alkoxy group, (c) $C_{1-6}$ alkyl group optionally having 1 or 2 $C_{6-10}$ aryl group (s) or (d) pyridyl group, $W^{a1}$ is hydrogen atom, hydroxy group or $C_{1-6}$ alkyl group),

(ii) the formula

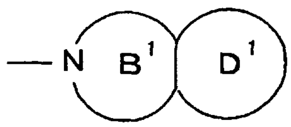

wherein ring $B^1$ is a monocyclic or bicyclic 5 to 10-membered ring optionally substituted by oxo group or 1 or 2 $C_{1-6}$ alkyl group(s), ring $D^1$ is a benzene ring optionally having 1 or 2 substituent(s) selected from the group consisting of $C_{1-6}$ alkyl group, $C_{1-6}$ alkoxy group and $C_{1-6}$ alkyl-carbonyl group or

(iii) the formula

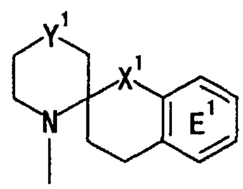

wherein ring $E^1$ is a benzene ring optionally having 1 to 3 substituent(s) selected from the group consisting of $C_{1-3}$ alkylenedioxy group, nitro group, $C_{1-6}$ alkoxy group, amino group, $C_{1-6}$ alkyl-carbonylamino group and $C_{1-6}$ alkoxy-carbonyl group,

$X^1$ is -$CH_2$- or -CO-, and

$Y^1$ is -$CH_2$- or -O-,

$R^3$ is

(i) hydrogen atom,

(ii) a group represented by the formula -CO-$R^5$ ($R^5$ is (a) hydrogen atom, (b) carboxyl group, (c) $C_{1-6}$ alkyl group, (d) $C_{5-6}$ cycloalkyl group optionally having $C_{1-6}$ alkoxy, and which is fused with benzene ring or (e) 5 or 6-membered aromatic heterocyclic group containing, besides carbon atom, 1 to 3 heteroatom(s) selected from the group consisting of nitrogen atom, oxygen atom and sulfur atom, which optionally has 1 or 2 substituent (s) selected from the group consisting of a halogen atom, $C_{6-10}$ aryl group, $C_{6-10}$ aryl-carbonylamino group),

(iii) a group represented by the formula -CO-$Alk_0$-$R^6$ [$Alk_0$ is $C_{1-6}$ alkylene group optionally having hydroxy group, $R^6$ is (a) $C_{6-14}$ aryl group optionally having 1 or 2 substituent(s) selected from the group consisting of a halogen atom, optionally halogenated $C_{1-6}$ alkyl, nitro, $C_{1-6}$ alkoxy, $C_{1-3}$ alkylenedioxy and $C_{6-10}$ aryl group, (b) $C_{6-10}$ aryloxy group, (c) 5 or 6-membered aromatic heterocyclic group containing, besides carbon atom, 1 or 2 heteroatom(s) selected from nitrogen atom, oxygen atom and sulfur atom (d) $C_{1-6}$ alkyl-carbonyl group, (e) carboxyl group, (f) $C_{1-6}$ alkoxy-carbonyl group, (g) amino group optionally having 1 or 2 substituent(s) selected from the group consisting of $C_{1-6}$ alkyl and $C_{1-6}$ alkyl-carbonyl, (h) 5 to 7-membered heterocyclic ring optionally having hydroxy, (i) $C_{7-16}$ aralkyloxy group, (j) $C_{6-10}$ aryl-carbonyl group or (k) $C_{1-6}$ alkyl-carbonyloxy group],

(iv) a group represented by the formula

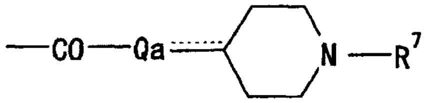

wherein Qa is a group represented by the formula $-(CH_2)s-$ (s is an integer of 1 to 3) or $-(CH_2)t-CH=$ (t is an integer of 0 to 2) and $R^7$ is hydrogen atom or $C_{1-6}$ alkoxy-carbonyl group,
(v) a group represented by the formula

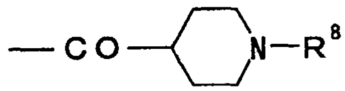

wherein $R^8$ is (a) hydrogen atom, (b) $C_{1-6}$ alkyl group optionally having substituents selected from the group consisting of $C_{1-6}$ alkoxy-carbonyl, morpholino and mono- or di-$C_{1-6}$ alkylamino, (c) $C_{1-6}$ alkoxy-carbonyl group, (d) a group represented by the formula $-CO-R^d$ ($R^d$ is $C_{6-10}$ aryl group optionally having halogen atom or 5 or 6-membered heterocyclic group containing, besides carbon atom, 1 or 2 heteroatom(s) selected from nitrogen atom, oxygen atom and sulfur atom), (e) a group represented by the formula $-CO-(CH_2)r^1-R^e$ ($r^1$ is an integer of 1 to 3, $R^e$ is $C_{1-6}$ alkoxy-carbonyl group or 5 or 6-membered heterocyclic group containing, besides carbon atom, 1 or 2 heteroatom(s) selected from nitrogen atom, oxygen atom and sulfur atom) or (f) a group represented by $-CONH-R^f$ ($R^f$ is $C_{1-6}$ alkyl group or $C_{6-14}$ aryl group),
(vi) a group represented by the formula

$$-COOR^9$$

($R^9$ is optionally halogenated $C_{1-6}$ alkyl group),
(vii) a group represented by the formula

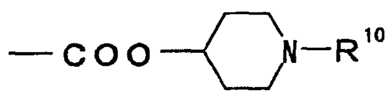

wherein $R^{10}$ is hydrogen atom, $C_{1-6}$ alkoxy-carbonyl group, mono- or di-$C_{1-6}$ alkyl-carbamoyl group, optionally halogenated nicotinoyl group or optionally halogenated isonicotinoyl group,
(viii) a group represented by the formula

$$-CONR^{11}-R^{12}$$

($R^{11}$ is hydrogen atom or $C_{1-6}$ alkyl group, $R^{12}$ is $C_{1-6}$ alkyl group optionally having substituents selected from the group consisting of (a) hydroxy, (b) amino, (c) a mono- or di-$C_{1-6}$ alkyl-amino, (d) $C_{1-6}$ alkyl-carbonyl, (e) $C_{1-6}$ alkoxy-carbonyl, (f) $C_{1-6}$ alkyl-carbonyloxy, (g) sulfamoyl and (h) 5 to 7-membered heterocyclic group optionally substituted by oxo, and (i) $C_{6-14}$ aryl group) ,
(ix) a group represented by the formula

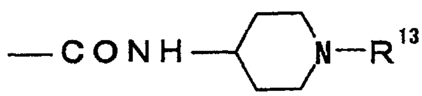

wherein $R^{13}$ is (a) hydrogen atom, (b) $C_{1-6}$ alkyl group optionally having substituents selected from the group consisting of a hydroxy and $C_{1-6}$ alkoxy-carbonyl, (c) $C_{7-16}$ aralkyl group, (d) $C_{1-6}$ alkyl-carbonyl group optionally having substituents selected from the group consisting of a halogen atom and $C_{1-6}$ alkoxy-carbonyl or (e) $C_{1-6}$ alkyl-carbamoyl group optionally having $C_{1-6}$ alkoxy-carbonyl,

(x) a group represented by the formula

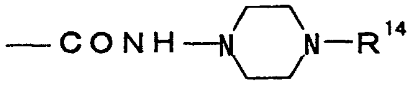

wherein $R^{14}$ is $C_{1-6}$ alkyl group or $C_{7-16}$ aralkyl group,
(xi) a group represented by the formula

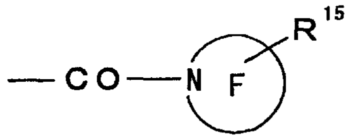

wherein ring F is 5 to 7-membered non-aromatic heterocyclic group optionally fused with benzene ring and $R^{15}$ is hydrogen atom, $C_{1-6}$ alkoxy-carbonylamino group or optionally halogenated $C_{1-6}$ alkyl-carbonylamino group,
(xii) a group represented by the formula

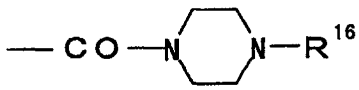

wherein $R^{16}$ is (a) $C_{1-6}$ alkyl group optionally having substituents selected from the group consisting of a hydroxy and $C_{1-6}$ alkoxy-carbonyl, (b) a formyl group, (c) $C_{1-6}$ alkoxy-carbonyl group or (d) 5 or 6-membered heterocyclic ring-carbonyl group containing, besides carbon atom, 1 to 3 heteroatom(s) selected from the group consisting of nitrogen atom, oxygen atom and sulfur atom,
(xiii) a group represented by the formula

$$-SO_2-R^{17}$$

($R^{17}$ is (i) $C_{1-6}$ alkyl group optionally having 5 or 6-membered heterocyclic group, (ii) $C_{2-6}$ alkenyl group or (iii) $C_{6-14}$ aryl group optionally having $C_{1-6}$ alkyl),
(xiv) $C_{7-16}$ aralkyl group optionally having 1 to 3 halogen atom(s) or $C_{1-6}$ alkoxy group,
(xv) $C_{1-6}$ alkyl group substituted by 5 or 6-membered heterocyclic group containing, besides carbon atom, 1 to 3 heteroatom(s) selected from the group consisting of nitrogen atom, oxygen atom and sulfur atom,
(xvi) a group represented by the formula

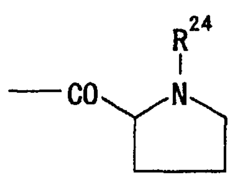

wherein $R^{24}$ is hydrogen atom or $C_{7-16}$ aralkyloxy-carbonyl group;
(xvii) a group represented by the formula

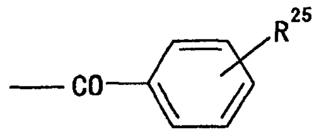

wherein $R^{25}$ is hydrogen atom, $C_{6-10}$ aryl group, $C_{7-16}$ aralkyloxy group, $C_{6-10}$ aryloxy group, halogen atom, $C_{6-10}$ aryl-carbonylamino group or $C_{6-10}$ aryl-carbamoyl group;
(xviii) a group represented by the formula

$$-CO-Alk-NR^{27}-CO-Alk_2-O-Alk_3-R^{28}$$

[Alk is $C_{1-6}$ alkylene group optionally having substituents; $R^{27}$ is hydrogen atom or $C_{1-6}$ alkyl group; $Alk_2$ and $Alk_3$ are the same or different and each is a bond or $C_{1-6}$ alkylene group optionally having substituents; $R^{28}$ is $C_{6-10}$ aryl group optionally having substituents or hydrogen atom];
(xix) a group represented by the formula

$$-CO-Alk_2-NR^{27}-CO-Alk_3-R^{29}$$

[$Alk_2$, $Alk_3$ and $R^{27}$ are as defined above; $R^{29}$ is (1) $C_{6-10}$ aryl group optionally having substituent or (2) 5 to 10-membered aromatic heterocyclic group optionally having substituent, which contains, besides carbon atom, 1 to 3 heteroatom(s) selected from the group consisting of nitrogen atom, oxygen atom and sulfur atom];
(xx) a group represented by the formula

$$-CO-Alk-NR^{27}-CO-Alk_2-NR^{30}-Alk_3-R^{31}$$

[Alk, $R^{27}$, $Alk_2$, $Alk_3$ are as defined above; $R^{30}$ is hydrogen atom, $C_{1-6}$ alkyl group or optionally halogenated $C_{1-6}$ alkyl-carbonyl group; and $R^{31}$ is $C_{6-10}$ aryl group optionally having substituents];
(xxi) a group represented by the formula

$$-CO-Alk-NR^{27}-CO-Alk_2-NR^{32}-CO-O-Alk_3-R^{31}$$

[Alk, $R^{27}$, $Alk_2$, $Alk_3$ and $R^{31}$ are as defined above; and $R^{32}$ is the same as the aforementioned $R^{27}$];
(xxii) a group represented by the formula

$$-CO-Alk-CO-NR^{27}-Alk_2-R^{31}$$

[Alk, $R^{27}$, $Alk_2$ and $R^{31}$ are as defined above] ; or
(xxiii) a group represented by the formula

$$-CO-Alk-O-CO-O-Alk_2-R^{31}$$

[Alk, $Alk_2$ and $R^{31}$ are as defined above] ;

$R^4$ is hydrogen atom or $C_{1-6}$ alkyl group;
or $R^3$ and $R^4$ may form, together with the adjacent nitrogen atom, a group represented by the formula

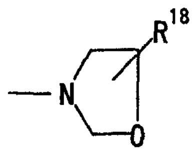

wherein $R^{18}$ is halogen atom, oxo group, optionally halogenated $C_{1-6}$ alkyl group or optionally halogenated $C_{1-6}$ alkoxy group.

**8.** The antagonist of claim 1, wherein $R^1$ is (i) $C_{1-6}$ alkyl group optionally having 5 or 6-membered nitrogen-containing heterocyclic group, (ii) $C_{7-16}$ aralkyl group optionally having nitro, amino or $C_{1-6}$ alkoxy-carbonyl or (iii) cyclohexyl group fused with benzene ring optionally having $C_{1-6}$ alkoxy, $R^2$ is (i) hydrogen atom, (ii) $C_{1-6}$ alkyl group or (iii) $C_{7-16}$ aralkyl group, or $R^1$ and $R^2$ may form, together with the adjacent nitrogen atom, a nitrogen-containing heterocyclic group represented by

(i) the formula

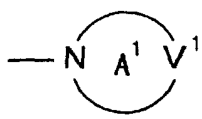

wherein ring $A^1$ is a 4 to 8-membered ring optionally substituted by hydroxy or oxo, $V^1$ is a group represented by the formula >O, >C-($W^1$)$W^{a1}$ or >N-$W^1$ ($W^1$ is (a) hydrogen atom, (b) $C_{6-14}$ aryl group optionally having 1 or 2 substituent(s) selected from the group consisting of halogen atom, optionally halogenated $C_{1-6}$ alkyl group and $C_{1-6}$ alkoxy group or (c) $C_{1-6}$ alkyl group optionally having 1 or 2 $C_{6-10}$ aryl group(s), $W^{a1}$ is hydrogen atom or hydroxy group),
(ii) the formula

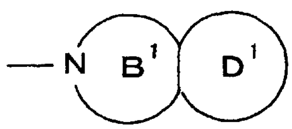

wherein ring $B^1$ is a monocyclic or bicyclic 5 to 10-membered ring optionally substituted by oxo group or 1 or 2 $C_{1-6}$ alkyl group(s), ring $D^1$ is a benzene ring optionally having 1 or 2 substituent(s) selected from the group consisting of $C_{1-6}$ alkyl group, $C_{1-6}$ alkoxy group and $C_{1-6}$ alkyl-carbonyl group or
(iii) the formula

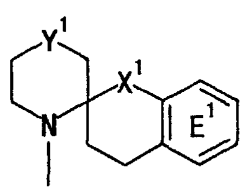

wherein ring $E^1$ is a benzene ring optionally having 1 or 2 substituent(s) selected from the group consisting of $C_{1-3}$ alkylenedioxy group, nitro group, $C_{1-6}$ alkoxy group, amino group, $C_{1-6}$ alkyl-carbonylamino group and $C_{1-6}$ alkoxy-carbonyl group
$X^1$ is $-CH_2-$ or -CO-,
$Y^1$ is $-CH_2-$ or -O-,
$R^3$ is

(i) hydrogen atom,

(ii) a group represented by the formula -CO-$R^5$ ($R^5$ is (a) hydrogen atom, (b) carboxyl group, (c) $C_{1-6}$ alkyl group, (d) $C_{5-7}$ cycloalkyl group optionally having alkoxy, and which is fused with benzene ring or (e) 5 or 6-membered aromatic heterocyclic group containing, besides carbon atom, 1 to 3 heteroatom(s) selected from the group consisting of nitrogen atom, oxygen atom and sulfur atom),

(iii) a group represented by the formula -CO-$(CH_2)r^1$-$R^6$ ($r^1$ is an integer of 1 to 3, $R^6$ is (a) $C_{6-14}$ aryl group optionally having 1 or 2 substituent(s) selected from the group consisting of halogen atom, optionally halogenated $C_{1-6}$ alkyl, nitro, $C_{1-6}$ alkoxy and $C_{1-3}$ alkylenedioxy, (b) $C_{6-14}$ aryloxy group, (c) 5 or 6-membered aromatic heterocyclic group containing, besides carbon atom, 1 or 2 heteroatom(s) selected from nitrogen atom, oxygen atom and sulfur atom, (d) $C_{1-6}$ alkyl-carbonyl group, (e) carboxyl group, (f) $C_{1-6}$ alkoxy-carbonyl group, (g) amino group optionally having 1 or 2 substituent(s) selected from the group consisting of $C_{1-6}$ alkyl and $C_{1-6}$ alkyl-carbonyl or (h) 5 or 6-membered cyclic amino group optionally having hydroxy),

(iv) a group represented by the formula

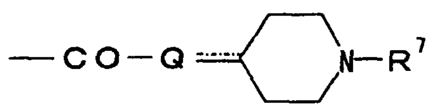

(Q is a group represented by the formula -$(CH_2)$s- (s is an integer of 1 to 3) or -$(CH_2)$t-CH= (t is an integer of 0 to 2), $R^7$ is hydrogen atom or $C_{1-6}$ alkoxy-carbonyl group),

(v) a group represented by the formula

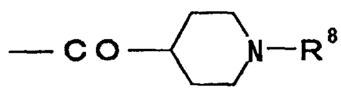

($R^8$ is (a) hydrogen atom, (b) $C_{1-6}$ alkyl group optionally having substituents selected from the group consisting of $C_{1-6}$ alkoxy-carbonyl, morpholino and mono- or di-$C_{1-6}$ alkylamino, (c) $C_{1-6}$ alkoxy-carbonyl group, (d) a group represented by the formula -CO-$R^d$ ($R^d$ is $C_{6-14}$ aryl group optionally having halogen atom or 5 or 6-membered heterocyclic group containing, besides carbon atom, 1 or 2 heteroatom(s) selected from nitrogen atom, oxygen atom and sulfur atom),

(e) a group represented by the formula -CO-$(CH_2)r^1$-$R^e$ ($r^1$ is an integer of 1 to 3, $R^e$ is $C_{1-6}$ alkoxy-carbonyl group or 5 or 6-membered heterocyclic group containing, besides carbon atom, 1 or 2 heteroatom(s) selected from nitrogen atom, oxygen atom and sulfur atom) or (f) a group represented by -CONH-$R^f$ ($R^f$ is $C_{1-6}$ alkyl group or $C_{6-14}$ aryl group)),

(vi) a group represented by the formula

$$-COOR^9$$

($R^9$ is optionally halogenated $C_{1-6}$ alkyl group),

(vii) a group represented by the formula

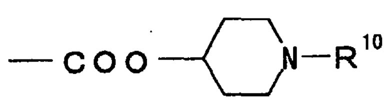

wherein $R^{10}$ is hydrogen atom, $C_{1-6}$ alkoxy-carbonyl group, mono or di-$C_{1-6}$ alkyl-carbamoyl group, optionally halogenated nicotinoyl group or optionally halogenated isonicotinoyl group,

(viii) a group represented by the formula -$CONR^{11}$-$R^{12}$ ($R^{11}$ is hydrogen atom or $C_{1-6}$ alkyl group, $R^{12}$ is $C_{1-6}$ alkyl optionally having substituents selected from the group consisting of (a) hydroxy, (b) amino, (c) a mono- or di-$C_{1-6}$ alkyl-amino, (d) $C_{1-6}$ alkyl-carbonyl, (e) $C_{1-6}$ alkoxy-carbonyl, (f) $C_{1-6}$ alkyl-carbonyloxy, (g) sulfamoyl and (f) 5 or 6-membered cyclic amine optionally substituted by oxo),

(ix) a group represented by the formula

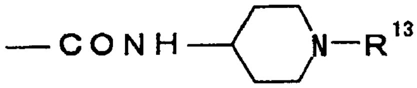

wherein $R^{13}$ is (a) a hydrogen atom, (b) $C_{1-6}$ alkyl group optionally having substituents selected from the group consisting of a hydroxy and $C_{1-6}$ alkoxy-carbonyl, (c) $C_{7-16}$ aralkyl group, (d) $C_{1-6}$ alkyl-carbonyl group optionally having substituents selected from the group consisting of a halogen and $C_{1-6}$ alkoxy-carbonyl or (e) $C_{1-6}$ alkyl-carbamoyl group optionally having $C_{1-6}$ alkoxy-carbonyl,
(x) a group represented by the formula

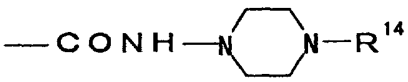

wherein $R^{14}$ is $C_{1-6}$ alkyl group or $C_{7-16}$ aralkyl group,
(xi) a group represented by the formula

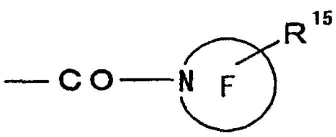

wherein ring F is 5 to 7-membered cyclic amino group optionally fused with benzene ring, $R^{15}$ is hydrogen atom, $C_{1-6}$ alkoxy-carbonylamino group or optionally halogenated $C_{1-6}$ alkyl-carbonylamino group,
(xii) a group represented by the formula

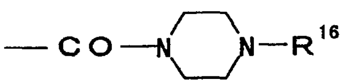

wherein $R^{16}$ is (a) $C_{1-6}$ alkyl group optionally having substituents selected from the group consisting of a hydroxy and $C_{1-6}$ alkoxy-carbonyl, (b) a formyl group, (c) $C_{1-6}$ alkoxy-carbonyl group or (d) a 5 or 6-membered heterocyclic ring-carbonyl group containing, besides carbon atom, 1 to 3 heteroatom(s) selected from the group consisting of nitrogen atom, oxygen atom and sulfur atom,
(xiii) a group represented by the formula

$$-SO_2-R^{17}$$

($R^{17}$ is (i) $C_{1-6}$ alkyl group optionally having 5 or 6-membered nitrogen-containing ring group, (ii) $C_{2-6}$ alkenyl group or (iii) $C_{6-14}$ aryl group optionally having $C_{1-6}$ alkyl),
(xiv) $C_{7-16}$ aralkyl group optionally having 1 to 3 halogen atom (s) , or
(xv) $C_{1-6}$ alkyl group substituted by 5 or 6-membered heterocyclic group containing, besides carbon atom, 1 to 3 heteroatom(s) selected from the group consisting of nitrogen atom, oxygen atom and sulfur atom,

$R^4$ is hydrogen atom or $C_{1-6}$ alkyl group,
or $R^3$ and $R^4$ may form, together with the adjacent nitrogen atom, a group of the formula

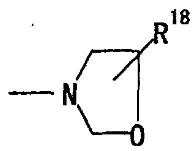

wherein $R^{18}$ is halogen atom, oxo group, optionally halogenated $C_{1-6}$ alkyl group or optionally halogenated $C_{1-6}$ alkoxy group.

**9.** The antagonist of claim 1, wherein $R^1$ and $R^2$ form, together with the adjacent nitrogen atom, a nitrogen-containing heterocyclic group represented by

(i) the formula

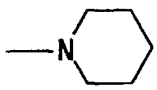 , 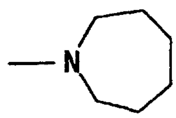 , 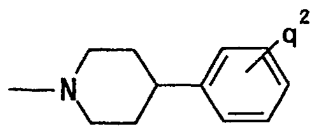 , 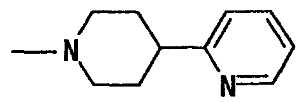 ,

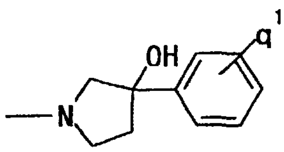 , 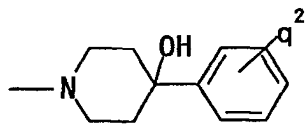 , 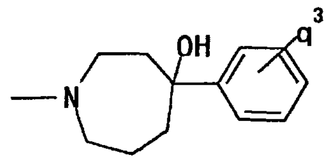 ,

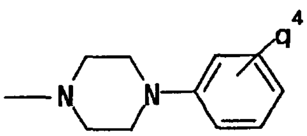 , 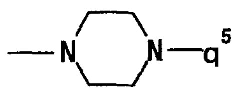 or 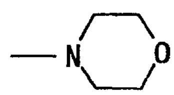

wherein $q^1$ is hydrogen atom or halogen atom, $q^2$ is hydrogen atom, halogen atom, optionally halogenated $C_{1-6}$ alkyl group or $C_{1-6}$ alkoxy group, $q^3$ is hydrogen atom or halogen atom, $q^4$ is hydrogen atom, halogen atom or $C_{1-6}$ alkoxy group, $q^5$ is hydrogen atom or $C_{1-6}$ alkyl group optionally having 1 or 2 $C_{6-10}$ aryl group(s),

(ii) the formula

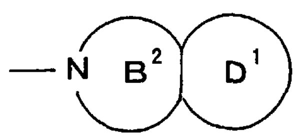

wherein ring $B^2$ is represented by the formula

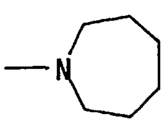 , 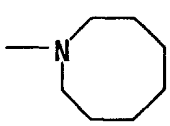 , 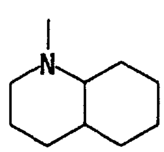 or 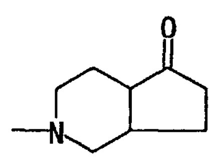

wherein ring $D^1$ is benzene ring optionally having 1 or 2 substituent(s) selected from the group consisting of $C_{1-6}$ alkyl group, $C_{1-6}$ alkoxy group and $C_{1-6}$ alkyl-carbonyl group or
(iii) the formula

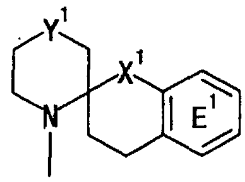

wherein ring $E^1$ is benzene ring optionally having 1 or 2 substituent(s) selected from the group consisting of $C_{1-3}$ alkylenedioxy group, nitro group, $C_{1-6}$ alkoxy group, amino group, $C_{1-6}$ alkyl-carbonylamino group and $C_{1-6}$ alkoxy-carbonyl group, $X^1$ is $-CH_2-$ or -CO-, and $Y^1$ is $-CH_2-$ or -O-.

**10.** The antagonist of claim 1, wherein the compound is represented by the formula

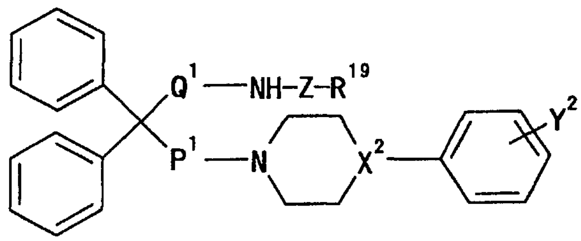

wherein $R^{19}$ is (i) hydrogen atom, (ii) carboxyl, (iii) $C_{1-6}$ alkoxy-carbonyl group, (iv) $C_{1-6}$ alkyl group optionally having substituents selected from the group consisting of carboxyl, $C_{1-6}$ alkyl-carbonyl, $C_{1-6}$ alkoxy-carbonyl, $C_{1-6}$ alkoxy-carbonylamino and $C_{7-16}$ aralkyloxy-carbonylamino, (v) a mono- or di-$C_{1-6}$ alkylamino group or (iv) $C_{6-14}$ aryloxy group; $P^1$ is $C_{1-3}$ alkylene group; $Q^1$ is $C_{1-3}$ alkylene group; $X^2$ is CH, C-OH or N; $Y^2$ is hydrogen atom, halogen atom, optionally halogenated $C_{1-6}$ alkyl group or $C_{1-6}$ alkoxy group; and Z is CO, SO or $SO_2$.

**11.** The antagonist of claim 1, wherein the compound is represented by the formula

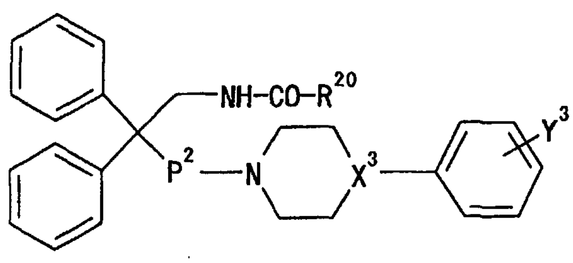

wherein $R^{20}$ is (i) hydrogen atom or (ii) $C_{1-6}$ alkyl group optionally having substituents selected from the group consisting of $C_{1-6}$ alkoxy-carbonylamino and $C_{7-16}$ aralkyloxy-carbonylamino; $P^2$ is $C_{1-3}$ alkylene group; $X^3$ is CH, C-OH or N; $Y^3$ is hydrogen atom, halogen atom or $C_{1-6}$ alkoxy group.

**12.** The antagonist of claim 1, wherein the compound is represented by the formula

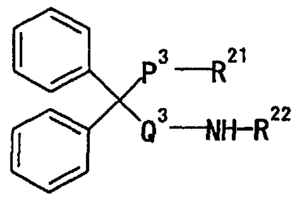

wherein $R^{21}$ is a nitrogen-containing heterocyclic group represented by (i) the formula

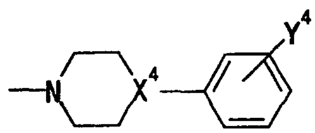

wherein $X^4$ is CH or N, $Y^4$ is hydrogen atom, halogen atom or $C_{1-6}$ alkoxy group or (ii) the formula

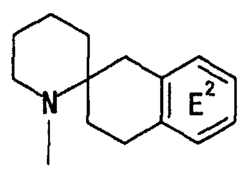

wherein ring $E^2$ is benzene ring optionally having 1 to 3 $C_{1-6}$ alkoxy,
$R^{22}$ is (i) hydrogen atom, (ii) $C_{7-16}$ aralkyl group, (iii) formyl group, (iv) $C_{1-6}$ alkyl-carbonyl group, (v) $C_{6-14}$ aryl-carbonyl group optionally having $C_{1-6}$ alkyl or (vi) $C_{6-14}$ aryl-sulfonyl group optionally having 1 to 4 $C_{1-6}$ alkyl; $P^3$ is $C_{1-3}$ alkylene group; and $Q^3$ is $C_{1-3}$ alkylene group.

**13.** The antagonist of claim 1, wherein the compound is

1-(5-amino-4,4-diphenylpentyl)-4-phenylpiperidine or a salt thereof,
3,4-dihydro-6-methoxy-1'-(5-amino-4,4-diphenylpentyl)-spiro[naphthalene-2(1H),2'-piperidine] or a salt thereof,
1-[5-amino-4-(4-methoxyphenyl)-4-phenylpentyl]-4-phenylpiperidine or a salt thereof,
1-[5-amino-4,4-bis(4-chlorophenyl)pentyl]-4-(4-fluorophenyl)-piperazine or a salt thereof,
3,4-dihydro-6-methoxy-1'-(6-amino-4,4-diphenylhexyl)-spiro[naphthalene-2(1H),2'-piperidine] or a salt thereof,
3,4-dihydro-6,7-dimethoxy-1'-(7-amino-4,4-diphenylheptyl)-spiro[naphthalene-2(1H),2'-piperidine] or a salt thereof,
4,4-diphenyl-5-formylamino-1-(4-phenylpiperidino)pentane or a salt thereof,
1-[4-(4-fluorophenyl)piperazin-1-yl]-5-formylamino-4,4-diphenylpentane or a salt thereof,
4,4-diphenyl-1-(4-phenylpiperazin-1-yl)-5-(tosylamino)pentane or a salt thereof,
4,4-diphenyl-1-[4-(2-methoxyphenyl)piperazin-1-yl]-5-(tosylamino)pentane or a salt thereof,
4- (4-chlorophenyl) -5-formylamino-4-phenyl-1- (4-phenylpiperidino)pentane or a salt thereof,
4- (4-chlorophenyl)-5-formylamino-4-phenyl-1-(4-phenylpiperazin-1-yl)pentane or a salt thereof,
4-(4-chlorophenyl)-1-[4-(4-fluorophenyl)piperazin-1-yl]-5-formylamino-4-phenylpentane or a salt thereof,
4-(4-chlorophenyl)-1-[4-(diphenylmethyl)piperazin-1-yl]-5-formylamino-4-phenylpentane or a salt thereof,
5-formylamino-4-(4-methoxyphenyl)-4-phenyl-1- (4-phenylpiperidino)pentane or a salt thereof,
4,4-bis(4-chlorophenyl)-1-[4-(4-fluorophenyl)piperazin-1-yl]-5-(formylamino)pentane or a salt thereof,
1- [4-(4-fluorophenyl)piperazin-1-yl]-6-formylamino-5,5-diphenylhexane or a salt thereof,
1- [4-(4-fluorophenyl)piperazin-1-yl]-6-formylamino-4,4-diphenylhexane or a salt thereof,
4,4-diphenyl-1-(4-phenylpiperidino]-6-(tosylamino)hexane or a salt thereof,
5- [4-(4-chlorophenyl)-4-hydroxypiperidino]-1-formylamino-2,2-diphenylpentane or a salt thereof,
5- [4-(4-fluorophenyl)piperazin-1-yl]-1-formylamino-2,2-diphenylpentane or a salt thereof,

1-formylamino-5-(4-hydroxy-4-phenylpiperidino)-2,2-diphenylpentane or a salt thereof,
5-[4-(4-trifluoromethylphenyl)-4-hydroxypiperidino]-1-formylamino-2,2-diphenylpentane or a salt thereof,
5- [4-[3,5-bis(trifluoromethyl)phenyl]-4-hydroxypiperidino]-1-formylamino-2,2-diphenylpentane or a salt thereof,
5- [4-(3,5-dichlorophenyl)-4-hydroxypiperidino]-1-formylamino-2,2-diphenylpentane or a salt thereof,
5- [4-(4-chlorophenyl) -1,2,3,6-tetrahydropyridin-1-yl]-1-formylamino-2,2-diphenylpentane or a salt thereof,
1-formylamino-2,2-diphenyl-5-(4-phenylpiperidino)pentane or a salt thereof,
5-[4-(4-chlorophenyl)piperidino]-1-formylamino-2,2-diphenylpentane or a salt thereof,
7-[4-(4-chlorophenyl)-4-hydroxypiperidino]-1-formylamino-4,4-diphenylheptane or a salt thereof,
5-[4-(4-fluorophenyl)-4-hydroxypiperidino]-1-formylamino-2,2-diphenylpentane or a salt thereof,
1-formylamino-5-[4-hydroxy-4-(4-methoxyphenyl)piperidino]-2,2-diphenylpentane or a salt thereof,
1-formylamino-5-[4-hydroxy-4-(2-pyridyl)piperidino]-2,2-diphenylpentane or a salt thereof,
1-acetylamino-5-[4-(4-chlorophenyl)-4-hydroxypiperidino]-2,2-diphenylpentane or a salt thereof,
1-acetoacetylamino-5-[4-(4-chlorophenyl)-4-hydroxypiperidino]-2,2-diphenylpentane or a salt thereof,
ethyl N-[5-[4-(4-chlorophenyl)-4-hydroxypiperidino]-2,2-diphenylpentyl]succinamate or a salt thereof,
N-[5-[4-(4-chlorophenyl)-4-hydroxypiperidino]-2,2-diphenylpentyl]succinamic acid or a salt thereof,
1-[5- [4-(4-chlorophenyl)-4-hydroxypiperidino]-2,2-diphenylpentyl]-3-ethylurea or a salt thereof,
N-[5-[4-(4-chlorophenyl)-4-hydroxypiperidino]-2,2-diphenylpentyl]methanesulfonamide or a salt thereof,
phenyl N-[5-[4-(4-chlorophenyl)-4-hydroxypiperidino]-2,2-diphenylpentyl]carbamate or a salt thereof,
1-acetylamino-5-[4-(4-chlorophenyl)-4-hydroxypiperidino]-2-phenyl-2-(2-pyridyl)pentane or a salt thereof,
ethyl N- [5-[4-(4-chlorophenyl)-4-hydroxypiperidino]-2,2-diphenylpentyl]oxamate or a salt thereof,
ethyl N-[5-[4-(4-chlorophenyl)-4-hydroxypiperidino]-2,2-diphenylpentyl]malonamate or a salt thereof,
ethyl N-[5-[4-(4-chlorophenyl)-4-hydroxypiperidino]-2,2-diphenylpentyl]glutaramate or a salt thereof,
benzyl 2-((2,2-diphenyl-5-(4-phenylpiperidino)pentyl)amino)-2-oxoethylcarbamate or a salt thereof,
tert-butyl 2-((2,2-diphenyl-5-(4-phenylpiperidino)-pentyl)amino)-2-oxoethylcarbamate or a salt thereof,
4,4-diphenyl-7-(4-phenylpiperidino)heptylamine or a salt thereof,
N-(4,4-diphenyl-7-(4-phenylpiperidino)heptyl)-4-methylbenzenesulfonamide or a salt thereof,
N-(4,4-diphenyl-7-(4-phenylpiperidino)heptyl)acetamide or a salt thereof,
N-benzyl-N-(4,4-diphenyl-7-(4-phenylpiperidino)heptyl)amine or a salt thereof,
N-(4,4-diphenyl-7-(4-phenylpiperidino)heptyl)-N-(3-methoxybenzyl)amine or a salt thereof,
N-(4,4-diphenyl-7-(4-phenylpiperidino)heptyl)-N-(2-methoxybenzyl)amine or a salt thereof,
N-(4,4-diphenyl-7-(4-phenylpiperidino)heptyl)-N-(2-fluorobenzyl)amine or a salt thereof,
N-(4,4-diphenyl-7-(4-phenylpiperidino)heptyl)-2-thiophenecarboxamide or a salt thereof,
N-(4,4-diphenyl-7-(4-phenylpiperidino)heptyl)-2-phenylacetamide or a salt thereof,
N-(4,4-diphenyl-7-(4-phenylpiperidino)heptyl)-N-(2-thienylmethyl)amine or a salt thereof, or
N-benzyl-N-(4,4-diphenyl-7-(4-phenylpiperidino)heptyl)-N-methylamine or a salt thereof.

**14.** The antagonist of claim 1, which is an agent for the prophylaxis or therapy of a disease caused by melanin-concentrating hormone.

**15.** The antagonist of claim 1, which is an agent for the prophylaxis or therapy of obesity.

**16.** The antagonist of claim 1, which is an agent for suppressing food intake.

**17.** A compound represented by the formula

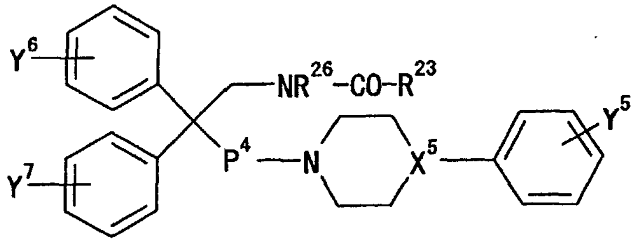

(Ia)

wherein $R^{23}$ is $C_{1-6}$ alkyl group having $C_{7-16}$ aralkyloxy-carbonylamino optionally having substituents selected from the group consisting of halogen atom, $C_{1-6}$ alkoxy and $C_{1-6}$ alkyl; $P^4$ is $C_{1-3}$ alkylene group; $X^5$ is CH, C-OH or N;

$Y^5$ is hydrogen atom, halogen atom or $C_{1-6}$ alkoxy group; $R^{26}$ is hydrogen atom or $C_{1-6}$ alkyl group; $Y^6$ and $Y^7$ are the same or different and each is hydrogen atom, halogen atom, optionally halogenated $C_{1-6}$ alkyl group or optionally halogenated $C_{1-6}$ alkoxy group, or a salt thereof or a prodrug thereof.

**18.** The compound of claim 17, wherein $R^{26}$ is hydrogen atom.

**19.** Benzyl 2-((2,2-diphenyl-5-(4-phenylpiperidino)-pentyl)amino)-2-oxoethylcarbamate or a salt thereof,

4-chlorobenzyl 2-((2,2-diphenyl-5-(4-phenylpiperidino)-pentyl)amino)-2-oxoethylcarbamate or a salt thereof,
3-chlorobenzyl 2-((2,2-diphenyl-5-(4-phenylpiperidino)-pentyl)amino)-2-oxoethylcarbamate or a salt thereof,
benzyl 2-(N-(2,2-diphenyl-5-(4-phenylpiperidino)pentyl)-N-methylamino)-2-oxoethylcarbamate or a salt thereof,
benzyl 2-((5-(4-(3-fluorophenyl)piperidino)-2,2-diphenylpentyl)amino)-2-oxoethylcarbamate or a salt thereof,
benzyl 2-((5-(4-(2-fluorophenyl)piperidino)-2,2-diphenylpentyl)amino)-2-oxoethylcarbamate or a salt thereof,
benzyl 2-((5-(4-(2-methoxyphenyl)piperidino)-2,2-diphenylpentyl)amino)-2-oxoethylcarbamate or a salt thereof, or
3-chlorobenzyl 2-((2,2-bis(4-chlorophenyl)-5-(4-phenylpiperidino)pentyl)amino)-2-oxoethylcarbamate or a salt thereof.

**20.** A production method of a compound of claim 17, which comprises reacting a compound represented by the formula

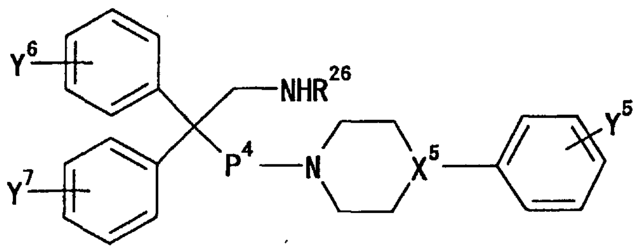

wherein each symbol is as defined in claim 17 or a salt thereof with a reactive derivative of an organic acid of the formula

$$R^{23}\text{-COOH}$$

wherein $R^{23}$ is as defined in claim 17.

**21.** A production method of a compound of claim 17, which comprises reacting a compound represented by the formula

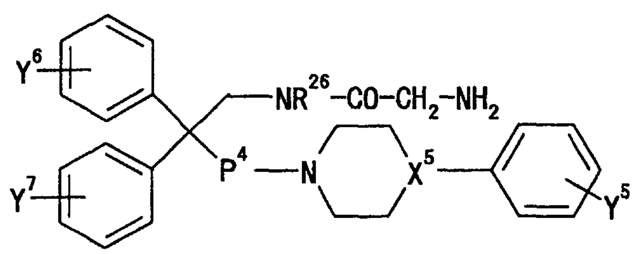

wherein each symbol is as defined in claim 17, or a salt thereof with a reactive derivative of the formula

$$R^{32}\text{-X}$$

wherein $R^{32}$ is $C_{7-16}$ aralkyloxy-carbonyl group, and X is a leaving group.

**22.** A pharmaceutical composition containing a compound of claim 17.

**23.** A compound represented by the formula

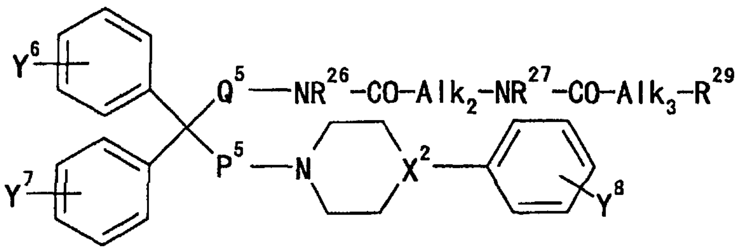

(Ib)

wherein $R^{26}$ and $R^{27}$ are the same or different and each is hydrogen atom or $C_{1-6}$ alkyl group; $Alk_2$ and $Alk_3$ are the same or different and each is a bond or $C_{1-6}$ alkylene group optionally having substituents; $R^{29}$ is (1) $C_{6-10}$ aryl group optionally having substituents or (2) 5 to 10-membered aromatic heterocyclic group optionally having substituents, which contains, besides carbon atom, 1 to 3 heteroatom(s) selected from the group consisting of nitrogen atom, oxygen atom and sulfur atom; $X^2$ is CH, C-OH or N; $P^5$ and $Q^5$ are the same or different and each is $C_{1-6}$ alkylene group; $Y^6$, $Y^7$ and $Y^8$ are the same or different and each is hydrogen atom, halogen atom, optionally halogenated $C_{1-6}$ alkyl group or optionally halogenated $C_{1-6}$ alkoxy group, or a salt thereof or a prodrug thereof.

**24.** The compound of claim 23, wherein $Alk_2$ and $Alk_3$ are the same or different and each is a bond, or $C_{1-6}$ alkylene group optionally having substituents selected from the group consisting of halogen atom, hydroxy, amino and $C_{6-10}$ aryl; $R^{29}$ is (1) $C_{6-10}$ aryl group or (2) 5 to 10-membered aromatic heterocyclic group containing, besides carbon atom, 1 to 3 heteroatom(s) selected from the group consisting of nitrogen atom, oxygen atom and sulfur atom, which optionally has substituents selected from the group consisting of nitro, halogen atom, $C_{1-6}$ alkyl, hydroxy, $C_{1-6}$ alkoxy and $C_{6-10}$ aryl.

**25.** The compound of claim 23 or 24, wherein $R^{29}$ is indol-2-yl optionally having substituents.

**26.** The compound of claim 23 or 24, wherein $R^{29}$ is indol-2-yl optionally having substituents selected from halogen atom, $C_{1-6}$ alkyl, $C_{1-6}$ alkoxy and hydroxy.

**27.** N-(2-((2,2-Diphenyl-5-(4-phenylpiperidino)pentyl)amino)-2-oxoethyl)indole-2-carboxamide or a salt thereof,

N-(2-((2,2-diphenyl-5-(4-phenylpiperidino)pentyl)amino)-2-oxoethyl)-1-methylindole-2-carboxamide or a salt thereof,
5-chloro-N-(2-((2,2-diphenyl-5-(4-phenylpiperidino)-pentyl)amino)-2-oxoethyl)indole-2-carboxamide or a salt thereof,
N- (2- ((2,2-bis(4-chlorophenyl)-5-(4-phenylpiperidino)-pentyl)amino)-2-oxoethyl)indole-2-carboxamide or a salt thereof,
N-(2-((2,2-bis(4-chlorophenyl)-5-(4-phenylpiperidino)-pentyl)amino)-2-oxoethyl)-5-chloroindole-2-carboxamide or a salt thereof,
N- (2-((2,2-bis(4-chlorophenyl)-5-(4-phenylpiperidino)-pentyl)amino)-2-oxoethyl)-1-methylindole-2-carboxamide or a salt thereof,
N-(2-((2,2-bis(4-chlorophenyl)-5-(4-phenylpiperidino)-pentyl)amino)-2-oxoethyl)-5-fluoroindole-2-carboxamide or a salt thereof,
N-(2-((2,2-bis(4-chlorophenyl)-5-(4-phenylpiperidino)-pentyl)amino)-2-oxoethyl)-5-methoxyindole-2-carboxamide or a salt thereof,
N- (2- ((2,2-bis(4-chlorophenyl)-5-(4-phenylpiperidino)-pentyl)amino)-2-oxoethyl)-5-hydroxyindole-2-carboxamide or a salt thereof,
N-(2-((5-(4-(2-fluorophenyl)piperidino)-2,2-diphenylpentyl)-amino)-2-oxoethyl)indole-2-carboxamide or a salt thereof,
N-(2-((5-(4-(2-fluorophenyl)piperidino)-2,2-diphenylpentyl)-amino)-2-oxoethyl)-1-methylindole-2-carboxamide or a salt thereof,
5-chloro-N-(2-((5-(4-(2-fluorophenyl)piperidino)-2,2-diphenylpentyl)amino)-2-oxoethyl)-1-methylindole-2-carboxamide or a salt thereof,
5-chloro-N-(2-((5-(4-(2-fluorophenyl)piperidino)-2,2-diphenylpentyl)amino)-2-oxoethyl)indole-2-carboxamide or a salt thereof,
N-(2-((2,2-bis(4-chlorophenyl)-5-(4-(2-fluorophenyl)-piperidino)pentyl)amino)-2-oxoethyl)-5-fluoroindole-2-carboxamide or a salt thereof,
N-(2-((2,2-bis(4-chlorophenyl)-5-(4-(2-fluorophenyl)-piperidino)pentyl)amino)-2-oxoethyl)-5-methoxyindole-2-carboxamide or a salt thereof,
N- (2- ((2,2-bis(4-chlorophenyl)-5-(4-(2-fluorophenyl)-piperidino)pentyl)amino)-2-oxoethyl)indole-2-carboxamide or a salt thereof,
N- (2-((2,2-bis(4-fluorophenyl)-5-(4-phenylpiperidino)-pentyl)amino)-2-oxoethyl)indole-2-carboxamide or a salt thereof,
N- (2-((2,2-bis(4-fluorophenyl)-5-(4-phenylpiperidino)-pentyl)amino)-2-oxoethyl)-5-chloroindole-2-carboxamide or a salt thereof,
N-(2-((2,2-bis(4-fluorophenyl)-5-(4-(2-methoxyphenyl)-piperidino)pentyl)amino)-2-oxoethyl)indole-2-carboxamide or a salt thereof,
N- (2- ((2,2-bis(4-fluorophenyl)-5-(4-(2-methoxyphenyl)-piperidino)pentyl)amino)-2-oxoethyl)-5-chloroindole-2-carboxamide or a salt thereof,
N-(2-((2,2-bis(4-fluorophenyl)-5-(4-(2-fluorophenyl)-piperidino)pentyl)amino)-2-oxoethyl)indole-2-carboxamide or a salt thereof, or
N- (2- ((2,2-bis(4-fluorophenyl)-5-(4-(2-fluorophenyl)-piperidino)pentyl)amino)-2-oxoethyl)-5-chloroindole-2-carboxamide or a salt thereof.

**28.** A production method of a compound of claim 23, which comprises reacting a compound represented by the formula

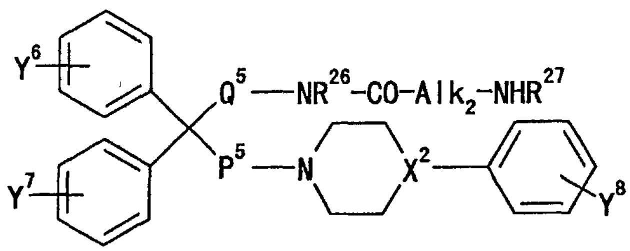

wherein each symbol is as defined in claim 23, or a salt thereof with a reactive derivative of an organic acid of the formula

$$R^{29}\text{-}Alk_3\text{-}COOH$$

wherein each symbol is as defined in claim 23.

**29.** A pharmaceutical composition containing a compound of claim 23.

**30.** A compound represented by the formula

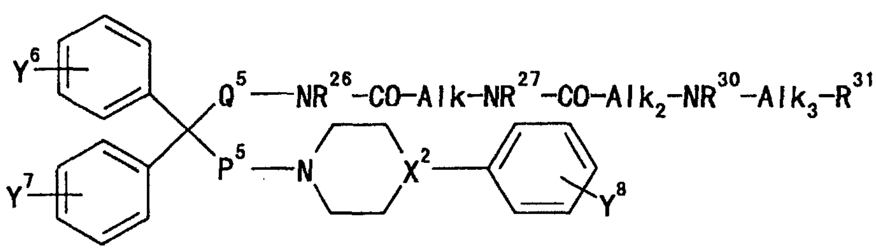

(Ic)

wherein $R^{26}$ and $R^{27}$ are the same or different and each is hydrogen atom or $C_{1-6}$ alkyl group; $R^{30}$ is hydrogen atom, $C_{1-6}$ alkyl group or optionally halogenated $C_{1-6}$ alkyl-carbonyl group; Alk is $C_{1-6}$ alkylene group optionally

having substituents; $Alk_2$ and $Alk_3$ are the same or different and each is a bond or $C_{1-6}$ alkylene group optionally having substituents; $R^{31}$ is $C_{6-10}$ aryl group optionally having substituents; $X^2$ is CH, C-OH or N; $P^5$ and $Q^5$ are the same or different and each is $C_{1-6}$ alkylene group; $Y^6$, $Y^7$ and $Y^8$ are the same or different and each is hydrogen atom, halogen atom, optionally halogenated $C_{1-6}$ alkyl group or optionally halogenated $C_{1-6}$ alkoxy group, or a salt thereof or a prodrug thereof.

**31.** The compound of claim 30, wherein Alk is $C_{1-6}$ alkylene group optionally having substituents selected from the group consisting of halogen atom, hydroxy, amino and $C_{6-10}$ aryl; $Alk_2$ and $Alk_3$ are the same or different and each is a bond or $C_{1-6}$ alkylene group optionally having substituents selected from the group consisting of halogen atom, hydroxy, amino and $C_{6-10}$ aryl; $R^{31}$ is $C_{6-10}$ aryl group optionally having substituents selected from the group consisting of halogen atom, $C_{1-6}$ alkyl, hydroxy, $C_{1-6}$ alkoxy and $C_{6-10}$ aryl.

**32.** N-(2-((2-((2,2-Diphenyl-5-(4-phenylpiperidino)pentyl)-amino)-2-oxoethyl)amino)-2-oxoethyl)-2,2,2-trifluoro-N-phenylacetamide or a salt thereof,

2-anilino-N-(2-((2,2-diphenyl-5-(4-phenylpiperidino)-pentyl)amino)-2-oxoethyl)acetamide or a salt thereof, or 2-(((benzylamino)carbonyl)amino)-N-(2,2-diphenyl-5-(4-phenylpiperidino)pentyl)acetamide or a salt thereof.

**33.** A production method of a compound of claim 30, which comprises reacting a compound represented by the formula

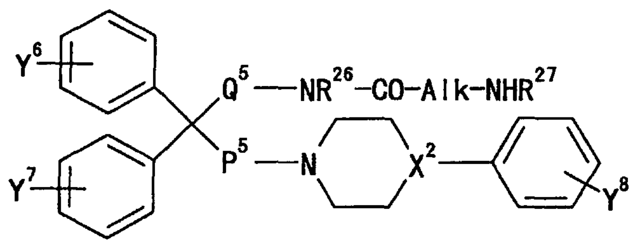

wherein each symbol is as defined in claim 30, or a salt thereof, with,

(1) when $Alk_2$ is $C_{1-6}$ alkylene group optionally having substituents, a reactive derivative of an organic acid compound of the formula

$$R^{31}\text{-}Alk_3\text{-}NR^{30}\text{-}Alk_2\text{-}COOH$$

wherein each symbol is as defined in claim 30,

(2) when $Alk_2$ is a bond, a reactive derivative of the formula

$$R^{31}\text{-}Alk_3\text{-}NR^{30}\text{-}CO\text{-}X$$

or

$$R^{31}\text{-}Alk_3\text{-}NCO$$

wherein X is leaving group, and other symbols are as defined in claim 30.

**34.** A pharmaceutical composition containing a compound of claim 30.

**35.** A method for antagonizing melanin-concentrating hormone, comprising administering, to a mammal, an effective amount of a compound of the formula

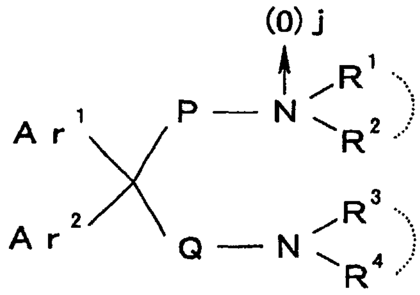

wherein

Ar$^1$ and Ar$^2$ are each an aromatic group optionally having substituents,
P and Q are each a divalent aliphatic hydrocarbon group which optionally contains ether oxygen or ether sulfur in a carbon chain and which optionally has substituents,
R$^1$ and R$^3$ are each (i) hydrogen atom, (ii) acyl group or (iii) a hydrocarbon group optionally having substituents,
R$^2$ and R$^4$ are each (i) hydrogen atom, (ii) an alkyl group optionally having substituents or (iii) an alkylcarbonyl group optionally having substituents, R$^1$ and R$^2$ or R$^3$ and R$^4$ optionally form, together with the adjacent nitrogen atom, a monocyclic or fused nitrogen-containing heterocyclic group optionally having substituents, and
j is 0 or 1, or a salt thereof or a prodrug thereof.

**36.** Use of a compound of the formula

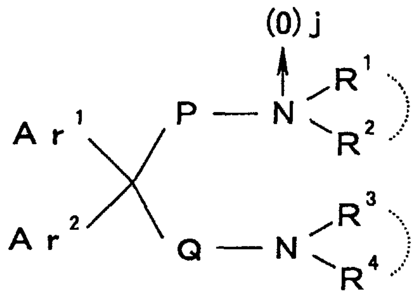

wherein

Ar$^1$ and Ar$^2$ are each an aromatic group optionally having substituents,
P and Q are each a divalent aliphatic hydrocarbon group which optionally contains ether oxygen or ether sulfur in a carbon chain and which optionally has substituents,
R$^1$ and R$^3$ are each (i) hydrogen atom, (ii) acyl group or (iii) hydrocarbon group optionally having substituents,
R$^2$ and R$^4$ are each (i) hydrogen atom, (ii) an alkyl group optionally having substituents or (iii) an alkylcarbonyl group optionally having substituents, R$^1$ and R$^2$ or R$^3$ and R$^4$ optionally form, together with the adjacent nitrogen atom, a monocyclic or fused nitrogen-containing heterocyclic group optionally having substituents, and
j is 0 or 1, or a salt thereof or a prodrug thereof, for production of a melanin-concentrating hormone antagonist.

**37.** A compound represented by the formula

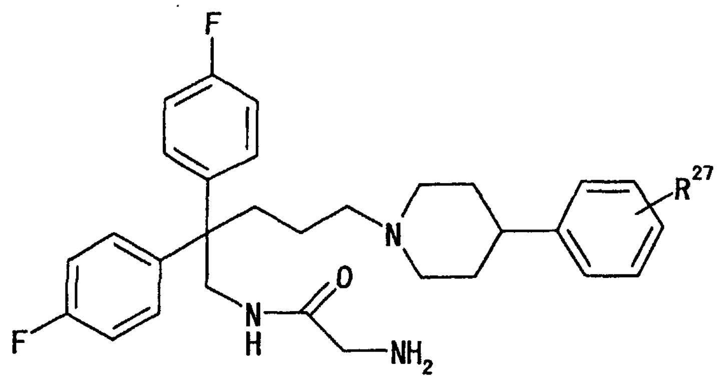

wherein $R^{27}$ is hydrogen atom, halogen atom, optionally halogenated $C_{1-6}$ alkyl group or optionally halogenated $C_{1-6}$ alkoxy group, or a salt thereof.

## INTERNATIONAL SEARCH REPORT

International application No.
PCT/JP00/06376

**A. CLASSIFICATION OF SUBJECT MATTER**

Int.Cl$^7$ A61K31/137, 31/27, 31/4035, 31/44, 31/445, 31/4453, 31/472, A61P43/00, 31/04, C07D211/14, 211/18, 211/46, 211/58, 211/70, 401/12, 405/12, 409/12, 417/12

According to International Patent Classification (IPC) or to both national classification and IPC

**B. FIELDS SEARCHED**

Minimum documentation searched (classification system followed by classification symbols)

Int.Cl$^7$ A61K31/137, 31/27, 31/4035, 31/44, 31/445, 31/4453, 31/472, A61P43/00, 31/04, C07D211/14, 211/18, 211/46, 211/58, 211/70, 401/12, 405/12, 409/12, 417/12

Documentation searched other than minimum documentation to the extent that such documents are included in the fields searched

Electronic data base consulted during the international search (name of data base and, where practicable, search terms used)

CAPLUS(STN),MEDLINE(STN),EMBASE(STN)

**C. DOCUMENTS CONSIDERED TO BE RELEVANT**

| Category* | Citation of document, with indication, where appropriate, of the relevant passages | Relevant to claim No. |
|---|---|---|
| X<br>X,Y<br><br>A | WO, 97/24325, A1 (Takeda Chemical Industries, Ltd.), 10 July, 1997 (10.07.97), & JP, 10-81665, A & Database CAPLUS on STN, AMERICAN CHEMICAL SOCIETY (ACS), (Columbus, OH, USA), DN.127:161698 especially compounds of RN:193541-68-3 | 14<br>17-22,23-34,37<br><br>1-13,15,16,36 |
| X<br>Y<br><br>A | JP, 11-71350, A (Takeda Chemical Industries, Ltd.), 16 March, 1999 (16.03.99) (Family: none) & Database CAPLUS on STN, AMERICAN CHEMICAL SOCIETY (ACS), (Columbus, OH, USA), DN.130:237480 | 14<br>17-22,23-34,37<br><br>1-13,15,16,<br>36 |
| Y<br><br>A | EP, 712845, A1 (Takeda Chemical Industries, Ltd.), 22 May, 1996 (22.05.96) & US, 5633248, A & JP, 8-253447, A & Database CAPLUS on STN, AMERICAN CHEMICAL SOCIETY (ACS), (Columbus, OH, USA), DN.125:114489 | 17-22,23-34,37<br><br>1-16,36 |
| Y<br><br>A | JP, 8-208595, A (Takeda Chemical Industries, Ltd.), 13 August, 1996 (13.08.96) (Family: none) & Database CAPLUS on STN, AMERICAN CHEMICAL SOCIETY (ACS), | 17-22,23-34,37<br><br>1-16,36 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 November, 2000 (17.11.00) | 28 November, 2000 (28.11.00) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP00/06376

C (Continuation). DOCUMENTS CONSIDERED TO BE RELEVANT

| Category* | Citation of document, with indication, where appropriate, of the relevant passages | Relevant to claim No. |
|---|---|---|
| | (Columbus, OH, USA), DN.125:300606 | |
| A | EP, 871463, A1 (JOSLIN DIABETES CENTER, INC.), 21 October, 1998 (21.10.98) & US, 5849708, A & JP, 11-507517, A | 1-16,36 |

Form PCT/ISA/210 (continuation of second sheet) (July 1992)

**INTERNATIONAL SEARCH REPORT**

International application No.
PCT/JP00/06376

**Box I Observations where certain claims were found unsearchable (Continuation of item 1 of first sheet)**

This international search report has not been established in respect of certain claims under Article 17(2)(a) for the following reasons:

1. ☒ Claims Nos.: 35
because they relate to subject matter not required to be searched by this Authority, namely:

Claim 35 pertains to methods for treatment of the human body by therapy, and thus relates to a subject matter which this International Searching Authority is not required, under the provisions of Article 17(2)(a)(i) of the PCT and Rule 39(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

(See extra sheet.)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)

**INTERNATIONAL SEARCH REPORT**

International application No.
PCT/JP00/06376

Continuation of Box No.II of continuation of first sheet(1)

Claims 1 to 16 and 36 relate to melanin concentrating hormone (MCH) antagonists containing as the active ingredient compounds represented by the general formula in claims 1 or preventive and therapeutic agents for diseases attributable to MCH.

Claims 17 to 22 and 23 to 34 relate to diphenyl compounds represented by general formula (Ia) in claim 17 or by general formula (Ib) in claim 23, processes for the preparation of the compounds, or drugs for nonspecified use containing the compounds. Furthermore, claim 37 relates to fluorinated diphenyl compounds represented by the general formula therein.

All of the compounds of general formulae (Ia) and (Ib) and those of claim 37 are included among the compounds represented by the general formula in claim 1. Additionally, it is considered on the basis of the results of prior art search that two inventive concepts, i.e., "use of novel compounds as drugs" and "use of publicly known compounds as novel drugs" are intermingled in claims 1 to 16 and 36. Such being the case, a group of inventions of claims 1 to 16 and 36 and a group of inventions of claims 17 to 22, 23 to 34 and 37 are not considered as relating to a group of inventions so linked as to form a single general inventive concept.

Form PCT/ISA/210 (extra sheet) (July 1992)